(19) Europäisches Patentamt
European Patent Office
Office européen des brevets

(11) EP 1 427 833 B1

(12) # EUROPÄISCHE PATENTSCHRIFT

(45) Veröffentlichungstag und Bekanntmachung des Hinweises auf die Patenterteilung:
**28.10.2009 Patentblatt 2009/44**

(21) Anmeldenummer: **02767458.9**

(22) Anmeldetag: **30.08.2002**

(51) Int Cl.:
***C12N 15/82*** *(2006.01)* ***C12N 15/29*** *(2006.01)*
***A01H 5/00*** *(2006.01)*

(86) Internationale Anmeldenummer:
**PCT/EP2002/009719**

(87) Internationale Veröffentlichungsnummer:
**WO 2003/020939 (13.03.2003 Gazette 2003/11)**

(54) **NEUE NUKLEINSAEURESEQUENZEN UND DEREN VERWENDUNG IN VERFAHREN ZUM ERREICHEN EINER PATHOGENRESISTENZ IN PFLANZEN**

NOVEL NUCLEIC ACID SEQUENCES AND THEIR USE IN METHODS FOR ACHIEVING PATHOGEN RESISTANCE IN PLANTS

NOUVELLES SEQUENCES D'ACIDES NUCLEIQUES ET LEUR UTILISATION DANS DES PROCEDES POUR OBTENIR UNE RESISTANCE AUX AGENTS PATHOGENES CHEZ DES VEGETAUX

(84) Benannte Vertragsstaaten:
**AT BE BG CH CY CZ DE DK EE ES FI FR GB GR IE IT LI LU MC NL PT SE SK TR**
Benannte Erstreckungsstaaten:
**AL LT LV MK RO SI**

(30) Priorität: **03.09.2001 DE 10142579**
**02.07.2002 DE 10229729**

(43) Veröffentlichungstag der Anmeldung:
**16.06.2004 Patentblatt 2004/25**

(73) Patentinhaber: **BASF Plant Science GmbH**
**67056 Ludwigshafen (DE)**

(72) Erfinder:
- **KOGEL, Karl-Heinz**
  **35457 Lollar (DE)**
- **HÜCKELHOVEN, Ralph**
  **35392 Giessen (DE)**
- **SCHULTHEISS, Holger**
  **61169 Friedberg (DE)**
- **FRANK, Markus**
  **68163 Mannheim (DE)**

(74) Vertreter: **Bieberbach, Andreas**
**BASF SE**
**Global Intellectual Property**
**GVX / K - C 6**
**67056 Ludwigshafen (DE)**

(56) Entgegenhaltungen:
**WO-A-00/15815**

- **DATABASE EMBL [Online] 2. August 2000 (2000-08-02) MI Z ET AL.: "Oryza sativa small GTP-binding protein RACBP (RACB) mRNA" Database accession no. AF250327 XP002226365**
- **KAWASAKI ET AL: "The small GTP-binding protein Rac is a regulator of cell death in plants" PROCEEDINGS OF THE NATIONAL ACADEMY OF SCIENCES OF USA, NATIONAL ACADEMY OF SCIENCE. WASHINGTON, US, Bd. 96, September 1999 (1999-09), Seiten 10922-10926, XP002131367 ISSN: 0027-8424**
- **ONO EIICHIRO ET AL: "Essential role of the small GTPase Rac in disease resistance of rice." PROCEEDINGS OF THE NATIONAL ACADEMY OF SCIENCES OF THE UNITED STATES, Bd. 98, Nr. 2, 16. Januar 2001 (2001-01-16), Seiten 759-764, XP002226363 January 16, 2001 ISSN: 0027-8424**
- **SCHULTHEISS HOLGER ET AL: "A small GTP-binding host protein is required for entry of powdery mildew fungus into epidermal cells of barley." PLANT PHYSIOLOGY (ROCKVILLE), Bd. 128, Nr. 4, April 2002 (2002-04), Seiten 1447-1454, XP002226364 April, 2002 ISSN: 0032-0889**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 427 833 B1

SEQUENZPROTOKOLL

<110> BASF Plant Science GmbH

<120> Neue Nukleinsäuresequenzen und deren Verwendung in Verfahren zum Erreichen einer Pathogenresistenz in Pflanzen.

<130> NAE 369/02 / NAE 761/01 AT

<140>
<141>

<160> 76

<170> PatentIn Ver. 2.1

<210> 1
<211> 857
<212> DNA
<213> Hordeum vulgare

<220>
<221> CDS
<222> (90)..(680)

<400> 1

```
atcttaacca gctccctacc tccccttctt cttcctcctc ctcccctgtc tcgcccgcag 60

cttcaccggc agcgagggaa agagggagg atg agc gcg tcc agg ttc ata aag   113
                                Met Ser Ala Ser Arg Phe Ile Lys
                                1               5

tgc gtc acc gtg ggg gac ggc gcc gtc ggc aag acc tgc atg ctc atc   161
Cys Val Thr Val Gly Asp Gly Ala Val Gly Lys Thr Cys Met Leu Ile
    10                  15                  20

tcc tac acc tcc aac acc ttc ccc acc gac tat gtg ccc acg gtg ttt   209
Ser Tyr Thr Ser Asn Thr Phe Pro Thr Asp Tyr Val Pro Thr Val Phe
25                  30                  35                  40

gac aac ttc agt gct aat gtt gtg gtt gat ggc aac act gtc aac ctt   257
Asp Asn Phe Ser Ala Asn Val Val Val Asp Gly Asn Thr Val Asn Leu
                45                  50                  55

ggg cta tgg gat act gca ggt cag gaa gac tac aac aga ctg aga ccg   305
Gly Leu Trp Asp Thr Ala Gly Gln Glu Asp Tyr Asn Arg Leu Arg Pro
            60                  65                  70

ctg agt tat cgt gga gct gat gtc ttc ctt ctg gcc ttc tcg ctt atc   353
Leu Ser Tyr Arg Gly Ala Asp Val Phe Leu Leu Ala Phe Ser Leu Ile
        75                  80                  85

agc aag gct agc tat gag aat gtt tca aag aag tgg ata cct gaa ctg   401
Ser Lys Ala Ser Tyr Glu Asn Val Ser Lys Lys Trp Ile Pro Glu Leu
    90                  95                  100

aag cat tat gca cca ggt gtg cct att atc ctc gtg gga aca aag ctt   449
Lys His Tyr Ala Pro Gly Val Pro Ile Ile Leu Val Gly Thr Lys Leu
105                 110                 115                 120

gat ctt cga gat gac aag cag ttc ttt gtg gac cat cct ggt gct gtt   497
Asp Leu Arg Asp Asp Lys Gln Phe Phe Val Asp His Pro Gly Ala Val
                125                 130                 135

cct atc act act gct cag ggg gag gaa cta aaa aag tta ata ggc gca   545
Pro Ile Thr Thr Ala Gln Gly Glu Glu Leu Lys Lys Leu Ile Gly Ala
            140                 145                 150
```

```
ccc tac tac atc gaa tgc agc tcg aag acc caa cta aat gtc aag ggt    593
Pro Tyr Tyr Ile Glu Cys Ser Ser Lys Thr Gln Leu Asn Val Lys Gly
        155                 160                 165

gta ttt gat gcg gca ata aag gtg gta ctg cag cca cca aag gca aag    641
Val Phe Asp Ala Ala Ile Lys Val Val Leu Gln Pro Pro Lys Ala Lys
    170                 175                 180

aag aag aaa aag gcg cag agg ggg gct tgc tcc atc ttg tgatctaatc     690
Lys Lys Lys Lys Ala Gln Arg Gly Ala Cys Ser Ile Leu
185                 190                 195

aatcggtaga caaagaacaa gggcgaagtt gccgccatgc tatattattg ttacgtcttg 750

cttcagcgga gctgcactct catggtcgtn ctnccttccc tcaccccac cccaccctag 810

gttacccacc ggcagctgca acaaggtctc tttgtcgagg catcggg                857
```

<210> 2
<211> 197
<212> PRT
<213> Hordeum vulgare

<400> 2

```
Met Ser Ala Ser Arg Phe Ile Lys Cys Val Thr Val Gly Asp Gly Ala
1               5                   10                  15

Val Gly Lys Thr Cys Met Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asp Gly Asn Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Leu Leu Ala Phe Ser Leu Ile Ser Lys Ala Ser Tyr Glu Asn Val
                85                  90                  95

Ser Lys Lys Trp Ile Pro Glu Leu Lys His Tyr Ala Pro Gly Val Pro
            100                 105                 110

Ile Ile Leu Val Gly Thr Lys Leu Asp Leu Arg Asp Asp Lys Gln Phe
        115                 120                 125

Phe Val Asp His Pro Gly Ala Val Pro Ile Thr Thr Ala Gln Gly Glu
    130                 135                 140

Glu Leu Lys Lys Leu Ile Gly Ala Pro Tyr Tyr Ile Glu Cys Ser Ser
145                 150                 155                 160

Lys Thr Gln Leu Asn Val Lys Gly Val Phe Asp Ala Ala Ile Lys Val
                165                 170                 175

Val Leu Gln Pro Pro Lys Ala Lys Lys Lys Lys Lys Ala Gln Arg Gly
            180                 185                 190

Ala Cys Ser Ile Leu
        195
```

<210> 3
<211> 1113
<212> DNA
<213> Oryza sativa

<220>
<221> CDS
<222> (158)..(748)
<400> 3

```
ccttgctttg ctcctccttc aaccttcttc tttcttggag tttcttgaga gagagagaga 60
gagagagaga gagagagaga gagagagaga gggggggag cggtcgcagg aggaggagga 120
cggcggcgtc tgctgcgacc gacggggagc ggcgagg atg agc gcg tcc agg ttc  175
                                         Met Ser Ala Ser Arg Phe
                                          1               5

ata aag tgc gtc acc gtc ggg gac ggc gcc gtc ggc aag acc tgc atg   223
Ile Lys Cys Val Thr Val Gly Asp Gly Ala Val Gly Lys Thr Cys Met
            10                  15                  20

ctc atc tcc tac acc tcc aac acc ttc ccc act gat tat gtt ccg acg   271
Leu Ile Ser Tyr Thr Ser Asn Thr Phe Pro Thr Asp Tyr Val Pro Thr
        25                  30                  35

gtg ttt gac aac ttc agt gcc aac gtc gtg gtt gat ggt aac acc gtc   319
Val Phe Asp Asn Phe Ser Ala Asn Val Val Val Asp Gly Asn Thr Val
    40                  45                  50

aac ctc ggg cta tgg gac act gca ggt cag gag gat tac aac aga ctg   367
Asn Leu Gly Leu Trp Asp Thr Ala Gly Gln Glu Asp Tyr Asn Arg Leu
55                  60                  65                  70

aga cca ctg agt tat cgt gga gct gat gtt ttc ctt ctg gcc ttc tcg   415
Arg Pro Leu Ser Tyr Arg Gly Ala Asp Val Phe Leu Leu Ala Phe Ser
                75                  80                  85

cta atc agc aag gcc agc tat gag aat gtt tca aag aag tgg ata cct   463
Leu Ile Ser Lys Ala Ser Tyr Glu Asn Val Ser Lys Lys Trp Ile Pro
            90                  95                  100

gag ctg aag cat tat gca cct ggt gtt cct atc atc ctt gtg gga aca   511
Glu Leu Lys His Tyr Ala Pro Gly Val Pro Ile Ile Leu Val Gly Thr
        105                 110                 115

aag ctt gat ctt cga gat gac aag cag ttt ttt gtg gac cat cct ggt   559
Lys Leu Asp Leu Arg Asp Asp Lys Gln Phe Phe Val Asp His Pro Gly
    120                 125                 130

gct gtt cct atc acc act gct cag gga gag gaa cta aga aag caa ata   607
Ala Val Pro Ile Thr Thr Ala Gln Gly Glu Glu Leu Arg Lys Gln Ile
135                 140                 145                 150

ggc gcc cca tac tac atc gaa tgc agc tca aag acc caa cta aac gtc   655
Gly Ala Pro Tyr Tyr Ile Glu Cys Ser Ser Lys Thr Gln Leu Asn Val
                155                 160                 165

aag ggc gtt ttc gat gcg gca ata aag gtg gtg ctg cag cca ccc aag   703
Lys Gly Val Phe Asp Ala Ala Ile Lys Val Val Leu Gln Pro Pro Lys
            170                 175                 180

gcg aag aag aag aaa aag gcg caa agg ggg gcg tgc tcc att ttg       748
Ala Lys Lys Lys Lys Lys Ala Gln Arg Gly Ala Cys Ser Ile Leu
        185                 190                 195

tgatctaatc atcagtagac gacgaagaag aagaacgatg aagttgccag gctttattat 808
tgttgcgtct tgcttcagcg aaacagcatt catggtccgg ggatcctagt ttactggcag 868
ctgcagcaag gcctctttgt cgaggcaatg agcgatccgt ttgtttcatt ttctcctttc 928
tgccttgtga ttatctcgtg tgactgacaa gtcgtggcaa ttaggtaact ttcctagatg 988
```

gtatttcctg tgtttgagaa aaaaaattct tgttatccct gtttcataag tagacatgat 1048
gtaatcgcac tcagtttatt cttttccttc ttatttcact tcaatggaaa attatgtttc 1108
ccttc 1113

<210> 4
<211> 197
<212> PRT
<213> Oryza sativa

<400> 4

Met Ser Ala Ser Arg Phe Ile Lys Cys Val Thr Val Gly Asp Gly Ala
1 5 10 15

Val Gly Lys Thr Cys Met Leu Ile Ser Tyr Thr Ser Asn Thr Phe Pro
20 25 30

Thr Asp Tyr Val Pro Thr Val Phe Asp Asn Phe Ser Ala Asn Val Val
35 40 45

Val Asp Gly Asn Thr Val Asn Leu Gly Leu Trp Asp Thr Ala Gly Gln
50 55 60

Glu Asp Tyr Asn Arg Leu Arg Pro Leu Ser Tyr Arg Gly Ala Asp Val
65 70 75 80

Phe Leu Leu Ala Phe Ser Leu Ile Ser Lys Ala Ser Tyr Glu Asn Val
85 90 95

Ser Lys Lys Trp Ile Pro Glu Leu Lys His Tyr Ala Pro Gly Val Pro
100 105 110

Ile Ile Leu Val Gly Thr Lys Leu Asp Leu Arg Asp Asp Lys Gln Phe
115 120 125

Phe Val Asp His Pro Gly Ala Val Pro Ile Thr Thr Ala Gln Gly Glu
130 135 140

Glu Leu Arg Lys Gln Ile Gly Ala Pro Tyr Tyr Ile Glu Cys Ser Ser
145 150 155 160

Lys Thr Gln Leu Asn Val Lys Gly Val Phe Asp Ala Ala Ile Lys Val
165 170 175

Val Leu Gln Pro Pro Lys Ala Lys Lys Lys Lys Lys Ala Gln Arg Gly
180 185 190

Ala Cys Ser Ile Leu
195

<210> 5
<211> 1393
<212> DNA
<213> Zea mays

<220>
<221> CDS
<222> (398)..(988)

<400> 5

```
gtcgacccac gcgtccgcgg acgcgtgggc ggacgcgtgg gtcccccaccc accaccgcgc 60
cgggccacca ccacccactc taccctcccc tccccaccac cactagcacc caccgtcccg 120
gcgcggagac cgcttccctc cctccgcctc cgcaaccctc tcccgcctcg cccgcgcctc 180
cctccatttg tccgcggctc ccctccctcc cgatcttaac cacccgccac ccggcttcct 240
```

```
ctcccccttc ttcctccctc aaaccagacg ctcgcccccc tttcctccac gcctatcttc 300
ttcagacgac cagcaggagg tacgaggaag accacctagg aggcctctct ctctctctcc 360
ccagccaccc ccgtagcgag agggagggcg gaagagg atg agc gcg tcc agg ttc  415
                                        Met Ser Ala Ser Arg Phe
                                        1               5

ata aag tgc gtc acg gtc ggg gac ggc gcc gtc ggc aag acc tgc atg   463
Ile Lys Cys Val Thr Val Gly Asp Gly Ala Val Gly Lys Thr Cys Met
            10                  15                  20

ctc atc tcc tac acc tcc aac acc ttc ccc acc gac tat gtt ccg aca   511
Leu Ile Ser Tyr Thr Ser Asn Thr Phe Pro Thr Asp Tyr Val Pro Thr
        25                  30                  35

gtg ttt gat aac ttc agt gcc aac gtt gtg gtt gat ggt aat act gtc   559
Val Phe Asp Asn Phe Ser Ala Asn Val Val Val Asp Gly Asn Thr Val
    40                  45                  50

aac ctc ggc ctc tgg gac act gca ggt caa gag gat tac aac aga ctg   607
Asn Leu Gly Leu Trp Asp Thr Ala Gly Gln Glu Asp Tyr Asn Arg Leu
55                  60                  65                  70

aga cca ctg agc tat cgt gga gct gat gtt ttt ctt ctg gct ttc tca   655
Arg Pro Leu Ser Tyr Arg Gly Ala Asp Val Phe Leu Leu Ala Phe Ser
                75                  80                  85

ctg atc agt aag gcc agc tat gag aat gtt tcg aag aag tgg ata cct   703
Leu Ile Ser Lys Ala Ser Tyr Glu Asn Val Ser Lys Lys Trp Ile Pro
            90                  95                  100

gaa ctg aag cat tat gca cct ggt gtg cca att att ctc gta ggg aca   751
Glu Leu Lys His Tyr Ala Pro Gly Val Pro Ile Ile Leu Val Gly Thr
        105                 110                 115

aag ctt gat ctt cga gac gac aag cag ttc ttt gtg gac cat cct ggt   799
Lys Leu Asp Leu Arg Asp Asp Lys Gln Phe Phe Val Asp His Pro Gly
    120                 125                 130

gct gtc cct atc act act gct cag gga gag gag cta aga aag caa ata   847
Ala Val Pro Ile Thr Thr Ala Gln Gly Glu Glu Leu Arg Lys Gln Ile
135                 140                 145                 150

ggc gct cca tac tac atc gaa tgc agc tcg aag acc caa cta aac gtg   895
Gly Ala Pro Tyr Tyr Ile Glu Cys Ser Ser Lys Thr Gln Leu Asn Val
                155                 160                 165

aag ggc gtc ttc gat gcg gcg ata aag gtt gtg ctg cag ccg cct aag   943
Lys Gly Val Phe Asp Ala Ala Ile Lys Val Val Leu Gln Pro Pro Lys
            170                 175                 180

gcg aag aag aag aaa aag gtg cag agg ggg gcg tgc tcc att ttg       988
Ala Lys Lys Lys Lys Lys Val Gln Arg Gly Ala Cys Ser Ile Leu
        185                 190                 195

tgatctaatc atcggtagat gaagaaacaa gggcgaaggt gccatggctt tatcatcgtc 1048
gcgtcttgct tcagtggaac agcatgaatg gtccccaccc cctctaggtt tactggcggc 1108
tcggctgcag cgagttctca tctctttgtc gaggcattga gcgatatgtt tgtttcattt 1168
tcctccttcc tgccttgtga ttatctggtg tgtgtgtgtg tgtgactgac gaagtcgcgg 1228
cgattaggta actcgcttag aaggtatttc ccgtgtttga gcaaaagaaa gtatccctgt 1288
tatctctgtt ccataagtta gacatgatgt aatcgtacta agtttatttt tacttatttc 1348
acttgaatgg aaaagtatgc ttcccattta aaaaaaaaaa aaaaa                 1393
```

<210> 6
<211> 197
<212> PRT
<213> Zea mays

<400> 6

Met Ser Ala Ser Arg Phe Ile Lys Cys Val Thr Val Gly Asp Gly Ala
1               5                   10                  15

Val Gly Lys Thr Cys Met Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asp Gly Asn Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Leu Leu Ala Phe Ser Leu Ile Ser Lys Ala Ser Tyr Glu Asn Val
                85                  90                  95

Ser Lys Lys Trp Ile Pro Glu Leu Lys His Tyr Ala Pro Gly Val Pro
            100                 105                 110

Ile Ile Leu Val Gly Thr Lys Leu Asp Leu Arg Asp Asp Lys Gln Phe
        115                 120                 125

Phe Val Asp His Pro Gly Ala Val Pro Ile Thr Thr Ala Gln Gly Glu
    130                 135                 140

Glu Leu Arg Lys Gln Ile Gly Ala Pro Tyr Tyr Ile Glu Cys Ser Ser
145                 150                 155                 160

Lys Thr Gln Leu Asn Val Lys Gly Val Phe Asp Ala Ala Ile Lys Val
                165                 170                 175

Val Leu Gln Pro Pro Lys Ala Lys Lys Lys Lys Lys Val Gln Arg Gly
            180                 185                 190

Ala Cys Ser Ile Leu
        195

<210> 7
<211> 197
<212> PRT
<213> Künstliche Sequenz

<220>
<223> Beschreibung der künstlichen Sequenz:
dominant-negative mutant of barley RacB protein

<220>
<221> SITE
<222> (20)
<223> Thr/Asn mutation for dominant-negative phenotype

<400> 7

Met Ser Ala Ser Arg Phe Ile Lys Cys Val Thr Val Gly Asp Gly Ala
1 5 10 15

Val Gly Lys Asn Cys Met Leu Ile Ser Tyr Thr Ser Asn Thr Phe Pro
20 25 30

Thr Asp Tyr Val Pro Thr Val Phe Asp Asn Phe Ser Ala Asn Val Val
35 40 45

Val Asp Gly Asn Thr Val Asn Leu Gly Leu Trp Asp Thr Ala Gly Gln
50 55 60

Glu Asp Tyr Asn Arg Leu Arg Pro Leu Ser Tyr Arg Gly Ala Asp Val
65 70 75 80

Phe Leu Leu Ala Phe Ser Leu Ile Ser Lys Ala Ser Tyr Glu Asn Val
85 90 95

Ser Lys Lys Trp Ile Pro Glu Leu Lys His Tyr Ala Pro Gly Val Pro
100 105 110

Ile Ile Leu Val Gly Thr Lys Leu Asp Leu Arg Asp Asp Lys Gln Phe
115 120 125

Phe Val Asp His Pro Gly Ala Val Pro Ile Thr Thr Ala Gln Gly Glu
130 135 140

Glu Leu Lys Lys Leu Ile Gly Ala Pro Tyr Tyr Ile Glu Cys Ser Ser
145 150 155 160

Lys Thr Gln Leu Asn Val Lys Gly Val Phe Asp Ala Ala Ile Lys Val
165 170 175

Val Leu Gln Pro Pro Lys Ala Lys Lys Lys Lys Lys Ala Gln Arg Gly
180 185 190

Ala Cys Ser Ile Leu
195

<210> 8
<211> 197
<212> PRT
<213> Künstliche Sequenz

<220>
<223> Beschreibung der künstlichen Sequenz: : dominant-negative mutant of rice RacB protein

<220>
<221> SITE
<222> (20)
<223> Thr/Asn mutation for dominant-negative phenotype

<400> 8

Met Ser Ala Ser Arg Phe Ile Lys Cys Val Thr Val Gly Asp Gly Ala
1 5 10 15

Val Gly Lys Asn Cys Met Leu Ile Ser Tyr Thr Ser Asn Thr Phe Pro
20 25 30

Thr Asp Tyr Val Pro Thr Val Phe Asp Asn Phe Ser Ala Asn Val Val
35 40 45

Val Asp Gly Asn Thr Val Asn Leu Gly Leu Trp Asp Thr Ala Gly Gln
50 55 60

Glu Asp Tyr Asn Arg Leu Arg Pro Leu Ser Tyr Arg Gly Ala Asp Val
65 70 75 80

Phe Leu Leu Ala Phe Ser Leu Ile Ser Lys Ala Ser Tyr Glu Asn Val
85 90 95

Ser Lys Lys Trp Ile Pro Glu Leu Lys His Tyr Ala Pro Gly Val Pro
100 105 110

Ile Ile Leu Val Gly Thr Lys Leu Asp Leu Arg Asp Asp Lys Gln Phe
115 120 125

Phe Val Asp His Pro Gly Ala Val Pro Ile Thr Thr Ala Gln Gly Glu
130 135 140

Glu Leu Arg Lys Gln Ile Gly Ala Pro Tyr Tyr Ile Glu Cys Ser Ser
145 150 155 160

Lys Thr Gln Leu Asn Val Lys Gly Val Phe Asp Ala Ala Ile Lys Val
165 170 175

Val Leu Gln Pro Pro Lys Ala Lys Lys Lys Lys Lys Ala Gln Arg Gly
180 185 190

Ala Cys Ser Ile Leu
195

<210> 9
<211> 197
<212> PRT
<213> Künstliche Sequenz

<220>
<221> SITE
<222> (20)
<223> Thr/Asn mutation for dominant-negative phenotype

<220>
<223> Beschreibung der künstlichen Sequenz:
dominant-negative mutant of maize RacB protein

<400> 9

```
Met Ser Ala Ser Arg Phe Ile Lys Cys Val Thr Val Gly Asp Gly Ala
 1               5                  10                  15

Val Gly Lys Asn Cys Met Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asp Gly Asn Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Leu Leu Ala Phe Ser Leu Ile Ser Lys Ala Ser Tyr Glu Asn Val
                85                  90                  95

Ser Lys Lys Trp Ile Pro Glu Leu Lys His Tyr Ala Pro Gly Val Pro
            100                 105                 110

Ile Ile Leu Val Gly Thr Lys Leu Asp Leu Arg Asp Asp Lys Gln Phe
        115                 120                 125

Phe Val Asp His Pro Gly Ala Val Pro Ile Thr Thr Ala Gln Gly Glu
    130                 135                 140

Glu Leu Arg Lys Gln Ile Gly Ala Pro Tyr Tyr Ile Glu Cys Ser Ser
145                 150                 155                 160

Lys Thr Gln Leu Asn Val Lys Gly Val Phe Asp Ala Ala Ile Lys Val
                165                 170                 175

Val Leu Gln Pro Pro Lys Ala Lys Lys Lys Lys Lys Val Gln Arg Gly
            180                 185                 190

Ala Cys Ser Ile Leu
        195
```

<210> 10
<211> 24
<212> DNA
<213> Künstliche Sequenz

<220>
<223> Beschreibung der künstlichen Sequenz:
oligonucleotide primer

<400> 10
ggatccgatg agcgcgtcca ggtt 24

<210> 11
<211> 26
<212> DNA
<213> Künstliche Sequenz

<220>
<223> Beschreibung der künstlichen Sequenz:
oligonucleotide primer

<400> 11

gtcgaccttc gcccttgttc tttgtc 26

<210> 12
<211> 26
<212> DNA
<213> Künstliche Sequenz

<220>
<223> Beschreibung der künstlichen Sequenz: oligonucleotide primer

<400> 12
gtgggcacat agtcggtggg gaaggt 26

<210> 13
<211> 23
<212> DNA
<213> Künstliche Sequenz

<220>
<223> Beschreibung der künstlichen Sequenz: oligonucleotide primer

<400> 13
cgactggagc acgaggacac tga 23

<210> 14
<211> 26
<212> DNA
<213> Künstliche Sequenz

<220>
<223> Beschreibung der künstlichen Sequenz: oligonucleotide primer

<400> 14
ggacactgac atggactgaa ggagta 26

<210> 15
<211> 22
<212> DNA
<213> Künstliche Sequenz

<220>
<223> Beschreibung der künstlichen Sequenz: oligonucleotide primer

<400> 15
gttcatcaag tgcgtcaccg tg 22

<210> 16
<211> 23
<212> DNA
<213> Künstliche Sequenz

<220>
<223> Beschreibung der künstlichen Sequenz: oligonucleotide primer

<400> 16
ttagcttcct cagttcttcc ctg 23

<210> 17
<211> 21
<212> DNA
<213> Künstliche Sequenz

<220>
<223> Beschreibung der künstlichen Sequenz:
oligonucleotide primer

<400> 17
cgcgccgcag ccgagtacga c 21

<210> 18
<211> 21
<212> DNA
<213> Künstliche Sequenz

<220>
<223> Beschreibung der künstlichen Sequenz:
oligonucleotide primer

<400> 18
gtcacaaaaa cacatgtaac c 21

<210> 19
<211> 20
<212> DNA
<213> Künstliche Sequenz

<220>
<223> Beschreibung der künstlichen Sequenz:
oligonucleotide primer

<400> 19
ggccgacatg cattcaccag 20

<210> 20
<211> 21
<212> DNA
<213> Künstliche Sequenz

<220>
<223> Beschreibung der künstlichen Sequenz:
oligonucleotide primer

<400> 20
catctgatat tgctgggtct g 21

<210> 21
<211> 21
<212> DNA
<213> Künstliche Sequenz

<220>
<223> Beschreibung der künstlichen Sequenz:

oligonucleotide primer

<400> 21
ccaagatgca gatcttcgtg a 21

<210> 22
<211> 21
<212> DNA
<213> Künstliche Sequenz

<220>
<223> Beschreibung der künstlichen Sequenz:
oligonucleotide primer

<400> 22
ttcgcgatag gtaaaagagc a 21

<210> 23
<211> 18
<212> DNA
<213> Künstliche Sequenz

<220>
<223> Beschreibung der künstlichen Sequenz:
oligonucleotide primer

<400> 23
gtaaaacgac ggccagtg 18

<210> 24
<211> 19
<212> DNA
<213> Künstliche Sequenz

<220>
<223> Beschreibung der künstlichen Sequenz:
oligonucleotide primer

<400> 24
ggaaacagct atgaccatg 19

<210> 25
<211> 17
<212> DNA
<213> Künstliche Sequenz

<220>
<223> Beschreibung der künstlichen Sequenz:
oligonucleotide primer

<400> 25
gtggaggcgc ggcgaga 17

<210> 26
<211> 22
<212> DNA
<213> Künstliche Sequenz

<220>
<223> Beschreibung der künstlichen Sequenz:
oligonucleotide primer

<400> 26
ccatgcttca tctccatagt ca 22

<210> 27
<211> 26
<212> DNA
<213> Künstliche Sequenz

<220>
<223> Beschreibung der künstlichen Sequenz:
oligonucleotide primer

<400> 27
ggatcccgat tccatcagga aagcat 26

<210> 28
<211> 26
<212> DNA
<213> Künstliche Sequenz

<220>
<223> Beschreibung der künstlichen Sequenz:
oligonucleotide primer

<400> 28
gtcgacgcga gacactgcaa aacaaa 26

<210> 29
<211> 26
<212> DNA
<213> Künstliche Sequenz

<220>
<223> Beschreibung der künstlichen Sequenz:
oligonucleotide primer

<400> 29
ggatccttct cgtccattta gccggc 26

<210> 30
<211> 27
<212> DNA
<213> Künstliche Sequenz

<220>
<223> Beschreibung der künstlichen Sequenz:
oligonucleotide primer

<400> 30
gtcgactgat cacttgaagc atgccag 27

<210> 31
<211> 24
<212> DNA

<213> Künstliche Sequenz

<220>
<223> Beschreibung der künstlichen Sequenz: oligonucleotide primer

<400> 31
ggatccgatg agcgcgtcca ggtt 24

<210> 32
<211> 26
<212> DNA
<213> Künstliche Sequenz

<220>
<223> Beschreibung der künstlichen Sequenz: oligonucleotide primer

<400> 32
gtcgaccttc gcccttgttc tttgtc 26

<210> 33
<211> 29
<212> DNA
<213> Künstliche Sequenz

<220>
<223> Beschreibung der künstlichen Sequenz: oligonucleotide primer

<400> 33
accgtggggg acgtcgccgt cggcaagac 29

<210> 34
<211> 721
<212> DNA
<213> Hordeum vulgare

<220>
<221> CDS
<222> (38) .. (673)
<223> coding for RacB homologue (Rop6)

<400> 34

```
gtggaggcgc ggcgagagcg gcggaggcgg aggagag atg agc gtg acc aag ttc   55
                                         Met Ser Val Thr Lys Phe
                                         1               5

atc aag tgc gtc acg gtg ggg gac ggc gcc gtc ggc aag acc tgc atg   103
Ile Lys Cys Val Thr Val Gly Asp Gly Ala Val Gly Lys Thr Cys Met
            10                  15                  20

ctc atc tgc tac acc agc aac agg ttc ccc agt gat tac atc ccc acg   151
Leu Ile Cys Tyr Thr Ser Asn Arg Phe Pro Ser Asp Tyr Ile Pro Thr
        25                  30                  35

gtg ttc gac aac ttc agc gcc aac gtc tcc gtc gac ggc aac atc gtc   199
Val Phe Asp Asn Phe Ser Ala Asn Val Ser Val Asp Gly Asn Ile Val
    40                  45                  50

aac ctc ggc cta tgg gac acc gcc ggg caa gaa gac tac agc cgg ctg   247
Asn Leu Gly Leu Trp Asp Thr Ala Gly Gln Glu Asp Tyr Ser Arg Leu
55                  60                  65                  70

agg ccg ctg agc tac aga ggc gcc gac gtg ttc gtg ctc gcc ttc tcc   295
Arg Pro Leu Ser Tyr Arg Gly Ala Asp Val Phe Val Leu Ala Phe Ser
                75                  80                  85

ctc atc agc agc gcc agc tac gag aat gtt ctt aag aag tgg atg cca   343
Leu Ile Ser Ser Ala Ser Tyr Glu Asn Val Leu Lys Lys Trp Met Pro
            90                  95                  100

gag ctc cgc cgg ttc gcg ccg aat gtc ccc att gtt ctt gtt ggg acc   391
Glu Leu Arg Arg Phe Ala Pro Asn Val Pro Ile Val Leu Val Gly Thr
        105                 110                 115

aag cta gat ctg cgt gac cac aga gcc tac ctc gcc gac cac ccc ggt   439
Lys Leu Asp Leu Arg Asp His Arg Ala Tyr Leu Ala Asp His Pro Gly
    120                 125                 130

gct tca gca atc aca act gca cag ggt gaa gaa ctt agg aag cag atc   487
Ala Ser Ala Ile Thr Thr Ala Gln Gly Glu Glu Leu Arg Lys Gln Ile
135                 140                 145                 150

ggc gcc gcg gct tac atc gag tgc agc tcc aag aca cag cag aac gtc   535
Gly Ala Ala Ala Tyr Ile Glu Cys Ser Ser Lys Thr Gln Gln Asn Val
                155                 160                 165

aag gct gtg ttt gac acc gcc ata aag gtg gtc ctc cag ccg ccg agg   583
Lys Ala Val Phe Asp Thr Ala Ile Lys Val Val Leu Gln Pro Pro Arg
            170                 175                 180

aga agg gag gtg atg tcc gcc agg aag aaa acc agg cga agc tct gga   631
Arg Arg Glu Val Met Ser Ala Arg Lys Lys Thr Arg Arg Ser Ser Gly
        185                 190                 195

tgc tcc atc aag cac ttg atc tgc ggg agt acg tgc gct gct           673
Cys Ser Ile Lys His Leu Ile Cys Gly Ser Thr Cys Ala Ala
    200                 205                 210

tgaattagca ccatggaggc ctggactgac tatggagatg aagcatgg              721
```

<210> 35
<211> 212
<212> PRT
<213> Hordeum vulgare

<400> 35

```
Met Ser Val Thr Lys Phe Ile Lys Cys Val Thr Val Gly Asp Gly Ala
1               5                   10                  15

Val Gly Lys Thr Cys Met Leu Ile Cys Tyr Thr Ser Asn Arg Phe Pro
            20                  25                  30

Ser Asp Tyr Ile Pro Thr Val Phe Asp Asn Phe Ser Ala Asn Val Ser
        35                  40                  45

Val Asp Gly Asn Ile Val Asn Leu Gly Leu Trp Asp Thr Ala Gly Gln
    50                  55                  60

Glu Asp Tyr Ser Arg Leu Arg Pro Leu Ser Tyr Arg Gly Ala Asp Val
65                  70                  75                  80

Phe Val Leu Ala Phe Ser Leu Ile Ser Ser Ala Ser Tyr Glu Asn Val
                85                  90                  95

Leu Lys Lys Trp Met Pro Glu Leu Arg Arg Phe Ala Pro Asn Val Pro
            100                 105                 110

Ile Val Leu Val Gly Thr Lys Leu Asp Leu Arg Asp His Arg Ala Tyr
        115                 120                 125

Leu Ala Asp His Pro Gly Ala Ser Ala Ile Thr Thr Ala Gln Gly Glu
    130                 135                 140

Glu Leu Arg Lys Gln Ile Gly Ala Ala Ala Tyr Ile Glu Cys Ser Ser
145                 150                 155                 160

Lys Thr Gln Gln Asn Val Lys Ala Val Phe Asp Thr Ala Ile Lys Val
                165                 170                 175

Val Leu Gln Pro Pro Arg Arg Arg Glu Val Met Ser Ala Arg Lys Lys
            180                 185                 190

Thr Arg Arg Ser Ser Gly Cys Ser Ile Lys His Leu Ile Cys Gly Ser
        195                 200                 205

Thr Cys Ala Ala
    210
```

<210> 36
<211> 699
<212> DNA
<213> Hordeum vulgare

<220>
<221> CDS
<222> (67)..(657)
<223> coding for RacB homologue (RacD)

<400> 36

```
ggatcccgat tccatcagga aagcatatag actagcccag taaatagaaa taagnaaaga 60

tcggcg atg agc gca tct cgg ttc atc aag tgc gtg acg gtg ggg gac     108
       Met Ser Ala Ser Arg Phe Ile Lys Cys Val Thr Val Gly Asp
        1               5                  10

ggc gcc gtg gga aag aca tgc ctc ctc atc tca tac aca tcc aac acc    156
Gly Ala Val Gly Lys Thr Cys Leu Leu Ile Ser Tyr Thr Ser Asn Thr
 15                  20                  25                  30

ttc ccc aca gac tat gtc cca aca gtt ttc gac aac ttc agc gct aac    204
Phe Pro Thr Asp Tyr Val Pro Thr Val Phe Asp Asn Phe Ser Ala Asn
                 35                  40                  45

gtc gtg gtt gac ggc agc acc gtc aac ctc gga tta tgg gat act gca    252
Val Val Val Asp Gly Ser Thr Val Asn Leu Gly Leu Trp Asp Thr Ala
             50                  55                  60

gga caa gaa gac tat aat cga cta cgc cca cta agc tac cgt ggt gcc    300
Gly Gln Glu Asp Tyr Asn Arg Leu Arg Pro Leu Ser Tyr Arg Gly Ala
         65                  70                  75

gat gtc ttc ctg ctc gcc ttt tct ctc atc agc aaa gca agc tac gag    348
Asp Val Phe Leu Leu Ala Phe Ser Leu Ile Ser Lys Ala Ser Tyr Glu
     80                  85                  90

aat gtc act aag aag tgg att cca gag tta cgg cac tat gct cct ggc    396
Asn Val Thr Lys Lys Trp Ile Pro Glu Leu Arg His Tyr Ala Pro Gly
 95                 100                 105                 110

gtg ccc ata att ctt gtt gga aca aag ctt gat ctg cgg gat gac aag    444
Val Pro Ile Ile Leu Val Gly Thr Lys Leu Asp Leu Arg Asp Asp Lys
                115                 120                 125

cag ttt ttt gtg gat cac cct ggg gcg gtt cct att tcc act gct cag    492
Gln Phe Phe Val Asp His Pro Gly Ala Val Pro Ile Ser Thr Ala Gln
            130                 135                 140

ggt gaa gag ctg aag aag gtg att ggc gcg act gcc tac atc gag tgc    540
Gly Glu Glu Leu Lys Lys Val Ile Gly Ala Thr Ala Tyr Ile Glu Cys
        145                 150                 155

agc tca aaa aca cag cag aac atc aag gcg gtg ttt gat gcg gcg atc    588
Ser Ser Lys Thr Gln Gln Asn Ile Lys Ala Val Phe Asp Ala Ala Ile
    160                 165                 170

aag gtg gtc ctc cag cct ccg aag cag aag cgg aag aag agg aag tca    636
Lys Val Val Leu Gln Pro Pro Lys Gln Lys Arg Lys Lys Arg Lys Ser
175                 180                 185                 190

cag aaa gga tgc agc atc ttg taaagctaaa atccctttg ttttgcagtg        687
Gln Lys Gly Cys Ser Ile Leu
                195

tctcgcgtcg ac                                                      699
```

<210> 37
<211> 197
<212> PRT
<213> Hordeum vulgare

<400> 37

```
Met Ser Ala Ser Arg Phe Ile Lys Cys Val Thr Val Gly Asp Gly Ala
1               5                   10                  15

Val Gly Lys Thr Cys Leu Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asp Gly Ser Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Leu Leu Ala Phe Ser Leu Ile Ser Lys Ala Ser Tyr Glu Asn Val
                85                  90                  95

Thr Lys Lys Trp Ile Pro Glu Leu Arg His Tyr Ala Pro Gly Val Pro
            100                 105                 110

Ile Ile Leu Val Gly Thr Lys Leu Asp Leu Arg Asp Asp Lys Gln Phe
        115                 120                 125

Phe Val Asp His Pro Gly Ala Val Pro Ile Ser Thr Ala Gln Gly Glu
    130                 135                 140

Glu Leu Lys Lys Val Ile Gly Ala Thr Ala Tyr Ile Glu Cys Ser Ser
145                 150                 155                 160

Lys Thr Gln Gln Asn Ile Lys Ala Val Phe Asp Ala Ala Ile Lys Val
                165                 170                 175

Val Leu Gln Pro Pro Lys Gln Lys Arg Lys Lys Arg Lys Ser Gln Lys
            180                 185                 190

Gly Cys Ser Ile Leu
        195
```

<210> 38
<211> 677
<212> DNA
<213> Hordeum vulgare

<220>
<221> CDS
<222> (27)..(665)
<223> coding for RacB homologue (Rop4)

<400> 38

```
ggatccttct cgtccattta gccggc atg gcg tcc agc gcc tcc cgg ttc atc  53
                             Met Ala Ser Ser Ala Ser Arg Phe Ile
                             1               5

aag tgc gtc acc gtc ggg gac ggc gcc gtc ggc aag acc tgc atg ctc  101
Lys Cys Val Thr Val Gly Asp Gly Ala Val Gly Lys Thr Cys Met Leu
10                  15                  20                  25

atc tgc tac acc agc aac aag ttc ccc acc gac tac gtg ccc acc gtg  149
Ile Cys Tyr Thr Ser Asn Lys Phe Pro Thr Asp Tyr Val Pro Thr Val
                30                  35                  40

ttc gac aat ttc agc gcg aac gtg gtg gtg gac ggc acc acc gtg aac  197
Phe Asp Asn Phe Ser Ala Asn Val Val Val Asp Gly Thr Thr Val Asn
            45                  50                  55

ctg ggc ctc tgg gac act gca ggg cag gag gac tac aac aga ttg aga  245
Leu Gly Leu Trp Asp Thr Ala Gly Gln Glu Asp Tyr Asn Arg Leu Arg
        60                  65                  70

ccg ctg agc tac cgg gga gcc gac gtc ttc gtg ctc tcc ttc tcg ctc  293
Pro Leu Ser Tyr Arg Gly Ala Asp Val Phe Val Leu Ser Phe Ser Leu
    75                  80                  85

gtc agc cga gcc agc tac gag aat gtc atg aag aag tgg cta ccg gag  341
Val Ser Arg Ala Ser Tyr Glu Asn Val Met Lys Lys Trp Leu Pro Glu
90                  95                  100                 105

ctt cag cac cat gca ccc ggc gtg cca aca gtg ctg gtt ggt aca aag  389
Leu Gln His His Ala Pro Gly Val Pro Thr Val Leu Val Gly Thr Lys
                110                 115                 120

cta gat cta cgt gaa gac aag caa tac tta ctt gac cac ccc ggc gtg  437
Leu Asp Leu Arg Glu Asp Lys Gln Tyr Leu Leu Asp His Pro Gly Val
            125                 130                 135

gtg cct gtt act aca gct cag ggg gag gaa ctc cgc aag cac atc ggt  485
Val Pro Val Thr Thr Ala Gln Gly Glu Glu Leu Arg Lys His Ile Gly
        140                 145                 150

gca act tgt tat gtc gaa tgc agc tca aag aca cag cag aat gtc aaa  533
Ala Thr Cys Tyr Val Glu Cys Ser Ser Lys Thr Gln Gln Asn Val Lys
    155                 160                 165

gct gtg ttt gat gct gcc atc aag gta gtg atc aaa cct cca aca aag  581
Ala Val Phe Asp Ala Ala Ile Lys Val Val Ile Lys Pro Pro Thr Lys
170                 175                 180                 185

cag agg gaa agg agg aag aag aaa gca cgg caa gga tgt gca tca ttg  629
Gln Arg Glu Arg Arg Lys Lys Lys Ala Arg Gln Gly Cys Ala Ser Leu
                190                 195                 200

ggt acc ctg tca aga agg aag ctg gca tgc ttc aag tgatcagtcg ac    677
Gly Thr Leu Ser Arg Arg Lys Leu Ala Cys Phe Lys
            205                 210
```

<210> 39
<211> 213
<212> PRT
<213> Hordeum vulgare

<400> 39

```
Met Ala Ser Ser Ala Ser Arg Phe Ile Lys Cys Val Thr Val Gly Asp
1               5                   10                  15

Gly Ala Val Gly Lys Thr Cys Met Leu Ile Cys Tyr Thr Ser Asn Lys
            20                  25                  30

Phe Pro Thr Asp Tyr Val Pro Thr Val Phe Asp Asn Phe Ser Ala Asn
        35                  40                  45

Val Val Val Asp Gly Thr Thr Val Asn Leu Gly Leu Trp Asp Thr Ala
    50                  55                  60

Gly Gln Glu Asp Tyr Asn Arg Leu Arg Pro Leu Ser Tyr Arg Gly Ala
65                  70                  75                  80

Asp Val Phe Val Leu Ser Phe Ser Leu Val Ser Arg Ala Ser Tyr Glu
                85                  90                  95

Asn Val Met Lys Lys Trp Leu Pro Glu Leu Gln His His Ala Pro Gly
            100                 105                 110

Val Pro Thr Val Leu Val Gly Thr Lys Leu Asp Leu Arg Glu Asp Lys
        115                 120                 125

Gln Tyr Leu Leu Asp His Pro Gly Val Val Pro Val Thr Thr Ala Gln
    130                 135                 140

Gly Glu Glu Leu Arg Lys His Ile Gly Ala Thr Cys Tyr Val Glu Cys
145                 150                 155                 160

Ser Ser Lys Thr Gln Gln Asn Val Lys Ala Val Phe Asp Ala Ala Ile
                165                 170                 175

Lys Val Val Ile Lys Pro Pro Thr Lys Gln Arg Glu Arg Arg Lys Lys
            180                 185                 190

Lys Ala Arg Gln Gly Cys Ala Ser Leu Gly Thr Leu Ser Arg Arg Lys
        195                 200                 205

Leu Ala Cys Phe Lys
    210
```

<210> 40
<211> 945
<212> DNA
<213> Zea mays

<220>
<221> CDS
<222> (37)..(672)
<223> coding for RacB homologue (Rop6)

<400> 40

```
gagaagaagg gggcctgccg gccggggctg ggagac atg agc gtg acc aag ttc      54
                                        Met Ser Val Thr Lys Phe
                                         1               5

atc aag tgc gtc acg gtg ggc gac ggc gcg gtg ggc aag acc tgc atg      102
Ile Lys Cys Val Thr Val Gly Asp Gly Ala Val Gly Lys Thr Cys Met
            10                  15                  20

ctc atc tgc tac acc agc aac aag ttc ccc acg gac tac atc ccc acg      150
Leu Ile Cys Tyr Thr Ser Asn Lys Phe Pro Thr Asp Tyr Ile Pro Thr
        25                  30                  35

gtg ttc gac aac ttc agc gcc aac gtc tcc gtg gac ggc agc atc gtc      198
Val Phe Asp Asn Phe Ser Ala Asn Val Ser Val Asp Gly Ser Ile Val
    40                  45                  50

aac ctg ggc ctc tgg gac acc gcg ggg caa gag gac tac agc agg ctg      246
Asn Leu Gly Leu Trp Asp Thr Ala Gly Gln Glu Asp Tyr Ser Arg Leu
55                  60                  65                  70

cgg ccg ctg agc tac agg ggc gcg gac gtg ttc gtg ctg gcc ttc tcc      294
Arg Pro Leu Ser Tyr Arg Gly Ala Asp Val Phe Val Leu Ala Phe Ser
                75                  80                  85

ctg atc agc agg gcg agc tac gag aac gtt ctt aag aag tgg gtg cca      342
Leu Ile Ser Arg Ala Ser Tyr Glu Asn Val Leu Lys Lys Trp Val Pro
            90                  95                  100

gag ctt cgc aga ttc gcg ccc aac gtc ccg gtc gtt ctt gtt ggg acc      390
Glu Leu Arg Arg Phe Ala Pro Asn Val Pro Val Val Leu Val Gly Thr
        105                 110                 115

aag tta gat ctc cgc gac cac aga gcc tac ctc gcc gac cat cct gga      438
Lys Leu Asp Leu Arg Asp His Arg Ala Tyr Leu Ala Asp His Pro Gly
    120                 125                 130

gct tca gca gtc acc acg gcg cag ggt gag gaa ctg agg aag cag atc      486
Ala Ser Ala Val Thr Thr Ala Gln Gly Glu Glu Leu Arg Lys Gln Ile
135                 140                 145                 150
```

```
ggc gct gcg gcc tac atc gag tgc agt tcc aaa acc cag cag aac gtc     534
Gly Ala Ala Ala Tyr Ile Glu Cys Ser Ser Lys Thr Gln Gln Asn Val
                155                 160                 165

aag tct gtc ttc gat acg gcc atc aaa gtg gtc ctt cag ccc cca cgg     582
Lys Ser Val Phe Asp Thr Ala Ile Lys Val Val Leu Gln Pro Pro Arg
            170                 175                 180

agg agg gag gca gtg cct gcc agg aag aag aac agg cgt ggc tcc gga     630
Arg Arg Glu Ala Val Pro Ala Arg Lys Lys Asn Arg Arg Gly Ser Gly
        185                 190                 195

tgc tct ata atg aac ctt gtg tgt ggc agc aca tgt gct gct             672
Cys Ser Ile Met Asn Leu Val Cys Gly Ser Thr Cys Ala Ala
    200                 205                 210

tagggagtct actagaacac tgaaccggaa gggaggtgaa ggcgtgattc atggtgtgta 732

atgtgctgtg gcaactggca agttagtttg ctatagatga ggatgactgc tgcttttgtt 792

ttccttggcc catctgctgt agttcgtcag gctcttcaag ggctgacttt ttaccagact 852

gcagtgttgt gtaagaagtt tgctagacgc tgtaactgta atgttctccg ctgatgtggt 912

ataactaaga tacgagtaag cttgagcgtg ttc                              945
```

<210> 41
<211> 212
<212> PRT
<213> Zea mays

<400> 41

Met Ser Val Thr Lys Phe Ile Lys Cys Val Thr Val Gly Asp Gly Ala
1               5                   10                  15

Val Gly Lys Thr Cys Met Leu Ile Cys Tyr Thr Ser Asn Lys Phe Pro
            20                  25                  30

Thr Asp Tyr Ile Pro Thr Val Phe Asp Asn Phe Ser Ala Asn Val Ser
        35                  40                  45

Val Asp Gly Ser Ile Val Asn Leu Gly Leu Trp Asp Thr Ala Gly Gln
    50                  55                  60

Glu Asp Tyr Ser Arg Leu Arg Pro Leu Ser Tyr Arg Gly Ala Asp Val
65                  70                  75                  80

Phe Val Leu Ala Phe Ser Leu Ile Ser Arg Ala Ser Tyr Glu Asn Val
                85                  90                  95

Leu Lys Lys Trp Val Pro Glu Leu Arg Arg Phe Ala Pro Asn Val Pro
            100                 105                 110

Val Val Leu Val Gly Thr Lys Leu Asp Leu Arg Asp His Arg Ala Tyr
        115                 120                 125

Leu Ala Asp His Pro Gly Ala Ser Ala Val Thr Thr Ala Gln Gly Glu
    130                 135                 140

Glu Leu Arg Lys Gln Ile Gly Ala Ala Ala Tyr Ile Glu Cys Ser Ser
145                 150                 155                 160

Lys Thr Gln Gln Asn Val Lys Ser Val Phe Asp Thr Ala Ile Lys Val
                165                 170                 175

Val Leu Gln Pro Pro Arg Arg Arg Glu Ala Val Pro Ala Arg Lys Lys
            180                 185                 190

Asn Arg Arg Gly Ser Gly Cys Ser Ile Met Asn Leu Val Cys Gly Ser
        195                 200                 205

Thr Cys Ala Ala
    210

<210> 42
<211> 850
<212> DNA
<213> Oryza sativa

<220>
<221> CDS
<222> (112)..(702)
<223> coding for RacB homologue (RACDP / RACD)

<400> 42

```
agcaagcagc agctgaggtg aggtccgtgg cgttggagtg aggactgagg aggaagaaga 60
gggcgggatc tagggtaccg gatgcgctgg ctgtgctgag tgagagtaga g atg agc  117
                                                        Met Ser
                                                        1
gcg tct cgg ttc atc aag tgc gtc acc gtg ggg gac ggc gcc gtg ggc   165
Ala Ser Arg Phe Ile Lys Cys Val Thr Val Gly Asp Gly Ala Val Gly
        5                   10                  15
aag acc tgc atg ctc atc tcc tac acc tcc aac acc ttc ccc acg gac   213
Lys Thr Cys Met Leu Ile Ser Tyr Thr Ser Asn Thr Phe Pro Thr Asp
    20                  25                  30
tat gtt cca act gtt ttt gat aac ttc agt gca aat gtt gtg gtc gat   261
Tyr Val Pro Thr Val Phe Asp Asn Phe Ser Ala Asn Val Val Val Asp
35                  40                  45                  50
ggg agc act gtg aac ttg ggg ttg tgg gat aca gca gga caa gag gac   309
Gly Ser Thr Val Asn Leu Gly Leu Trp Asp Thr Ala Gly Gln Glu Asp
                55                  60                  65
tac aat agg cta cgc ccg ttg agc tat cgt ggc gct gat gtt ttc ctg   357
Tyr Asn Arg Leu Arg Pro Leu Ser Tyr Arg Gly Ala Asp Val Phe Leu
            70                  75                  80
ctg gcc ttt tct ctg atc agc aaa gca agc tat gag aat gtt tct aaa   405
Leu Ala Phe Ser Leu Ile Ser Lys Ala Ser Tyr Glu Asn Val Ser Lys
        85                  90                  95
aag tgg ata cct gaa tta agg cat tat gct cct ggt gtg cca ata att   453
Lys Trp Ile Pro Glu Leu Arg His Tyr Ala Pro Gly Val Pro Ile Ile
    100                 105                 110
ctc gtt gga aca aag ctt gat ctg cgg gat gat aag caa ttt ttc gta   501
Leu Val Gly Thr Lys Leu Asp Leu Arg Asp Asp Lys Gln Phe Phe Val
115                 120                 125                 130
gat cac cct ggt gct gta cct att tcc act gct cag ggc gaa gag ctg   549
Asp His Pro Gly Ala Val Pro Ile Ser Thr Ala Gln Gly Glu Glu Leu
                135                 140                 145
agg aaa ctc att ggt gca gcg gca tac att gaa tgc agt tca aaa aca   597
Arg Lys Leu Ile Gly Ala Ala Ala Tyr Ile Glu Cys Ser Ser Lys Thr
            150                 155                 160
cag caa aac atc aag gca gtt ttc gat gct gcg att aag gtg gtt ctc   645
Gln Gln Asn Ile Lys Ala Val Phe Asp Ala Ala Ile Lys Val Val Leu
        165                 170                 175
cag cct cca aag caa aag aag aag aag aaa aag gcg cag aaa gga tgt   693
Gln Pro Pro Lys Gln Lys Lys Lys Lys Lys Lys Ala Gln Lys Gly Cys
    180                 185                 190

gcc atc ttg taattaaatg gtagacagtg cagtgcagat cgatgtatcc           742
Ala Ile Leu
195
cttcatttgt agcctctggc ttcaatcgtc gcttgtttgt ataattacgc tagatgccac 802
cggcagaaga tataatatag tcctcctgcc tttgtggtgt tggtctct              850
```

<210> 43
<211> 197

<212> PRT
<213> Oryza sativa

<400> 43

```
Met Ser Ala Ser Arg Phe Ile Lys Cys Val Thr Val Gly Asp Gly Ala
1               5                   10                  15
Val Gly Lys Thr Cys Met Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asp Gly Ser Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Leu Leu Ala Phe Ser Leu Ile Ser Lys Ala Ser Tyr Glu Asn Val
                85                  90                  95
Ser Lys Lys Trp Ile Pro Glu Leu Arg His Tyr Ala Pro Gly Val Pro
            100                 105                 110
Ile Ile Leu Val Gly Thr Lys Leu Asp Leu Arg Asp Asp Lys Gln Phe
        115                 120                 125
Phe Val Asp His Pro Gly Ala Val Pro Ile Ser Thr Ala Gln Gly Glu
    130                 135                 140
Glu Leu Arg Lys Leu Ile Gly Ala Ala Ala Tyr Ile Glu Cys Ser Ser
145                 150                 155                 160
Lys Thr Gln Gln Asn Ile Lys Ala Val Phe Asp Ala Ala Ile Lys Val
                165                 170                 175
Val Leu Gln Pro Pro Lys Gln Lys Lys Lys Lys Lys Lys Ala Gln Lys
            180                 185                 190
Gly Cys Ala Ile Leu
        195
```

<210> 44
<211> 1169
<212> DNA
<213> Oryza sativa

<220>
<221> CDS
<222> (169)..(813)
<223> coding for RacB homologue (Rop 4)

<400> 44

```
aacagttcag agaggaagca tgtgacactt ccctctgtcc ctctctctct ctctagcctc 60
caaccatcgc ttcaccaaga agccatcacc tcctcctctc tatcaagttc tctcccctct 120
```

```
cttgctgtct ctgcttgctg ctgctgctgc tcgattcggc cggcggcc atg gcg tcc  177
                                                      Met Ala Ser
                                                        1

agc gcg tcg cgg ttc atc aag tgc gtc acg gtc ggg gac ggc gcc gtc  225
Ser Ala Ser Arg Phe Ile Lys Cys Val Thr Val Gly Asp Gly Ala Val
    5                   10                  15

ggc aag acc tgc atg ctc atc tgc tac acc agc aac aag ttc ccc act  273
Gly Lys Thr Cys Met Leu Ile Cys Tyr Thr Ser Asn Lys Phe Pro Thr
20                  25                  30                  35

gat tac gta ccc act gtt ttt gac aat ttc agt gca aac gtg gtg gtc  321
Asp Tyr Val Pro Thr Val Phe Asp Asn Phe Ser Ala Asn Val Val Val
                40                  45                  50

gac ggc acc acg gtg aat ttg ggt ctc tgg gat act gca ggg cag gaa  369
Asp Gly Thr Thr Val Asn Leu Gly Leu Trp Asp Thr Ala Gly Gln Glu
            55                  60                  65

gat tac aac aga ttg agg ccg cta agc tac cgt ggc gcc gat gtc ttt  417
Asp Tyr Asn Arg Leu Arg Pro Leu Ser Tyr Arg Gly Ala Asp Val Phe
        70                  75                  80

gtg ctt gcc ttc tcc cta gtg agc cga gct agc tat gag aat gtc atg  465
Val Leu Ala Phe Ser Leu Val Ser Arg Ala Ser Tyr Glu Asn Val Met
    85                  90                  95

aag aag tgg tta cca gag ctt cag cat tat gca cca ggg gtg cca att  513
Lys Lys Trp Leu Pro Glu Leu Gln His Tyr Ala Pro Gly Val Pro Ile
100                 105                 110                 115

gtg ttg gtt ggg acc aaa ttg gat ctt cgt gaa gat aaa cac tac tta  561
Val Leu Val Gly Thr Lys Leu Asp Leu Arg Glu Asp Lys His Tyr Leu
                120                 125                 130

ctt gac cat cct agc ttg gtg cct gtg act aca gca cag gga gag gaa  609
Leu Asp His Pro Ser Leu Val Pro Val Thr Thr Ala Gln Gly Glu Glu
            135                 140                 145

ctc cgc aag cac att ggc gca acg tgt tac atc gaa tgc agc tca aag  657
Leu Arg Lys His Ile Gly Ala Thr Cys Tyr Ile Glu Cys Ser Ser Lys
        150                 155                 160

aca cag cag aat gta aaa gct gtg ttt gat gct gcc atc aag gta gta  705
Thr Gln Gln Asn Val Lys Ala Val Phe Asp Ala Ala Ile Lys Val Val
    165                 170                 175

atc aag cct cca aca aag cag agg gac agg aag aag aag aaa aca cgg  753
Ile Lys Pro Pro Thr Lys Gln Arg Asp Arg Lys Lys Lys Lys Thr Arg
180                 185                 190                 195

cgc gga tgt tct ttc ttc tgc aag ggt gtc atg tcc aga aga agg cta  801
Arg Gly Cys Ser Phe Phe Cys Lys Gly Val Met Ser Arg Arg Arg Leu
                200                 205                 210

gta tgc ttc aag tgaacaagag gggttctttg atgagcagag cagaggtctg       853
Val Cys Phe Lys
            215

tgagacaaaa tgatgtcttg tgtttgataa ttgctttatc tcaaaagttc cagtttgata 913

gttgcatttc caacctatat atatcctgtt tggcaattaa ctactacctc cgtcccaaaa 973

tataacaact tttggctatg aatctgaacg cacagttatc cagattcata gctaaaaata 1033

cttatatttt gggacggagg gagtactagt agtagattac tatcctgtcc atgtaatgta 1093
```

ttaggaaggt taatagcact ccctacatct cagaatggaa gttgttttgg ttttaaaaaa 1153

aaaaaaaaaa aaaaaa 1169

<210> 45
<211> 215
<212> PRT
<213> Oryza sativa

<400> 45

Met Ala Ser Ser Ala Ser Arg Phe Ile Lys Cys Val Thr Val Gly Asp
1 5 10 15

Gly Ala Val Gly Lys Thr Cys Met Leu Ile Cys Tyr Thr Ser Asn Lys
20 25 30

Phe Pro Thr Asp Tyr Val Pro Thr Val Phe Asp Asn Phe Ser Ala Asn
35 40 45

Val Val Val Asp Gly Thr Thr Val Asn Leu Gly Leu Trp Asp Thr Ala
50 55 60

Gly Gln Glu Asp Tyr Asn Arg Leu Arg Pro Leu Ser Tyr Arg Gly Ala
65 70 75 80

Asp Val Phe Val Leu Ala Phe Ser Leu Val Ser Arg Ala Ser Tyr Glu
85 90 95

Asn Val Met Lys Lys Trp Leu Pro Glu Leu Gln His Tyr Ala Pro Gly
100 105 110

Val Pro Ile Val Leu Val Gly Thr Lys Leu Asp Leu Arg Glu Asp Lys
115 120 125

His Tyr Leu Leu Asp His Pro Ser Leu Val Pro Val Thr Thr Ala Gln
130 135 140

Gly Glu Glu Leu Arg Lys His Ile Gly Ala Thr Cys Tyr Ile Glu Cys
145 150 155 160

Ser Ser Lys Thr Gln Gln Asn Val Lys Ala Val Phe Asp Ala Ala Ile
165 170 175

Lys Val Val Ile Lys Pro Pro Thr Lys Gln Arg Asp Arg Lys Lys Lys
180 185 190

Lys Thr Arg Arg Gly Cys Ser Phe Phe Cys Lys Gly Val Met Ser Arg
195 200 205

Arg Arg Leu Val Cys Phe Lys
210 215

<210> 46
<211> 1127
<212> DNA
<213> Zea mays

<220>
<221> CDS
<222> (190)..(831)
<223> coding for RacB homologue (RacA)

<400> 46

```
gtcgacccac gcgtccgccc agaagtcacg caccaaacac caccaccaaa gaaggcgaga 60
acgtactccg tccctcccct cccctcccct ccccttcccc tcgaggctcc aggaccgtct 120
cctcgcctgc tcatccgccg ctgcttccct tctctgggct cggagaaccg gagagaagcg 180
```

```
cgcgcggcc atg gcg tcc agc gcc tct cgg ttc atc aag tgc gtc acg gtc 231
          Met Ala Ser Ser Ala Ser Arg Phe Ile Lys Cys Val Thr Val
           1               5                  10

ggc gac ggt gcc gtg ggc aag aca tgt atg ctc atc tgc tac acc agc   279
Gly Asp Gly Ala Val Gly Lys Thr Cys Met Leu Ile Cys Tyr Thr Ser
 15                  20                  25                  30

aac aag ttc ccc act gac tac ata cct acg gtg ttc gac aat ttc agt   327
Asn Lys Phe Pro Thr Asp Tyr Ile Pro Thr Val Phe Asp Asn Phe Ser
                 35                  40                  45

gca aat gta gtt gtg gat ggc acc act gtg aat ttg ggc ctt tgg gat   375
Ala Asn Val Val Val Asp Gly Thr Thr Val Asn Leu Gly Leu Trp Asp
             50                  55                  60

acc gct ggg cag gaa gat tac aac cgc ctg agg cct cta agc tac cga   423
Thr Ala Gly Gln Glu Asp Tyr Asn Arg Leu Arg Pro Leu Ser Tyr Arg
         65                  70                  75

ggt gca gat gtt ttc gtg ctt gca ttc tca ctt gtg agc cga gct agc   471
Gly Ala Asp Val Phe Val Leu Ala Phe Ser Leu Val Ser Arg Ala Ser
     80                  85                  90

tat gag aat atc atg aag aag tgg ata cca gag ctt caa cat tat gca   519
Tyr Glu Asn Ile Met Lys Lys Trp Ile Pro Glu Leu Gln His Tyr Ala
 95                 100                 105                 110

cct ggg gtg ccc gtt gtt ttg gca ggc aca aaa ttg gat ctt cgt gaa   567
Pro Gly Val Pro Val Val Leu Ala Gly Thr Lys Leu Asp Leu Arg Glu
                115                 120                 125

gac aag cac tac ttg atg gac cat cct gga ttg gtg cct gtt acc act   615
Asp Lys His Tyr Leu Met Asp His Pro Gly Leu Val Pro Val Thr Thr
            130                 135                 140

gca cag ggg gag gaa ctt cgt aga caa att ggt gct atg tat tac att   663
Ala Gln Gly Glu Glu Leu Arg Arg Gln Ile Gly Ala Met Tyr Tyr Ile
        145                 150                 155

gaa tgc agc tca aag aca cag cag aat gtc aaa gct gtg ttc gat gct   711
Glu Cys Ser Ser Lys Thr Gln Gln Asn Val Lys Ala Val Phe Asp Ala
    160                 165                 170

gcc atc aag gta gta atc cag cct cca act aaa ata aga gaa aag aag   759
Ala Ile Lys Val Val Ile Gln Pro Pro Thr Lys Ile Arg Glu Lys Lys
175                 180                 185                 190

aag aaa aaa tca cgc aaa gga tgt tct atg atg aac atc ttc ggt gga   807
Lys Lys Lys Ser Arg Lys Gly Cys Ser Met Met Asn Ile Phe Gly Gly
                195                 200                 205

aga aaa atg cta tgc ttc aag tcc tgaatggttc aagggggtct tacatggact  861
Arg Lys Met Leu Cys Phe Lys Ser
            210

gataccacga gtgtgacccc gagtttgcga agcttgaaat cttgatgtgc tcgttgcgca 921
tgtgtatatt tgcacctttg gttattaatg actagaggta ggtaattgaa actagtctgc 981
ttaagcgttc tgcactgctg gtgtggttag ctctatgagt taagcagttc gacagaggcc 1041
aaaccgacag tgagattttg ttctttcatg gaaatgtgcc aatgtcacag ctttttcgtg 1101
aaaaaaaaaa aaaaaaaaaa aaaaaa                                      1127
```

<210> 47
<211> 214
<212> PRT
<213> Zea mays

<400> 47

```
Met Ala Ser Ser Ala Ser Arg Phe Ile Lys Cys Val Thr Val Gly Asp
1               5                   10                  15

Gly Ala Val Gly Lys Thr Cys Met Leu Ile Cys Tyr Thr Ser Asn Lys
            20                  25                  30

Phe Pro Thr Asp Tyr Ile Pro Thr Val Phe Asp Asn Phe Ser Ala Asn
        35                  40                  45

Val Val Val Asp Gly Thr Thr Val Asn Leu Gly Leu Trp Asp Thr Ala
    50                  55                  60

Gly Gln Glu Asp Tyr Asn Arg Leu Arg Pro Leu Ser Tyr Arg Gly Ala
65                  70                  75                  80

Asp Val Phe Val Leu Ala Phe Ser Leu Val Ser Arg Ala Ser Tyr Glu
                85                  90                  95

Asn Ile Met Lys Lys Trp Ile Pro Glu Leu Gln His Tyr Ala Pro Gly
            100                 105                 110

Val Pro Val Val Leu Ala Gly Thr Lys Leu Asp Leu Arg Glu Asp Lys
        115                 120                 125

His Tyr Leu Met Asp His Pro Gly Leu Val Pro Val Thr Thr Ala Gln
    130                 135                 140

Gly Glu Glu Leu Arg Arg Gln Ile Gly Ala Met Tyr Tyr Ile Glu Cys
145                 150                 155                 160

Ser Ser Lys Thr Gln Gln Asn Val Lys Ala Val Phe Asp Ala Ala Ile
                165                 170                 175

Lys Val Val Ile Gln Pro Pro Thr Lys Ile Arg Glu Lys Lys Lys Lys
            180                 185                 190

Lys Ser Arg Lys Gly Cys Ser Met Met Asn Ile Phe Gly Gly Arg Lys
        195                 200                 205

Met Leu Cys Phe Lys Ser
    210
```

<210> 48
<211> 901
<212> DNA
<213> Hordeum vulgare

<220>
<221> misc_feature
<222> (1) .. (901)
<223> coding for RacB homologue (RacA)

<400> 48

```
cccgggctgc aggaattcgg cacgaggcaa gaagtcacgc accaaacacc accccccatc 60
accgccgctc cgctccccag tcccccaccc ctcctccgcc cccttcctcg agccgagctc 120
cgggggaagg aatcggagag gccggcgcgc ggcgagccat ggcgtccagc gcctcccggt 180
tcatcaagtg cgtcacggtg ggcgacggcg ccgtcggcaa gacctgcatg ctcatctgct 240
acaccagcaa caagttcccc accgactaca tacccacggt gttcgacaat ttcagcgcga 300
acgtggtggc ggacggcacc acggtgaatt tgggcctttg ggacaccgcc gggcaggagg 360
attacaaccg gctgaggcct ctaagctacc gcggcgccga cgttttcgtg cttgccttct 420
cccttgtgag ccgagctagc tatgagaata tcatgaagaa gtggataccg gagcttcagc 480
```

```
attacgcgcc cggcgtacct gttgtgctgg taggcacaaa actggatctt cgtgaagata 540
agcactattt gctggaccac cctgggatga tacccgttac cacagcacag gggGaggaac 600
ttcgtaagca agttggtgct ttatattaca tagagtgcag ctcaaagaca caacagaatg 660
tcaaagctgt gtttgatgct gctatcaagg tagtaatcca gccccccact aaacaaagag 720
aaaagaagaa aaagaaacag cgtcggggat gttctatgat gaacttcagc ggaaggaaat 780
gctatgcttc aaatcctgaa tgatgaaaga gaaggttcct tgcctngaac gattgtcacg 840
gttgcgctgc accaatttga caacacctcc aaaccggttg aatgtgctgg attgcaccgt 900
c                                                                 901
```

<210> 49
<211> 594
<212> DNA
<213> Arabidopsis thaliana

<220>
<221> CDS
<222> (1)..(591)
<223> coding for RacB homologue

<400> 49

```
atg agc gct tcg agg ttc gta aag tgc gtg acg gtt ggt gat gga gct      48
Met Ser Ala Ser Arg Phe Val Lys Cys Val Thr Val Gly Asp Gly Ala
  1               5                  10                  15

gtc gga aaa act tgt ttg ttg att tct tac aca agc aac act ttc cct      96
Val Gly Lys Thr Cys Leu Leu Ile Ser Tyr Thr Ser Asn Thr Phe Pro
            20                  25                  30

acg gat tat gtg cct acc gtt ttc gat aat ttc agt gcc aat gtt gtg      144
Thr Asp Tyr Val Pro Thr Val Phe Asp Asn Phe Ser Ala Asn Val Val
        35                  40                  45

gtt aat gga agc act gtg aat ctt gga ttg tgg gac act gca ggg caa      192
Val Asn Gly Ser Thr Val Asn Leu Gly Leu Trp Asp Thr Ala Gly Gln
    50                  55                  60

gag gat tac aat aga tta aga cca ctg agt tac cgt gga gca gat gtt      240
Glu Asp Tyr Asn Arg Leu Arg Pro Leu Ser Tyr Arg Gly Ala Asp Val
 65                  70                  75                  80

ttc att ttg gcc ttc tct ctt atc agt aaa gcc agt tat gaa aac gtc      288
Phe Ile Leu Ala Phe Ser Leu Ile Ser Lys Ala Ser Tyr Glu Asn Val
                85                  90                  95

tcc aaa aag tgg atc ccg gag ttg aaa cat tac gcg cct ggt gtc ccc      336
Ser Lys Lys Trp Ile Pro Glu Leu Lys His Tyr Ala Pro Gly Val Pro
            100                 105                 110

atc gtc ctt gtt gga aca aag ctt gat ctt cga gat gat aaa cag ttc      384
Ile Val Leu Val Gly Thr Lys Leu Asp Leu Arg Asp Asp Lys Gln Phe
        115                 120                 125

ttt atc gac cat cct ggt gct gtt ccg att act act gct cag gga gag      432
Phe Ile Asp His Pro Gly Ala Val Pro Ile Thr Thr Ala Gln Gly Glu
    130                 135                 140

gag ctg agg aag caa ata gga gca cct act tac atc gaa tgc agt tcc      480
Glu Leu Arg Lys Gln Ile Gly Ala Pro Thr Tyr Ile Glu Cys Ser Ser
145                 150                 155                 160

aaa act caa gag aat gtg aag gcg gtg ttt gac gca gcc atc cga gtg      528
Lys Thr Gln Glu Asn Val Lys Ala Val Phe Asp Ala Ala Ile Arg Val
                165                 170                 175

gtg ttg caa ccg cca aag cag aag aag aag aag agc aaa gcg cag aag      576
Val Leu Gln Pro Pro Lys Gln Lys Lys Lys Lys Ser Lys Ala Gln Lys
            180                 185                 190

gca tgc tcc att cta tga                                              594
Ala Cys Ser Ile Leu
        195
```

<210> 50
<211> 197
<212> PRT
<213> Arabidopsis thaliana

<400> 50

Met Ser Ala Ser Arg Phe Val Lys Cys Val Thr Val Gly Asp Gly Ala
1               5                   10                  15

Val Gly Lys Thr Cys Leu Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asn Gly Ser Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Ile Leu Ala Phe Ser Leu Ile Ser Lys Ala Ser Tyr Glu Asn Val
                85                  90                  95

Ser Lys Lys Trp Ile Pro Glu Leu Lys His Tyr Ala Pro Gly Val Pro
            100                 105                 110

Ile Val Leu Val Gly Thr Lys Leu Asp Leu Arg Asp Asp Lys Gln Phe
        115                 120                 125

Phe Ile Asp His Pro Gly Ala Val Pro Ile Thr Thr Ala Gln Gly Glu
    130                 135                 140

Glu Leu Arg Lys Gln Ile Gly Ala Pro Thr Tyr Ile Glu Cys Ser Ser
145                 150                 155                 160

Lys Thr Gln Glu Asn Val Lys Ala Val Phe Asp Ala Ala Ile Arg Val
                165                 170                 175

Val Leu Gln Pro Pro Lys Gln Lys Lys Lys Lys Ser Lys Ala Gln Lys
            180                 185                 190

Ala Cys Ser Ile Leu
        195

<210> 51
<211> 594
<212> DNA
<213> Arabidopsis thaliana

<220>
<221> CDS
<222> (1) .. (591)
<223> coding for RacB homologue

<400> 51

atg agc gct tcg agg ttc ata aag tgt gtc acc gtt ggc gac gga gct     48
Met Ser Ala Ser Arg Phe Ile Lys Cys Val Thr Val Gly Asp Gly Ala
1               5                   10                  15

gtt ggt aaa acc tgt ttg ctg att tct tac acc agc aac act ttt cct     96
Val Gly Lys Thr Cys Leu Leu Ile Ser Tyr Thr Ser Asn Thr Phe Pro
            20                  25                  30

```
acg gat tat gta ccg act gtt ttc gat aac ttt agc gca aat gtg gtt    144
Thr Asp Tyr Val Pro Thr Val Phe Asp Asn Phe Ser Ala Asn Val Val
         35                  40                  45

gtt aat gga gcc act gtg aat ctt ggg cta tgg gat acc gca ggg cag    192
Val Asn Gly Ala Thr Val Asn Leu Gly Leu Trp Asp Thr Ala Gly Gln
     50                  55                  60

gag gat tat aac aga tta aga cct ttg agt tac cgc ggt gct gat gtt    240
Glu Asp Tyr Asn Arg Leu Arg Pro Leu Ser Tyr Arg Gly Ala Asp Val
 65                  70                  75                  80

ttc atc tta gca ttc tct ctt atc agt aag gct agt tat gag aat gtc    288
Phe Ile Leu Ala Phe Ser Leu Ile Ser Lys Ala Ser Tyr Glu Asn Val
                 85                  90                  95

tcc aag aag tgg atc cca gag ctg aag cat tat gcc cct ggt gtc cct    336
Ser Lys Lys Trp Ile Pro Glu Leu Lys His Tyr Ala Pro Gly Val Pro
            100                 105                 110

ata gtt ctt gtt gga acc aaa cta gat ctt cgg gat gac aaa cag ttc    384
Ile Val Leu Val Gly Thr Lys Leu Asp Leu Arg Asp Asp Lys Gln Phe
        115                 120                 125

ttc att gac cac cct ggc gct gta cca att act act gct cag gga gag    432
Phe Ile Asp His Pro Gly Ala Val Pro Ile Thr Thr Ala Gln Gly Glu
    130                 135                 140

gaa ctg aag aaa cta att gga gct ccc gca tac atc gag tgc agt tca    480
Glu Leu Lys Lys Leu Ile Gly Ala Pro Ala Tyr Ile Glu Cys Ser Ser
145                 150                 155                 160

aaa aca caa gag aac gtg aaa gga gta ttt gat gca gcg atc cga gtg    528
Lys Thr Gln Glu Asn Val Lys Gly Val Phe Asp Ala Ala Ile Arg Val
                165                 170                 175

gtt ctt caa cct cca aag cag aag aaa aag aaa agc aaa gca caa aaa    576
Val Leu Gln Pro Pro Lys Gln Lys Lys Lys Lys Ser Lys Ala Gln Lys
            180                 185                 190

gcc tgc tcc att ttg taa                                            594
Ala Cys Ser Ile Leu
        195
```

<210> 52
<211> 197
<212> PRT
<213> Arabidopsis thaliana

<400> 52

Met Ser Ala Ser Arg Phe Ile Lys Cys Val Thr Val Gly Asp Gly Ala
1               5                   10                  15

Val Gly Lys Thr Cys Leu Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asn Gly Ala Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Ile Leu Ala Phe Ser Leu Ile Ser Lys Ala Ser Tyr Glu Asn Val
                85                  90                  95

Ser Lys Lys Trp Ile Pro Glu Leu Lys His Tyr Ala Pro Gly Val Pro
            100                 105                 110

Ile Val Leu Val Gly Thr Lys Leu Asp Leu Arg Asp Asp Lys Gln Phe
        115                 120                 125

Phe Ile Asp His Pro Gly Ala Val Pro Ile Thr Thr Ala Gln Gly Glu
    130                 135                 140

Glu Leu Lys Lys Leu Ile Gly Ala Pro Ala Tyr Ile Glu Cys Ser Ser
145                 150                 155                 160

Lys Thr Gln Glu Asn Val Lys Gly Val Phe Asp Ala Ala Ile Arg Val
                165                 170                 175

Val Leu Gln Pro Pro Lys Gln Lys Lys Lys Lys Ser Lys Ala Gln Lys
            180                 185                 190

Ala Cys Ser Ile Leu
        195

<210> 53
<211> 594
<212> DNA
<213> Arabidopsis thaliana

<220>
<221> CDS
<222> (1) .. (591)
<223> coding for RacB homologue

<400> 53

```
atg agc gca tca agg ttc ata aag tgc gtc acc gtt ggt gat gga gct     48
Met Ser Ala Ser Arg Phe Ile Lys Cys Val Thr Val Gly Asp Gly Ala
 1               5                  10                  15

gtt ggt aaa acc tgt ttg ctg att tct tat acc agc aac acc ttt ccc     96
Val Gly Lys Thr Cys Leu Leu Ile Ser Tyr Thr Ser Asn Thr Phe Pro
            20                  25                  30

acg gat tat gtt ccg act gtt ttc gat aac ttt agt gca aat gtg gtt     144
Thr Asp Tyr Val Pro Thr Val Phe Asp Asn Phe Ser Ala Asn Val Val
        35                  40                  45

gtt aat gga gcc acg gtg aat ctt gga ttg tgg gat act gca ggg caa     192
Val Asn Gly Ala Thr Val Asn Leu Gly Leu Trp Asp Thr Ala Gly Gln
    50                  55                  60

gag gac tat aac aga tta aga cct ttg agt tac cgt ggt gct gat gtt     240
Glu Asp Tyr Asn Arg Leu Arg Pro Leu Ser Tyr Arg Gly Ala Asp Val
65                  70                  75                  80

ttc att ctt gcc ttc tct ctc att agt aag gct agt tat gag aat gtt     288
Phe Ile Leu Ala Phe Ser Leu Ile Ser Lys Ala Ser Tyr Glu Asn Val
                85                  90                  95

tcc aag aag tgg att cct gag ttg aag cac tat gct cct ggt gtc cca     336
Ser Lys Lys Trp Ile Pro Glu Leu Lys His Tyr Ala Pro Gly Val Pro
            100                 105                 110

att gtc ctt gtt gga acc aaa cta gat ctt cga gat gac aaa cag ttt     384
Ile Val Leu Val Gly Thr Lys Leu Asp Leu Arg Asp Asp Lys Gln Phe
        115                 120                 125

ttc atc gac cat cct ggt gct gtc cct att acc act gtt cag gga gag     432
Phe Ile Asp His Pro Gly Ala Val Pro Ile Thr Thr Val Gln Gly Glu
    130                 135                 140

gag ctg aag aag cta att gga gcg cca gct tac atc gag tgc agt tca     480
Glu Leu Lys Lys Leu Ile Gly Ala Pro Ala Tyr Ile Glu Cys Ser Ser
145                 150                 155                 160

aaa tca caa gag aac gtg aag ggc gtg ttt gat gca gcg atc aga gtg     528
Lys Ser Gln Glu Asn Val Lys Gly Val Phe Asp Ala Ala Ile Arg Val
                165                 170                 175

gtc ctt caa cct cca aag cag aag aaa aag aag aac aaa gca caa aag     576
Val Leu Gln Pro Pro Lys Gln Lys Lys Lys Lys Asn Lys Ala Gln Lys
            180                 185                 190

gcc tgc tcc atc ttg taa                                             594
Ala Cys Ser Ile Leu
        195
```

<210> 54
<211> 197
<212> PRT
<213> Arabidopsis thaliana

<400> 54

Met Ser Ala Ser Arg Phe Ile Lys Cys Val Thr Val Gly Asp Gly Ala
1 5 10 15

Val Gly Lys Thr Cys Leu Leu Ile Ser Tyr Thr Ser Asn Thr Phe Pro
20 25 30

Thr Asp Tyr Val Pro Thr Val Phe Asp Asn Phe Ser Ala Asn Val Val
35 40 45

Val Asn Gly Ala Thr Val Asn Leu Gly Leu Trp Asp Thr Ala Gly Gln
50 55 60

Glu Asp Tyr Asn Arg Leu Arg Pro Leu Ser Tyr Arg Gly Ala Asp Val
65 70 75 80

Phe Ile Leu Ala Phe Ser Leu Ile Ser Lys Ala Ser Tyr Glu Asn Val
85 90 95

Ser Lys Lys Trp Ile Pro Glu Leu Lys His Tyr Ala Pro Gly Val Pro
100 105 110

Ile Val Leu Val Gly Thr Lys Leu Asp Leu Arg Asp Asp Lys Gln Phe
115 120 125

Phe Ile Asp His Pro Gly Ala Val Pro Ile Thr Thr Val Gln Gly Glu
130 135 140

Glu Leu Lys Lys Leu Ile Gly Ala Pro Ala Tyr Ile Glu Cys Ser Ser
145 150 155 160

Lys Ser Gln Glu Asn Val Lys Gly Val Phe Asp Ala Ala Ile Arg Val
165 170 175

Val Leu Gln Pro Pro Lys Gln Lys Lys Lys Lys Asn Lys Ala Gln Lys
180 185 190

Ala Cys Ser Ile Leu
195

<210> 55
<211> 591
<212> DNA
<213> Arabidopsis thaliana

<220>
<221> CDS
<222> (1)..(588)
<223> coding for RacB homologue

<400> 55

```
atg agt gct tcg agg ttt ata aag tgt gtc acc gtc ggc gat ggt gcc      48
Met Ser Ala Ser Arg Phe Ile Lys Cys Val Thr Val Gly Asp Gly Ala
 1               5                  10                  15

gtc gga aaa act tgt atg ctg att tct tac aca agc aac act ttc cct      96
Val Gly Lys Thr Cys Met Leu Ile Ser Tyr Thr Ser Asn Thr Phe Pro
            20                  25                  30

acg gac tat gtt cca act gtt ttc gac aac ttc agt gct aat gtg gtt     144
Thr Asp Tyr Val Pro Thr Val Phe Asp Asn Phe Ser Ala Asn Val Val
        35                  40                  45

gta gat ggg aac acg gtg aat ctt gga ttg tgg gat aca gct ggt caa     192
Val Asp Gly Asn Thr Val Asn Leu Gly Leu Trp Asp Thr Ala Gly Gln
    50                  55                  60

gaa gac tat aac agg tta aga ccg ttg agt tac cgt ggt gcc gat gtc     240
Glu Asp Tyr Asn Arg Leu Arg Pro Leu Ser Tyr Arg Gly Ala Asp Val
65                  70                  75                  80

ttc att ctt gca ttc tcg ctt att agc aaa gct agc tac gag aat gta     288
Phe Ile Leu Ala Phe Ser Leu Ile Ser Lys Ala Ser Tyr Glu Asn Val
                85                  90                  95

gcc aag aag tgg att cct gag ctt agg cat tat gcc cct ggt gtt cct     336
Ala Lys Lys Trp Ile Pro Glu Leu Arg His Tyr Ala Pro Gly Val Pro
            100                 105                 110

ata atc ctc gtt gga acg aaa ctc gat ctt cga gat gac aag caa ttc     384
Ile Ile Leu Val Gly Thr Lys Leu Asp Leu Arg Asp Asp Lys Gln Phe
        115                 120                 125

ttc ata gac cat cct ggt gca gtg cct att act aca aac cag gga gag     432
Phe Ile Asp His Pro Gly Ala Val Pro Ile Thr Thr Asn Gln Gly Glu
    130                 135                 140

gaa cta aag aaa ctg ata gga tca cca atc tac att gaa tgt agt tca     480
Glu Leu Lys Lys Leu Ile Gly Ser Pro Ile Tyr Ile Glu Cys Ser Ser
145                 150                 155                 160

aag act cag cag aat gtg aaa gca gtc ttt gac gca gcc ata aaa gtg     528
Lys Thr Gln Gln Asn Val Lys Ala Val Phe Asp Ala Ala Ile Lys Val
                165                 170                 175

gtg ctt cag cca ccg aaa cag aag aag aag aaa aag aac aag aac cgc     576
Val Leu Gln Pro Pro Lys Gln Lys Lys Lys Lys Lys Asn Lys Asn Arg
            180                 185                 190

tgc gtg ttc ttg tga                                                 591
Cys Val Phe Leu
        195
```

<210> 56
<211> 196
<212> PRT
<213> Arabidopsis thaliana

<400> 56

Met Ser Ala Ser Arg Phe Ile Lys Cys Val Thr Val Gly Asp Gly Ala
1 5 10 15

Val Gly Lys Thr Cys Met Leu Ile Ser Tyr Thr Ser Asn Thr Phe Pro
20 25 30

Thr Asp Tyr Val Pro Thr Val Phe Asp Asn Phe Ser Ala Asn Val Val
35 40 45

Val Asp Gly Asn Thr Val Asn Leu Gly Leu Trp Asp Thr Ala Gly Gln
50 55 60

Glu Asp Tyr Asn Arg Leu Arg Pro Leu Ser Tyr Arg Gly Ala Asp Val
65 70 75 80

Phe Ile Leu Ala Phe Ser Leu Ile Ser Lys Ala Ser Tyr Glu Asn Val
85 90 95

Ala Lys Lys Trp Ile Pro Glu Leu Arg His Tyr Ala Pro Gly Val Pro
100 105 110

Ile Ile Leu Val Gly Thr Lys Leu Asp Leu Arg Asp Asp Lys Gln Phe
115 120 125

Phe Ile Asp His Pro Gly Ala Val Pro Ile Thr Thr Asn Gln Gly Glu
130 135 140

Glu Leu Lys Lys Leu Ile Gly Ser Pro Ile Tyr Ile Glu Cys Ser Ser
145 150 155 160

Lys Thr Gln Gln Asn Val Lys Ala Val Phe Asp Ala Ala Ile Lys Val
165 170 175

Val Leu Gln Pro Pro Lys Gln Lys Lys Lys Lys Lys Asn Lys Asn Arg
180 185 190

Cys Val Phe Leu
195

<210> 57
<211> 597
<212> DNA
<213> Arabidopsis thaliana

<220>
<221> CDS
<222> (1) .. (594)
<223> coding for RacB homologue

<400> 57

```
atg agt gct tca agg ttt atc aag tgt gtc act gtc ggc gac ggt gct    48
Met Ser Ala Ser Arg Phe Ile Lys Cys Val Thr Val Gly Asp Gly Ala
 1               5                  10                  15

gtt gga aag act tgt ctt ctc atc tcc tac act agc aac act ttc ccc    96
Val Gly Lys Thr Cys Leu Leu Ile Ser Tyr Thr Ser Asn Thr Phe Pro
            20                  25                  30

acg gat tat gtg cca act gtg ttc gat aat ttc agt gcc aat gtg att    144
Thr Asp Tyr Val Pro Thr Val Phe Asp Asn Phe Ser Ala Asn Val Ile
        35                  40                  45

gtt gat ggc aac act atc aac ttg gga ttg tgg gat act gca ggg caa    192
Val Asp Gly Asn Thr Ile Asn Leu Gly Leu Trp Asp Thr Ala Gly Gln
    50                  55                  60

gag gac tac aat aga cta aga cct ttg agc tat cgc ggt gca gat gtc    240
Glu Asp Tyr Asn Arg Leu Arg Pro Leu Ser Tyr Arg Gly Ala Asp Val
65                  70                  75                  80

ttc tta ctt gca ttc tca ctt gtc agc aaa gct agc tat gaa aat gtt    288
Phe Leu Leu Ala Phe Ser Leu Val Ser Lys Ala Ser Tyr Glu Asn Val
                85                  90                  95

tct aaa aag tgg gtt cct gaa ctg aga cat tat gct cct ggt gtt ccc    336
Ser Lys Lys Trp Val Pro Glu Leu Arg His Tyr Ala Pro Gly Val Pro
            100                 105                 110

atc atc ctc gtt gga aca aag ctt gat ctt cga gat gat aag caa ttc    384
Ile Ile Leu Val Gly Thr Lys Leu Asp Leu Arg Asp Asp Lys Gln Phe
        115                 120                 125

ttt gcc gag cac cct ggt gct gtg cct atc tct acc gct cag ggt gaa    432
Phe Ala Glu His Pro Gly Ala Val Pro Ile Ser Thr Ala Gln Gly Glu
    130                 135                 140

gaa cta aag aag ctg att ggg gcg cct gct tat atc gaa tgc agt gca    480
Glu Leu Lys Lys Leu Ile Gly Ala Pro Ala Tyr Ile Glu Cys Ser Ala
145                 150                 155                 160

aaa act caa cag aat gtg aaa gca gtg ttt gat gcg gct atc aag gtc    528
Lys Thr Gln Gln Asn Val Lys Ala Val Phe Asp Ala Ala Ile Lys Val
                165                 170                 175

gtt ctc cag cca cca aaa aac aag aag aag aag aag aga aaa tct cag    576
Val Leu Gln Pro Pro Lys Asn Lys Lys Lys Lys Lys Arg Lys Ser Gln
            180                 185                 190

aaa ggt tgt tct ata ctc tga                                        597
Lys Gly Cys Ser Ile Leu
        195
```

<210> 58
<211> 198
<212> PRT
<213> Arabidopsis thaliana

<400> 58

Met Ser Ala Ser Arg Phe Ile Lys Cys Val Thr Val Gly Asp Gly Ala
1               5                   10                  15

Val Gly Lys Thr Cys Leu Leu Ile Ser Tyr Thr Ser Asn Thr Phe Pro
            20                  25                  30

Thr Asp Tyr Val Pro Thr Val Phe Asp Asn Phe Ser Ala Asn Val Ile
        35                  40                  45

Val Asp Gly Asn Thr Ile Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Leu Leu Ala Phe Ser Leu Val Ser Lys Ala Ser Tyr Glu Asn Val
                85                  90                  95

Ser Lys Lys Trp Val Pro Glu Leu Arg His Tyr Ala Pro Gly Val Pro
            100                 105                 110

Ile Ile Leu Val Gly Thr Lys Leu Asp Leu Arg Asp Asp Lys Gln Phe
        115                 120                 125

Phe Ala Glu His Pro Gly Ala Val Pro Ile Ser Thr Ala Gln Gly Glu
    130                 135                 140

Glu Leu Lys Lys Leu Ile Gly Ala Pro Ala Tyr Ile Glu Cys Ser Ala
145                 150                 155                 160

Lys Thr Gln Gln Asn Val Lys Ala Val Phe Asp Ala Ala Ile Lys Val
                165                 170                 175

Val Leu Gln Pro Pro Lys Asn Lys Lys Lys Lys Lys Arg Lys Ser Gln
            180                 185                 190

Lys Gly Cys Ser Ile Leu
        195

<210> 59
<211> 588
<212> DNA
<213> Arabidopsis thaliana

<220>
<221> CDS
<222> (1) .. (585)
<223> coding for RacB homologue

<400> 59

```
atg gcg tca agg ttt ata aag tgt gtg acc gtc gga gat ggt gcc gtc      48
Met Ala Ser Arg Phe Ile Lys Cys Val Thr Val Gly Asp Gly Ala Val
 1               5                  10                  15

gga aaa act tgc atg ctc att tct tac act agc aat act ttt cct act      96
Gly Lys Thr Cys Met Leu Ile Ser Tyr Thr Ser Asn Thr Phe Pro Thr
            20                  25                  30

gat tat gtg cca act gtt ttc gac aac ttc agt gct aat gtg gtt gtt     144
Asp Tyr Val Pro Thr Val Phe Asp Asn Phe Ser Ala Asn Val Val Val
        35                  40                  45

gat ggc aac act gtc aat ctt gga ttg tgg gat act gct ggt caa gag     192
Asp Gly Asn Thr Val Asn Leu Gly Leu Trp Asp Thr Ala Gly Gln Glu
    50                  55                  60

gac tac aac agg tta cga cct ttg agt tac cgt ggt gct gat gtt ttc     240
Asp Tyr Asn Arg Leu Arg Pro Leu Ser Tyr Arg Gly Ala Asp Val Phe
65                  70                  75                  80

att ctt gct ttc tct ctt att agc aag gct agc tat gag aat ata gcc     288
Ile Leu Ala Phe Ser Leu Ile Ser Lys Ala Ser Tyr Glu Asn Ile Ala
                85                  90                  95

aag aag tgg att cct gag ctc agg cat tat gct cct ggt gtt ccc att     336
Lys Lys Trp Ile Pro Glu Leu Arg His Tyr Ala Pro Gly Val Pro Ile
            100                 105                 110

atc ctt gtt ggg aca aaa ctc gat ctt cga gat gac aag caa ttc ttt     384
Ile Leu Val Gly Thr Lys Leu Asp Leu Arg Asp Asp Lys Gln Phe Phe
        115                 120                 125

ata gat cat cct ggt gct gtg cca att act aca aac cag gga gag gaa     432
Ile Asp His Pro Gly Ala Val Pro Ile Thr Thr Asn Gln Gly Glu Glu
    130                 135                 140

ctg aag aaa ctg att gga tct gct gtc tac att gaa tgt agt tca aag     480
Leu Lys Lys Leu Ile Gly Ser Ala Val Tyr Ile Glu Cys Ser Ser Lys
145                 150                 155                 160

aca cag cag aac gtg aag gca gtg ttt gat gca gct ata aaa gtg gtg     528
Thr Gln Gln Asn Val Lys Ala Val Phe Asp Ala Ala Ile Lys Val Val
                165                 170                 175

ctt cag cca cca aag cag aag aag aag aaa aag aat aag aac cgt tgc     576
Leu Gln Pro Pro Lys Gln Lys Lys Lys Lys Lys Asn Lys Asn Arg Cys
            180                 185                 190

gcg ttc ttg tga                                                      588
Ala Phe Leu
        195
```

<210> 60
<211> 195
<212> PRT
<213> Arabidopsis thaliana

<400> 60

Met Ala Ser Arg Phe Ile Lys Cys Val Thr Val Gly Asp Gly Ala Val
1               5                   10                  15

Gly Lys Thr Cys Met Leu Ile Ser Tyr Thr Ser Asn Thr Phe Pro Thr
            20                  25                  30

Asp Tyr Val Pro Thr Val Phe Asp Asn Phe Ser Ala Asn Val Val Val
        35                  40                  45

Asp Gly Asn Thr Val Asn Leu Gly Leu Trp Asp Thr Ala Gly Gln Glu
    50                  55                  60

Asp Tyr Asn Arg Leu Arg Pro Leu Ser Tyr Arg Gly Ala Asp Val Phe
65                  70                  75                  80

Ile Leu Ala Phe Ser Leu Ile Ser Lys Ala Ser Tyr Glu Asn Ile Ala
                85                  90                  95

Lys Lys Trp Ile Pro Glu Leu Arg His Tyr Ala Pro Gly Val Pro Ile
            100                 105                 110

Ile Leu Val Gly Thr Lys Leu Asp Leu Arg Asp Asp Lys Gln Phe Phe
        115                 120                 125

Ile Asp His Pro Gly Ala Val Pro Ile Thr Thr Asn Gln Gly Glu Glu
    130                 135                 140

Leu Lys Lys Leu Ile Gly Ser Ala Val Tyr Ile Glu Cys Ser Ser Lys
145                 150                 155                 160

Thr Gln Gln Asn Val Lys Ala Val Phe Asp Ala Ala Ile Lys Val Val
                165                 170                 175

Leu Gln Pro Pro Lys Gln Lys Lys Lys Lys Lys Asn Lys Asn Arg Cys
            180                 185                 190

Ala Phe Leu
        195

<210> 61
<211> 606
<212> DNA
<213> Arabidopsis thaliana

<220>
<221> CDS
<222> (1) .. (603)
<223> coding for RacB homologue

<400> 61

atg agc aca gca aga ttc att aag tgt gtg act gtc gga gat gga gca 48
Met Ser Thr Ala Arg Phe Ile Lys Cys Val Thr Val Gly Asp Gly Ala
1               5                   10                  15

gtg gga aag act tgt atg ctc att tca tat acc agc aat acg ttt cct 96
Val Gly Lys Thr Cys Met Leu Ile Ser Tyr Thr Ser Asn Thr Phe Pro
            20                  25                  30

acg gat tat gtt cca aca gtt ttc gac aac ttc agc gca aat gtg gtg 144
Thr Asp Tyr Val Pro Thr Val Phe Asp Asn Phe Ser Ala Asn Val Val
        35                  40                  45

```
gtc gac ggg agt acc gtg aac ctt ggc ctg tgg gat act gcc ggt cag      192
Val Asp Gly Ser Thr Val Asn Leu Gly Leu Trp Asp Thr Ala Gly Gln
     50                  55                  60

gaa gat tat aat agg ctt agg cct ttg agt tac aga gga gca gat gtc      240
Glu Asp Tyr Asn Arg Leu Arg Pro Leu Ser Tyr Arg Gly Ala Asp Val
 65                  70                  75                  80

ttc tta tta gca ttt tcc ctt ata agc aag gcc agt tac gag aat att      288
Phe Leu Leu Ala Phe Ser Leu Ile Ser Lys Ala Ser Tyr Glu Asn Ile
                 85                  90                  95

cac aaa aag tgg ctt ccg gag ctg aaa cat tat gct cct ggc atc ccc      336
His Lys Lys Trp Leu Pro Glu Leu Lys His Tyr Ala Pro Gly Ile Pro
            100                 105                 110

att gtg ctc gtc gga aca aaa tta gat ttg agg gat gac aag cag ttc      384
Ile Val Leu Val Gly Thr Lys Leu Asp Leu Arg Asp Asp Lys Gln Phe
        115                 120                 125

ttg aag gat cat cca gga gca gct tct ata aca act gct cag gga gaa      432
Leu Lys Asp His Pro Gly Ala Ala Ser Ile Thr Thr Ala Gln Gly Glu
    130                 135                 140

gaa tta agg aaa atg att gga gct gtt agg tac tta gag tgc agc tcc      480
Glu Leu Arg Lys Met Ile Gly Ala Val Arg Tyr Leu Glu Cys Ser Ser
145                 150                 155                 160

aaa acc caa cag aat gtg aag gca gtg ttt gat aca gcg ata agg gta      528
Lys Thr Gln Gln Asn Val Lys Ala Val Phe Asp Thr Ala Ile Arg Val
                165                 170                 175

gct ttg agg cca cca aag gca aag aaa aag ata aaa cca ttg aag act      576
Ala Leu Arg Pro Pro Lys Ala Lys Lys Lys Ile Lys Pro Leu Lys Thr
            180                 185                 190

aag aga tca aga ata tgc ttt ttc cta taa                              606
Lys Arg Ser Arg Ile Cys Phe Phe Leu
        195                 200
```

<210> 62
<211> 201
<212> PRT
<213> Arabidopsis thaliana

<400> 62

Met Ser Thr Ala Arg Phe Ile Lys Cys Val Thr Val Gly Asp Gly Ala
1               5                   10                  15

Val Gly Lys Thr Cys Met Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asp Gly Ser Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Leu Leu Ala Phe Ser Leu Ile Ser Lys Ala Ser Tyr Glu Asn Ile
                85                  90                  95

His Lys Lys Trp Leu Pro Glu Leu Lys His Tyr Ala Pro Gly Ile Pro
            100                 105                 110

Ile Val Leu Val Gly Thr Lys Leu Asp Leu Arg Asp Asp Lys Gln Phe
        115                 120                 125

Leu Lys Asp His Pro Gly Ala Ala Ser Ile Thr Thr Ala Gln Gly Glu
    130                 135                 140

Glu Leu Arg Lys Met Ile Gly Ala Val Arg Tyr Leu Glu Cys Ser Ser
145                 150                 155                 160

Lys Thr Gln Gln Asn Val Lys Ala Val Phe Asp Thr Ala Ile Arg Val
                165                 170                 175

Ala Leu Arg Pro Pro Lys Ala Lys Lys Lys Ile Lys Pro Leu Lys Thr
            180                 185                 190

Lys Arg Ser Arg Ile Cys Phe Phe Leu
        195                 200

<210> 63
<211> 606
<212> DNA
<213> Arabidopsis thaliana

<220>
<221> CDS
<222> (1) .. (603)
<223> coding for RacB homologue

<400> 63

```
atg gct tcg agt gct tca aaa ttc atc aaa tgt gtg act gtt gga gat      48
Met Ala Ser Ser Ala Ser Lys Phe Ile Lys Cys Val Thr Val Gly Asp
1               5                   10                  15

ggt gcc gtt gga aaa act tgt atg ctc atc tgc tac act agc aac aaa      96
Gly Ala Val Gly Lys Thr Cys Met Leu Ile Cys Tyr Thr Ser Asn Lys
            20                  25                  30

ttc cct act gac tac ata cca aca gtt ttt gac aac ttt agt gtt aat      144
Phe Pro Thr Asp Tyr Ile Pro Thr Val Phe Asp Asn Phe Ser Val Asn
        35                  40                  45

gtt gtg gtt gaa ggc atc act gtg aac tta ggc ctt tgg gac act gcc      192
Val Val Val Glu Gly Ile Thr Val Asn Leu Gly Leu Trp Asp Thr Ala
    50                  55                  60

ggg caa gaa gac tat aac aga cta agg cct tta agt tac aga gga gca      240
Gly Gln Glu Asp Tyr Asn Arg Leu Arg Pro Leu Ser Tyr Arg Gly Ala
65                  70                  75                  80

gat gtt ttt gtg ttg gct ttc tca ttg atc agc cga gct agc tat gag      288
Asp Val Phe Val Leu Ala Phe Ser Leu Ile Ser Arg Ala Ser Tyr Glu
                85                  90                  95

aat gtg ttt aaa aag tgg atc cct gaa ctc caa cac ttt gca cca gga      336
Asn Val Phe Lys Lys Trp Ile Pro Glu Leu Gln His Phe Ala Pro Gly
            100                 105                 110

gtc ccc att gtg ctt gtt ggt acc aaa atg gat ctt cgt gaa gat aga      384
Val Pro Ile Val Leu Val Gly Thr Lys Met Asp Leu Arg Glu Asp Arg
        115                 120                 125

cat tac ttg tct gat cat cct gga ctg tcc ccg gta act aca tca cag      432
His Tyr Leu Ser Asp His Pro Gly Leu Ser Pro Val Thr Thr Ser Gln
    130                 135                 140

gga gag gaa ctc cgc aag cat atc gga gcg act tat tac att gaa tgt      480
Gly Glu Glu Leu Arg Lys His Ile Gly Ala Thr Tyr Tyr Ile Glu Cys
145                 150                 155                 160

agc tca aaa act caa cag aat gtg aaa gcc gta ttt gat gct gct att      528
Ser Ser Lys Thr Gln Gln Asn Val Lys Ala Val Phe Asp Ala Ala Ile
                165                 170                 175

aaa gta gta att aaa cca gca gtg aaa caa aag gag aag aag aag aag      576
Lys Val Val Ile Lys Pro Ala Val Lys Gln Lys Glu Lys Lys Lys Lys
            180                 185                 190

cag aag cct cgc agc gga tgt ctc tcg taa                              606
Gln Lys Pro Arg Ser Gly Cys Leu Ser
        195                 200
```

<210> 64
<211> 201
<212> PRT
<213> Arabidopsis thaliana

<400> 64

Met Ala Ser Ser Ala Ser Lys Phe Ile Lys Cys Val Thr Val Gly Asp
1               5                   10                  15

Gly Ala Val Gly Lys Thr Cys Met Leu Ile Cys Tyr Thr Ser Asn Lys
            20                  25                  30

Phe Pro Thr Asp Tyr Ile Pro Thr Val Phe Asp Asn Phe Ser Val Asn
        35                  40                  45

Val Val Val Glu Gly Ile Thr Val Asn Leu Gly Leu Trp Asp Thr Ala
    50                  55                  60

Gly Gln Glu Asp Tyr Asn Arg Leu Arg Pro Leu Ser Tyr Arg Gly Ala
65                  70                  75                  80

Asp Val Phe Val Leu Ala Phe Ser Leu Ile Ser Arg Ala Ser Tyr Glu
                85                  90                  95

Asn Val Phe Lys Lys Trp Ile Pro Glu Leu Gln His Phe Ala Pro Gly
            100                 105                 110

Val Pro Ile Val Leu Val Gly Thr Lys Met Asp Leu Arg Glu Asp Arg
        115                 120                 125

His Tyr Leu Ser Asp His Pro Gly Leu Ser Pro Val Thr Thr Ser Gln
    130                 135                 140

Gly Glu Glu Leu Arg Lys His Ile Gly Ala Thr Tyr Tyr Ile Glu Cys
145                 150                 155                 160

Ser Ser Lys Thr Gln Gln Asn Val Lys Ala Val Phe Asp Ala Ala Ile
                165                 170                 175

Lys Val Val Ile Lys Pro Ala Val Lys Gln Lys Glu Lys Lys Lys Lys
            180                 185                 190

Gln Lys Pro Arg Ser Gly Cys Leu Ser
        195                 200

<210> 65
<211> 648
<212> DNA
<213> Arabidopsis thaliana

<220>
<221> CDS
<222> (1) .. (645)
<223> coding for RacB homologue

<400> 65

```
atg gct tca agt gct tca aag ttc atc aag tgt gtg act gtt ggt gat      48
Met Ala Ser Ser Ala Ser Lys Phe Ile Lys Cys Val Thr Val Gly Asp
 1               5                  10                  15

ggt gct gtt ggt aaa acc tgt atg ctc atc tgc tac acc agc aat aaa      96
Gly Ala Val Gly Lys Thr Cys Met Leu Ile Cys Tyr Thr Ser Asn Lys
            20                  25                  30

ttc ccc act gac tac ata cca aca gtt ttt gac aac ttt agt gca aat     144
Phe Pro Thr Asp Tyr Ile Pro Thr Val Phe Asp Asn Phe Ser Ala Asn
        35                  40                  45

gtt gtt gtt gaa ggc acc act gtc aat ttg ggg ctt tgg gac act gct     192
Val Val Val Glu Gly Thr Thr Val Asn Leu Gly Leu Trp Asp Thr Ala
    50                  55                  60

ggg caa gaa gac tat aac aga tta agg cct tta agt tac agg gga gca     240
Gly Gln Glu Asp Tyr Asn Arg Leu Arg Pro Leu Ser Tyr Arg Gly Ala
65                  70                  75                  80

gat gtt ttc gtc ttg tct ttc tca tta gtc agc cga gct agc tac gag     288
Asp Val Phe Val Leu Ser Phe Ser Leu Val Ser Arg Ala Ser Tyr Glu
                85                  90                  95

aat gtt ttt aaa aag tgg atc cct gaa ctc caa cac ttt gct cca gga     336
Asn Val Phe Lys Lys Trp Ile Pro Glu Leu Gln His Phe Ala Pro Gly
            100                 105                 110

gtt ccc ctt gtc ctt gtt ggt acc aaa tta gat ctt cgt gaa gat aag     384
Val Pro Leu Val Leu Val Gly Thr Lys Leu Asp Leu Arg Glu Asp Lys
        115                 120                 125

cat tat ttg gct gat cat cct gga cta tcc cct gta act act gca cag     432
His Tyr Leu Ala Asp His Pro Gly Leu Ser Pro Val Thr Thr Ala Gln
    130                 135                 140

gga gag gag ttg cgt aag cta att ggt gcg acg tat tac att gag tgt     480
Gly Glu Glu Leu Arg Lys Leu Ile Gly Ala Thr Tyr Tyr Ile Glu Cys
145                 150                 155                 160

agt tca aaa act caa cag aat gtg aaa gca gtt ttt gat tct gcg ata     528
Ser Ser Lys Thr Gln Gln Asn Val Lys Ala Val Phe Asp Ser Ala Ile
                165                 170                 175

aag gaa gtg atc aaa cct ctg gtt aaa caa aag gag aag act aag aag     576
Lys Glu Val Ile Lys Pro Leu Val Lys Gln Lys Glu Lys Thr Lys Lys
            180                 185                 190

aag aag aag caa aag tcg aat cac ggc tgt tta tca aat gtt ctg tgt     624
Lys Lys Lys Gln Lys Ser Asn His Gly Cys Leu Ser Asn Val Leu Cys
        195                 200                 205

ggg agg ata gtg act cgg cat tga                                     648
Gly Arg Ile Val Thr Arg His
    210                 215
```

<210> 66
<211> 215
<212> PRT
<213> Arabidopsis thaliana

<400> 66

```
Met Ala Ser Ser Ala Ser Lys Phe Ile Lys Cys Val Thr Val Gly Asp
1               5                   10                  15

Gly Ala Val Gly Lys Thr Cys Met Leu Ile Cys Tyr Thr Ser Asn Lys
            20                  25                  30

Phe Pro Thr Asp Tyr Ile Pro Thr Val Phe Asp Asn Phe Ser Ala Asn
        35                  40                  45

Val Val Val Glu Gly Thr Thr Val Asn Leu Gly Leu Trp Asp Thr Ala
    50                  55                  60

Gly Gln Glu Asp Tyr Asn Arg Leu Arg Pro Leu Ser Tyr Arg Gly Ala
65                  70                  75                  80

Asp Val Phe Val Leu Ser Phe Ser Leu Val Ser Arg Ala Ser Tyr Glu
                85                  90                  95

Asn Val Phe Lys Lys Trp Ile Pro Glu Leu Gln His Phe Ala Pro Gly
            100                 105                 110

Val Pro Leu Val Leu Val Gly Thr Lys Leu Asp Leu Arg Glu Asp Lys
        115                 120                 125

His Tyr Leu Ala Asp His Pro Gly Leu Ser Pro Val Thr Thr Ala Gln
    130                 135                 140

Gly Glu Glu Leu Arg Lys Leu Ile Gly Ala Thr Tyr Tyr Ile Glu Cys
145                 150                 155                 160

Ser Ser Lys Thr Gln Gln Asn Val Lys Ala Val Phe Asp Ser Ala Ile
                165                 170                 175

Lys Glu Val Ile Lys Pro Leu Val Lys Gln Lys Glu Lys Thr Lys Lys
            180                 185                 190

Lys Lys Lys Gln Lys Ser Asn His Gly Cys Leu Ser Asn Val Leu Cys
        195                 200                 205

Gly Arg Ile Val Thr Arg His
    210                 215
```

<210> 67
<211> 630
<212> DNA
<213> Arabidopsis thaliana

<220>
<221> CDS
<222> (1) .. (627)
<223> coding for RacB homologue

<400> 67

```
atg agt gct tcg aag ttc ata aaa tgt gtt act gtt gga gat ggg gct     48
Met Ser Ala Ser Lys Phe Ile Lys Cys Val Thr Val Gly Asp Gly Ala
 1               5                  10                  15

gtt ggg aag aca tgt atg ctt atc tgt tac act agc aac aag ttt cct     96
Val Gly Lys Thr Cys Met Leu Ile Cys Tyr Thr Ser Asn Lys Phe Pro
            20                  25                  30

act gat tat ata ccg act gtg ttc gac aat ttc agt gcc aat gta gct    144
Thr Asp Tyr Ile Pro Thr Val Phe Asp Asn Phe Ser Ala Asn Val Ala
        35                  40                  45

gtg gat gga caa atc gtt aat tta ggg cta tgg gac act gcc ggt caa    192
Val Asp Gly Gln Ile Val Asn Leu Gly Leu Trp Asp Thr Ala Gly Gln
    50                  55                  60

gaa gat tac agt agg tta aga cca ttg agt tat aga gga gct gat atc    240
Glu Asp Tyr Ser Arg Leu Arg Pro Leu Ser Tyr Arg Gly Ala Asp Ile
65                  70                  75                  80

ttc gtc tta gcc ttt tcg ctt att agc aag gcg agt tac gaa aat gta    288
Phe Val Leu Ala Phe Ser Leu Ile Ser Lys Ala Ser Tyr Glu Asn Val
                85                  90                  95

ctc aag aag tgg atg cct gaa ctt cgt cgg ttt gcg cca aat gtt ccc    336
Leu Lys Lys Trp Met Pro Glu Leu Arg Arg Phe Ala Pro Asn Val Pro
            100                 105                 110

ata gtt ctt gtt ggt aca aag cta gat ctc cgg gat gac aag gga tac    384
Ile Val Leu Val Gly Thr Lys Leu Asp Leu Arg Asp Asp Lys Gly Tyr
        115                 120                 125

ctc gcg gat cac acc aat gtc att acc tct act cag gga gag gaa ttg    432
Leu Ala Asp His Thr Asn Val Ile Thr Ser Thr Gln Gly Glu Glu Leu
    130                 135                 140

agg aag caa att ggt gca gct gct tat att gag tgt agt tcc aag act    480
Arg Lys Gln Ile Gly Ala Ala Ala Tyr Ile Glu Cys Ser Ser Lys Thr
145                 150                 155                 160

caa caa aat gtg aaa gca gtg ttt gat aca gcg atc aag gtg gtt ctt    528
Gln Gln Asn Val Lys Ala Val Phe Asp Thr Ala Ile Lys Val Val Leu
                165                 170                 175

cag cct cca agg agg aaa gag gtc ccg agg agg agg aag aat cat aga    576
Gln Pro Pro Arg Arg Lys Glu Val Pro Arg Arg Arg Lys Asn His Arg
            180                 185                 190

aga tcc ggt tgc tcc att gcg agt att gtc tgt gga ggt tgc acc gct    624
Arg Ser Gly Cys Ser Ile Ala Ser Ile Val Cys Gly Gly Cys Thr Ala
        195                 200                 205

gct taa                                                            630
Ala
```

<210> 68
<211> 209
<212> PRT
<213> Arabidopsis thaliana

<400> 68

```
Met Ser Ala Ser Lys Phe Ile Lys Cys Val Thr Val Gly Asp Gly Ala
1               5                   10                  15

Val Gly Lys Thr Cys Met Leu Ile Cys Tyr Thr Ser Asn Lys Phe Pro
            20                  25                  30

Thr Asp Tyr Ile Pro Thr Val Phe Asp Asn Phe Ser Ala Asn Val Ala
        35                  40                  45

Val Asp Gly Gln Ile Val Asn Leu Gly Leu Trp Asp Thr Ala Gly Gln
    50                  55                  60

Glu Asp Tyr Ser Arg Leu Arg Pro Leu Ser Tyr Arg Gly Ala Asp Ile
65                  70                  75                  80

Phe Val Leu Ala Phe Ser Leu Ile Ser Lys Ala Ser Tyr Glu Asn Val
                85                  90                  95

Leu Lys Lys Trp Met Pro Glu Leu Arg Arg Phe Ala Pro Asn Val Pro
            100                 105                 110

Ile Val Leu Val Gly Thr Lys Leu Asp Leu Arg Asp Asp Lys Gly Tyr
        115                 120                 125

Leu Ala Asp His Thr Asn Val Ile Thr Ser Thr Gln Gly Glu Glu Leu
    130                 135                 140

Arg Lys Gln Ile Gly Ala Ala Ala Tyr Ile Glu Cys Ser Ser Lys Thr
145                 150                 155                 160

Gln Gln Asn Val Lys Ala Val Phe Asp Thr Ala Ile Lys Val Val Leu
                165                 170                 175

Gln Pro Pro Arg Arg Lys Glu Val Pro Arg Arg Arg Lys Asn His Arg
            180                 185                 190

Arg Ser Gly Cys Ser Ile Ala Ser Ile Val Cys Gly Gly Cys Thr Ala
        195                 200                 205

Ala
```

<210> 69
<211> 600
<212> DNA
<213> Arabidopsis thaliana

<220>
<221> CDS
<222> (1)..(597)
<223> coding for RacB homologue

<400> 69

```
atg gct gca aca tca aca tca tca gca aca gct aca acg ttt ata aag     48
Met Ala Ala Thr Ser Thr Ser Ser Ala Thr Ala Thr Thr Phe Ile Lys
 1               5                  10                  15

tgt gtc act gtt ggc gat gga gct ctt ttg gtg act gtt gag atc ttg     96
Cys Val Thr Val Gly Asp Gly Ala Leu Leu Val Thr Val Glu Ile Leu
            20                  25                  30

tta tta cag gat tat gtt cca aca gtg ttc gac aat ttc aat gct aat    144
Leu Leu Gln Asp Tyr Val Pro Thr Val Phe Asp Asn Phe Asn Ala Asn
        35                  40                  45

gtt tta gtc gat ggt aaa act gtc aat ctg ggt ctc tgg gat act gct    192
Val Leu Val Asp Gly Lys Thr Val Asn Leu Gly Leu Trp Asp Thr Ala
    50                  55                  60

ggt caa gaa gac tac aat agg gtt aga cca ttg agt tac aga gga gca    240
Gly Gln Glu Asp Tyr Asn Arg Val Arg Pro Leu Ser Tyr Arg Gly Ala
65                  70                  75                  80

gat gtt ttc att ctt gcc ttc tca ctt att agc agg cct agc ttt gag    288
Asp Val Phe Ile Leu Ala Phe Ser Leu Ile Ser Arg Pro Ser Phe Glu
                85                  90                  95

aac att gct aaa aag tgg gta ccc gag ctg aga cat tat gcc ccg act    336
Asn Ile Ala Lys Lys Trp Val Pro Glu Leu Arg His Tyr Ala Pro Thr
            100                 105                 110

gtg cct att gtt ctt gtg gga acc aaa tca gat cta aga gac aac atg    384
Val Pro Ile Val Leu Val Gly Thr Lys Ser Asp Leu Arg Asp Asn Met
        115                 120                 125

cag ttc cca aag aat tat cca ggt gct tgc aca atc ttc cca gaa cag    432
Gln Phe Pro Lys Asn Tyr Pro Gly Ala Cys Thr Ile Phe Pro Glu Gln
    130                 135                 140

ggt caa gaa cta aga aag gaa ata gga gca tta gca tac ata gag tgc    480
Gly Gln Glu Leu Arg Lys Glu Ile Gly Ala Leu Ala Tyr Ile Glu Cys
145                 150                 155                 160

agc tca aaa gca caa atg aac gta aaa gcc gtg ttt gat gaa gcg atc    528
Ser Ser Lys Ala Gln Met Asn Val Lys Ala Val Phe Asp Glu Ala Ile
                165                 170                 175

aaa gta gtt tta cat cct cct tca aag act aag aag cga aag aga aag    576
Lys Val Val Leu His Pro Pro Ser Lys Thr Lys Lys Arg Lys Arg Lys
            180                 185                 190

atc ggt tta tgc cat gtt ctt tga                                    600
Ile Gly Leu Cys His Val Leu
        195
```

<210> 70
<211> 199
<212> PRT
<213> Arabidopsis thaliana

<400> 70

```
Met Ala Ala Thr Ser Thr Ser Ser Ala Thr Ala Thr Thr Phe Ile Lys
1               5                   10                  15

Cys Val Thr Val Gly Asp Gly Ala Leu Leu Val Thr Val Glu Ile Leu
            20                  25                  30

Leu Leu Gln Asp Tyr Val Pro Thr Val Phe Asp Asn Phe Asn Ala Asn
        35                  40                  45

Val Leu Val Asp Gly Lys Thr Val Asn Leu Gly Leu Trp Asp Thr Ala
    50                  55                  60

Gly Gln Glu Asp Tyr Asn Arg Val Arg Pro Leu Ser Tyr Arg Gly Ala
65                  70                  75                  80

Asp Val Phe Ile Leu Ala Phe Ser Leu Ile Ser Arg Pro Ser Phe Glu
                85                  90                  95

Asn Ile Ala Lys Lys Trp Val Pro Glu Leu Arg His Tyr Ala Pro Thr
            100                 105                 110

Val Pro Ile Val Leu Val Gly Thr Lys Ser Asp Leu Arg Asp Asn Met
        115                 120                 125

Gln Phe Pro Lys Asn Tyr Pro Gly Ala Cys Thr Ile Phe Pro Glu Gln
    130                 135                 140

Gly Gln Glu Leu Arg Lys Glu Ile Gly Ala Leu Ala Tyr Ile Glu Cys
145                 150                 155                 160

Ser Ser Lys Ala Gln Met Asn Val Lys Ala Val Phe Asp Glu Ala Ile
                165                 170                 175

Lys Val Val Leu His Pro Pro Ser Lys Thr Lys Lys Arg Lys Arg Lys
            180                 185                 190

Ile Gly Leu Cys His Val Leu
        195
```

<210> 71
<211> 21
<212> DNA
<213> Künstliche Sequenz

<220>
<223> Beschreibung der künstlichen Sequenz: oligonucleotide primer

<400> 71
atgagcgcgt ccaggttcat a 21

<210> 72
<211> 21
<212> DNA
<213> Künstliche Sequenz

<220>
<223> Beschreibung der künstlichen Sequenz: oligonucleotide primer

<400> 72
atcaaacacg cccttcacgt t 21

<210> 73
<211> 10828
<212> DNA
<213> Künstliche Sequenz

<220>
<223> Beschreibung der künstlichen Sequenz: transgenic expression vector pSUN3NIT_AtRacB_s for expression of A-thaliana RacB sense RNA

<400> 73

```
ttccatggac atacaaatgg acgaacggat aaaccttttc acgccctttt aaatatccga 60
ttattctaat aaacgctctt ttctcttagg tttacccgcc aatatatcct gtcaaacact 120
gatagtttaa actgaaggcg ggaaacgaca atcagatcta gtaggaaaca gctatgacca 180
tgattacgcc aagcttgcat gcctgcaggt cgactctaga ggatccccca tcaagatctt 240
ggtgatgtag caagagctaa gttgtacttc gatycggttg gacattactc gagaccagat 300
gttttacact tgaccgtaaa tgagcacccg aagaaaccgg taacattcat ttcgaaggta 360
gagaaagcgg aagatgactc aaacaagtaa tcggttgtga ttcgtcagtt catgtcactc 420
ctatgaagga gtcaagttca aaatgttatg ttgagtttca aacttttatg ctaaactttt 480
tttcttaatt ttcgttaata atggaagaga accaattctc ttgtatctaa agattatcca 540
tctatcatcc aatttgagtg ttcaattctg gatgttgtgt taccctacat tctacaacca 600
tgtagccaat tattatgaat ctggctttga tttcagttgt gttcttttct tttttctttt 660
gcatatttgc atttagaatg tttaataatt aagttactgt atttccacat acattagttc 720
caagaatata catatattaa tttatttttc ttaaaaatgt tttggaatga ctaatattga 780
caacgaaaat agaagctatg ctaaaccatt acgtatatgt gacttcacat gttgttgttt 840
tacattccct atatatatgg atggctgtca caatcagaaa cgtgatcgaa aaaagacaaa 900
cagtgtttgc ataaaaagac tatttcgttt cattgacaat ttgtgtttat ttgtaaagaa 960
aagtggcaaa gtggaatttg agttcctgca agtaagaaag atgaaataaa agacttgagt 1020
gtgtgttttt ttcttttatc tgaaagctgc aatgaaatat tcctaccaag cccgtttgat 1080
tattaattgg ggtttggttt tcttgatgcg aactaattgg ttatataaga aactatacaa 1140
tccatgttaa ttcaaaaatt ttgatttctc ttgtaggaat atgatttact atatgagact 1200
ttcttttcgc caataatagt aaatccaaag atatttgacc ggaccaaaac acattgatct 1260
atttttagt ttatttaatc cagtttctct gagataattc attaaggaaa acttagtatt 1320
aacccatcct aagattaaat aggagccaaa ctcacatttc aaatattaaa taacataaaa 1380
tggatttaaa aaatctatac gtcaaatttt atttatgaca tttcttattt aaatttatat 1440
ttaatgaaat acagctaaga caaaccaaaa aaaaaatact ttctaagtgg tccaaaacat 1500
caattccgtt caatattatt aggtagaatc gtacgaccaa aaaaggtagg ttaatacgaa 1560
attacaaaca tatctatata catagtatat attattacct attatgagga atcaaaatgc 1620
atcaaatatg gatttaagga atccataaaa gaataaattc tacggaaaaa aaaaaaagaa 1680
taaattcttt taagttttta atttgttttt tatttggtag ttctccattt tgttttattt 1740
cgtttggatt tattgtgtcc aaatactttg taaaccaccg ttgtaattct taaacggggt 1800
tttcacttct tttttatatt cagacataaa gcatcggctg gtttaatcaa tcaatagatt 1860
ttatttttct tctcaattat tagtaggttt gatgtgaact ttacaaaaaa aacaaaaaca 1920
aatcaatgca gagaaaagaa accacgtggg ctagtcccac cttgtttcat ttccaccaca 1980
ggttcgatct tcgttaccgt ctccaatagg aaaataaacg tgaccacaaa aaaaaaacaa 2040
aaaaaaagtc tatatattgc ttctctcaag tctctgagtg tcatgaacca aagtaaaaaa 2100
caaagactcg agtggatccc cggaattcgc ccttatgagc gcatcaaggt tcataaagtg 2160
cgtcaccgtt ggtgatggag ctgttggtaa aacctgtttg ctgatttctt ataccagcaa 2220
cacctttccc acggntattc atcaatcatt ctccctcctt tttttgatat ctgattcatt 2280
```

```
tgattctgat tgtgccatat atgaattggg atccatacat actaaaaatg ttgtatacat 2340
ttccattgga acaggattat gttccgactg ttttcgataa ctttagtgca aatgtggttg 2400
tcaatggggc cacggtgaat cttggattgt gggatactgc aggtaaatga atgatgatcc 2460
aattcataat ccttggtgag agagctttcc gtgatgaatc agaggtcgaa attatgtttt 2520
tggtttatgc agggcaagag gactataaca gattaagacc tttgagttac cgtggtgctg 2580
atgttttcat tcttgccttc tctctcatta gtaaggctag ttatgagaat gtttccaaga 2640
aggtcagttt cgtccgaact ggtcgactat ttaacaattg agagttccaa attttgatgc 2700
ttcttttctt ttacagtgga ttcctgagtt gaagcactat gctcctggtg tcccaattgt 2760
ccttgttgga accaaactag gttacttcct cctctcacat ttgtccttgt ttatgcattt 2820
atttatatat atgtctgatt cccatgctta cactgccatt ttcctttca ctttattaag 2880
ctgctctggt ataatatata tcatgacatt agtggacata aaacttcacc ttctcttgat 2940
tgtggttaaa acttgtacat gttcaagatg tattcgttag gtgaaactga gggtagtttt 3000
cagagaatat cattggtcaa caggcttctt cttgtatctt gcacttcttg tgataaagca 3060
accgtatcct ataacacacg cctttaagca tcctccaatg aaatagctac tgtatagcaa 3120
gtgtatacct ttataaaaga cacttgcaag atcttcagtc aactcatgat cctggccttt 3180
ttgattgtct aaccttggtt gttgtcagat cttcgagatg acaaacagtt tttcatcgac 3240
catcctggtg ctgtccctat taccactgtt caggtaagaa tacagttatt tcctcagtgc 3300
aattttatca gctttaccac cgttaagcat tttccctctc tgcatggaag ggagaggagc 3360
tgaagaagct aattggagcg ccagcttaca tcgagtgcag ttcaaaatca caagaggtaa 3420
acgaataaaa gacatctcat gaatcatctt ttcggtgtta gattcttctt tttttgatga 3480
aaacaatgtg actataactg cagaacgtga agggcgtgtt tgataagggc gaattaattc 3540
actggccgtc gttttacaac gactcagagc ttgacaggag gcccgatcta gtaacataga 3600
tgacaccgcg cgcgataatt tatcctagtt tgcgcgctat attttgtttt ctatcgcgta 3660
ttaaatgtat aattgcggga ctctaatcat aaaaacccat ctcataaata acgtcatgca 3720
ttacatgtta attattacat gcttaacgta attcaacaga aattatatga taatcatcgc 3780
aagaccggca acaggattca atcttaagaa actttattgc caaatgtttg aacgatcggg 3840
gatcatccgg gtctgtggcg ggaactccac gaaaatatcc gaacgcagca agatctagag 3900
cttgggtccc gctcagaaga actcgtcaag aaggcgatag aaggcgatgc gctgcgaatc 3960
gggagcggcg ataccgtaaa gcacgaggaa gcggtcagcc cattcgccgc caagctcttc 4020
agcaatatca cgggtagcca acgctatgtc ctgatagcgg tccgccacac ccagccggcc 4080
acagtcgatg aatccagaaa agcggccatt ttccaccatg atattcggca agcaggcatc 4140
gccatgggtc acgacgagat cctcgccgtc gggcatgcgc gccttgagcc tggcgaacag 4200
ttcggctggc gcgagcccct gatgctcttc gtccagatca tcctgatcga caagaccggc 4260
ttccatccga gtacgtgctc gctcgatgcg atgtttcgct tggtggtcga atgggcaggt 4320
agccggatca agcgtatgca gccgccgcat tgcatcagcc atgatggata ctttctcggc 4380
aggagcaagg tgagatgaca ggagatcctg ccccggcact tcgcccaata gcagccagtc 4440
ccttcccgct tcagtgacaa cgtcgagcac agctgcgcaa ggaacgcccg tcgtggccag 4500
ccacgatagc cgcgctgcct cgtcctgcag ttcattcagg gcaccggaca ggtcggtctt 4560
gacaaaaaga accgggcgcc cctgcgctga cagccggaac acggcggcat cagagcagcc 4620
gattgtctgt tgtgcccagt catagccgaa tagcctctcc acccaagcgg ccggagaacc 4680
tgcgtgcaat ccatcttgtt caatcatgcg aaacgatcca gatccggtgc agattatttg 4740
gattgagagt gaatatgaga ctctaattgg ataccgaggg gaatttatgg aacgtcagtg 4800
gagcattttt gacaagaaat atttgctagc tgatagtgac cttaggcgac ttttgaacgc 4860
gcaataatgg tttctgacgt atgtgcttag ctcattaaac tccagaaacc cgcggctgag 4920
tggctccttc aacgttgcgg ttctgtcagt tccaaacgta aaacggcttg tcccgcgtca 4980
tcggcggggg tcataacgtg actcccttaa ttctccgctc atgatcagat tgtcgtttcc 5040
cgccttcagt ttaaactatc agtgtttgac aggatcctgc ttggtaataa ttgtcattag 5100
attgttttta tgcatagatg cactcgaaat cagccaattt tagacaagta tcaaacggat 5160
gttaattcag tacattaaag acgtccgcaa tgtgttatta agttgtctaa gcgtcaattt 5220
gtttacacca caatatatcc tgccaccagc cagccaacag ctccccgacc ggcagctcgg 5280
cacaaaatca ccacgcgtta ccaccacgcc ggccggccgc atggtgttga ccgtgttcgc 5340
cggcattgcc gagttcgagc gttccctaat catcgaccgc acccggagcg ggcgcgaggc 5400
cgccaaggcc cgaggcgtga agtttggccc ccgccctacc ctcaccccgg cacagatcgc 5460
gcacgcccgc gagctgatcg accaggaagg ccgcaccgtg aaagaggcgg ctgcactgct 5520
tggcgtgcat cgctcgaccc tgtaccgcgc acttgagcgc agcgaggaag tgacgcccac 5580
cgaggccagg cggcgcggtg ccttccgtga ggacgcattg accgaggccg acgccctggc 5640
ggccgccgag aatgaacgcc aagaggaaca agcatgaaac cgcaccagga cggccaggac 5700
```

gaaccgtttt tcattaccga agagatcgag gcggagatga tcgcggccgg gtacgtgttc 5760
gagccgcccg cgcacgtctc aaccgtgcgg ctgcatgaaa tcctggccgg tttgtctgat 5820
gccaagctgg cggcctggcc ggccagcttg gccgctgaag aaaccgagcg ccgccgtcta 5880
aaaaggtgat gtgtatttga gtaaaacagc ttgcgtcatg cggtcgctgc gtatatgatg 5940
cgatgagtaa ataaacaaat acgcaagggg aacgcatgaa ggttatcgct gtacttaacc 6000
agaaaggcgg gtcaggcaag acgaccatcg caacccatct agcccgcgcc ctgcaactcg 6060
ccggggccga tgttctgtta gtcgattccg atccccaggg cagtgcccgc gattgggcgg 6120
ccgtgcggga agatcaaccg ctaaccgttg tcggcatcga ccgcccgacg attgaccgcg 6180
acgtgaaggc catcggccgg cgcgacttcg tagtgatcga cggagcgccc caggcggcgg 6240
acttggctgt gtccgcgatc aaggcagccg acttcgtgct gattccggtg cagccaagcc 6300
cttacgacat atgggccacc gccgacctgg tggagctggt taagcagcgc attgaggtca 6360
cggatggaag gctacaagcg gcctttgtcg tgtcgcgggc gatcaaaggc acgcgcatcg 6420
gcggtgaggt tgccgaggcg ctggccgggt acgagctgcc cattcttgag tcccgtatca 6480
cgcagcgcgt gagctaccca ggcactgccg ccgccggcac aaccgttctt gaatcagaac 6540
ccgagggcga cgctgcccgc gaggtccagg cgctggccgc tgaaattaaa tcaaaactca 6600
tttgagttaa tgaggtaaag agaaaatgag caaaagcaca aacacgctaa gtgccggccg 6660
tccgagcgca cgcagcagca aggctgcaac gttggccagc ctggcagaca cgccagccat 6720
gaagcgggtc aactttcagt tgccggcgga ggatcacacc aagctgaaga tgtacgcggt 6780
acgccaaggc aagaccatta ccgagctgct atctgaatac atcgcgcagc taccagagta 6840
aatgagcaaa tgaataaatg agtagatgaa ttttagcggc taaaggaggc ggcatggaaa 6900
atcaagaaca accaggcacc gacgccgtgg aatgccccat gtgtggagga acgggcggtt 6960
ggccaggcgt aagcggctgg gttgtctgcc ggccctgcaa tggcactgga acccccaagc 7020
ccgaggaatc ggcgtgagcg gtcgcaaacc atccggcccg gtacaaatcg gcgcggcgct 7080
gggtgatgac ctggtggaga agttgaaggc cgcgcaggcc gcccagcggc aacgcatcga 7140
ggcagaagca cgccccggtg aatcgtggca agcggccgct gatcgaatcc gcaaagaatc 7200
ccggcaaccg ccggcagccg gtgcgccgtc gattaggaag ccgcccaagg gcgacgagca 7260
accagatttt ttcgttccga tgctctatga cgtgggcacc cgcgatagtc gcagcatcat 7320
ggacgtggcc gttttccgtc tgtcgaagcg tgaccgacga gctggcgagg tgatccgcta 7380
cgagcttcca gacgggcacg tagaggtttc cgcagggccg gccggcatgg ccagtgtgtg 7440
ggattacgac ctggtactga tggcggtttc ccatctaacc gaatccatga accgataccg 7500
ggaagggaag ggagacaagc ccggccgcgt gttccgtcca cacgttgcgg acgtactcaa 7560
gttctgccgg cgagccgatg gcggaaagca gaaagacgac ctggtagaaa cctgcattcg 7620
gttaaacacc acgcacgttg ccatgcagcg tacgaagaag gccaagaacg gccgcctggt 7680
gacggtatcc gagggtgaag ccttgattag ccgctacaag atcgtaaaga gcgaaaccgg 7740
gcggccggag tacatcgaga tcgagctagc tgattggatg taccgcgaga tcacagaagg 7800
caagaacccg gacgtgctga cggttcaccc cgattacttt ttgatcgatc ccggcatcgg 7860
ccgttttctc taccgcctgg cacgccgcgc cgcaggcaag gcagaagcca gatggttgtt 7920
caagacgatc tacgaacgca gtggcagcgc cggagagttc aagaagttct gtttcaccgt 7980
gcgcaagctg atcgggtcaa atgacctgcc ggagtacgat ttgaaggagg aggcggggca 8040
ggctggcccg atcctagtca tgcgctaccg caacctgatc gagggcgaag catccgccgg 8100
ttcctaatgt acggagcaga tgctagggca aattgcccta gcaggggaaa aaggtcgaaa 8160
aggtctcttt cctgtggata gcacgtacat tgggaaccca aagccgtaca ttgggaaccg 8220
gaacccgtac attgggaacc caaagccgta cattgggaac cggtcacaca tgtaagtgac 8280
tgatataaaa gagaaaaaag gcgatttttc cgcctaaaac tctttaaaac ttattaaaac 8340
tcttaaaacc cgcctggcct gtgcataact gtctggccag cgcacagccg aagagctgca 8400
aaaagcgcct acccttcggt cgctgcgctc cctacgcccc gccgcttcgc gtcggcctat 8460
cgcggccgct ggccgctcaa aaatggctgg cctacggcca ggcaatctac cagggcgcgg 8520
acaagccgcg ccgtcgccac tcgaccgccg gcgcccacat caaggcaccc tgcctcgcgc 8580
gtttcggtga tgacggtgaa aacctctgac acatgcagct cccggagacg gtcacagctt 8640
gtctgtaagc ggatgccggg agcagacaag cccgtcaggg cgcgtcagcg ggtgttggcg 8700
ggtgtcgggg cgcagccatg acccagtcac gtagcgatag cggagtgtat actggcttaa 8760
ctatgcggca tcagagcaga ttgtactgag agtgcaccat atgcggtgtg aaataccgca 8820
cagatgcgta aggagaaaat accgcatcag gcgctcttcc gcttcctcgc tcactgactc 8880
gctgcgctcg gtcgttcggc tgcggcgagc ggtatcagct cactcaaagg cggtaatacg 8940
gttatccaca gaatcagggg ataacgcagg aaagaacatg tgagcaaaag gccagcaaaa 9000
ggccaggaac cgtaaaaagg ccgcgttgct ggcgtttttc cataggctcc gcccccctga 9060
cgagcatcac aaaaatcgac gctcaagtca gaggtggcga aacccgacag gactataaag 9120

ataccaggcg tttccccctg gaagctccct cgtgcgctct cctgttccga ccctgccgct 9180
taccggatac ctgtccgcct ttctcccttc gggaagcgtg gcgctttctc atagctcacg 9240
ctgtaggtat ctcagttcgg tgtaggtcgt tcgctccaag ctgggctgtg tgcacgaacc 9300
ccccgttcag cccgaccgct gcgccttatc cggtaactat cgtcttgagt ccaacccggt 9360
aagacacgac ttatcgccac tggcagcagc cactggtaac aggattagca gagcgaggta 9420
tgtaggcggt gctacagagt tcttgaagtg gtggcctaac tacggctaca ctagaaggac 9480
agtatttggt atctgcgctc tgctgaagcc agttaccttc ggaaaaagag ttggtagctc 9540
ttgatccggc aaacaaacca ccgctggtag cggtggtttt tttgtttgca agcagcagat 9600
tacgcgcaga aaaaaaggat ctcaagaaga tcctttgatc ttttctacgg ggtctgacgc 9660
tcagtggaac gaaaactcac gttaagggat tttggtcatg catgatatat ctcccaattt 9720
gtgtagggct tattatgcac gcttaaaaat aataaaagca gacttgacct gatagtttgg 9780
ctgtgagcaa ttatgtgctt agtgcatcta acgcttgagt taagccgcgc cgcgaagcgg 9840
cgtcggcttg aacgaatttc tagctagaca ttatttgccg actaccttgg tgatctcgcc 9900
tttcacgtag tggacaaatt cttccaactg atctgcgcgc gaggccaagc gatcttcttc 9960
ttgtccaaga taagcctgtc tagcttcaag tatgacgggc tgatactggg ccggcaggcg 10020
ctccattgcc cagtcggcag cgacatcctt cggcgcgatt ttgccggtta ctgcgctgta 10080
ccaaatgcgg gacaacgtaa gcactacatt tcgctcatcg ccagcccagt cgggcggcga 10140
gttccatagc gttaaggttt catttagcgc ctcaaataga tcctgttcag gaaccggatc 10200
aaagagttcc tccgccgctg gacctaccaa ggcaacgcta tgttctcttg cttttgtcag 10260
caagatagcc agatcaatgt cgatcgtggc tggctcgaag atacctgcaa gaatgtcatt 10320
gcgctgccat tctccaaatt gcagttcgcg cttagctgga taacgccacg gaatgatgtc 10380
gtcgtgcaca acaatggtga cttctacagc gcggagaatc tcgctctctc caggggaagc 10440
cgaagtttcc aaaaggtcgt tgatcaaagc tcgccgcgtt gtttcatcaa gccttacggt 10500
caccgtaacc agcaaatcaa tatcactgtg tggcttcagg ccgccatcca ctgcggagcc 10560
gtacaaatgt acggccagca acgtcggttc gagatggcgc tcgatgacgc caactacctc 10620
tgatagttga gtcgatactt cggcgatcac cgcttccccc atgatgttta actttgtttt 10680
agggcgactg ccctgctgcg taacatcgtt gctgctccat aacatcaaac atcgacccac 10740
ggcgtaacgc gcttgctgct tggatgcccg aggcatagac tgtaccccaa aaaaacagtc 10800
ataacaagcc atgaaaaccg ccactgcg 10828

<210> 74
<211> 10828
<212> DNA
<213> Künstliche Sequenz

<220>
<223> Beschreibung der künstlichen Sequenz: transgenic expression vector pSUN3NIT_AtRacB_as for expression of A-thaliana RacB antisense RNA

<400> 74

```
ttccatggac atacaaatgg acgaacggat aaacctttc acgccctttt aaatatccga 60
ttattctaat aaacgctctt ttctcttagg tttacccgcc aatatatcct gtcaaacact 120
gatagtttaa actgaaggcg ggaaacgaca atcagatcta gtaggaaaca gctatgacca 180
tgattacgcc aagcttgcat gcctgcaggt cgactctaga ggatccccca tcaagatctt 240
ggtgatgtag caagagctaa gttgtacttc gatycggttg gacattactc gagaccagat 300
gttttacact tgaccgtaaa tgagcacccg aagaaaccgg taacattcat ttcgaaggta 360
gagaaagcgg aagatgactc aaacaagtaa tcggttgtga ttcgtcagtt catgtcactc 420
ctatgaagga gtcaagttca aaatgttatg ttgagtttca aacttttatg ctaaactttt 480
tttcttaatt ttcgttaata atggaagaga accaattctc ttgtatctaa agattatcca 540
tctatcatcc aatttgagtg ttcaattctg gatgttgtgt taccctacat tctacaacca 600
tgtagccaat tattatgaat ctggctttga tttcagttgt gttcttttct ttttttcttt 660
gcatatttgc atttagaatg tttaataatt aagttactgt atttccacat acattagttc 720
caagaatata catatattaa tttatttttc ttaaaaatgt tttggaatga ctaatattga 780
caacgaaaat agaagctatg ctaaaccatt acgtatatgt gacttcacat gttgttgttt 840
tacattccct atatatatgg atggctgtca caatcagaaa cgtgatcgaa aaaagacaaa 900
cagtgtttgc ataaaaagac tatttcgttt cattgacaat ttgtgtttat ttgtaaagaa 960
aagtggcaaa gtggaatttg agttcctgca agtaagaaag atgaaataaa agacttgagt 1020
gtgtgttttt ttcttttatc tgaaagctgc aatgaaatat tcctaccaag cccgtttgat 1080
```

```
tattaattgg ggtttggttt tcttgatgcg aactaattgg ttatataaga aactatacaa 1140
tccatgttaa ttcaaaaatt ttgatttctc ttgtaggaat atgatttact atatgagact 1200
ttcttttcgc caataatagt aaatccaaag atatttgacc ggaccaaaac acattgatct 1260
atttttagt ttatttaatc cagtttctct gagataattc attaaggaaa acttagtatt 1320
aacccatcct aagattaaat aggagccaaa ctcacatttc aaatattaaa taacataaaa 1380
tggatttaaa aaatctatac gtcaaatttt atttatgaca tttcttattt aaatttatat 1440
ttaatgaaat acagctaaga caaaccaaaa aaaaaatact ttctaagtgg tccaaaacat 1500
caattccgtt caatattatt aggtagaatc gtacgaccaa aaaaggtagg ttaatacgaa 1560
attacaaaca tatctatata catagtatat attattacct attatgagga atcaaaatgc 1620
atcaaatatg gatttaagga atccataaaa gaataaattc tacggaaaaa aaaaaaagaa 1680
taaattcttt taagttttta atttgttttt tatttggtag ttctccattt tgttttattt 1740
cgtttggatt tattgtgtcc aaatactttg taaaccaccg ttgtaattct taaacggggt 1800
tttcacttct tttttatatt cagacataaa gcatcggctg gtttaatcaa tcaatagatt 1860
ttatttttct tctcaattat tagtaggttt gatgtgaact ttacaaaaaa aacaaaaaca 1920
aatcaatgca gagaaaagaa accacgtggg ctagtcccac cttgtttcat ttccaccaca 1980
ggttcgatct tcgttaccgt ctccaatagg aaaataaacg tgaccacaaa aaaaaaacaa 2040
aaaaaaagtc tatatattgc ttctctcaag tctctgagtg tcatgaacca aagtaaaaaa 2100
caaagactcg agtggatccc cggaattcgc ccttatcaaa cacgcccttc acgttctgca 2160
gttatagtca cattgttttc atcaaaaaaa gaagaatcta acaccgaaaa gatgattcat 2220
gagatgtctt ttattcgttt acctcttgtg attttgaact gcactcgatg taagctggcg 2280
ctccaattag cttcttcagc tcctctccct tccatgcaga gagggaaaat gcttaacggt 2340
ggtaaagctg ataaaattgc actgaggaaa taactgtatt cttacctgaa cagtggtaat 2400
agggacagca ccaggatggt cgatgaaaaa ctgtttgtca tctcgaagat ctgacaacaa 2460
ccaaggttag acaatcaaaa aggccaggat catgagttga ctgaagatct tgcaagtgtc 2520
ttttataaag gtatacactt gctatacagt agctatttca ttggaggatg cttaaaggcg 2580
tgtgttatag gatacggttg ctttatcaca agaagtgcaa gatacaagaa gaagcctgtt 2640
gaccaatgat attctctgaa aactaccctc agtttcacct aacgaataca tcttgaacat 2700
gtacaagttt taaccacaat caagagaagg tgaagtttta tgtccactaa tgtcatgata 2760
tatattatac cagagcagct taataaagtg aaaaggaaaa tggcagtgta agcatgggaa 2820
tcagacatat atataaataa atgcataaac aaggacaaat gtgagaggag gaagtaacct 2880
agtttggttc caacaaggac aattgggaca ccaggagcat agtgcttcaa ctcaggaatc 2940
cactgtaaaa gaaaagaagc atcaaaattt ggaactctca attgttaaat agtcgaccag 3000
ttcggacgaa actgaccttc ttggaaacat tctcataact agccttacta atgagagaga 3060
aggcaagaat gaaaacatca gcaccacggt aactcaaagg tcttaatctg ttatagtcct 3120
cttgccctgc ataaaccaaa aacataattt cgacctctga ttcatcacgg aaagctctct 3180
caccaaggat tatgaattgg atcatcattc atttacctgc agtatcccac aatccaagat 3240
tcaccgtggc cccattgaca accacatttg cactaaagtt atcgaaaaca gtcggaacat 3300
aatcctgttc caatggaaat gtatacaaca tttttagtat gtatggatcc caattcatat 3360
atggcacaat cagaatcaaa tgaatcagat atcaaaaaaa ggagggagaa tgattgatga 3420
atanccgtgg gaaaggtgtt gctggtataa gaaatcagca aacaggtttt accaacagct 3480
ccatcaccaa cggtgacgca ctttatgaac cttgatgcgc tcataagggc gaattaattc 3540
actggccgtc gttttacaac gactcagagc ttgacaggag gcccgatcta gtaacataga 3600
tgacaccgcg cgcgataatt tatcctagtt tgcgcgctat attttgtttt ctatcgcgta 3660
ttaaatgtat aattgcggga ctctaatcat aaaaacccat ctcataaata acgtcatgca 3720
ttacatgtta attattacat gcttaacgta attcaacaga aattatatga taatcatcgc 3780
aagaccggca acaggattca atcttaagaa actttattgc caaatgtttg aacgatcggg 3840
gatcatccgg gtctgtggcg ggaactccac gaaaatatcc gaacgcagca agatctagag 3900
cttgggtccc gctcagaaga actcgtcaag aaggcgatag aaggcgatgc gctgcgaatc 3960
gggagcggcg ataccgtaaa gcacgaggaa gcggtcagcc cattcgccgc caagctcttc 4020
agcaatatca cgggtagcca acgctatgtc ctgatagcgg tccgccacac ccagccggcc 4080
acagtcgatg aatccagaaa agcggccatt ttccaccatg atattcggca agcaggcatc 4140
gccatgggtc acgacgagat cctcgccgtc gggcatgcgc gccttgagcc tggcgaacag 4200
ttcggctggc gcgagcccct gatgctcttc gtccagatca tcctgatcga caagaccggc 4260
ttccatccga gtacgtgctc gctcgatgcg atgtttcgct tggtggtcga atgggcaggt 4320
agccggatca agcgtatgca gccgccgcat tgcatcagcc atgatggata ctttctcggc 4380
aggagcaagg tgagatgaca ggagatcctg ccccggcact tcgcccaata gcagccagtc 4440
ccttcccgct tcagtgacaa cgtcgagcac agctgcgcaa ggaacgcccg tcgtggccag 4500
```

ccacgatagc cgcgctgcct cgtcctgcag ttcattcagg gcaccggaca ggtcggtctt 4560
gacaaaaaga accgggcgcc cctgcgctga cagccggaac acggcggcat cagagcagcc 4620
gattgtctgt tgtgcccagt catagccgaa tagcctctcc acccaagcgg ccggagaacc 4680
tgcgtgcaat ccatcttgtt caatcatgcg aaacgatcca gatccggtgc agattatttg 4740
gattgagagt gaatatgaga ctctaattgg ataccgaggg gaatttatgg aacgtcagtg 4800
gagcattttt gacaagaaat atttgctagc tgatagtgac cttaggcgac ttttgaacgc 4860
gcaataatgg tttctgacgt atgtgcttag ctcattaaac tccagaaacc cgcggctgag 4920
tggctccttc aacgttgcgg ttctgtcagt tccaaacgta aaacggcttg tcccgcgtca 4980
tcggcggggg tcataacgtg actcccttaa ttctccgctc atgatcagat tgtcgtttcc 5040
cgccttcagt ttaaactatc agtgtttgac aggatcctgc ttggtaataa ttgtcattag 5100
attgttttta tgcatagatg cactcgaaat cagccaattt tagacaagta tcaaacggat 5160
gttaattcag tacattaaag acgtccgcaa tgtgttatta agttgtctaa gcgtcaattt 5220
gtttacacca caatatatcc tgccaccagc cagccaacag ctccccgacc ggcagctcgg 5280
cacaaaatca ccacgcgtta ccaccacgcc ggccggccgc atggtgttga ccgtgttcgc 5340
cggcattgcc gagttcgagc gttccctaat catcgaccgc acccggagcg ggcgcgaggc 5400
cgccaaggcc cgaggcgtga agtttggccc ccgccctacc ctcaccccgg cacagatcgc 5460
gcacgcccgc gagctgatcg accaggaagg ccgcaccgtg aaagaggcgg ctgcactgct 5520
tggcgtgcat cgctcgaccc tgtaccgcgc acttgagcgc agcgaggaag tgacgcccac 5580
cgaggccagg cggcgcggtg ccttccgtga ggacgcattg accgaggccg acgccctggc 5640
ggccgccgag aatgaacgcc aagaggaaca agcatgaaac cgcaccagga cggccaggac 5700
gaaccgtttt tcattaccga agagatcgag gcggagatga tcgcggccgg gtacgtgttc 5760
gagccgcccg cgcacgtctc aaccgtgcgg ctgcatgaaa tcctggccgg tttgtctgat 5820
gccaagctgg cggcctggcc ggccagcttg gccgctgaag aaaccgagcg ccgccgtcta 5880
aaaaggtgat gtgtatttga gtaaaacagc ttgcgtcatg cggtcgctgc gtatatgatg 5940
cgatgagtaa ataaacaaat acgcaagggg aacgcatgaa ggttatcgct gtacttaacc 6000
agaaaggcgg gtcaggcaag acgaccatcg caacccatct agcccgcgcc ctgcaactcg 6060
ccggggccga tgttctgtta gtcgattccg atccccaggg cagtgcccgc gattgggcgg 6120
ccgtgcggga agatcaaccg ctaaccgttg tcggcatcga ccgcccgacg attgaccgcg 6180
acgtgaaggc catcggccgg cgcgacttcg tagtgatcga cggagcgccc caggcggcgg 6240
acttggctgt gtccgcgatc aaggcagccg acttcgtgct gattccggtg cagccaagcc 6300
cttacgacat atgggccacc gccgacctgg tggagctggt taagcagcgc attgaggtca 6360
cggatggaag gctacaagcg gcctttgtcg tgtcgcgggc gatcaaaggc acgcgcatcg 6420
gcggtgaggt tgccgaggcg ctggccgggt acgagctgcc cattcttgag tcccgtatca 6480
cgcagcgcgt gagctaccca ggcactgccg ccgccggcac aaccgttctt gaatcagaac 6540
ccgagggcga cgctgcccgc gaggtccagg cgctggccgc tgaaattaaa tcaaaactca 6600
tttgagttaa tgaggtaaag agaaaatgag caaaagcaca aacacgctaa gtgccggccg 6660
tccgagcgca cgcagcagca aggctgcaac gttggccagc ctggcagaca cgccagccat 6720
gaagcgggtc aactttcagt tgccggcgga ggatcacacc aagctgaaga tgtacgcggt 6780
acgccaaggc aagaccatta ccgagctgct atctgaatac atcgcgcagc taccagagta 6840
aatgagcaaa tgaataaatg agtagatgaa ttttagcggc taaaggaggc ggcatggaaa 6900
atcaagaaca accaggcacc gacgccgtgg aatgccccat gtgtggagga acgggcggtt 6960
ggccaggcgt aagcggctgg gttgtctgcc ggccctgcaa tggcactgga acccccaagc 7020
ccgaggaatc ggcgtgagcg gtcgcaaacc atccggcccg gtacaaatcg gcgcggcgct 7080
gggtgatgac ctggtggaga agttgaaggc cgcgcaggcc gcccagcggc aacgcatcga 7140
ggcagaagca cgccccggtg aatcgtggca agcggccgct gatcgaatcc gcaaagaatc 7200
ccggcaaccg ccggcagccg gtgcgccgtc gattaggaag ccgcccaagg gcgacgagca 7260
accagatttt ttcgttccga tgctctatga cgtgggcacc cgcgatagtc gcagcatcat 7320
ggacgtggcc gttttccgtc tgtcgaagcg tgaccgacga gctggcgagg tgatccgcta 7380
cgagcttcca gacgggcacg tagaggtttc cgcagggccg gccggcatgg ccagtgtgtg 7440
ggattacgac ctggtactga tggcggtttc ccatctaacc gaatccatga accgataccg 7500
ggaagggaag ggagacaagc ccggccgcgt gttccgtcca cacgttgcgg acgtactcaa 7560
gttctgccgg cgagccgatg gcggaaagca gaaagacgac ctggtagaaa cctgcattcg 7620
gttaaacacc acgcacgttg ccatgcagcg tacgaagaag gccaagaacg gccgcctggt 7680
gacggtatcc gagggtgaag ccttgattag ccgctacaag atcgtaaaga gcgaaaccgg 7740
gcggccggag tacatcgaga tcgagctagc tgattggatg taccgcgaga tcacagaagg 7800
caagaacccg gacgtgctga cggttcaccc cgattacttt ttgatcgatc ccggcatcgg 7860
ccgttttctc taccgcctgg cacgccgcgc cgcaggcaag gcagaagcca gatggttgtt 7920

caagacgatc tacgaacgca gtggcagcgc cggagagttc aagaagttct gtttcaccgt 7980
gcgcaagctg atcgggtcaa atgacctgcc ggagtacgat ttgaaggagg aggcggggca 8040
ggctggcccg atcctagtca tgcgctaccg caacctgatc gagggcgaag catccgccgg 8100
ttcctaatgt acggagcaga tgctagggca aattgcccta gcaggggaaa aaggtcgaaa 8160
aggtctcttt cctgtggata gcacgtacat tgggaaccca aagccgtaca ttgggaaccg 8220
gaacccgtac attgggaacc caaagccgta cattgggaac cggtcacaca tgtaagtgac 8280
tgatataaaa gagaaaaaag gcgatttttc cgcctaaaac tctttaaaac ttattaaaac 8340
tcttaaaacc cgcctggcct gtgcataact gtctggccag cgcacagccg aagagctgca 8400
aaaagcgcct acccttcggt cgctgcgctc cctacgcccc gccgcttcgc gtcggcctat 8460
cgcggccgct ggccgctcaa aaatggctgg cctacggcca ggcaatctac cagggcgcgg 8520
acaagccgcg ccgtcgccac tcgaccgccg gcgcccacat caaggcaccc tgcctcgcgc 8580
gtttcggtga tgacggtgaa aacctctgac acatgcagct cccggagacg gtcacagctt 8640
gtctgtaagc ggatgccggg agcagacaag cccgtcaggg cgcgtcagcg ggtgttggcg 8700
ggtgtcgggg cgcagccatg acccagtcac gtagcgatag cggagtgtat actggcttaa 8760
ctatgcggca tcagagcaga ttgtactgag agtgcaccat atgcggtgtg aaataccgca 8820
cagatgcgta aggagaaaat accgcatcag gcgctcttcc gcttcctcgc tcactgactc 8880
gctgcgctcg gtcgttcggc tgcggcgagc ggtatcagct cactcaaagg cggtaatacg 8940
gttatccaca gaatcagggg ataacgcagg aaagaacatg tgagcaaaag gccagcaaaa 9000
ggccaggaac cgtaaaaagg ccgcgttgct ggcgtttttc cataggctcc gcccccctga 9060
cgagcatcac aaaaatcgac gctcaagtca gaggtggcga aacccgacag gactataaag 9120
ataccaggcg tttccccctg gaagctccct cgtgcgctct cctgttccga ccctgccgct 9180
taccggatac ctgtccgcct ttctcccttc gggaagcgtg gcgctttctc atagctcacg 9240
ctgtaggtat ctcagttcgg tgtaggtcgt tcgctccaag ctgggctgtg tgcacgaacc 9300
ccccgttcag cccgaccgct gcgccttatc cggtaactat cgtcttgagt ccaacccggt 9360
aagacacgac ttatcgccac tggcagcagc cactggtaac aggattagca gagcgaggta 9420
tgtaggcggt gctacagagt tcttgaagtg gtggcctaac tacggctaca ctagaaggac 9480
agtatttggt atctgcgctc tgctgaagcc agttaccttc ggaaaaagag ttggtagctc 9540
ttgatccggc aaacaaacca ccgctggtag cggtggtttt tttgtttgca agcagcagat 9600
tacgcgcaga aaaaaaggat ctcaagaaga tcctttgatc ttttctacgg ggtctgacgc 9660
tcagtggaac gaaaactcac gttaagggat tttggtcatg catgatatat ctcccaattt 9720
gtgtagggct tattatgcac gcttaaaaat aataaaagca gacttgacct gatagtttgg 9780
ctgtgagcaa ttatgtgctt agtgcatcta acgcttgagt taagccgcgc cgcgaagcgg 9840
cgtcggcttg aacgaatttc tagctagaca ttatttgccg actaccttgg tgatctcgcc 9900
tttcacgtag tggacaaatt cttccaactg atctgcgcgc gaggccaagc gatcttcttc 9960
ttgtccaaga taagcctgtc tagcttcaag tatgacgggc tgatactggg ccggcaggcg 10020
ctccattgcc cagtcggcag cgacatcctt cggcgcgatt ttgccggtta ctgcgctgta 10080
ccaaatgcgg gacaacgtaa gcactacatt tcgctcatcg ccagcccagt cgggcggcga 10140
gttccatagc gttaaggttt catttagcgc ctcaaataga tcctgttcag gaaccggatc 10200
aaagagttcc tccgccgctg gacctaccaa ggcaacgcta tgttctcttg cttttgtcag 10260
caagatagcc agatcaatgt cgatcgtggc tggctcgaag atacctgcaa gaatgtcatt 10320
gcgctgccat tctccaaatt gcagttcgcg cttagctgga taacgccacg gaatgatgtc 10380
gtcgtgcaca acaatggtga cttctacagc gcggagaatc tcgctctctc caggggaagc 10440
cgaagtttcc aaaaggtcgt tgatcaaagc tcgccgcgtt gtttcatcaa gccttacggt 10500
caccgtaacc agcaaatcaa tatcactgtg tggcttcagg ccgccatcca ctgcggagcc 10560
gtacaaatgt acggccagca acgtcggttc gagatggcgc tcgatgacgc caactacctc 10620
tgatagttga gtcgatactt cggcgatcac cgcttccccc atgatgttta actttgtttt 10680
agggcgactg ccctgctgcg taacatcgtt gctgctccat aacatcaaac atcgacccac 10740
ggcgtaacgc gcttgctgct tggatgcccg aggcatagac tgtaccccaa aaaaacagtc 10800
ataacaagcc atgaaaaccg ccactgcg 10828

<210> 75
<211> 10036
<212> DNA
<213> Künstliche Sequenz

<220>
<223> Beschreibung der künstlichen Sequenz: transgenic expression vector pSUN3NIT_HvRacB_s for expression of barley RacB sense RNA fragment

<400> 75

```
ttccatggac atacaaatgg acgaacggat aaaccttttc acgccctttt aaatatccga 60
ttattctaat aaacgctctt ttctcttagg tttacccgcc aatatatcct gtcaaacact 120
gatagtttaa actgaaggcg ggaaacgaca atcagatcta gtaggaaaca gctatgacca 180
tgattacgcc aagcttgcat gcctgcaggt cgactctaga ggatccccca tcaagatctt 240
ggtgatgtag caagagctaa gttgtacttc gatycggttg gacattactc gagaccagat 300
gttttacact tgaccgtaaa tgagcacccg aagaaaccgg taacattcat ttcgaaggta 360
gagaaagcgg aagatgactc aaacaagtaa tcggttgtga ttcgtcagtt catgtcactc 420
ctatgaagga gtcaagttca aaatgttatg ttgagtttca aacttttatg ctaaactttt 480
tttcttaatt ttcgttaata atggaagaga accaattctc ttgtatctaa agattatcca 540
tctatcatcc aatttgagtg ttcaattctg gatgttgtgt taccctacat tctacaacca 600
tgtagccaat tattatgaat ctggctttga tttcagttgt gttcttttct tttttctttt 660
gcatatttgc atttagaatg tttaataatt aagttactgt atttccacat acattagttc 720
caagaatata catatattaa tttatttttc ttaaaaatgt tttggaatga ctaatattga 780
caacgaaaat agaagctatg ctaaaccatt acgtatatgt gacttcacat gttgttgttt 840
tacattccct atatatatgg atggctgtca caatcagaaa cgtgatcgaa aaaagacaaa 900
cagtgtttgc ataaaaagac tatttcgttt cattgacaat ttgtgtttat ttgtaaagaa 960
aagtggcaaa gtggaatttg agttcctgca agtaagaaag atgaaataaa agacttgagt 1020
gtgtgttttt ttcttttatc tgaaagctgc aatgaaatat tcctaccaag cccgtttgat 1080
tattaattgg ggtttggttt tcttgatgcg aactaattgg ttatataaga aactatacaa 1140
tccatgttaa ttcaaaaatt ttgatttctc ttgtaggaat atgatttact atatgagact 1200
ttcttttcgc caataatagt aaatccaaag atatttgacc ggaccaaaac acattgatct 1260
atttttagt ttatttaatc cagtttctct gagataattc attaaggaaa acttagtatt 1320
aacccatcct aagattaaat aggagccaaa ctcacatttc aaatattaaa taacataaaa 1380
tggatttaaa aaatctatac gtcaaatttt atttatgaca tttcttattt aaatttatat 1440
ttaatgaaat acagctaaga caaaccaaaa aaaaaatact ttctaagtgg tccaaaacat 1500
caattccgtt caatattatt aggtagaatc gtacgaccaa aaaaggtagg ttaatacgaa 1560
attacaaaca tatctatata catagtatat attattacct attatgagga atcaaaatgc 1620
atcaaatatg gatttaagga atccataaaa gaataaattc tacggaaaaa aaaaaaagaa 1680
taaattcttt taagttttta atttgttttt tatttggtag ttctccattt tgttttattt 1740
cgtttggatt tattgtgtcc aaatactttg taaaccaccg ttgtaattct taaacggggt 1800
tttcacttct tttttatatt cagacataaa gcatcggctg gtttaatcaa tcaatagatt 1860
ttatttttct tctcaattat tagtaggttt gatgtgaact ttacaaaaaa aacaaaaaca 1920
aatcaatgca gagaaaagaa accacgtggg ctagtcccac cttgtttcat ttccaccaca 1980
ggttcgatct tcgttaccgt ctccaataggg aaaataaacg tgaccacaaa aaaaaaacaa 2040
aaaaaaagtc tatatattgc ttctctcaag tctctgagtg tcatgaacca aagtaaaaaa 2100
caaagactcg agtggatccc cgggccgcca tggccgcggg atggatccga tgagcgcgtc 2160
caggttcata aagtgcgtca cggtcgggga cggcgccgtc ggcaagacct gcatgctcat 2220
ctcctacacc tccaacacct tccccaccga ctatgttccg acagtgtttg ataacttcag 2280
tgccaacgtt gtggttgatg gtaatactgt caacctcggc ctctgggaca ctgcaggtca 2340
agaggattac aacagactga gaccactgag ctatcgtgga gctgatgttt ttcttctggc 2400
tttctcactg atcagtaagg ccagctatga gaatgtttcg aagaagtgga tacctgaact 2460
gaagcattat gcacctggtg tgccaattat tctcgtaggg acaaagcttg atcttcgaga 2520
cgacaagcag ttctttgtgg accatcctgg tgctgtccct atcactactg ctcagggaga 2580
ggagctaaga aagcaaatag gcgctccata ctacatcgaa tgcagctcga agacccaact 2640
aaacgtgaag ggcgtcttcg atgcggcgat aaaggttgtg ctgcagccgc ctaaggcgaa 2700
gaagaagaaa aaggtgcaga gggggcgtg ctccattttg tgaaattcac tggccgtcgt 2760
tttacaacga ctcagagctt gacaggaggc ccgatctagt aacatagatg acaccgcgcg 2820
cgataattta tcctagtttg cgcgctatat tttgttttct atcgcgtatt aaatgtataa 2880
ttgcgggact ctaatcataa aaacccatct cataaataac gtcatgcatt acatgttaat 2940
tattacatgc ttaacgtaat tcaacagaaa ttatatgata atcatcgcaa gaccggcaac 3000
aggattcaat cttaagaaac tttattgcca aatgtttgaa cgatcgggga tcatccgggt 3060
ctgtggcggg aactccacga aaatatccga acgcagcaag atctagagct tgggtcccgc 3120
tcagaagaac tcgtcaagaa ggcgatagaa ggcgatgcgc tgcgaatcgg gagcggcgat 3180
accgtaaagc acgaggaagc ggtcagccca ttcgccgcca agctcttcag caatatcacg 3240
```

ggtagccaac gctatgtcct gatagcggtc cgccacaccc agccggccac agtcgatgaa 3300
tccagaaaag cggccatttt ccaccatgat attcggcaag caggcatcgc catgggtcac 3360
gacgagatcc tcgccgtcgg gcatgcgcgc cttgagcctg gcgaacagtt cggctggcgc 3420
gagcccctga tgctcttcgt ccagatcatc ctgatcgaca agaccggctt ccatccgagt 3480
acgtgctcgc tcgatgcgat gtttcgcttg gtggtcgaat gggcaggtag ccggatcaag 3540
cgtatgcagc cgccgcattg catcagccat gatggatact ttctcggcag gagcaaggtg 3600
agatgacagg agatcctgcc ccggcacttc gcccaatagc agccagtccc ttcccgcttc 3660
agtgacaacg tcgagcacag ctgcgcaagg aacgcccgtc gtggccagcc acgatagccg 3720
cgctgcctcg tcctgcagtt cattcagggc accggacagg tcggtcttga caaaaagaac 3780
cgggcgcccc tgcgctgaca gccggaacac ggcggcatca gagcagccga ttgtctgttg 3840
tgcccagtca tagccgaata gcctctccac ccaagcggcc ggagaacctg cgtgcaatcc 3900
atcttgttca atcatgcgaa acgatccaga tccggtgcag attatttgga ttgagagtga 3960
atatgagact ctaattggat accgagggga atttatggaa cgtcagtgga gcatttttga 4020
caagaaatat ttgctagctg atagtgacct taggcgactt ttgaacgcgc aataatggtt 4080
tctgacgtat gtgcttagct cattaaactc cagaaacccg cggctgagtg gctccttcaa 4140
cgttgcggtt ctgtcagttc caaacgtaaa acggcttgtc ccgcgtcatc ggcgggggtc 4200
ataacgtgac tcccttaatt ctccgctcat gatcagattg tcgtttcccg ccttcagttt 4260
aaactatcag tgtttgacag gatcctgctt ggtaataatt gtcattagat tgtttttatg 4320
catagatgca ctcgaaatca gccaatttta gacaagtatc aaacggatgt taattcagta 4380
cattaaagac gtccgcaatg tgttattaag ttgtctaagc gtcaatttgt ttacaccaca 4440
atatatcctg ccaccagcca gccaacagct ccccgaccgg cagctcggca caaaatcacc 4500
acgcgttacc accacgccgg ccggccgcat ggtgttgacc gtgttcgccg gcattgccga 4560
gttcgagcgt tccctaatca tcgaccgcac ccggagcggg cgcgaggccg ccaaggcccg 4620
aggcgtgaag tttggccccc gccctaccct caccccggca cagatcgcgc acgcccgcga 4680
gctgatcgac caggaaggcc gcaccgtgaa agaggcggct gcactgcttg gcgtgcatcg 4740
ctcgaccctg taccgcgcac ttgagcgcag cgaggaagtg acgcccaccg aggccaggcg 4800
gcgcggtgcc ttccgtgagg acgcattgac cgaggccgac gccctggcgg ccgccgagaa 4860
tgaacgccaa gaggaacaag catgaaaccg caccaggacg gccaggacga accgtttttc 4920
attaccgaag agatcgaggc ggagatgatc gcggccgggt acgtgttcga gccgcccgcg 4980
cacgtctcaa ccgtgcggct gcatgaaatc ctggccggtt tgtctgatgc caagctggcg 5040
gcctggccgg ccagcttggc cgctgaagaa accgagcgcc gccgtctaaa aggtgatgt 5100
gtatttgagt aaaacagctt gcgtcatgcg gtcgctgcgt atatgatgcg atgagtaaat 5160
aaacaaatac gcaaggggaa cgcatgaagg ttatcgctgt acttaaccag aaaggcgggt 5220
caggcaagac gaccatcgca acccatctag cccgcgccct gcaactcgcc ggggccgatg 5280
ttctgttagt cgattccgat ccccagggca gtgcccgcga ttgggcggcc gtgcgggaag 5340
atcaaccgct aaccgttgtc ggcatcgacc gcccgacgat tgaccgcgac gtgaaggcca 5400
tcggccggcg cgacttcgta gtgatcgacg gagcgcccca ggcggcggac ttggctgtgt 5460
ccgcgatcaa ggcagccgac ttcgtgctga ttccggtgca gccaagccct tacgacatat 5520
gggccaccgc cgacctggtg gagctggtta agcagcgcat tgaggtcacg gatggaaggc 5580
tacaagcggc ctttgtcgtg tcgcgggcga tcaaaggcac gcgcatcggc ggtgaggttg 5640
ccgaggcgct ggccgggtac gagctgccca ttcttgagtc ccgtatcacg cagcgcgtga 5700
gctacccagg cactgccgcc gccggcacaa ccgttcttga atcagaaccc gagggcgacg 5760
ctgcccgcga ggtccaggcg ctggccgctg aaattaaatc aaaactcatt tgagttaatg 5820
aggtaaagag aaaatgagca aaagcacaaa cacgctaagt gccggccgtc cgagcgcacg 5880
cagcagcaag gctgcaacgt tggccagcct ggcagacacg ccagccatga agcgggtcaa 5940
ctttcagttg ccggcggagg atcacaccaa gctgaagatg tacgcggtac gccaaggcaa 6000
gaccattacc gagctgctat ctgaatacat cgcgcagcta ccagagtaaa tgagcaaatg 6060
aataaatgag tagatgaatt ttagcggcta aaggaggcgg catggaaaat caagaacaac 6120
caggcaccga cgccgtggaa tgccccatgt gtggaggaac gggcggttgg ccaggcgtaa 6180
gcggctgggt tgtctgccgg ccctgcaatg gcactggaac ccccaagccc gaggaatcgg 6240
cgtgagcggt cgcaaaccat ccggcccggt acaaatcggc gcggcgctgg gtgatgacct 6300
ggtggagaag ttgaaggccg cgcaggccgc ccagcggcaa cgcatcgagg cagaagcacg 6360
ccccggtgaa tcgtggcaag cggccgctga tcgaatccgc aaagaatccc ggcaaccgcc 6420
ggcagccggt gcgccgtcga ttaggaagcc gcccaagggc gacgagcaac cagatttttt 6480
cgttccgatg ctctatgacg tgggcacccg cgatagtcgc agcatcatgg acgtggccgt 6540
tttccgtctg tcgaagcgtg accgacgagc tggcgaggtg atccgctacg agcttccaga 6600
cgggcacgta gaggtttccg cagggccggc cggcatggcc agtgtgtggg attacgacct 6660

ggtactgatg gcggtttccc atctaaccga atccatgaac cgataccggg aagggaaggg 6720
agacaagccc ggccgcgtgt tccgtccaca cgttgcggac gtactcaagt tctgccggcg 6780
agccgatggc ggaaagcaga aagacgacct ggtagaaacc tgcattcggt taaacaccac 6840
gcacgttgcc atgcagcgta cgaagaaggc caagaacggc cgcctggtga cggtatccga 6900
gggtgaagcc ttgattagcc gctacaagat cgtaaagagc gaaaccgggc ggccggagta 6960
catcgagatc gagctagctg attggatgta ccgcgagatc acagaaggca agaacccgga 7020
cgtgctgacg gttcaccccg attacttttt gatcgatccc ggcatcggcc gttttctcta 7080
ccgcctggca cgccgcgccg caggcaaggc agaagccaga tggttgttca agacgatcta 7140
cgaacgcagt ggcagcgccg gagagttcaa gaagttctgt ttcaccgtgc gcaagctgat 7200
cgggtcaaat gacctgccgg agtacgattt gaaggaggag gcggggcagg ctggcccgat 7260
cctagtcatg cgctaccgca acctgatcga gggcgaagca tccgccggtt cctaatgtac 7320
ggagcagatg ctagggcaaa ttgccctagc aggggaaaaa ggtcgaaaag gtctctttcc 7380
tgtggatagc acgtacattg ggaacccaaa gccgtacatt gggaaccgga acccgtacat 7440
tgggaaccca aagccgtaca ttgggaaccg gtcacacatg taagtgactg atataaaaga 7500
gaaaaaaggc gatttttccg cctaaaactc tttaaaactt attaaaactc ttaaaacccg 7560
cctggcctgt gcataactgt ctggccagcg cacagccgaa gagctgcaaa aagcgcctac 7620
ccttcggtcg ctgcgctccc tacgccccgc cgcttcgcgt cggcctatcg cggccgctgg 7680
ccgctcaaaa atggctggcc tacggccagg caatctacca gggcgcggac aagccgcgcc 7740
gtcgccactc gaccgccggc gcccacatca aggcaccctg cctcgcgcgt ttcggtgatg 7800
acggtgaaaa cctctgacac atgcagctcc cggagacggt cacagcttgt ctgtaagcgg 7860
atgccgggag cagacaagcc cgtcagggcg cgtcagcggg tgttggcggg tgtcggggcg 7920
cagccatgac ccagtcacgt agcgatagcg gagtgtatac tggcttaact atgcggcatc 7980
agagcagatt gtactgagag tgcaccatat gcggtgtgaa ataccgcaca gatgcgtaag 8040
gagaaaatac cgcatcaggc gctcttccgc ttcctcgctc actgactcgc tgcgctcggt 8100
cgttcggctg cggcgagcgg tatcagctca ctcaaaggcg gtaatacggt tatccacaga 8160
atcaggggat aacgcaggaa agaacatgtg agcaaaaggc cagcaaaagg ccaggaaccg 8220
taaaaaggcc gcgttgctgg cgtttttcca taggctccgc ccccctgacg agcatcacaa 8280
aaatcgacgc tcaagtcaga ggtggcgaaa cccgacagga ctataaagat accaggcgtt 8340
tccccctgga agctccctcg tgcgctctcc tgttccgacc ctgccgctta ccggatacct 8400
gtccgccttt ctcccttcgg gaagcgtggc gctttctcat agctcacgct gtaggtatct 8460
cagttcggtg taggtcgttc gctccaagct gggctgtgtg cacgaacccc ccgttcagcc 8520
cgaccgctgc gccttatccg gtaactatcg tcttgagtcc aacccggtaa gacacgactt 8580
atcgccactg gcagcagcca ctggtaacag gattagcaga gcgaggtatg taggcggtgc 8640
tacagagttc ttgaagtggt ggcctaacta cggctacact agaaggacag tatttggtat 8700
ctgcgctctg ctgaagccag ttaccttcgg aaaaagagtt ggtagctctt gatccggcaa 8760
acaaaccacc gctggtagcg gtggtttttt tgtttgcaag cagcagatta cgcgcagaaa 8820
aaaaggatct caagaagatc ctttgatctt ttctacgggg tctgacgctc agtggaacga 8880
aaactcacgt taagggattt tggtcatgca tgatatatct cccaatttgt gtagggctta 8940
ttatgcacgc ttaaaaataa taaaagcaga cttgacctga tagtttggct gtgagcaatt 9000
atgtgcttag tgcatctaac gcttgagtta agccgcgccg cgaagcggcg tcggcttgaa 9060
cgaatttcta gctagacatt atttgccgac taccttggtg atctcgcctt tcacgtagtg 9120
gacaaattct tccaactgat ctgcgcgcga ggccaagcga tcttcttctt gtccaagata 9180
agcctgtcta gcttcaagta tgacgggctg atactgggcc ggcaggcgct ccattgccca 9240
gtcggcagcg acatccttcg gcgcgatttt gccggttact gcgctgtacc aaatgcggga 9300
caacgtaagc actacatttc gctcatcgcc agcccagtcg ggcggcgagt tccatagcgt 9360
taaggtttca tttagcgcct caaatagatc ctgttcagga accggatcaa agagttcctc 9420
cgccgctgga cctaccaagg caacgctatg ttctcttgct tttgtcagca agatagccag 9480
atcaatgtcg atcgtggctg gctcgaagat acctgcaaga atgtcattgc gctgccattc 9540
tccaaattgc agttcgcgct tagctggata acgccacgga atgatgtcgt cgtgcacaac 9600
aatggtgact tctacagcgc ggagaatctc gctctctcca ggggaagccg aagtttccaa 9660
aaggtcgttg atcaaagctc gccgcgttgt ttcatcaagc cttacggtca ccgtaaccag 9720
caaatcaata tcactgtgtg gcttcaggcc gccatccact gcggagccgt acaaatgtac 9780
ggccagcaac gtcggttcga gatggcgctc gatgacgcca actacctctg atagttgagt 9840
cgatacttcg gcgatcaccg cttcccccat gatgtttaac tttgttttag ggcgactgcc 9900
ctgctgcgta acatcgttgc tgctccataa catcaaacat cgacccacgg cgtaacgcgc 9960
ttgctgcttg gatgcccgag gcatagactg taccccaaaa aaacagtcat aacaagccat 10020
gaaaaccgcc actgcg 10036

<210> 76
<211> 10036
<212> DNA
<213> Künstliche Sequenz

<220>
<223> Beschreibung der künstlichen Sequenz: transgenic expression vector pSUN3NIT_HvRacB_as for expression of barley RacB anti sense RNA fragment

<400> 76

ttccatggac atacaaatgg acgaacggat aaaccttttc acgccctttt aaatatccga 60
ttattctaat aaacgctctt ttctcttagg tttacccgcc aatatatcct gtcaaacact 120
gatagtttaa actgaaggcg ggaaacgaca atcagatcta gtaggaaaca gctatgacca 180
tgattacgcc aagcttgcat gcctgcaggt cgactctaga ggatccccca tcaagatctt 240
ggtgatgtag caagagctaa gttgtacttc gatycggttg gacattactc gagaccagat 300
gttttacact tgaccgtaaa tgagcacccg aagaaaccgg taacattcat ttcgaaggta 360
gagaaagcgg aagatgactc aaacaagtaa tcggttgtga ttcgtcagtt catgtcactc 420
ctatgaagga gtcaagttca aaatgttatg ttgagtttca aacttttatg ctaacttttt 480
tttcttaatt ttcgttaata atggaagaga accaattctc ttgtatctaa agattatcca 540
tctatcatcc aatttgagtg ttcaattctg gatgttgtgt taccctacat tctacaacca 600
tgtagccaat tattatgaat ctggctttga tttcagttgt gttcttttct tttttctttt 660
gcatatttgc atttagaatg tttaataatt aagttactgt atttccacat acattagttc 720
caagaatata catatattaa tttatttttc ttaaaaatgt tttggaatga ctaatattga 780
caacgaaaat agaagctatg ctaaaccatt acgtatatgt gacttcacat gttgttgttt 840
tacattccct atatatatgg atggctgtca caatcagaaa cgtgatcgaa aaaagacaaa 900
cagtgtttgc ataaaaagac tatttcgttt cattgacaat ttgtgtttat ttgtaaagaa 960
aagtggcaaa gtggaatttg agttcctgca agtaagaaag atgaaataaa agacttgagt 1020
gtgtgttttt ttcttttatc tgaaagctgc aatgaaatat tcctaccaag cccgtttgat 1080
tattaattgg ggtttggttt tcttgatgcg aactaattgg ttatataaga aactatacaa 1140
tccatgttaa ttcaaaaatt ttgatttctc ttgtaggaat atgatttact atatgagact 1200
ttcttttcgc caataatagt aaatccaaag atatttgacc ggaccaaaac acattgatct 1260
atttttagt ttatttaatc cagtttctct gagataattc attaaggaaa acttagtatt 1320
aacccatcct aagattaaat aggagccaaa ctcacatttc aaatattaaa taacataaaa 1380
tggatttaaa aaatctatac gtcaaatttt atttatgaca tttcttattt aaatttatat 1440
ttaatgaaat acagctaaga caaaccaaaa aaaaaatact ttctaagtgg tccaaaacat 1500
caattccgtt caatattatt aggtagaatc gtacgaccaa aaaaggtagg ttaatacgaa 1560
attacaaaca tatctatata catagtatat attattacct attatgagga atcaaaatgc 1620
atcaaatatg gatttaagga atccataaaa gaataaattc tacggaaaaa aaaaaaagaa 1680
taaattcttt taagtttta atttgttttt tatttggtag ttctccattt tgttttattt 1740
cgtttggatt tattgtgtcc aaatactttg taaaccaccg ttgtaattct taaacggggt 1800
tttcacttct tttttatatt cagacataaa gcatcggctg gtttaatcaa tcaatagatt 1860
ttatttttct tctcaattat tagtaggttt gatgtgaact ttacaaaaaa aacaaaaaca 1920
aatcaatgca gagaaaagaa accacgtggg ctagtcccac cttgtttcat ttccaccaca 1980
ggttcgatct tcgttaccgt ctccaatagg aaaataaacg tgaccacaaa aaaaaaacaa 2040
aaaaaaagtc tatatattgc ttctctcaag tctctgagtg tcatgaacca aagtaaaaaa 2100
caaagactcg agtggatccc cggtcacaaa atggagcacg ccccctctg caccttttc 2160
ttcttcttcg ccttaggcgg ctgcagcaca acctttatcg ccgcatcgaa gacgcccttc 2220
acgtttagtt gggtcttcga gctgcattcg atgtagtatg gagcgcctat ttgctttctt 2280
agctcctctc cctgagcagt agtgataggg acagcaccag gatggtccac aaagaactgc 2340
ttgtcgtctc gaagatcaag ctttgtccct acgagaataa ttggcacacc aggtgcataa 2400
tgcttcagtt caggtatcca cttcttcgaa acattctcat agctggcctt actgatcagt 2460
gagaaagcca gaagaaaaac atcagctcca cgatagctca gtggtctcag tctgttgtaa 2520
tcctcttgac ctgcagtgtc ccagaggccg aggttgacag tattaccatc aaccacaacg 2580
ttggcactga agttatcaaa cactgtcgga acatagtcgg tggggaaggt gttggaggtg 2640
taggagatga gcatgcaggt cttgccgacg gcgccgtccc cgaccgtgac gcactttatg 2700
aacctggacg cgctcatcgg atccatcccg cggccatggc ggcaattcac tggccgtcgt 2760
tttacaacga ctcagagctt gacaggaggc ccgatctagt aacatagatg acaccgcgcg 2820

cgataattta tcctagtttg cgcgctatat tttgttttct atcgcgtatt aaatgtataa 2880
ttgcgggact ctaatcataa aaacccatct cataaataac gtcatgcatt acatgttaat 2940
tattacatgc ttaacgtaat tcaacagaaa ttatatgata atcatcgcaa gaccggcaac 3000
aggattcaat cttaagaaac tttattgcca aatgtttgaa cgatcgggga tcatccgggt 3060
ctgtggcggg aactccacga aaatatccga acgcagcaag atctagagct tgggtcccgc 3120
tcagaagaac tcgtcaagaa ggcgatagaa ggcgatgcgc tgcgaatcgg gagcggcgat 3180
accgtaaagc acgaggaagc ggtcagccca ttcgccgcca agctcttcag caatatcacg 3240
ggtagccaac gctatgtcct gatagcggtc cgccacaccc agccggccac agtcgatgaa 3300
tccagaaaag cggccatttt ccaccatgat attcggcaag caggcatcgc catgggtcac 3360
gacgagatcc tcgccgtcgg gcatgcgcgc cttgagcctg gcgaacagtt cggctggcgc 3420
gagcccctga tgctcttcgt ccagatcatc ctgatcgaca agaccggctt ccatccgagt 3480
acgtgctcgc tcgatgcgat gtttcgcttg gtggtcgaat gggcaggtag ccggatcaag 3540
cgtatgcagc cgccgcattg catcagccat gatggatact ttctcggcag gagcaaggtg 3600
agatgacagg agatcctgcc ccggcacttc gcccaatagc agccagtccc ttcccgcttc 3660
agtgacaacg tcgagcacag ctgcgcaagg aacgcccgtc gtggccagcc acgatagccg 3720
cgctgcctcg tcctgcagtt cattcagggc accggacagg tcggtcttga caaaaagaac 3780
cgggcgcccc tgcgctgaca gccggaacac ggcggcatca gagcagccga ttgtctgttg 3840
tgcccagtca tagccgaata gcctctccac ccaagcggcc ggagaacctg cgtgcaatcc 3900
atcttgttca atcatgcgaa acgatccaga tccggtgcag attatttgga ttgagagtga 3960
atatgagact ctaattggat accgagggga atttatggaa cgtcagtgga gcatttttga 4020
caagaaatat ttgctagctg atagtgacct taggcgactt ttgaacgcgc aataatggtt 4080
tctgacgtat gtgcttagct cattaaactc cagaaacccg cggctgagtg gctccttcaa 4140
cgttgcggtt ctgtcagttc caaacgtaaa acggcttgtc ccgcgtcatc ggcgggggtc 4200
ataacgtgac tcccttaatt ctccgctcat gatcagattg tcgtttcccg ccttcagttt 4260
aaactatcag tgtttgacag gatcctgctt ggtaataatt gtcattagat tgtttttatg 4320
catagatgca ctcgaaatca gccaatttta gacaagtatc aaacggatgt taattcagta 4380
cattaaagac gtccgcaatg tgttattaag ttgtctaagc gtcaatttgt ttacaccaca 4440
atatatcctg ccaccagcca gccaacagct ccccgaccgg cagctcggca caaaatcacc 4500
acgcgttacc accacgccgg ccggccgcat ggtgttgacc gtgttcgccg gcattgccga 4560
gttcgagcgt tccctaatca tcgaccgcac ccggagcggg cgcgaggccg ccaaggcccg 4620
aggcgtgaag tttggccccc gccctaccct caccccggca cagatcgcgc acgcccgcga 4680
gctgatcgac caggaaggcc gcaccgtgaa agaggcggct gcactgcttg gcgtgcatcg 4740
ctcgaccctg taccgcgcac ttgagcgcag cgaggaagtg acgcccaccg aggccaggcg 4800
gcgcggtgcc ttccgtgagg acgcattgac cgaggccgac gccctggcgg ccgccgagaa 4860
tgaacgccaa gaggaacaag catgaaaccg caccaggacg gccaggacga accgtttttc 4920
attaccgaag agatcgaggc ggagatgatc gcggccgggt acgtgttcga gccgcccgcg 4980
cacgtctcaa ccgtgcggct gcatgaaatc ctggccggtt tgtctgatgc caagctggcg 5040
gcctggccgg ccagcttggc cgctgaagaa accgagcgcc gccgtctaaa aggtgatgt 5100
gtatttgagt aaaacagctt gcgtcatgcg gtcgctgcgt atatgatgcg atgagtaaat 5160
aaacaaatac gcaaggggaa cgcatgaagg ttatcgctgt acttaaccag aaaggcgggt 5220
caggcaagac gaccatcgca acccatctag cccgcgccct gcaactcgcc ggggccgatg 5280
ttctgttagt cgattccgat ccccagggca gtgcccgcga ttgggcggcc gtgcgggaag 5340
atcaaccgct aaccgttgtc ggcatcgacc gcccgacgat tgaccgcgac gtgaaggcca 5400
tcggccggcg cgacttcgta gtgatcgacg gagcgcccca ggcggcggac ttggctgtgt 5460
ccgcgatcaa ggcagccgac ttcgtgctga ttccggtgca gccaagccct tacgacatat 5520
gggccaccgc cgacctggtg gagctggtta agcagcgcat tgaggtcacg gatggaaggc 5580
tacaagcggc ctttgtcgtg tcgcgggcga tcaaaggcac gcgcatcggc ggtgaggttg 5640
ccgaggcgct ggccgggtac gagctgccca ttcttgagtc ccgtatcacg cagcgcgtga 5700
gctacccagg cactgccgcc gccggcacaa ccgttcttga atcagaaccc gagggcgacg 5760
ctgcccgcga ggtccaggcg ctggccgctg aaattaaatc aaaactcatt tgagttaatg 5820
aggtaaagag aaaatgagca aaagcacaaa cacgctaagt gccggccgtc cgagcgcacg 5880
cagcagcaag gctgcaacgt tggccagcct ggcagacacg ccagccatga agcgggtcaa 5940
ctttcagttg ccggcggagg atcacaccaa gctgaagatg tacgcggtac gccaaggcaa 6000
gaccattacc gagctgctat ctgaatacat cgcgcagcta ccagagtaaa tgagcaaatg 6060
aataaatgag tagatgaatt ttagcggcta aaggaggcgg catggaaaat caagaacaac 6120
caggcaccga cgccgtggaa tgccccatgt gtggaggaac gggcggttgg ccaggcgtaa 6180
gcggctgggt tgtctgccgg ccctgcaatg gcactggaac ccccaagccc gaggaatcgg 6240

cgtgagcggt cgcaaaccat ccggcccggt acaaatcggc gcggcgctgg gtgatgacct 6300
ggtggagaag ttgaaggccg cgcaggccgc ccagcggcaa cgcatcgagg cagaagcacg 6360
ccccggtgaa tcgtggcaag cggccgctga tcgaatccgc aaagaatccc ggcaaccgcc 6420
ggcagccggt gcgccgtcga ttaggaagcc gcccaagggc gacgagcaac cagatttttt 6480
cgttccgatg ctctatgacg tgggcacccg cgatagtcgc agcatcatgg acgtggccgt 6540
tttccgtctg tcgaagcgtg accgacgagc tggcgaggtg atccgctacg agcttccaga 6600
cgggcacgta gaggtttccg cagggccggc cggcatggcc agtgtgtggg attacgacct 6660
ggtactgatg gcggtttccc atctaaccga atccatgaac cgataccggg aagggaaggg 6720
agacaagccc ggccgcgtgt tccgtccaca cgttgcggac gtactcaagt tctgccggcg 6780
agccgatggc ggaaagcaga aagacgacct ggtagaaacc tgcattcggt taaacaccac 6840
gcacgttgcc atgcagcgta cgaagaaggc caagaacggc cgcctggtga cggtatccga 6900
gggtgaagcc ttgattagcc gctacaagat cgtaaagagc gaaaccgggc ggccggagta 6960
catcgagatc gagctagctg attggatgta ccgcgagatc acagaaggca agaacccgga 7020
cgtgctgacg gttcaccccg attacttttt gatcgatccc ggcatcggcc gttttctcta 7080
ccgcctggca cgccgcgccg caggcaaggc agaagccaga tggttgttca agacgatcta 7140
cgaacgcagt ggcagcgccg gagagttcaa gaagttctgt ttcaccgtgc gcaagctgat 7200
cgggtcaaat gacctgccgg agtacgattt gaaggaggag gcggggcagg ctggcccgat 7260
cctagtcatg cgctaccgca acctgatcga gggcgaagca tccgccggtt cctaatgtac 7320
ggagcagatg ctagggcaaa ttgccctagc aggggaaaaa ggtcgaaaag gtctctttcc 7380
tgtggatagc acgtacattg ggaacccaaa gccgtacatt gggaaccgga acccgtacat 7440
tgggaaccca aagccgtaca ttgggaaccg gtcacacatg taagtgactg atataaaaga 7500
gaaaaaaggc gatttttccg cctaaaactc tttaaaactt attaaaactc ttaaaacccg 7560
cctggcctgt gcataactgt ctggccagcg cacagccgaa gagctgcaaa aagcgcctac 7620
ccttcggtcg ctgcgctccc tacgccccgc cgcttcgcgt cggcctatcg cggccgctgg 7680
ccgctcaaaa atggctggcc tacggccagg caatctacca gggcgcggac aagccgcgcc 7740
gtcgccactc gaccgccggc gcccacatca aggcaccctg cctcgcgcgt ttcggtgatg 7800
acggtgaaaa cctctgacac atgcagctcc cggagacggt cacagcttgt ctgtaagcgg 7860
atgccgggag cagacaagcc cgtcagggcg cgtcagcggg tgttggcggg tgtcggggcg 7920
cagccatgac ccagtcacgt agcgatagcg gagtgtatac tggcttaact atgcggcatc 7980
agagcagatt gtactgagag tgcaccatat gcggtgtgaa ataccgcaca gatgcgtaag 8040
gagaaaatac cgcatcaggc gctcttccgc ttcctcgctc actgactcgc tgcgctcggt 8100
cgttcggctg cggcgagcgg tatcagctca ctcaaaggcg gtaatacggt tatccacaga 8160
atcaggggat aacgcaggaa agaacatgtg agcaaaaggc cagcaaaagg ccaggaaccg 8220
taaaaaggcc gcgttgctgg cgtttttcca taggctccgc ccccctgacg agcatcacaa 8280
aaatcgacgc tcaagtcaga ggtggcgaaa cccgacagga ctataaagat accaggcgtt 8340
tccccctgga agctccctcg tgcgctctcc tgttccgacc ctgccgctta ccggatacct 8400
gtccgccttt ctcccttcgg gaagcgtggc gctttctcat agctcacgct gtaggtatct 8460
cagttcggtg taggtcgttc gctccaagct gggctgtgtg cacgaacccc ccgttcagcc 8520
cgaccgctgc gccttatccg gtaactatcg tcttgagtcc aacccggtaa gacacgactt 8580
atcgccactg gcagcagcca ctggtaacag gattagcaga gcgaggtatg taggcggtgc 8640
tacagagttc ttgaagtggt ggcctaacta cggctacact agaaggacag tatttggtat 8700
ctgcgctctg ctgaagccag ttaccttcgg aaaaagagtt ggtagctctt gatccggcaa 8760
acaaaccacc gctggtagcg gtggtttttt tgtttgcaag cagcagatta cgcgcagaaa 8820
aaaaggatct caagaagatc ctttgatctt ttctacgggg tctgacgctc agtggaacga 8880
aaactcacgt taagggattt tggtcatgca tgatatatct cccaatttgt gtagggctta 8940
ttatgcacgc ttaaaaataa taaaagcaga cttgacctga tagtttggct gtgagcaatt 9000
atgtgcttag tgcatctaac gcttgagtta agccgcgccg cgaagcggcg tcggcttgaa 9060
cgaatttcta gctagacatt atttgccgac taccttggtg atctcgcctt tcacgtagtg 9120
gacaaattct tccaactgat ctgcgcgcga ggccaagcga tcttcttctt gtccaagata 9180
agcctgtcta gcttcaagta tgacgggctg atactgggcc ggcaggcgct ccattgccca 9240
gtcggcagcg acatccttcg gcgcgatttt gccggttact gcgctgtacc aaatgcggga 9300
caacgtaagc actacatttc gctcatcgcc agcccagtcg ggcggcgagt tccatagcgt 9360
taaggtttca tttagcgcct caaatagatc ctgttcagga accggatcaa agagttcctc 9420
cgccgctgga cctaccaagg caacgctatg ttctcttgct tttgtcagca agatagccag 9480
atcaatgtcg atcgtggctg gctcgaagat acctgcaaga atgtcattgc gctgccattc 9540
tccaaattgc agttcgcgct tagctggata acgccacgga atgatgtcgt cgtgcacaac 9600
aatggtgact tctacagcgc ggagaatctc gctctctcca ggggaagccg aagtttccaa 9660

```
aaggtcgttg atcaaagctc gccgcgttgt ttcatcaagc cttacggtca ccgtaaccag 9720
caaatcaata tcactgtgtg gcttcaggcc gccatccact gcggagccgt acaaatgtac 9780
ggccagcaac gtcggttcga gatggcgctc gatgacgcca actacctctg atagttgagt 9840
cgatacttcg gcgatcaccg cttcccccat gatgtttaac tttgttttag ggcgactgcc 9900
ctgctgcgta acatcgttgc tgctccataa catcaaacat cgacccacgg cgtaacgcgc 9960
ttgctgcttg gatgcccgag gcatagactg taccccaaaa aaacagtcat aacaagccat 10020
gaaaaccgcc actgcg                                                 10036
```

**Beschreibung**

Die Erfindung betrifft Verfahren zur Erzeugung oder Erhöhung einer Pathogenresistenz in Pflanzen durch Verminderung der Expression eines RacB-Protein oder eines funktionellen Äquivalentes desselben, sowie mit diesem Verfahren erhaltene Organismen, Zellen, Zellkulturen, Gewebe, Pflanzen und Vermehrungsgut davon.

Ziel biotechnologischer Arbeiten an Pflanzen ist die Herstellung von Pflanzen mit vorteilhaften, neuen Eigenschaften zum Beispiel zur Steigerung der landwirtschaftlichen Produktivität, zur Qualitätssteigerung bei Nahrungsmitteln oder zur Produktion bestimmter Chemikalien oder Pharmazeutika (Dunwell JM (2000) J Exp Bot 51 Spec No:487-96) . Oft sinddie natürlichenAbwehrmechanismen der Pflanze gegen Pathogene unzureichend. Allein Pilzerkrankungen führen zu Ernteverlusten in der Höhe von vielen Milliarden US-$ jährlich. Die Einführung fremder Gene aus Pflanzen, Tieren oder mikrobiellen Quellen kann die Abwehr verstärken. Beispiele sind der Schutz gegen Insektenfrass in Tabak durch Expression von *Bacillus thuringiensis* Endotoxinen unter Kontrolle des 35 S CaMV Promoters (Vaeck et al. (1987) Nature 328:33-37) oder der Schutz des Tabaks gegen Pilzbefall durch Expression einer Chitinase aus der Bohne unter Kontrolle des CaMV Promoters (Broglie et al. (1991) Science 254:1194-1197). Die meisten der beschriebenen Ansätze gewähren jedoch nur eine Resistenz gegen ein einzelnes Pathogen oder gegen ein schmales Spektrum von Pathogenen.

Es gibt nur wenige Ansätze, die Pflanzen eine Resistenz gegen ein breiteres Spektrum von Pathogenen, vor allem Pilzpathogene, verleihen. Die systemische erworbene Resistenz ("systemic acquired resistance"; SAR) - ein Abwehrmechanismus bei verschiedenen Pflanze/Pathogen-Interaktionen - kann durch Applikation von endogenen Botenstoffen wie Jasmonat (JA) oder Salicylsäure (SA) vermittelt werden (Ward, et al. (1991) Plant Cell 3:1085-1094; Uknes, et al. (1992) Plant Cell 4(6):645-656). Ähnliche Effekte können auch durch synthetische Verbindungen wie 2,6-Dichlorisonikotinsäure (INA) oder Benzo(1,2,3)thiadiazol-7-thiocarbonsäure-S-methylester (BTH; Bion®) (Friedrich et al. (1996) Plant J 10(1):61-70; Lawton et al. (1996) Plant J. 10:71-82) bewirkt werden. Auch die Expression der im Rahmen eines SAR hochregulierten "pathogenesis related" (PR) Proteine vermag zum Teil eine Pathogenresistenz zu bewirken.

In Gerste ist bereits seit längerem der Mlo-Locus als negativer Regulator der Pathogenabwehr beschrieben. Der Verlust oder Funktionsverlust ("loss-of-function") des Mlo-Gens bedingt eine erhöhte und vor allem rassen-unspezifische Resistenz beispielsweise gegen zahlreiche Arten von Mehltau (Büschges R et al. (1997) Cell 88:695-705; Jorgensen JH (1977) Euphytica 26:55-62; Lyngkjaer MF et al. (1995) Plant Pathol 44:786-790). Der Mlo-Phänotyp wird rezessiv vererbt, was ebenfalls für eine Funktion als Suszeptibilitätsgen spricht. Durch klassische Züchtung erhaltene Mlo-defiziente Gerstensorten werden bereits breit in der Landwirtschaft verwendet. Vermutlich aufgrund der Rezessivität hat sich trotz eines intensiven Anbaus diese Resistenz als außerordentlich dauerhaft erwiesen. Durchbrechungen der Resistenz sind bislang nicht aufgetreten. Mlo-ähnliche Resistenzen in anderen Pflanzen v.a. in Getreidearten sind nicht beschrieben, obgleich auch Weizen, Roggen und andere Getreide von vergleichbaren Mehltau-Pathogenen befallen werden. Ursache bei Weizen kann zum Beispiel das Vorliegen eines hexaploiden Genoms sein, was die Identifizierung von Mutanten, bei denen jede der sechs Genkopien inaktiviert wurde, extrem erschwert.

Das Mlo-Gen wurde erst kürzlich kloniert (Büschges R et al. (1997) Cell 88:695-705; WO 98/04586; Schulze-Lefert P, Vogel J (2000) Trends Plant Sci. 5:343-348). Infolge verschiedener Homologe aus anderen Getreidearten isoliert. Verschiedene Verfahren unter Verwendung dieser Gene zum Erzielen einer Pathogenresistenz sind beschrieben (WO 98/04586; WO 00/01722; WO 99/47552).

Die Mlo-bedingte Resistenz einer Pflanze gegen Mehltau-Pathogene äußert sich in zwei wesentlichen Ereignissen, die beide eine Penetrationsresistenz bewirken: Eine Papillenbildung ("cell wall apposition"; CWA) unterhalb des Penetrationsortes des Pathogens, der epidermalen Zellwand. Die Ausbreitung des Pilzpathogens bleibt fast ausschließlich auf diese subzellulare Struktur beschränkt (Jorgensen JH und Mortensen K (1977) Phytopathology 67:678-685; Freialdenhoven A et al. (1996) Plant Cell 8:5-14). Bedingt wird diese Reaktion durch die für die Mlo-Wirkung erforderlichen Gene Ror1 und Ror2 (Peterhänsel C et al. (1997) 9:1397-1409). Nachteilig bei der Mlo-bedingten Pathogenresistenz ist, dass Mlo-defiziente Pflanzen - auch in Abwesenheit eines Pathogens - einen Abwehrmechanismus initiieren, der sich beispielsweise in einem spontanen Absterben von Blattzellen äußert (Wolter M et al. (1993).Mol Gen Genet 239: 122-128). Nachteilig ist ferner, dass die Mlo-defizienten Genotypen eine Hypersuszeptibilität gegen hemibiotrophe Pathogene wie *Magnaporte grisea (M. grisea)* sowie *Cochliobolus sativus* (Bipolaris sorokiniana) zeigen (Jarosch B et al. (1999) Mol Plant Microbe Interact 12:508-514; Kumar J et al. (2001) Phytopathology 91:127-133). Das Mlo-Gen scheint also ein negativer Regulator des Zelltods zu sein. Ursache ist ebenfalls vermutlich die Induktion von Zelltod bei Abwesenheit des Mlo-Gens, was die Suszeptibilität gegenüber diesen eher nekrotrophen Pathogenen steigert. Diese ambivalente Wirkung, die die biotechnologische Anwendung von Mlo limitiert, beruht vermutlich darauf, dass nekrotrophe Pilze die stärkere HR der Mlo-defizienten Wirtspflanze für ihren Infektionsprozess ausnutzen können. Wünschenswert wäre eine der Mlo-Defizienz vergleichbare Resistenz, der jedoch das Merkmal der Zelltodinduktion fehlt.

Die Proteine Rho, Rac und Cdc42 sind Mitglieder der Familie der kleinen GTP-(Guanosintriphosphat) Bindeproteine und regulieren als "molekulare Schalter" zahlreiche intrazelluläre Prozesse, sowohl in pflanzlichen als auch in tierischen Organismen. Sie haben als Bausteine der Signaltransduktion eine wichtige Rolle bei der Umsetzung extrazellulärer Stimuli. Sie regulieren beispielsweise die NADPH Oxidase und damit die Freisetzung reaktiver Sauerstoffmoleküle ("oxidative burst"). Tierisches oder menschliches Rac1 ist essentiell für die Bildung des aktiven NADPH Oxidasekomplexes, der wiederum wichtig für die Bildung von Superoxid ist, und so einen Beitrag zur Pathogenabwehr liefert (Irani K und Goldschmidt-Clermont PJ (1998) Biochem Pharmacol 55: 1339-1346). Die Funktion in der Pathogenabwehr ist weitgehend analog in Pflanzen und in Tieren (Kwong et al. (1995) J Biol Chem 270(34): 19868-19872; Dusi et al. (1996) Biochem J 314:409-412; Diekmann et al. (1994) Science 265:531-533; Purgin et al. (1997) The Plant Cell 9:2077-2091; Kleinberg et al. (1994) Biochemistry 33:2490-2495; Prigmore et al. (1995) Journal of Biol Chem 27(18): 10717-10722; Irani et al. (1997) Science 275:1649-1652; Low et al. (1994) Advances in Molecular Genetics of Plant-Microbe Interactions 3:361-369 (1994) eds. MJ Daniels, Kluwer Acadmic Publishers, Netherlands; Mehdy et al. (1994) Plant Physiol 105: 467-472; Sundaresan et al. (1996) Biochem J 318:379-382). Darüber hinaus haben GTP-Bindeproteine eine Funktion bei der Umstrukturierung des Zytoskeletts und der Zelltransformation (Symon M. (1996) TIBS 21: 178-181), sowie bei der Aktivierung der Transkription (Hill et al. (1995) Cell 81:1159-1170; Chandra et al. (1996) Proc Natl Acad Sci USA 93:13393-13397).

In Pflanzen besteht eine größere Familie an Rac-ähnlichen Proteinen (Winge et al. (1997) Plant Mol Biol 35: 483-495), die auch als Rop-Familie bezeichnet werden (Lin et al. (1997) The Plant Cell 9:1647-1659). In Pflanzen scheinen die Rac Proteine eine Funktion in der Freisetzung reaktiver Sauerstoffmoleküle infolge eines Pathogenbefalls zu haben (Groom QJ et al. (1996) Plant J 10: 515-522; Hassanain HH et al. (2000) Biochem Biophys Res Commun 272 (3):783-788; Ono E et al. (2001) Proc Natl Acad Sci USA 98: 759-764). Rac moduliert u.a. die Zellwandarchitektur, die Signaltransduktion im Meristem und die Abwehr von Pathogenen (Valster AH et al. (2000) Trends Cell Biol 10(4): 141-146). Rac1 aus Reis vermag bei Überexpression der konstitutiv-aktiven Form eine hypersensitive Antwort ("hypersensitive response"; HR) an den Stellen eines M. grisea-Befalls zu induzieren und bewirkt so eine Pathogenresistenz. Analog bewirkt die Expression einer dominant-negative Form von Rac1 eine erhöhte Suszeptibilität gegen M. grisea (Kawasaki T et al. (1999) Proc Natl Acad Sci USA 96:10922-10926; Ono E et al. (2001) Proc Natl Acad Sci USA 98: 759-764). Diese Befunde deuten darauf hin, dass durch eine Überexpression von Rac-Proteinen in der Pflanze vorteilhafte Effekte bezüglich einer Pathogenabwehr bewirkt werden können.

WO 00/15815 beschreibt fünf Rac-Gene aus Mais. Obgleich hier sowohl Verfahren für eine Hoch- als auch Runterregulation von Rac-Proteinen beschrieben und spekulativ in den Zusammenhang mit der Erzielen einer Pathogenresistenz gebracht werden (S.55/Z.25ff.), ist die einzige technische Lehre die diese Verwendung konkret beschreibt allein auf eine Überexpression der beanspruchten Rac-Gene zum Erreichen einer Pathogenresistenz gerichtet (S.60/Z. 21ff.). Ganz eindeutig - und im Einklang mit dem im Stand der Technik beschriebenen Sachverhalt - wird hier postuliert (S.60/Z.31ff): "Demzufolge ist die Erfindung nützlich, um Pflanzen vor Pathogenen zu schützen. Sobald eine Pflanze mit einem Polynukleotid kodierend für ein Rac Polypeptid transformiert ist, wird die Expression des Polypeptides in der Pflanze eine Resistenz gegen Pflanzenpathogene bewirken." Das hinter dieser Hypothese stehende Rational (Pathogenabwehr über reaktive Sauerstoffmoleküle) wird nachfolgend dargelegt und mit zahlreichen Literaturstellen untermauert. Darüberhinaus wird keine Differenzierung zwischen den fünf beanspruchten Rac-Genen vorgenommen.

Der vorliegenden Erfindung liegt die Aufgabe zugrunde, neue Verfahren zur Pathogenabwehr in Pflanzen bereitzustellen, die eine effiziente Abwehr eines möglichst breiten Spektrum von Pathogenen in möglichst vielen verschiedene Pflanzenarten, bevorzugt den in der Landwirtschaft verwendeten Kulturpflanzen bewirken. Diese Aufgabe wird durch das erfindungsgemäße Verfahren gelöst.

Ein erster Gegenstand der Erfindung umfasst ein Verfahren zur Erzeugung oder Erhöhung der Resistenz gegen mindestens ein Pathogen in Pflanzen, dadurch gekennzeichnet, dass nachfolgende Arbeitsschritte umfasst sind

a) Verminderung der Proteinmenge, Aktivität oder Funktion eines RacB-Proteins in einer Pflanze oder einem Gewebe, Organ, Teil oder Zelle derselben und

b) Auswahl der Pflanzen, bei denen - im Unterschied oder Vergleich zur Ausgangspflanze - die Resistenz gegen mindestens ein Pathogen besteht oder erhöht ist.

Überraschenderweise zeigt das Rac-Homolog RacB aus Gerste (Hordeum vulgare) (SEQ ID NO: 1) (infolge: hvRacB) - trotz einer hohen Ähnlichkeit zu Reis-Rac1 - im Gegensatz zu diesem eine negative Kontrollfunktion bei einem Befall durch den Gersten-Mehltau *Blumeria (syn. Erysiphe) graminis* f.sp. *hordei* (Bgh): Die Verminderung der *hvRacB* Expression in der epidermalen Zelle durch einen sequenzespezifischen RNA-Interferenz Ansatz unter Verwendung doppelsträngiger *hvRacB*-dsRNA ("Gene-Silencing"), verhinderte signifikant die Haustorium-Ausbildung infolge einer *Bgh*-Infektion. Weitere Experimente zeigten (vgl. Beispiel 7), dass dieser Phänotyp nicht in einem *mlo5*-*ror1*-mutanten Genotyp - der Gerste A89 - beobachtet werden kann. Dies legt nahe, dass RacB in funktioneller Verbindung zu Mlo oder Ror1 oder beiden steht, also vermutlich in einer Signalkette wirkt.

Ähnlich wie der Funktionsverlust von Mlo vermittelt der von HvRacB eine breite Resistenz gegen verschiedene Isolate von Blumeria graminis f. sp. hordei. In transienten "Gene-Silencing"-Experimenten reduziert HvRacB die Penetrationseffizienz (Haustorien-Bildung) von Bgh um 44 % (vgl. Beispiel 7)- ein Effekt, der in seiner Stärke dem mittels Mlo-dsRNA erreichten entspricht (Schweizer et al. (2000) Plant J 24:895-903). In der Wildtyp Gerstensorte Ingrid führen ca. 60 % der Pilzpenetrationen zu einer Haustorien-Bildung, wohingegen die Penetrationsrate in BCIngrid-mlo5 nahezu 0 % beträgt. Die Gerstensorte A89 (mlo-rorl-Doppelmutante) zeigt eine Penetrationseffizienz von ca. 20 bis 35%. In keiner dieser Varianten war eine geänderte RacB Expression infolge der Bgh-Inokulation zu beobachten (vgl. Beispiel 7; Fig. 3). Die Tatsache, dass auch in pathogen-empfindlichen Wildtyp-Sorten wie Pallas oder Ingrid nur eine Penetration von ca. 50 % beobachtet werden kann, ist auf die stets vorhandene Basalresistenz zurückzuführen.

Interessanterweise verstärkt das "Gene-Silencing" von hvRacB nur die Papillenbildung, aber anscheinend nicht den spontanen Zelltod der Pflanze - im Gegensatz zu Mlo. Damit unterscheidet sich HvRacB von OsRac1, einem Reis-Homolog von Rac1 (Ono E et al. (2001) Proc Natl Acad Sci USA 98:759-764). HvRacB wirkt vorwiegend als negativer Regulator der Papillenbildung. Für die Verwendung zum Erreichen einer Pathogenresistenz in Pflanzen ist dieser Unterschied von herausragender Bedeutung. Wie bereits oben beschrieben wird die Mlo-Resistenz gegen biotrophe Pilze (z.B. Mehltaupilze) zwar auch durch eine verstärkte Papillenbildung bedingt, ist jedoch mit einer höheren Anfälligkeit gegen nekrotrophe Pilze teuer erkauft (Jarosch B et al. (1999) Mol Plant Microbe Interact 12:508-514; Kumar J et al. (2001) Phytopathology 91:127-133). Da HvRacB nur die Papillenbildung beeinflusst, kann dieses Ambivalenz-Problem umgangen werden.

RacB ist aufgrund der oben genannten Befunde als Schlüsselelement für das erfolgreiche Eindringen eines Pathogens wie Bgh in die pflanzliche Zelle zu verstehen. Das erfindungsgemäße Verfahren bietet demnach alle Vorzüge der Mlo-Defizienz, ohne zugleich deren größten Nachteil - den erhöhten spontanen Zelltod - aufzuweisen.

Darüberhinaus ist das Verfahren allen Verfahren überlegen, bei denen ein pathogen-resistenter Phänotyp durch Überexpression eines resistenzvermittelnden Proteins realisiert wird. Das Ausschalten eines Gens, lässt sich ohne Expression eines (Fremd)-Proteins realisieren. Im Idealfall wird lediglich das endogene Gen deaktiviert. Dies hat nicht zu vernachlässigende Vorteile bei der Zulassung und der Akzeptanz durch den Verbraucher, der Pflanzen mit Fremdproteinen oft mit Vorbehalt begegnet. Ganz besonders vorteilhaft ist in diesem Zusammenhang die Verwendung von induzierbaren Promotoren zur Verminderung der RacB-Proteinmenge, Aktivität oder Funktion, was beispielsweise bei Verwendung von pathogeninduzierbaren Promotoren eine Expression nur im Bedarfsfall (d.h. Pathogenbefall) ermöglicht.

Eine partielle Sequenz der Gerste RacB cDNA (HvRacB-cDNA) ist beschrieben (GenBank Acc.-No.: AJ290240), die hochkonserviert zum Reis-RacB (GenBank Acc.-No.: AF250327) und Mais RacB (GenBank Acc.-No.: AF126053) und sehr ähnlich zum Reis Rac1 ist. Mais RacB ist auch eines der fünf Rac-Gene in der oben genannten Anmeldung WO 00/15815 (Sequenz Nr. 3). Die vollständige kodierende Sequenz des HvRacB-Protein ist bislang nicht beschrieben (s. Beispiel 1). Gerste RacB hat eine Homologie von 95 % Identität zu Reis RacB und Mais RacB und ist mehr als 55 % identisch zu dem humanen RAC1 oder RAC2 (Hassanain et al. 2000, Fig. 1). HvRacB wird konstitutiv in den Primärblättern der Gerste (epidermis-spezifisch) exprimiert und wird durch einen *Bgh*-Befall nicht wesentlich in seiner Expressionshöhe beeinflusst. Insofern erfolgt die Expression in dem Gewebe, das unmittelbar mit dem Bgh-Pathogen interagiert.

Das erfindungsgemäße Verfahren kann im Prinzip auf alle Pflanzenarten angewendet werden. Bevorzugt auf solche, in denen natürlicherweise ein RacB-Protein oder ein funktionelles Äquivalent desselben exprimiert wird. Da die Funktion von RacB in engem funktionellem Zusammenhang mit dem Mlo-Gen steht und dieses in zahlreichen Pflanzen - auch in Dikotyledonen - identifiziert wurde (Devoto A et al. (1999) J Biol Chem 274(49):34993-5004), ist eine ähnlich breite Verteilung auch für RacB und seine Homologen zu vermuten. Die zu den im Rahmen dieser Erfindung offenbarten RacB Sequenzen homologen Sequenzen aus anderen Pflanzen (beispielsweise Arabidopsis thaliana) können z.B. durch Datenbanksuche oder Durchmustern von Gen-Banken - unter Verwendung der *RacB*-Sequenzen als Suchsequenz vzw. Sonde - leicht aufgefunden werden.

"Pflanze" im Rahmen der Erfindung meint alle Gattungen und Arten höherer und niedrigerer Pflanzen des Pflanzenreiches. Eingeschlossen unter dem Begriff sind die reifen Pflanzen, Saatgut, Sprossen und Keimlinge, sowie davon abgeleitete Teile, Vermehrungsgut, Pflanzenorgane, Gewebe, Protoplasten, Kallus und andere Kulturen, zum Beispiel Zellkulturen, sowie alle anderen Arten von Gruppierungen von Pflanzenzellen zu funktionellen oder strukturellen Einheiten. Reife Pflanzen meint Pflanzen zu jedem beliebigen Entwicklungsstadium jenseits des Keimlings. Keimling meint eine junge, unreife Pflanze in einem frühen Entwicklungsstadium. "Pflanze," umfasst alle einjährigen und mehrjährige, monokotyledonen und dikotyledonen Pflanzen und schließt beispielhaft jedoch nicht einschränkend solche der Gattungen Cucurbita, Rosa, Vitis, Juglans, Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersicon, Nicotiana, Solarium, Petunia, Digitalis, Majorana, Ciahorium, Helianthus, Lactuca, Bromus, Asparagus, Antirrhinum, Heterocallis, Nemesis, Pelargonium, Panieum, Pennisetum, Ranunculus, Senecio, Salpiglossis, Cucumis, Browaalia, Glycine, Pisum, Phaseolus, Lolium, Oryza, Zea, Avena, Hordeum, Secale, Triticum, Sorghum, Picea und Populus ein.

Der Begriff "Pflanze" umfasst bevorzugt monokotyle Kulturpflanzen, wie zum Beispiel Getreidearten wie Weizen, Gerste, Hirse, Roggen, Triticale, Mais, Reis, Sorghum oder Hafer sowie Zuckerrohr.

Ferner umfasst der Begriff dikotyle Kulturpflanzen, wie zum Beispiel

- Brassicacae wie Raps, Canola, Kresse, Arabidopsis, Kohlarten oder Canola, Leguminosae wie Soja, Alfalfa, Erbse, Bohnengewächsen oder Erdnuss
- Solanaceae wie Kartoffel, Tabak, Tomate, Aubergine oder Paprika, Asteraceae wie Sonnenblume, Tagetes, Salat oder Calendula,
- Cucurbitaceae wie Melone, Kürbis oder Zucchini,

sowie Lein, Baumwolle, Hanf, Klee, Spinat, Flachs, Roter Pfeffer, Möhre, Karotte, Rübe, Rettich, Zuckerrübe, Süßkartoffel, Gurke, Chicorée, Blumenkohl, Brokkoli, Spargel, Zwiebel, Knoblauch, Sellerie, Erdbeere, Himbeere, Brombeere, Ananas, Avocado, und den verschiedenen Baum-, Strauch-, Nuss- und Weinarten. Baumarten umfasst bevorzugt Pflaume, Kirsche, Pfirsich, Nektarine, Aprikose, Banane, Papaya, Mango, Apfel, Birne, Quitte.

Ferner umfasst sind Schmuckpflanzen, Nutz- oder Zierbäume, Blumen, Schnittblumen, Sträuchern oder Rasen wie beispielhaft aber nicht einschränkend die Familien der Rosaceae wie Rose, Ericaceae wie Rhododendrons und Azaleen, Euphorbiaceae wie Weihnachtssterne und Kroton, Caryophyllaceae wie Nelken, Solanaceae wie Petunien, Gesneriaceae wie das Usambaraveilchen, Balsaminaceae wie das Springkraut, Orchidaceae wie Orchideen, Iridaceae wie Gladiolen, Iris, Freesie und Krokus, Compositae wie Ringelblume, Geraniaceae wie Geranien, Liliaceae wie der Drachenbaum, Moraceae wie Ficus, Araceae wie Philodendron und andere mehr.

Im Rahmen der Erfindung sind solche Pflanzen bevorzugt, die als Nahrungs- oder Futtermittel zum Einsatz kommen, ganz besonders bevorzugt monokotyle Gattungen und Arten, wie die beschriebenen Getreidearten.

Ganz besonders bevorzugt wird das Verfahren auf monokotyle Pflanzen, am meisten bevorzugt auf Pflanzen mit landwirtschaftlicher Bedeutung wie Weizen, Hafer, Hirse, Gerste, Roggen, Mais, Reis, Buchweizen, Sorghum, Triticale, Dinkel, Leinsamen oder Zuckerrohr angewendet.

"Pathogenresistenz" meint das Vermindern oder Abschwächen von Krankheitssymptomen einer Pflanze infolge eines Befalls durch ein Pathogen. Die Symptome können vielfältiger Art sein, umfassen aber bevorzugt solche die direkt oder indirekt zu einer Beeinträchtigung Qualität der Pflanze, der Quantität des Ertrages, der Eignung zur Verwendung als Futter- oder Nahrungsmittel führen oder aber auch Aussaat, Anbau, Ernte oder Prozessierung des Erntegutes erschweren.

"Verleihen", "bestehen", "erzeugern" oder "erhöhen" einer Pathogenresistenz meint, dass die Abwehrmechanismen einer bestimmten Pflanzenart oder -sorte durch Anwendung des erfindungsgemäßen Verfahrens im Vergleich zu dem Wildtyp der Pflanze ("Ausgangspflanze"), auf den das erfindungsgemäße Verfahren nicht angewendet wurde, unter ansonsten gleichen Bedingungen (wie beispielsweise Klima- oder Anbaubedingungen, Pathogenart etc.) eine erhöhte Resistenz gegen ein und mehr Pathogene aufweist. Dabei äußert sich die erhöhte Resistenz bevorzugt in einer verminderten Ausprägung der Krankheitssymptome, wobei Krankheitssymptome - neben den oben erwähnten Beeinträchtigungen - auch beispielsweise die Penetrationseffizienz eines Pathogens in die Pflanze oder pflanzliche Zellen oder die Proliferationseffizienz in oder auf denselben umfasst. Dabei sind die Krankheitssymptome bevorzugt um mindestens 10 % oder mindestens 20 %, besonders bevorzugt um mindestens 40 % oder 60 %, ganz besonders bevorzugt um mindestens 70 % oder 80 %, am meisten bevorzugt um mindestens 90 % oder 95 % vermindert.

"Auswahl" meint in Bezug auf Pflanzen, bei denen - im Unterschied oder Vergleich zur Ausgangspflanze - die Resistenz gegen mindestens ein Pathogen besteht oder erhöht ist, all die Verfahren, die eine zur Erkennung einer vorliegenden oder erhöhten Pathogenresistenz geeignet sind. Dies können Symptome der Pathogeninfektion sein (z.B. Haustorium-Ausbildung bei Pilzinfektion) aber auch die oben beschriebenen Symptome umfassen, die die Qualität der Pflanze, die Quantität des Ertrages, die Eignung zur Verwendung als Futter- oder Nahrungsmittel usw. betreffen.

"Pathogen" meint im Rahmen der Erfindung beispielsweise jedoch nicht einschränkend Viren oder Viroide, Bakterien, Pilze, tierische Schädlinge, wie beispielsweise Insekten oder Nematoden.

Besonders bevorzugt sind Pilze wie beispielsweise der Mehltau. Es ist jedoch anzunehmen, dass die Verminderung der Expression eines RacB-Proteins, seiner Aktivität oder Funktion auch eine Resistenz gegen weitere Pathogene bewirkt. Veränderungen in der Zellwandstruktur können einen grundlegenden Mechanismus der Pathogenresistenz darstellen.

Beispielsweise jedoch nicht einschränkend seien nachfolgende Pathogene zu nennen:

1. Pilzpathogene oder pilz-ähnliche Pathogene:

Pilzpathogene oder pilz-ähnliche Pathogene (wie z.B. Chromista) stammen vorzugsweise aus der Gruppe umfassend Plasmodiophoramycota, Oomycota, Ascomycota, Chytridiomyceten, Zygomyceten, Basidiomycota und Deuteromyceten (Fungi imperfecti). Beispielhaft jedoch nicht einschränkend seien die in Tabelle 1 und 2 genannten Pathogene und die mit ihnen in Zusammenhang gebrachten Erkrankungen zu nennen.

Tabelle 1: Pflanzliche Pilzerkrankungen

| Erkrankung | Pathogen |
|---|---|
| Braunrost | Puccinia recondita |
| Gelbrost | P. striiformis |
| Echter Mehltau | Erysiphe graminis / Blumeria graminis |
| Spelzenbräune | Septoria nodorum |
| Blattdürre | Septoria tritici |
| Ährenfusariosen | Fusarium spp. |
| Halmbruchkrankheit | Pseudocercosporella herpotrichoides |
| Flugbrand | Ustilago spp. |
| Weizensteinbrand | Tilletia caries |
| Schwarzbeinigkeit | Gaeumannomyces graminis |
| Anthrocnose leaf blight<br>Anthracnose stalk rot | Colletotrichum graminicola (tele-omorph: Glomerella graminicola Politis); Glomerella tucumanensis (anamorph: Glomerella falcatum Went) |
| Aspergillus ear and kernel rot | Aspergillus flavus |
| Banded leaf and sheath spot ("Wurzeltöter") | Rhizoctonia solani Kuhn = Rhizoctonia microsclerotia J. Matz (telomorph: Thanatephorus cucumeris) |
| Black bundle disease | Acremonium strictum W. Gams = Cephalosporium acremonium Auct. non Corda |
| Black kernel rot | Lasiodiplodia theobromae = Botryodiplodia theobromae |
| Borde blanco | Marasmiellus sp. |
| Brown spot (black spot, stalk rot) | Physoderma maydis |
| Cephalosporium kernel rot | Acremonium strictum = Cephalosporium acremonium |
| Charcoal rot | Macrophomina phaseolina |
| Corticium ear rot | Thanatephorus cucumeris = Corticium sasakii |
| Curvularia leaf spot | Curvularia clavata, C. eragrostidis, = C. maculans (teleomorph: Cochliobolus eragrostidis), Curvularia inaequalis, C. intermedia (teleomorph: Cochliobolus intermedius), Curvularia lunata (teleomorph: Cochliobolus lunatus), Curvularia pallescens (tele-omorph: Cochliobolus pallescens), Curvularia senegalensis, C. tuberculata (teleomorph: Cochliobolus tuberculatus) |
| Didymella leaf spot | Didymella exitalis |
| Diplodia ear rot and stalk rot | Diplodia frumenti (teleomorph: Botryosphaeria festucae) |
| Diplodia ear rot, stalk rot, seed rot and seedling blight | Diplodia maydis = Stenocarpella maydis |
| Diplodia leaf spot or streak | Stenocarpella macrospora = Diplodialeaf macrospora |

Tabelle 2: Falscher Mehltau

| Erkrankung | Pathogen |
|---|---|
| Brown stripe downy mildew | Sclerophthora rayssiae var. zeae |

(fortgesetzt)

| Erkrankung | Pathogen |
|---|---|
| Crazy top downy mildew | Sclerophthora macrospora = Sclerospora macrospora |
| Green ear downy mi ldew (graminicola downy mildew) | Sclerospora graminicola |
| Java downy mildew | Peronosclerospora maydis = Sclerospora maydis |
| Philippine downy mildew | Peronosclerospora philippinensis = Sclerospora philippinensis |
| Sorghum downy mildew | Peronosclerospora sorghi = Sclerospora sorghi |
| Spontaneum downy mildew | Peronosclerospora spontanea = Sclerospora spontanea |
| Sugarcane downy mildew | Peronosclerospora sacchari = Sclerospora sacchari |
| Dry ear rot (cob, kernel and stalk rot) | Nigrospora oryzae (teleomorph: Khuskia oryzae) |
| Ear rots, minor | Alternaria alternata = A. tenuis, Aspergillus glaucus, A. niger, -Aspergillus spp., Botrytis cinerea (teleomorph: Botryotinia fuckeliana), Cunninghamella sp., Curvularia pallescens, Doratomyces stemonitis = Cephalotrichum stemonitis, Fusarium culmorum, Gonatobotrys simplex, Pithomyces maydicus, Rhizopus microsporus Tiegh., R. stolonifer = R. nigricans, Scopulariopsis brumptii |
| Ergot(horse's tooth) | Claviceps gigantea (anamorph: Sphacelia sp.) |
| Eyespot | Aureobasidium zeae = Kabatiella zeae |
| Fusarium ear and stalk rot | Fusarium subglutinans = F. moniliforme var.subglutinans |
| Fusarium kernel, root and stalk rot, seed rot and seedling blight | Fusarium moniliforme (teleomorph: Gibberella fujikuroi) |
| Fusarium stalk rot, seedling root rot | Fusarium avenaceum (teleomorph: Gibberlla avenacea) |
| Gibberella ear and stalk rot (Ähren- u. Stengelfäule) | Gibberella zeae (anamorph: Fusarium graminearum) |
| Gray ear rot | Botryosphaeria zeae = Physalospora zeae (anamorph: Macrophoma zeae) |
| Gray leaf spot (Cercospora leaf spot) | Cercospora sorghi = C. sorghi var. maydis, C. zeae-maydis |
| Helminthosporium root rot | Exserohilum pedicellatum = Helminthosporium pedicellatum (teleomorph: Setosphaeria pedicellata) |
| Hormodendrum ear rot (Cladosporium rot) | Cladosporium cladosporioides = Hormodendrum cladosporioides, C. herbarum (teleomorph: Mycosphaerella tassiana) |
| Hyalothyridium leaf spot | Hyalothyridium maydis |
| Late wilt | Cephalosporium maydis |
| Leaf spots, minor | Alternaria alternata, Ascochyta maydis, A. tritici, A. zeicola, Bipolaris victoriae = Helminthosporium victoriae (teleomorph: Cochliobolus victoriae), C. sativus (anamorph: Bipolaris sorokiniana = H. sorokinianum = H. sativum), Epicoccum nigrum, Exserohilum prolatum = Drechslera prolata (teleomorph: Setosphaeria prolata) Graphium penicillioides, Leptosphaeria maydis, Leptothyrium zeae, Ophiosphaerella herpotricha, (anamorph: Scolecosporiella sp.), Paraphaeosphaeria michotii, Phoma sp., Septoria zeae, S. zeicola, S. zeina |

(fortgesetzt)

| Erkrankung | Pathogen |
|---|---|
| Northern corn leaf blight (white blast, crown stalk rot, stripe) | Setosphaeria turcica (anarnorph: Exserohilum turcicum = Helminthosporium turcicum) |
| Northern corn leaf spot Helminthosporium ear rot (race 1) | Cochliobolus carbonum (anamorph: Bipolaris zeicola = Helminthosporium carbonum) |
| Penicillium ear rot (blue eye, blue mold) | Penicillium spp., P. chrysogenum, P. expansum, P. oxalicum |
| Phaeocytostroma stalk rot and root rot | Phaeocytostroma ambiguum, = Phaeocytosporella zeae |
| Phaeosphaeria leaf spot | Phaeosphaeria maydis = Sphaerulina maydis |
| Physalospora ear rot (Botryosphaeria ear rot) | Botryosphaeria festucae = Physalospora zeicola (anamorph: Diplodia frumenti) |
| Purple leaf sheath | Hemiparasitic bacteria and fungi |
| Pyrenochaeta stalk rot and root rot | Phoma terrestris = Pyrenochaeta terrestris |
| Pythium root rot | Pythium spp., P. arrhenomanes, P. graminicola |
| Pythium stalk rot | Pythium aphanidermatum = P. butleri L. |
| Red kernel disease (ear mold, leaf and seed rot) | Epicoccum nigrum |
| Rhizoctonia ear rot (sclerotial rot) | Rhizoctonia zeae (teleomorph: Waitea circinata) |
| Rhizoctonia root rot and stalk rot | Rhizoctonia solani, Rhizoctonia zeae |
| Root rots, minor | Alternaria alternata, Cercospora sorghi, Dictochaeta fertilis, Fusarium acuminatum (teleomorph: Gibberella acuminata), F. equiseti (teleomorph: G. intricans), F. oxysporum, F. pallidoroseum, F. poae, F. roseum, G. cyanogena, (anamorph: F. sulphureum), Microdochium bolleyi, Mucor sp., Periconia circinata, Phytophthora cactorum, P. drechsleri, P. nicotianae var. parasitica, Rhizopus arrhizus |
| Rostratum leaf spot (Helminthosporium leaf disease, ear and stalk rot) | Setosphaeria rostrata, (anamorph: Exserohilum rostratum = He/minthosporium rostratum) |
| Rust, commön corn | Puccinia sorghi |
| Rust, southern corn | Puccinia polysora |
| Rust, tropical corn | Physopella pallescens, P. zeae = Angiopsora zeae |
| Sclerotium ear rot (southern blight) | Sclerotium rolfsii Sacc. (teleomorph: Athelia rolfsii) |
| Seed rot-seedling blight | Bipolaris sorokiniana, B. zeicola = Helminthosporium carbonum, Diplodia maydis, Exserohilum pedicillatum, Exserohilum turcicum = Helminthosporium turcicum, Fusarium avenaceum, F. culmorum, F. moniliforme, Gibberella zeae (anamorph: F. graminearum), Macrophomina phaseolina, Penicillium spp., Phomopsis sp., Pythium spp., Rhizoctonia solani, R. zeae, Sclerotium rolfsii, Spicaria sp. |
| Selenophoma leaf spot | Selenophoma sp. |
| Sheath rot | Gaeumannomyces graminis |
| Shuck rot | Myrothecium gramineum |
| Silage mold | Monascus purpureus, M ruber |
| Smut, common | Ustilago zeae = U. maydis |

(fortgesetzt)

| Erkrankung | Pathogen |
|---|---|
| Smut, false | Ustilaginoidea virens |
| Smut, head | Sphacelotheca reiliana = Sporisorium holcisorghi |
| Southern corn leaf blight and stalk rot | Cochliobolus heterostrophus (anamorph: Bipolaris maydis = Helminthosporium maydis) |
| Southern leaf spot | Stenocarpella macrospora = Diplodia macrospora |
| Stalk rots, minor | Cercospora sorghi, Fusarium episphaeria, F. merismoides, F. oxysporum Schlechtend, F. poae, F. roseum, F. solani (teleomorph: Nectria haematococca), F. tricinctum, Mariannaea elegans, Mucor sp., Rhopographus zeae, Spicaria sp. |
| Storage rots | Aspergillus spp., Penicillium spp. and other fungi |
| Tar spot | Phyllachora maydis |
| Trichoderma ear rot and root rot | Trichoderma viride = T. lignorum tele-omorph: Hypocrea sp. |
| White ear rot, root and stalk rot | Stenocarpella maydis = Diplodia zeae |
| Yellow leaf blight | Ascochyta ischaemi, Phyllosticta maydis (teleomorph: Mycosphaerella zeae-maydis) |
| Zonate leaf spot | Gloeocercospora sorghi |

Besonders bevorzugt sind

- Plasmodiophoromycota wie Plasmodiophora brassicae (Kohlhernie, clubroot of crucifers), Spongospora subterranea (powdery scab of potato tubers), Polymyxa graminis (root disease of cereals and grasses),

- Oomycota wie Bremia lactucae (Falscher Mehltau an Salat), Peronospora (Falscher Mehltau) bei snapdragon (P. antirrhini), Zwiebel (P. destructor), Spinat (P. effusa), Sojabohne (P. manchurica), Tabak ("blue mold" = Blauschimmel; P. tabacina) Alfalfa und Klee (P. trifolium), Pseudoperonospora humuli (Falscher Mehltau an Hopfen), Plasmopara (Falscher Mehltau bei Trauben) (P. viticola) und Sonnenblume (P. halstedii), Sclerophtohra macrospora (Falscher Mehltau bei Cerealien und Gäsern), Pythium (seed rot, seedling damping-off, and root rot and all types of plants, z.B. Wurzelbrand an Beta-Rübe durch P. debaryanum), Phytophthora infestans (Kraut- und Knollenfäule bei Kartoffel, Braunfäule bei Tomate etc.), Albugo spec. (white rust on cruciferous plants.

- Ascomycota wie Microdochium nivale (Schneeschimmel an Roggen und Weizen), Fusarium graminearum, Fusarium culmorum (Ährenfäule v.a. bei Weizen), Fusarium oxysporum (Fusarium-Welke an Tomate), Blumeria graminis (Echter Mehltau an Gerste (f.sp. hordei) und Weizen (f.sp. tritici)), Erysiphe pisi (Erbsenmehltau), Nectria galligena (Obstbaumkrebs), Unicnula necator (Echter Mehltau der Weinrebe), Pseudopeziza tracheiphila (Roter Brenner der Weinrebe), Claviceps purpurea (Mutterkorn an z.B. Roggen und Gräsern), Gaeumannomyces graminis (Schwarzbeinigkeit an Weizen, Roggen u.a. Gräsern), Magnaporthe grisea (rice blast disease), Pyrenophora graminea (Streifenkrankheit an Gerste), Pyrenophora teres (Netzfleckenkrankheit an Gerste), Pyrenophora tritici-repentis (Blattfleckenkrankheit (Blattdürre) an Weizen), Venturia inaequalis (Apfelschorf), Sclerotinia sclerotium (Weißstengeligkeit, Rapskrebs), Pseudopeziza medicaginis (Klappenschorf an Luzerne, Weiß- und Rotklee).

- Basidiomyceten wie Typhula incarnata (Typhula-Fäule an Gerste, Roggen, Weizen), Ustilago maydis (Beulenbrand an. Mais), Ustilago nuda (Flugbrand an Gerste), Ustilago tritici (Flugbrand an Weizen, Dinkel), Ustilago avenae (Flugbrand an Hafer), Rhizoctonia solani (Wurzeltöter an Kartoffeln), Sphacelotheca spp. (head smut of sorghum), Melampsora lini (rust of flax), Puccinia graminis (Schwarzrost an Weizen, Gerste, Roggen, Hafer), Puccinia recondita (Braunrost an Weizen), Puccinia dispersa (Braunrost an Roggen), Puccinia hordei (Braunrost an Gerste), Puccinia coronata (Kronenrost an Hafer), Puccinia striiformis (Gelbrost an Weizen, Gerste, Roggen sowie zahlreichen Gräsern), Uromyces appendiculatus (Bohnenrost), Sclerotium rolfsii (root and stem rots of many plants).

- Deuteromyceten (Fungi imperfecti) wie Septoria nodorum (Spelzenbräune) an Weizen (Septoria tritici), Pseudocercosporella herpotrichoides (Halmbruchkrankheit an Weizen, Gerste, Roggen), Rynchosporium secalis (Blattfleckenkrankheit an Roggen und Gerste), Alternaria solani (Dürrfleckenkrankheit an Kartoffel, Tomate), Phoma betae (Wurzelbrand an Beta-Rübe), Cercospora beticola (Cercospora-Blattfleckenkrankheit an Beta-Rübe), (Alternaria brassicae (Rapsschwärze an Raps, Kohl u.a. Kreuzblütlern), Verticillium dahliae (Rapswelke und -stengelfäule), Colletotrichum lindemuthianum (Brennfleckenkrankheit an Bohne), Phoma lingam - Umfallkrankheit (Schwarzbeinigkeit an Kohl; Wurzelhals- oder Stengelfäule an Raps), Botrytis cinerea (Grauschimmel an Weinrebe, Erdbeere, Tomate, Hopfen etc.).

Am meisten bevorzugt sind Phytophthora infestans (Kraut- und Knollenfäule, Braunfäule bei Tomate etc.), Microdochium nivale (vormals Fusarium nivale; Schneeschimmel an Roggen und Weizen), Fusarium graminearum, Fusarium culmorum (Ährenfäule an Weizen), Fusarium oxysporum (Fusarium-Welke an Tomate), Blumeria graminis (Echter Mehltau an Gerste (f. sp. hordei) und Weizen (f. sp. tritici)), Magnaporthe.grisea (rice blast disease), Sclerotinia sclerotium (Weißstengeligkeit, Rapskrebs), Septoria nodorum und Septoria tritici (Spelzenbräune an Weizen), Alternaria brassicae (Rapsschwärze an Raps, Kohl u.a. Kreuzblütlern), Phoma lingam (Umfallkrankheit, Schwarzbeinigkeit an Kohl; Wurzelhals- oder Stengelfäule an Raps).

2. Bakterielle Pathogene:

Beispielhaft jedoch nicht einschränkend seien die in Tabelle 3 genannten Pathogene und die mit ihnen in Zusammenhang gebrachten Erkrankungen zu nennen.

Tabelle 3: Bakterielle Erkrankungen

| Erkrankung | Pathogen |
|---|---|
| Bacterial leaf blight and stalk rot | Pseudomonas avenae subsp. avenae |
| Bacterial leaf spot | Xanthomonas campestris pv. holcicola |
| Bacterial stalk rot | Enterobacter dissolvens = Erwinia dissolvens |
| Schwarzbeinigkeit ("Bacterial stalk and top rot") | Erwinia carotovora subsp. carotovora, Erwinia chrysanthemi pv. zeae |
| Bacterial stripe | Pseudomonas andropogonis |
| Chocolate spot | Pseudomonas syringae pv. coronafaciens |
| Goss's bacterial wilt and blight (leaf freckles and wilt) | Clavibacter michiganensis subsp. nebraskensis = Corynebacterium michiganense pv.andnebraskense |
| Holcus spot | Pseudomonas syringae pv. syringae |
| Purple leaf sheath | Hemiparasitic bacteria |
| Seed rot-seedling blight | Bacillus subtilis |
| Stewart's disease (bacterial wilt) | Pantoea stewartii = Erwinia stewartii |
| Corn stunt (achapparramiento,maize stunt, Mesa Central or Rio Grande maize stunt) | Spiroplasma kunkelii |

Ganz besonders bevorzugt sind nachfolgende pathogene Bakterien: Corynebacterium sepedonicum (Bakterienringfäule an Kartoffel), Erwinia carotovora (Schwarzbeinigkeit an Kartoffel), Erwinia amylovora (Feuerbrand an Birne, Apfel, Quitte), Streptomyces scabies (Kartoffelschorf), Pseudomonas syringae pv. tabaci (Wildfeuer an Tabak), Pseudomonas syringae pv. phaseolicola (Fettfleckenkrankheit an Buschbohne), Pseudomonas syringae pv. tomato ("bacterial speck" an Tomate), Xanthomonas campestris pv. malvacearum (Blattfleckenkrankheit an Baumwolle) und Xanthomonas campestris pv. oryzae (Bakterienfäule an Reis und anderen Gräsern).

3. Virale Pathogene:

"Virale Pathogene" schließt sämtliche Pflanzenviren ein wie beispielsweise Tabak- oder Cucumber-Mosaiv Virus, Ringspot-Virus, Nekrose-Virus, Mais Dwarf-Mosaic Virus etc.

Beispielhaft jedoch nicht einschränkend seien die in Tabelle 4 genannten Pathogene und die mit ihnen in Zusammenhang gebrachten Erkrankungen zu nennen.

Tabelle 4: Virale Erkrankungen

| Krankheit | Pathogen |
|---|---|
| American wheat striate (wheat striate mosaic) | American wheat striate mosaic virus (AWSMV) |
| Barley stripe mosaic | Barley stripe mosaic virus (BSMV) |
| Barley yellow dwarf | Barley yellow dwarf virus (BYDV) |
| Brome mosaic | Brome mosaic virus (BMV) |
| Cereal chlorotic mottle | Cereal chlorotic mottle virus (CCMV) |
| Corn chlorotic vein banding (Braizilian maize mosaic) | Corn chlorotic vein banding virus (CCVBV) |
| Corn lethal necrosis | Viruskomplex aus Maize chlorotic mottle virus (MCMV) und Maize dwarf mosaic virus (MDMV) A oder B oder Wheat streak mosaic virus (WSMV) |
| Cucumber mosaic | Cucumber mosaic virus (CMV) |
| Cynodon chlorotic streak | Cynodon chlorotic streak virus (CCSV) |
| Johnsongrass mosaic | Johnsongrass mosaic virus (JGMV) |
| Maize bushy stunt | Mycoplasma-like organism (MLO) associated |
| Maize chlorotic dwarf | Maize chlorotic dwarf virus (MCDV) |
| Maize chlorotic mottle | Maize chlorotic mottle virus (MCMV) |
| Maize dwarf mosaic | Maize dwarf mosaic virus (MDMV) strains A, D, E and F |
| Maize leaf fleck | Maize leaf fleck virus (MLFV) |
| Maize line | Maize line virus (MLV) |
| Maize mosaic (corn leaf stripe, enanismo rayado) | Maize mosaic virus (MMV) |
| Maize mottle and chlorotic stunt | Maize mottle and chlorotic stunt virus |
| Maize pellucid ringspot | Maize pellucid ringspot virus (MPRV) |
| Maize raya gruesa | Maize raya gruesa virus (MRGV) |
| maize rayado fino (fine striping disease) | Maize rayado fino virus (MRFV) |
| Maize red leaf and red stripe | Mollicute |
| Maize red stripe | Maize red stripe virus (MRSV) |
| Maize ring mottle | Maize ring mottle virus (MRMV) |
| Maize- rio IV | Maize rio cuarto virus, (MRCV). |
| Maize rough dwarf (nanismo ruvido) | Maize rough dwarf virus (MRDV) (Cereal tillering disease virus) |
| Maize sterile stunt | Maize sterile stunt virus (strains of barley yellow striate virus) |
| Maize streak | Maize streak virus (MSV) |
| Maize stripe (maize chlorotic stripe, maize hoja blanca) | Maize stripe virus |
| Maize stunting | Maize stunting virus |
| Maize tassel abortion | Maize tassel abortion virus (MTAV) |
| Maize vein enation | Maize vein enation virus (MVEV) |
| Maize wallaby ear | Maize wallaby ear virus (MWEV) |

(fortgesetzt)

| Krankheit | Pathogen |
|---|---|
| Maize white leaf | Maize white leaf virus |
| Maize white line mosaic | Maize white line mosaic virus (MWLMV) |
| Millet red leaf | Millet red leaf virus (MRLV) |
| Northern cereal mosaic | Northern cereal mosaic virus (NCMV) |
| Oat pseudorosette (zakuklivanie) | Oat pseudorosette virus |
| Oat sterile dwarf | Oat sterile dwarf virus (OSDV) |
| Rice black-streaked dwarf | Rice black-streaked dwarf virus (RBSDV) |
| Rice stripe | Rice stripe virus (RSV) |
| Sorghum mosaic | Sorghum mosaic virus (SrMV) (auch: sugarcane mosaic virus (SCMV) Stämme H, I and M) |
| Sugarcane Fiji disease | Sugarcane Fiji disease virus (FDV) |
| Sugarcane mosaic | Sugarcane mosaic virus (SCMV) strains A, B, D, E, SC, BC, Sabi and MB (formerly MDMV-B) |
| Wheat spot mosaic | Wheat spot mosaic virus (WSMV) |

4. Tierische Schädlinge

4.1 Insekten Pathogene:

Beispielhaft jedoch nicht einschränkend seien Insekten wie beispielsweise Käfer, Raupen, Läuse oder Milben zu nennen. Bevorzugt sind Insekten der Gattungen Coleoptera, Diptera, Hymenoptera, Lepidoptera, Mallophaga, Homoptera, Hemiptera, Orthoptera, Thysanoptera. Dermaptera, Isoptera, Anoplura, Siphonaptera, Trichoptera, etc.. Besonders bevorzugt sind Coleoptera and Lepidoptera Insekten, wie beispielsweise den Maiszünsler (European Corn Borer (ECB)), Diabrotica barberi ("northern corn rootworm"), Diabrotica undecimpunctata ("southern corn rootworm"), Diabrotica virgifera ("Western corn rootworm"), Agrotis ipsilon ("back cutworm"), Crymodes devastator ("glassy cutworm"), Feltia ducens ("dingy cutworm"), Agrotis gladiaria ("claybacked cutworm"), Melanotus spp., Aeolus mellillus ("wireworm"), Aeolus mancus ("wheat wireworm"), Horistonotus uhlerii ("sand wireworm"), Sphenophorus maidis ("maize billbug"), Sphenophorus zeae ("timothy billbug"), Sphenophorus parvulus ("bluegrass billbug"), Sphenophorus callosus ("southern corn billbug"), Phyllogphaga spp.("white grubs"), Anuraphis maidiradicis ("corn root aphid"), Delia platura ("seedcorn maggot"), Colaspis brunnea ("grape colaspis"), Stenolophus lecontei ("seedcorn beetle") und Clivinia impressifrons ("lender seedcorn beetle").

Ferner sind zu nennen: Das Getreidehähnchen (Oulema melanopus), die Fritfliege (Oscinella frit), Drahtwürmer (Agrotis lineatus) und Blattläuse (wie z.B. Haferblattlaus Rhopalosiphum padi, Grosse Getreideblattlaus Sitobion avenae).

4.2 Nematoden:

Beispielhaft jedoch nicht einschränkend seien die in Tabelle 6 genannten Pathogene und die mit ihnen in Zusammenhang gebrachten Erkrankungen zu nennen.

Tabelle 6: Parasitäre Nematoden

| Schädigung | Pathogene Nematode |
|---|---|
| Awl | Dolichodorus spp., D. heterocephalus |
| Stengel- oder Stockälchen, Rübenkopfälchen ("Bulb and stem"; Europe) | Ditylenchus dipsaci |
| Burrowing | Radopholus similis |
| Haferzystenälchen ("Cyst") | Heterodera avenae, H. zeae, Punctodera chalcoensis |

(fortgesetzt)

| Schädigung | Pathogene Nematode |
|---|---|
| Dagger | Xiphinema spp., X. americanum, X. mediterraneum |
| False root-knot | Nacobbus dorsalis |
| Lance, Columbia | Hoplolaimus columbus |
| Lance | Hoplolaimus spp., H. galeatus |
| Lesion | Pratylenchus spp., P. brachyurus, P. crenatus, P. hexincisus, P. neglectus, P. penetrans, P. scribneri, P. thornei, P. zeae |
| Needle | Longidorus spp., L. breviannulatus |
| Ring | Criconemella spp., C. ornata |
| Wurzelgallenälchen ("Root-knot") | Meloidogyne spp., M. chitwoodi, M. incognita, M. javanica |
| Spiral | Helicotylenchus spp. |
| Sting | Belonolaimus spp., B. longicaudatus |
| Stubby-root | Paratrichodorus spp., P. christiei, P. minor, Quinisulcius acutus, Trichodorus spp. |
| Stunt | Tylenchorhynchus dubius |

Ganz besonders bevorzugt sind Globodera rostochiensis und G. pallida (Zystenälchen an Kartoffel, Tomate u.a. Nachtschattengewächsen), Heterodera schachtii (Rübenzystenälchen an Zucker- und Futterrübe, Raps, Kohl etc.), Heterodera avenae (Haferzystenälchen an Hafer u.a. Getreidearten), Ditylenchus dipsaci (Stengel- oder Stockälchen, Rübenkopfälchen an Roggen, Hafer, Mais, Klee, Tabak, Rübe), Anguina tritici (Weizenälchen, Radekrankheit an Weizen (Dinkel, Roggen), Meloidogyne hapla (Wurzelgallenälchen an Möhre, Gurke, salat, tomate, Kartoffel, Zuckerrübe, Luzerne).

Als für die einzelnen Sorten bevorzugte Pilz- oder Virus-Pathogene sind beispielsweise zu nennen:

1. Gerste:

Pilz-, bakterielle und virale Pathogene: Puccinia graminis f.sp. hordei (barley stem rust), Blumeria (Erysiphe) graminis f.sp. hordei (Barley Powdery Mildew), barley yellow dwarf virus (BYDV), Pathogene Insekten / Nematoden: Ostrinia nubilalis (European corn borer); Agrotis ipsilon (black cutworm); Schizaphis graminum (greenbug); Blissus leucopterus leucopterus (chinch bug); Acrosternum hilare (green stink bug); Euschistus servus (brown stink bug); Deliaplatura (seedcorn maggot); Mayetiola destructor (Hessian fly); Petrobia latens (brown wheat mite).

2. Sojabohne:

Pilz-, bakterielle oder virale Pathogene: Phytophthora megasperma fsp.glycinea, Macrophomina phaseolina, Rhizoctonia solani, Sclerotinia sclerotiorum, Fusarium oxysporum, Diaporthe phaseolorum var. sojae (Phomopsis sojae), Diaporthe phaseolorum var. caulivora, Sclerotium rolfsii, Cercospora kikuchii, Cercospora sojina, Peronospora manshurica, Colletotrichum dematium (Colletotrichum truncatum), Corynespora cassiicola, Septoria glycines, Phyllosticta sojicola, Alternaria alternata, Pseudomonas syringae p.v. glycinea, Xanthomonas campestris p.v. phaseoli, Microsphaera diffussa, Fusarium semitectum, Phialophora gregata, Sojabohnen Mosaikvirus, Glomerella glycines, Tobacco Ring spot virus, Tobacco Streak virus, Phakopsorapachyrhizi, Pythium aphanidermatum, Pythium ultimum, Pythium debaryanum, Tomato spotted wilt virus, Heterodera glycines Fusarium solani.

Pathogene Insekten / Nematoden: Pseudoplusia includens (soybean looper); Anticarsia gemmatalis (velvetbean caterpillar); Plathypena scabra (green cloverworm); Ostrinia nubilalis (European corn borer); Agrotis ipsilon (black cutworm); Spodoptera exigua (beet armyworm); Heliothis virescens (cotton budworm); Helicoverpa zea (cotton bollworm); Epilachna varivestis (Mexican bean beetle); Myzus persicae (green peach aphid); Empoasca fabae (potato leaf hopper); Acrosternum hilare (green stink bug); Melanoplus femurrubrum (redlegged grasshopper); Melanoplus differentialis (differential grasshopper); Hylemya platura (seedcom maggot); Sericothrips variabilis (soybean thrips); Thrips tabaci (onion thrips); Tetranychus turkestani (strawberry spider mite); Tetranychus urticae (twospotted spider mite);

3. Canola:

Pilz-, bakterielle oder virale Pathogene: Albugo candida, Alternaria brassicae, Leptosphaeria maculans, Rhizoctonia solani, Sclerotinia sclerotiorum, Mycosphaerella brassiccola, Pythium ultimum, Peronospora parasitica, Fusarium roseum, Alternaria alternata.

4. Alfalfa:

Pilz,, bakterielle oder virale Pathogene: Clavibater michiganese subsp. insidiosum, Pythium ultimum, Pythium irregulare, Pythium splendens, Pythium debaryanum, Pythium aphanidermatum, Phytophthora megasperma, Peronospora trifoliorum, Phoma medicaginis var. medicaginis, Cercospora medicaginis, Pseudopeziza medicaginis, Leptotrochila medicaginis, Fusarium, Xanthomonas campestris p.v. alfalfae, Aphanomyces euteiches, Stemphylium herbarum, Stemphylium alfalfae.

5. Weizen:

Pilz-,bakterielle oder virale Pathogene: Pseudomonas syringae p.v. atrofaciens, Urocystis agropyri, Xanthomonas campestris p.v. translucens, Pseudomonas syringae p.v. syringae, Alternaria alternata, Cladesporium herbarum, Fusarium graminearum, Fusarium avenaceum, Fusarium culmorum, Ustilago tritici, Ascochyta tritici, Cephalosporium gramineum, Collotetrichum graminicola, Erysiphe graminis f.sp. tritici, Puccinia graminis f.sp. tritici, Puccinia recondita f.sp. tritici, Puccinia striiformis, Pyrenophora tritici-repentis, Septoria nodorum, Septoria tritici, Septoria avenae, Pseudocercosporella herpotrichoides, Rhizoctonia solani, Rhizoctonia cerealis, Gaeumannomyces graminis var. tritici, Pythium aphanidermatum, Pythium arrhenomanes, Pythium ultimum, Bipolaris sorokiniana, Barley Yellow Dwarf Virus, Brome Mosaic Virus, Soil Borne Wheat Mosaic Virus, Wheat Streak Mosaic Virus, Wheat Spindle Streak Virus, American Wheat Striate Virus, Claviceps purpurea, Tilletia tritici, Tilletia laevis, Ustilago tritici, Tilletia indica, Rhizoctonia solani, Pythium arrhenomannes, Pythium gramicola, Pythium aphanidermatum, High Plains Virus, European wheat striate virus, Puccinia graminis f.sp. tritici (Wheat stem rust), Blumeria (Erysiphe) graminis f.sp. tritici (Wheat Powdery Mildew)

Pathogene Insekten / Nematoden: Pseudaletia unipunctata (army worm); Spodoptera, frugiperda (fall armyworm); Elasmopalpus lignosellus (lesser cornstalk borer); Agrotis orthogonia (western cutworm); Elasmopalpus Zignosellus (lesser cornstalk borer); Oulema melanopus (cereal leaf beetle); Hypera punctata (clover leaf weevil); Diabrotica undecimpunctata howardi (southern corn rootworm); Russian wheat aphid; Schizaphis graminum (greenbug); Macrosiphum avenae (English grain aphid); Melanoplus femurrubrum (redlegged grasshopper); Melanoplus differentialis (differential grasshopper); Melanoplus sanguinipes (migratory grasshopper); Mayetiola destructor (Hessian fly); Sitodiplosis mosellana (wheat midge); Meromyza americana (wheat stem maggot); Hylemya coarctata (wheat bulb fly); Frankliniella fusca (tobacco thrips); Cephus cinctus (wheat stem sawfly); Aceria tulipae (wheat curl mite);

6. Sonnenblume:

Pilz-, bakterielle oder virale Pathogene: Plasmophora halstedii, Sclerotinia sclerotiorum, Aster Yellows, Septoria helianthi, Phomopsis helianthi, Alternaria helianthi, Alternaria zinniae, Botrytis cinerea, Phoma macdonaldii, Macrophomina phaseolina, Erysiphe cichoracearum, Rhizopus oryzae, Rhizopus arrhizus, Rhizopus stolonifer, Puccinia helianthi, Verticillium dahliae, Erwinia carotovorum p.v. Carotovora, Cephalosporium acremonium, Phytophthora cryptogea, Albugo tragopogonis.

Pathogene Insekten / Nematoden: Suleima helianthana (sunflower bud moth); Homoeosoma electellum (sunflower moth); zygogramma exclamationis (sunflower beetle); Bothyrus gibbosus (carrot beetle); Neolasioptera murtfeldtiana (sunflower seed midge);

7. Mais:

Pilz-, bakterielle oder virale Pathogene: Fusarium moniliforme var. subglutinans, Erwinia stewartii, Fusarium moniliforme, Gibberella zeae (Fusarium graminearum), Stenocarpella maydi (Diplodia maydis), Pythium irregulare, Pythium debaryanum, Pythium graminicola, Pythium splendens, Pythium ultimum, Pythium aphanidermatum, Aspergillus flavus, Bipolaris maydis 0, T (Cochliobolus heterostrophus), Helminthosporium carbonum I, II & III (Cochliobolus carbonum), Exserohilum turcicum I, II & III, Helminthosporium pedicellatum, Physoderma maydis, Phyllosticta maydis, Kabatiella maydis, Cercospora sorghi, Ustilago maydis, Puccinia sorghi, Puccinia polysora, Macrophomina phaseolina, Penicillium oxalicum, Nigrospora oryzae, Cladosporium herbarum, Curvularia lunata, Curvularia inaequalis, Curvularia pallescens, Clavibacter michiganese subsp. nebraskense, Trichoderma viride, Maize Dwarf Mosaic Virus A & B, Wheat Streak Mosaic Virus, Maize Chlorotic Dwarf Virus, Claviceps sorghi, Pseudonomas avenae, Erwinia chrysanthemi p.v.

Zea, Erwinia corotovora, Cornstunt spiroplasma, Diplodia macrospora, Sclerophthora macrospora, Peronosclerospora sorghi, Peronosclerospora philippinesis, Peronosclerospora maydis, Peronosclerospora sacchari, Spacelotheca reiliana, Physopella zeae, Cephalosporium maydis, Caphalosporium acremonium, Maize Chlorotic Mottle Virus, High Plains Virus, Maize Mosaic Virus, Maize Rayado Fino Virus, Maize Streak Virus (MSV, Maisstrichel-Virus), Maize Stripe Virus, Maize Rough Dwarf Virus.

Pathogene Insekten / Nematoden: Ostrinia nubilalis (European corn borer); Agrotis ipsilon (black cutworm); Helicoverpa zea (corn earworm); Spodoptera frugiperda. (fall armyworm); Diatraea grandiosella (southwestern corn borer); Elasmopalpus lignosellus (lesser cornstalk borer); Diatraea saccharalis (surgarcane borer); Diabrotica virgifera (western corn rootworm); Diabrotica longicornis barberi (northern corn rootworm); Diabrotica undecimpunctata howardi (southern corn rootworm); Melanotus spp. (wireworms); Cyclocephala borealis (northern masked chafer; white grub); Cyclocephala immaculata (southern masked chafer; white grub); Popillia japonica (Japanese beetle); Chaetocnema pulicaria (corn flea beetle); Sphenophorus maidis (maize billbug); Rhopalosiphum maidis (corn leaf aphid); Anuraphis maidiradicis (corn root aphid); Blissus leucopterus leucopterus (chinch bug); Melanoplus femurrubrum (redlegged grasshopper); Melanoplus sanguinipes (migratory grasshopper); Hylemva platura (seedcom maggot); Agromyza parvicornis (corn blot leafminer); Anaphothrips obscrurus (grass thrips); Solenopsis milesta (thief ant); Tetranychus urticae (twospotted spider mite).

8. Sorghum:

Pilz-, bakterielle oder virale Pathogene: Exserohilum turcicum, Colletotrichum graminicola (Glomerella graminicola), Cercospora sorghi, Gloeocercospora sorghi, Ascochyta sorghina, Pseudomonas syringae p.v. syringae, Xanthomonas campestris p.v. holcicola, Pseudomonas andropogonis, Puccinia purpurea, Macrophomina phaseolina, Perconia circinata, Fusarium monilifonne, Alternaria alternate, Bipolaris sorghicola, Helminthosporium sorghicola, Curvularia lunata, Phoma insidiosa, Pseudomonas avenae (Pseudomonas alboprecipitans), Ramulispora sorghi, Ramulispora sorghicola, Phyllachara sacchari, Sporisorium reilianum (Sphacelotheca reiliana), Sphacelotheca cruenta, Sporisorium sorghi, Sugarcane mosaic H, Maize Dwarf Mosaic Virus A & B, Claviceps sorghi, Rhizoctonia solani, Acremonium strictum, Sclerophthona macrospora, Peronosclerospora sorghi, Peronosclerospora philippinensis, Sclerospora graminicola, Fusarium graminearum, Fusarium oxysporum, Pythium arrhenomanes, Pythium graminicola.

Pathogene Insekten / Nematoden: Chilo partellus (sorghum borer); Spodoptera frugiperda (fall armyworm); Helicoverpa zea (corn ear-worm); Elasmopalpus lignosellus (lesser cornstalk borer); Feltia subterranea (granulate cutworm); Phvllophaga crinita (white grub); Eleodes, Conoderus und Aeolus spp. (wireworm); Oulema melanopus (cereal leaf beetle); Chaetocnema pulicaria (corn flea beetle); Sphenophorus maidis (maize billbug); Rhopalosiphum maidis (corn leaf aphid); Siphaflava (yellow sugarcane aphid); Blissus leucopterus leucopterus (chinch bug); Contarinia sorghicola (sorghummidge); Tetranychus cinnabarinus (carmine spider mite); Tetranychus urticae (two spotted spider mite).

9. Baumwolle:

Pathogene Insekten / Nematoden: Heliothis virescens (cotton budworm); Helicoverpa zea (cotton bollworm); Spodoptera exigua (beet armyworm); Pectinophora gossypiella (pink bollworm); Anthonomus grandis grandis (boll weevil); Aphis gossypii (cotton aphid); Pseudatomoscelis seriatus (cotton fleahopper); Trialeurodes abutilonea (bandedwinged whitefly); Lygus lineolaris (tarnished plant bug); Melanoplus femurrubrum (redlegged grasshopper); Melanoplus differentialis (differential grasshopper); Thrips tabaci (onion thrips); Franklinkiella fusca (tobacco thrips); Tetranychus cinnabarinus (carmine spider mite); Tetranychus urticae (twospotted spider mite);

10. Reis:

Pathogene Insekten / Nematoden: Diatraea saccharalis (sugarcane borer); Spodoptera frugiperda (fall armyworm); Helicoverpa zea (corn earworm); Colaspis brunnea (grape colaspis); Lissorhoptrus oryzophilus (rice water weevil); Sitophilus oryzae (rice weevil); Nephotettix nigropictus (rice leafhopper); Blissus leucopterus leucopterus (chinch bug); Acrosternum hilare (green stink bug);

11. Raps:

Pathogene Insekten / Nematoden: Brevicoryne brassicae (cabbage aphid); Phyilotreta cruciferae (Flea beetle); Mamestra conjgurata (Bertha armyworm); Plutella xylostella (Diamondback moth); Delia ssp. (Root maggots).

"RacB-Protein" meint im Rahmen der Erfindung das RacB-Protein aus Gerste gemäß SEQ ID NO: 2, sowie seine Homologen aus Reis (Oryza sative) gemäß SEQ ID NO: 4 und Mais (Zea mays) gemäß SEQ ID NO: 6 als auch funktionelle Äquivalente der vorgenannten.

"Proteinmenge" meint die Menge eines RacB-Polypeptides in einem Organismus, einem Gewebe, einer Zelle oder einem Zellkompartiment. "Verminderung" der Proteinmenge meint die mengenmäßige Verminderung der Menge eines RacB-Proteins in einem Organismus, einem Gewebe, einer Zelle oder einem Zellkompartiment - beispielsweise durch eines der unten beschriebenen Verfahren - im Vergleich zu dem Wildtyp derselben Gattung und Art auf den dieses Verfahren nicht angewendet wurde, unter ansonst gleichen Rahmenbedingungen (wie beispielsweise Kulturbedingungen, Alter der Pflanzen etc.). Der Verminderung beträgt dabei mindestens 10 %, bevorzugt mindestens 10 % oder mindestens 20 %, besonders bevorzugt um mindestens 40 % oder 60 %, ganz besonders bevorzugt um mindestens 70 % oder 80 %, am meisten bevorzugt um mindestens 90 % oder 95%.

"Aktivität" meint bevorzugt die GTPase Aktivität eines RacB-Polypeptides in einem Organismus, einem Gewebe, einer Zelle oder einem Zellkompartiment. "Verminderung" der Aktivität meint die Verminderung der Gesamt-Aktivität eines RacB-Proteins in einem Organismus, einem Gewebe, einer Zelle oder einem Zellkompartiment - beispielsweise durch eines der unten beschriebenen Verfahren - im Vergleich zu dem Wildtyp derselben Gattung und Art auf den dieses Verfahren nicht angewendet wurde, unter ansonst gleichen Rahmenbedingungen (wie beispielsweise Kulturbedingungen, Alter der Pflanzen etc.). Der Verminderung beträgt dabei mindestens 10 %, bevorzugt mindestens 10 % oder mindestens 20 %, besonders bevorzugt um mindestens 40 % oder 60 %, ganz besonders bevorzugt um mindestens 70 % oder 80 %, am meisten bevorzugt um mindestens 90 % oder 95 %.

"Funktion" meint bevorzugt die Substratbindekapazität eines RacB-Polypeptides in einem Organismus, einem Gewebe, einer Zelle oder einem Zellkompartiment. Als Substrate kommen niedermolekulare Verbindungen wie GTP aber auch die Proteininteraktionspartner eines RacB-Proteins in Frage. "Verminderung" der Funktion meint beispielsweise die mengenmäßige Verminderung der Bindekapazität oder Bindestärke eines RacB-Proteins zu mindestens einem Substrat in einem Organismus, einem Gewebe, einer Zelle oder einem Zellkompartiment - beispielsweise durch eines der unten beschriebenen Verfahren - im Vergleich zu dem Wildtyp derselben Gattung und Art auf den dieses Verfahren nicht angewendet wurde, unter ansonst gleichen Rahmenbedingungen (wie beispielsweise Kulturbedingungen, Alter der Pflanzen etc.). Unter Verminderung ist auch die Veränderung der Substratspezifität zu verstehen, wie sie beispielsweise durch den kcat/Km-Wert ausgedrückt werden kann. Der Verminderung beträgt dabei mindestens 10 %, bevorzugt mindestens 10 % oder mindestens 20%, besonders bevorzugt um mindestens 40 % oder 60 %, ganz besonders bevorzugt um mindestens 70 % oder 80 %, am meisten bevorzugt um mindestens 90 % oder 95 %. Bindepartner für RacB können beispielsweise durch das Hefe-2-Hybridsystem in der dem Fachmann geläufigen Weise identifiziert werden.

Verfahren zur Bestimmung der Proteinmenge, der Aktivität von GTPasen oder der Substratbindekapazität sind dem Fachmann bekannt und vielfach für GTPasen, wie auch für Rac-Proteine aus verschiedenen Gattungen und Arten beschrieben (u.a. Benard V et al. (1999) J Biol Chem 274(19):13198-204;Burstein ES (1998) Oncogene. 17(12):1617-23).

"Funktionelle Äquivalente" eines RacB-Proteins meint bevorzugt solche Sequenzen, die von einem RacB-Protein beschrieben durch SEQ ID NO: 2, 4 oder 6 abgeleitet oder zu diesem homolog sind und im wesentlichen die gleichen Eigenschaften aufweisen.

"Im wesentlichen gleiche Eigenschaften" eines funktionellen Äquivalentes meint vor allem die Verleihung eines pathogenresistenten Phänotypes oder die Verleihung oder Steigerung der Pathogenresistenz gegen zumindest ein Pathogen bei Verminderung der Proteinmenge, Aktivität oder Funktion des besagten funktionellen RacB Äquivalentes in einer Pflanze bzw. in einem Gewebe, Teil oder Zellen derselben. Ferner ist das Ausbleiben eines spontan-induzierten Zelltodes bei besagter Verminderung der Proteinmenge, Aktivität oder Funktion des funktionellen Äquivalentes als wesentliche Eigenschaft zu verstehen.

Dabei kann die Effizienz der Pathogenresistenz sowohl nach unten als auch nach oben im Vergleich zu einem Wert erhalten bei Verminderung eines der RacB-Proteine gemäß SEQ ID NO: 2, 4 oder 6 abweichen. Bevorzugt sind solche funktionelle Äquivalente, bei denen sich die Effizienz der Pathogenresistenz - gemessen beispielsweise an der Penetrationseffizienz eines Pathogens (Haustoriumbildung) - um nicht mehr als 50 %, bevorzugt 25 %, besonders bevorzugt 10 % von einem Vergleichswert erhalten unter Verminderung eines RacB-Proteins gemäß NO:2, 4 oder 6 unterscheidet. Besonders bevorzugt sind solche Sequenzen, bei deren Verminderung die Effizienz der Pathogenresistenz quantitativ um mehr als 50 %, bevorzugt 100 %, besonders bevorzugt 500 %, ganz besonders bevorzugt 1000 % einen Vergleichswert erhalten bei Verminderung eines der RacB-Protein gemäß SEQ ID NO: 2, 4 oder 6 übersteigt.

Der Vergleich wird bevorzugt unter analogen Bedingungen durch geführt. "Analoge Bedingungen- bedeutet, dass alle Rahmenbedingungen wie beispielsweise Kultur- oder Zuchtbedingungen, Assaybedingungen (wie Puffer, Temperatur, Substrate, Pathogenkonzentration etc.) zwischen den zu vergleichenden Versuchen identisch gehalten werden und die Ansätze sich allein durch die Sequenz der zu vergleichenden RacB-Polypeptide, ihrem Ursprungsorganismus und gegebenenfalls dem Pathogen unterscheiden. Bei Wahl des Pathogens ist für den Vergleich jeweils das Pathogen zu wählen, das dem jeweils anderen - unter Berücksichtigung der Artspezifität - am nächsten kommt.

"Funktionelle Äquivalente" meint insbesondere natürliche oder künstliche Mutationen der RacB-Polypeptide gemäß SEQ ID NO: 2, 4 oder 6 sowie homologe Polypeptide aus anderen Pflanzen, welche weiterhin im wesentlichen gleiche Eigenschaften aufweisen. Bevorzugt sind homologe Polypeptide aus oben beschriebenen bevorzugten Pflanzen. Die zu den im Rahmen dieser Erfindung offenbarten RacB Sequenzen homologen Sequenzen aus anderen Pflanzen (beispielsweise Arabidopsis thaliana) können z.B. durch Datenbanksuche oder Durchmustern von Gen-Banken - unter Verwendung der RacB-Sequenzen als Suchsequenz vzw. Sonde - leicht aufgefunden werden.

Mutationen umfassen Substitutionen, Additionen, Deletionen, Inversion oder Insertionen eines oder mehrerer Aminosäurereste. Somit werden beispielsweise auch solche Polypeptide durch die vorliegende Erfindung mit umfasst, welche man durch Modifikation eines Polypeptides gemäß SEQ ID NO: 2, 4 oder 6 erhält.

Unter Homologie zwischen zwei Nukleinsäuresequenzen wird die Identität der Nukleinsäuresequenz über die jeweils gesamte Sequenzlänge verstanden, die durch Vergleich mit Hilfe des Programmalgorithmus GAP (Wisconsin Package Version 10.0, University of Wisconsin, Genetics Computer Group (GCG), Madison, USA; Altschul et al. (1997) Nucleic Acids Res. 25:3389ff) unter Einstellung folgender Parameter berechnet wird:

| | |
|---|---|
| Gap Weight: 50 | Length Weight: 3 |
| Average Match: 10 | Average Mismatch: 0 |

Beispielhaft wird unter einer Sequenz, die eine Homologie von mindestens 80 % auf Nukleinsäurebasis mit der Sequenz SEQ ID NO: 1 aufweist, eine Sequenz verstanden, die bei einem Vergleich mit der Sequenz SEQ ID NO: 1 nach obigem Programmalgorithmus mit obigem Parametersatz eine Homologie von mindestens 80 % aufweist.

Unter Homologie zwischen zwei Polypeptiden wird die Identität der Aminosäuresequenz über die jeweils gesamte Sequenzlänge verstanden, die durch Vergleich mit Hilfe des Programmalgorithmus GAP (Wisconsin Package Version 10.0, University of Wisconsin, Genetics Computer Group (GCG), Madison, USA) unter Einstellung folgender Parameter berechnet wird:

| | |
|---|---|
| Gap Weight: 8 | Length Weight: 2 |
| Average Match: 2,912 | Average Mismatch:-2,003 |

Beispielhaft wird unter einer Sequenz, die eine Homologie von mindestens 80 % auf Proteinbasis mit der Sequenz SEQ ID NO: 2 aufweist, eine Sequenz verstanden, die bei einem Vergleich mit der Sequenz SEQ ID NO: 2 nach obigem Programmalgorithmus mit obigem Parametersatz eine Homologie von mindestens 80% aufweist.

Funktionelle Äquivalente, abgeleitet von einem Polypeptid gemäß SEQ ID NO: 2, 4 oder 6 durch Substitution, Insertion oder Deletion, haben eine Homologie von mindestens 60 %, bevorzugt mindestens 80 %, vorzugsweise mindestens 90 %, besonders bevorzugt mindestens 95 %, ganz besonders bevorzugt mindestens 98 % zu einem Polypeptid gemäß SEQ ID NO: 2, 4 oder 6 und zeichnen sich durch im wesentlichen gleiche Eigenschaften wie das Polypeptid gemäß SEQ ID NO: 2, 4 oder 6 aus.

Funktionelle Äquivalente, abgeleitet von der Nukleinsäuresequenz gemäß SEQ ID NO: 1, 3 oder 5 durch Substitution, Insertion oder Deletion, haben eine Homologie von mindestens 60 %, bevorzugt 80 %, vorzugsweise mindestens 90 %, besonders bevorzugt mindestens 95 %, ganz besonders bevorzugt mindestens 98 % zu einem Polypeptid gemäß SEQ ID NO: 1, 3 oder 5 und kodieren für Polypeptide mit im wesentlichen den gleiche Eigenschaften wie das Polypeptide gemäß SEQ ID NO: 2, 4 oder 6.

Bevorzugt weisen die als funktionelle Äquivalente umfassten racB-Proteine mindestens eines der nachfolgenden sequenzmotive auf:

a) Eine G1-Elementes GXXXXGKS/T bevorzugt im N-terminalen Bereich. Ganz besonders bevorzugt ein Element mit der Sequenz GDGAVGKT, am meisten bevorzugt ein Element mit der Sequenz KCVTVGDGAVGKTC.

b) Eine G2-Effektorregion beinhaltend ein Sequenzmotiv mit PTVFDN, besonders bevorzugt NTFPTDYVPTVFDNFSANW.

c) Ein G3-Elemente beinhaltend LWDTAGQ, besondes bevorzugt NLGLWDTAGQEDYN

d) Ein G4-Elementes TKXD, besonders bevorzugt TKLD, ganz besonders bevorzugt LVGTKLDLRDDKQ

e) Ein. G5 Elementes EXS, bevorzugt ECSS, ganz besonder bevorzugt ECSSKTG

f) Ein C-terminales Isoprenylierungsmotives (CXXX, Hassanain HH et al. (2000) Biochem Biophys Res Commun. 272(3):783-8.), besonders bevorzugt CSIL.

Besonders bevorzugt kommen mindestens 2 oder 3 dieser Motive (a bis f) in einem funktionell äquivalenten RacB-Protein vor, ganz besonders bevorzugt mindestens 4 oder 5, am meisten bevorzugt alle Motive a bis f. Weitere RacB typischen Sequenzmotive, insbesondere auch Motive zur Abgrenzung gegen Rac1-Proteine, kann der Fachmann unschwer aus dem Sequenzvergleich der bekannten RacB (bzw. Rac1) Proteine - wie in Fig. 1 dargestellt - ableiten.

Beispiele für die in dem erfindungsgemäßen Verfahren zu vermindernden funktionellen Äquivalente zu den RacB-Proteinen gemäß SEQ ID NO: 2, 4 oder 6 lassen sich beispielsweise aus verschiedenen Organismen, deren genomische Sequenz bekannt ist, wie beispielsweise aus Arabidopsis thaliana, Brassica napus, Nicotiana tabacum, Solanum tuberosum, Helianthinum, durch Homologievergleiche aus Datenbanken auffinden.

Auch die Durchmusterung von cDNA- oder genomischen-Bibliotheken anderer Organismen, bevorzugt von den weiter unten genannten als Wirt zur Transformation geeigneten Pflanzenarten, unter Verwendung der unter SEQ ID NO: 1, 3 oder 5 beschriebene Nukleinsäuresequenzen oder Teilen derselben als Sonde, ist ein dem Fachmann geläufiges Verfahren, um Homologe in anderen Arte zu identifizieren. Dabei haben die von den Nukleinsäuresequenzen gemäß SEQ ID NO: 1, 3 oder 5 abgeleiteten Sonden eine Länge von mindestens 20 bp, bevorzugt mindestens 50 bp, besonders bevorzugt mindestens 100 bp, ganz besonders bevorzugt mindestens 200 bp, am meisten bevorzugt mindestens 400 bp. Für die Durchmusterung der Bibliotheken kann auch ein zu den unter SEQ ID NO: 1, 3 oder 5 beschriebenen Sequenzen komplementärer DNA-Strang eingesetzt werden.

Funktionelle Äquivalente umfasst demgemäß DNA Sequenzen, die unter Standardbedingungen mit der durch SEQ ID NO:1, 3 oder 5 beschriebenen RacB Nukleinsäuresequenz, der zu ihr komplementären Nukleinsäuresequenz oder teilen der vorgenannten hybridisieren und als vollständige Sequenzen für Proteine kodieren, die die gleichen Eigenschaften, wie die unter SEQ ID NO: 2, 4 oder 6 beschriebenen Proteine verfügen.

"Standardhybridisierungsbedingungen" ist breit zu verstehen und meint stringente als auch weniger stringente Hybridisierungsbedingungen. Solche Hybridisierungsbedingungen sind unter anderem bei Sambrook J, Fritsch EF, Maniatis T et al., in Molecular Cloning (A Laboratory Manual), 2. Auflage, Cold Spring Harbor Laboratory Press, 1989, Seiten 9.31-9.57) oder in Current Protocols in Molecular Biology, John Wiley &Sons, N.Y. (1989), 6.3.1-6.3.6. beschrieben.

Beispielhaft können die Bedingungen während des Waschschrittes ausgewählt sein aus dem Bereich von Bedingungen begrenzt von solchen mit geringer Stringenz (mit ungefähr 2X SSC bei 50°C) und solchen mit hoher Stringenz (mit ungefähr 0.2X SSC bei 50°C bevorzugt bei 65°C) (20X SSC: 0,3M Natriumcitrat, 3M NaCl, pH 7.0). Darüberhinaus kann die Temperatur während des Waschschrittes von niedrig stringenten Bedingungen bei Raumtemperatur, ungefähr 22°C, bis zu stärker stringenten Bedingungen bei ungefähr 65°C angehoben werden. Beide Parameter, Salzkonzentration und Temperatur, können gleichzeitig variiert werden, auch kann einer der beiden Parameter konstant gehalten und nur der andere variiert werden. Während der Hybridisierung können auch denaturierende Agenzien wie zum Beispiel Formamid oder SDS eingesetzt werden. In Gegenwart von 50% Formamid wird die Hybridisierung bevorzugt bei 42°C ausgeführt. Einige beispielhafte Bedingungen für Hybridisierung und Waschschritt sind infolge gegeben:

(1) Hybridisierungbedingungen zum Beispiel aus nachfolgenden Bedingungen ausgewählt sein:

a) 4X SSC bei 65°C,

b) 6X SSC bei 45°C,

c) 6X SSC, 100 μg/ml denaturierter, fragmentierte Fischsperma-DNA bei 68°C,

d) 6X SSC, 0,5 % SDS, 100 μg/ml denaturierter, Lachssperma-DNA bei 68°C,

e) 6X SSC, 0,5 % SDS, 100 μg/ml denaturierter, fragmentierte Lachssperma-DNA, 50 % Formamid bei 42°C.

f) 50 % Formamid, 4XSSC bei 42°C, oder

g) 50 % (vol/vol) Formamid, 0,1% Rinderserumalbumin, 0,1 % Ficoll, 0,1 % Polyvinylpyrrolidon, 50 mM Natriumphosphatpuffer pH 6,5, 750 mM NaCl, 75 mM Natriumcitrate bei 42°C, oder

h) 2X oder 4X SSC bei 50°C (schwach stringente Bedingung),

i) 30 bis 40 % Formamid, 2X oder 4X SSC bei 42°C (schwach stringente Bedingung).

(2) Waschschritte können zum Beispiel aus nachfolgenden Bedingungen ausgewählt sein:

a) 0,015 M NaCl/0,0015 M Natriumcitrat/0,1 % SDS bei 50°C.

b) 0.1X SSC bei 65°C.

c) 0,1X SSC, 0,5 % SDS bei 68°C.

d) 0,1X SSC, 0,5 % SDS, 50 % Formamid bei 42°C.

e) 0,2X SSC, 0,1 % SDS bei 42°C.

f) 2X SSC bei 65°C (schwach stringente Bedingung).

Funktionelle Äquivalente abgeleitet von einem Polypeptid gemäß SEQ ID NO: 2, 4 oder 6 umfasst insbesondere auch die Proteine mit der SEQ ID NO: 35, 37, 39, 41, 43, 45, 47, 50, 52, 54, 56, 58, 60, 62, 64, 66, 68 oder 70. Insbesondere meint funktionelle Äquivalente Proteine, die durch eine Nukleinsäuresequenz mit der SEQ ID NO: 34, 36, 38, 40, 42, 44, 46, 48, 49, 51, 53, 55, 57, 61, 63, 65, 67 oder 69 kodiert werden.

Die Verminderung der Expression eines RacB-Proteins, der RacB-Aktivität oder der RacB-Funktion kann auf vielfältige Art und Weise realisiert werden.

"Verminderung" oder "vermindern" ist im Zusammenhang mit einem RacB Protein, einer RacB Aktivität oder RacB-Funktion weit auszulegen und umfasst die teilweise oder im wesentlichen vollständige, auf unterschiedliche zellbiologische Mechanismen beruhende Unterbindung oder Blockierung der Funktionalität eines RacB-Proteins in einer Pflanze oder einem davon abgeleiteten Teil, Gewebe, Organ, Zellen oder Samen. Eine Verminderung im Sinne der Erfindung umfasst auch eine mengenmäßige Verringerung eines RacB-Proteins bis hin zu einem im wesentlichen vollständigen Fehlen des RacB-Proteins (d.h. fehlende Nachweisbarkeit von RacB-Aktivität bzw. RacB-Funktion oder fehlende immunologische Nachweisbarkeit des RacB-Proteins). Dabei wird die Expression eines bestimmten RacB-Proteins oder die RacB-Aktivität bzw. RacB-Funktion in einer Zelle oder einem Organismus bevorzugt um mehr als 50 %, besonders bevorzugt um mehr als 80 %, ganz besonders bevorzugt um mehr als 90% vermindert.

Erfindungsgemäß sind verschiedene Strategien zur Verminderung der Expression eines RacB-Proteins, der RacB-Aktivität oder RacB-Funktion umfasst. Der Fachmann erkennt, dass eine Reihe verschiedener Methoden zur Verfügung stehen, um die Expression eines RacB-Proteins, die RacB-Aktivität oder die RacB-Funktion in gewünschter Weise zu beeinflussen.

Eine Verminderung der RacB-Aktivität oder der RacB-Funktion wird bevorzugt durch eine verminderte Expression eines endogenen RacB-Proteins erreicht.

Eine Verminderung der RacB-Proteinmenge, RacB-Aktivität oder RacB-Funktion kann unter Verwendung nachfolgender Verfahren realisiert werden:

a) Einbringung einer doppelsträngigen RacB RNA-Nukleinsäuresequenz (RacB-dsRNA) oder einer deren Expression gewährleistenden Expressionskassette oder Expressionskassetten;

b) Einbringung einer RacB antisense-Nukleinsäuresequenzen oder einer deren Expression gewährleistenden Expressionskassette. Umfasst sind solche Verfahren, bei denen die antisense-Nukleinsäuresequenz gegen ein RacB-Gen (also genomische DNA-Sequenzen) oder ein RacB-Gentranskript (also RNA-Sequenzen) gerichtet ist. Umfasst sind auch $\alpha$-anomere Nukleinsäuresequenzen.

c) Einbringung einer RacB antisense-Nukleinsäuresequenzen kombiniert mit einem Ribozym oder einer deren Expression gewährleistenden Expressionskassette

d) Einbringung von RacB sense-Nukleinsäuresequenzen zur Induktion einer Kosuppression oder einer deren Expression gewährleistenden Expressionskassette

e) Einbringung einer Nukleinsäuresequenz kodierend für dominant-negatives RacB Protein oder einer deren Expression gewährleistenden Expressionskassette

f) Einbringung DNA-oder Protein-bindende Faktoren gegen RacB -Gene, -RNAs oder -Proteine oder einer deren Expression gewährleistenden Expressionskassette

g) Einbringung von den RacB RNA-Abbau bewirkende virale Nukleinsäuresequenzen und Expressionskonstrukten oder einer deren Expression gewährleistenden Expressionskassette

h) Einbringung von Konstrukten zur Induktion einer homologen Rekombination an endogenen RacB-Genen beispielsweise zur Erzeugung von Knockout-Mutanten.

i) Einführung von Mutationen in endogenen RacB Gene zur Erzeugung eines Funktionsverlustes (z.B. Generierung von Stopp-Kodons, Verschiebungen im Leseraster etc.)

Dabei kann jedes einzelne dieser Verfahren eine Verminderung der RacB-Expression, RacB-Aktivität oder RacB-Funktion im Sinne der Erfindung bewirken. Auch eine kombinierte Anwendung ist denkbar. Weitere Methoden sind dem Fachmann bekannt und können die Behinderung oder Unterbindung der Prozessierung des RacB-Proteins, des Transports des RacB-Proteins oder dessen mRNA, Hemmung der Ribosomenanlagerung, Hemmung des RNA-Spleißens, Induktion eines RacB-RNA abbauenden Enzyms und/oder Hemmung der Translationselongation oder -termination umfassen.

Die einzelnen bevorzugten Verfahren seien infolge kurz beschrieben:

a) Einbringung einer doppelsträngigen RacB RNA-Nukleinsäuresequenz (RacB-dsRNA)
Das Verfahren der Genregulation mittels doppelsträngiger RNA ("double-stranded RNA interference"; dsRNAi) ist vielfach in tierischen und pflanzlichen Organismen beschrieben (z.B. Matzke MA et al. (2000) Plant Mol Biol 43: 401-415; Fire A. et al (1998) Nature 391:806-811; WO 99/32619; WO 99/53050; WO 00/68374; WO 00/44914; WO 00/44895; WO 00/49035; WO 00/63364). Auf die in den angegebenen Zitaten beschriebenen Verfahren und Methoden wird ausdrücklich Bezug genommen. Eine effiziente Gensuppression kann auch bei transienter Expression oder nach transienter Transformation beispielsweise infolge einer biolistischen Transformation gezeigt werden (Schweizer P et al. (2000) Plant J 2000 24: 895-903). dsRNAi-Verfahren beruhen auf dem Phänomen, dass durch gleichzeitiges Einbringen von komplementären Strang- und Gegenstrang eines Gentranskriptes eine hocheffiziente Unterdrückung der Expression des entsprechenden Gens bewirkt wird. Der bewirkte Phänotyp kommt dem einer entsprechenden knock-out Mutanten sehr ähnlich (Waterhouse PM et al. (1998) Proc Natl Acad Sci USA 95: 13959-64).
Das dsRNAi-Verfahren hat sich bei der Verminderung der RacB-Expression als besonders effizient und vorteilhaft erwiesen. Wie u.a. in WO 99/32619 beschrieben sind dsRNAi-Ansätze klassischen antisense-Ansätzen deutlich überlegen.
Ein weiterer Gegenstand der Erfindung bezieht sich daher auf doppelsträngige RNA-Moleküle (dsRNA-Moleküle), die bei Einführung in eine Pflanze (oder eine davon abgeleitete Zelle, Gewebe, Organ oder Samen) die Verminderung eines RacB bewirken.
Das doppelsträngiges RNA-Molekül zur Verminderung der Expression eines RacB Proteins ist dadurch gekennzeichnet, dass

a) einer der beiden RNA Stränge im wesentlichen identisch ist zu zumindest einem Teil einer RacB-Nukleinsäuresequenz, und

b) der jeweils andere RNA Strang im wesentlichen identisch ist zu zumindest einem Teil des komplementären Stranges einer RacB Nukleinsäuresequenz.

In einer weiterhin bevorzugten Ausführungsform umfasst das doppelsträngige RNA-Molekül zur Verminderung der Expression eines RacB Proteins

a) einen "sense"-RNA-Strang umfassend mindestens eine Ribonukleotidsequenz, die im wesentlichen identisch ist zu mindestens einem Teil des "sense"-RNA-Transkriptes einer Nukleinsäuresequenz kodierend für ein RacB Protein, und

b) einen "antisense"-RNA-Strang, der zu dem RNA-sense-Strang unter a) im wesentlichen - bevorzugt vollständig - komplementären ist.

In Bezug auf die doppelsträngigen RNA-Moleküle meint RacB-Nukleinsäuresequenz bevorzugt eine Sequenz gemäß SEQ ID NO: 1, 3 oder 5 oder ein funktionelles Äquivalent derselben gemäß SEQ ID NO: 34, 36, 38, 40, 42, 44, 46, 48, 49, 51, 53, 55, 57, 61, 63, 65, 67 oder 69.
Im wesentlichen identisch" meint, dass die dsRNA Sequenz auch Insertionen, Deletionen sowie einzelne Punktmutationen im Vergleich zu der RacB Zielsequenz oder einer funktionell äquivalenten Zielsequenz aufweisen kann und dennoch eine effiziente Verminderung der Expression bewirken. Bevorzugt beträgt die Homologie nach obiger Definition mindestens 75 %, bevorzugt mindestens 80 %, ganz besonders bevorzugt mindestens 90 % am meisten bevorzugt 100 % zwischen dem "sense"-Strang einer inhibitorischen dsRNA und mindestens einem Teil des "sense"-RNA-Transkriptes einer Nukleinsäuresequenz kodierend für ein RacB Protein oder ein funktionelles Äquivalent desselben (bzw. zwischen dem "antisense"-Strang dem komplementären Strang einer Nukleinsäuresequenz kodierend für ein RacB Protein oder ein funktionelles Äquivalent desselben).
Die Länge des Teilabschnittes beträgt mindestens 10 Basen, bevorzugt mindestens 25 Basen, besonders bevorzugt mindestens 50 Basen, ganz besonders bevorzugt mindestens 100 Basen, am meisten bevorzugt mindestens 200 Basen oder mindestens 300 Basen.
Alternativ, kann eine "im wesentlichen identische" dsRNA auch als Nukleinsäuresequenz definiert werden, die befähigt ist, mit einem Teil eines Speicherprotein Gentranskriptes zu hybridisieren (z.B. in 400 mM NaCl, 40 mM PIPES pH 6,4, 1 mM EDTA bei 50°C oder 70°C für 12 bis 16 h).
"Im wesentlichen komplementär" meint, dass der "antisense"-RNA-Strang auch Insertionen, Deletionen sowie einzelne Punktmutationen im Vergleich zu dem Komplement des "sense"-RNA-Stranges aufweisen kann. Bevorzugt beträgt die Homologie mindestens 80%, bevorzugt mindestens 90%, ganz besonders bevorzugt mindestens 95 %, am meisten bevorzugt 100% zwischen dem "antisense"-RNA-Strang und dem Komplement des"sense"-RNA-Strangs.
"Teil des "sense"-RNA-Transkriptes" einer Nukleinsäuresequenz kodierend für ein RacB Protein oder ein funktionelles Äquivalent desselben meint Fragmente einer RNA oder mRNA transkibiert von einer für ein RacB-Protein oder ein funktionelles Äquivalent desselben kodierenden Nukleinsäuresequenz, bevorzugt von einem RacB-Gen. Dabei haben die Fragmente bevorzugt eine Sequenzlänge von mindestens 20 Basen, bevorzugt mindestens 50 Basen, besonders bevorzugt mindestens 100 Basen, ganz besonders bevorzugt mindestens 200 Basen, am meisten bevorzugt mindestens 500 Basen. Umfasst ist auch die vollständige transkribierte RNA oder mRNA.
Umfasst ist auch die Verwendung der dsRNA-Moleküle in den erfindungsgemäßen Verfahren zur Erzeugung einer Pathogenresistenz in Pflanzen.
Die dsRNA kann aus einem oder mehr Strängen polymerisierter Ribonukleotide bestehen. Es können ferner Modifikationen sowohl des Zucker-Phosphat-Gerüstes als auch der Nukleoside vorliegen. Beispielsweise können die Phosphodiesterbindungen der natürlichen RNA dahingehend modifiziert sein, dass sie zumindest ein Stickstoff oder Schwefel-Heteroatom umfassen. Basen können dahingehend modifiziert werden, dass die Aktivität beispielsweise von Adenosindeaminase eingeschränkt wird. Solche und weitere Modifikationen sind weiter unten bei den Verfahren zur Stabilisierung von antisense-RNA beschrieben.
Natürlich können, um den gleichen Zweck zu erreichen, auch mehrere individuelle dsRNA Moleküle, die jeweils einen der oben definierten Ribonukleotidsequenzabschnitte umfassen, in die Zelle oder den Organismus eingebracht werden.
Die dsRNA kann enzymatisch oder ganz oder teilweise chemisch-synthetisch hergestellt werden.
Die doppelsträngige dsRNA Struktur kann ausgehend von zwei komplementären, separaten RNA-Strängen oder - bevorzugt - ausgehend von einem einzelnen, selbstkomplementären RNA-Strang gebildet werden.
Bei einem einzelnen, selbstkomplementären Strang, können "sense"- und "antisense"-Sequenz durch eine verbindende Sequenz ("Linker") verknüpft sein und beispielsweise eine Haarnadelstruktur ausbilden. Bevorzugt kann die verbindende Sequenz ein Intron sein, das nach Synthese der dsRNA herausgespleißt wird.
Die Nukleinsäuresequenz kodierend für eine dsRNA kann weitere Elemente beinhalten, wie beispielsweise Transkriptionsterminationssignale oder Polyadenylierungssignale.
Sollen die zwei Stränge der dsRNA in einer Zelle oder Pflanze zusammengebracht werden, so kann dies auf verschiedene Art geschehen:

a) Transformation der Zelle oder Pflanze mit einem Vektor, der beide Expressionskassetten umfasst,

b) Kotransformation der Zelle oder Pflanze mit zwei Vektoren, wobei der eine die Expressionskassetten mit dem "sense"-Strang, der andere die Expressionskassetten mit dem "antisense"-Strang umfasst.

c) Kreuzung von zwei Pflanzen, die mit jeweils einem Vektor transformiert wurden, wobei der eine die Expressionskassetten mit dem "sense"-Strang, der andere die Expressionskassetten mit dem "antisense"-Strang umfasst.

Die Bildung der RNA Duplex kann entweder außerhalb der Zelle oder innerhalb derselben initiiert werden. Wie in WO 99/53050 kann die dsRNA auch eine Haarnadelstruktur umfassen, indem "sense"- und "antisense"-Strang durch einen "Linker" (beispielsweise ein Intron) verbunden werden. Die selbstkomplementären dsRNA-Strukturen sind bevorzugt, da sie lediglich die Expression eines Konstruktes erfordern und die komplementären Stränge stets in einem äquimolaren Verhältnis umfassen.

Die Expressionskassetten kodierend für den "antisense"- oder "sense"-Strang einer dsRNA oder für den selbstkomplementären-Strang der dsRNA, werden bevorzugt in einen Vektor insertiert und mit den unten beschriebenen Verfahren stabil (beispielsweise unter Verwendung von Selektionsmarkern) in das Genom einer Pflanze insertiert, um eine dauerhafte Expression der dsRNA zu gewährleisten.

Die dsRNA kann unter Verwendung einer Menge eingeführt werden, die zumindest ein Kopie pro Zelle ermöglicht. Höhere Mengen (z.B. mindestens 5, 10, 100, 500 oder 1000 Kopien pro Zelle) können ggf. eine effizienter Verminderung bewirken.

Wie bereits beschrieben, ist eine 100%ige Sequenzidentität zwischen dsRNA und einem RacB Gentranskript oder dem Gentranskript eines funktionell äquivalenten Gens nicht zwingend erforderlich, um eine effiziente Verminderung der RacB Expression zu bewirken. Demzufolge besteht der Vorteil, dass das Verfahren tolerant ist gegenüber Sequenzabweichungen, wie sie infolge genetischer Mutationen, Polymorphismen oder evolutionärer Divergenzen vorliegen können. So ist es beispielsweise möglich mit der dsRNA, die ausgehend von der RacB Sequenz des einen Organismus generiert wurde, die RacB Expression in einem anderen Organismus zu unterdrücken. Die hohe Sequenzhomologie zwischen den RacB Sequenzen aus Reis, Mais und Gerste lässt auf einen hohen Konservierungsgrad dieses Proteins innerhalb von Pflanzen schließen, so dass die Expression einer dsRNA abgeleitet von einer der offenbarten RacB Sequenzen gemäß SEQ ID NO: 1, 3 oder 5 auch einen vorteilhaften Effekt in anderen Pflanzenarten haben dürfte.

Auch ist es aufgrund der hohen Homologie zwischen den einzelnen RacB-Proteinen und ihren funktionellen Äquivalenten möglich mit einer einzigen dsRNA, die ausgehend von einer bestimmten RacB-Sequenz eines Organismus generiert wurde, die Expression weiterer homologer RacB-Proteine und/oder deren funktioneller Äquivalente des gleichen Organismus oder aber auch die Expression von RacB-Proteinen in anderen verwandten Arten zu unterdrücken. Zu diesem Zweck umfasst die dsRNA bevorzugt Sequenzbereich von RacB-Gentranskripten, die konservierten Bereichen entsprechen. Besagte konservierte Bereiche können aus Sequenzvergleichen leicht abgeleitet werden.

Die dsRNA kann entweder in vivo oder in vitro synthetisiert werden. Dazu kann eine DNA-Sequenz kodierend für eine dsRNA in eine Expressionskassette unter Kontrolle mindestens eines genetischen Kontrollelementes (wie beispielsweise Promotor, Enhancer, Silencer, Splice-Donor oder -Akzeptor, Polyadenylierungssignal) gebracht werden. Entsprechend vorteilhafte Konstruktionen sind weiter unten beschrieben. Eine Polyadenylierung ist nicht erforderlich, ebenso müssen keine Elemente zur Initiierung einer Translation vorhanden sein.

Eine dsRNA kann chemisch oder enzymatisch synthetisiert werden. Dazu können zelluläre RNA Polymerasen oder Bakteriophagen RNA Polymerasen (wie z.B. T3-, T7- oder SP6 RNA-Polymerase) verwendet werden. Entsprechende Verfahren zu in vitro Expression von RNA sind beschrieben (WO 97/32016; US 5,593,874; US 5,698,425, US 5,712,135, US 5,789,214, US 5,804,693). Eine chemisch oder enzymatisch in vitro syntetisierte dsRNA kann vor der Einführung in eine Zelle, Gewebe oder Organismus aus dem Reaktionsgemisch beispielsweise durch Extraktion, Präzipitation, Elektrophorese, Chromatographie oder Kombinationen dieser Verfahren ganz oder teilweise aufgereinigt werden. Die dsRNA kann unmittelbar in die Zelle eingeführt werden oder aber auch extrazellulär (z.B. in den interstitialen Raum) appliziert werden.

Bevorzugt wird die Pflanze jedoch stabil mit einem Expressionskonstrukt, das die Expression der dsRNA realisiert, transformiert. Entsprechende Verfahren sind weiter unten beschrieben.

b) Einbringung einer RacB antisense-Nukleinsäuresequenz

Verfahren zur Suppression eines bestimmten Proteins durch Verhinderung der Akkumulation seiner mRNA durch die "antisense"-Technologie sind vielfach - auch in Pflanzen - beschrieben (Sheehy et al. (1988) Proc Natl Acad Sci USA 85: 8805-8809; US 4,801,340; Mol JN et al. (1990) FEBS Lett 268(2):427-430). Das antisense Nukleinsäuremolekül hybridisiert bzw. bindet mit der zellulären mRNA und/oder genomischen DNA kodierend für das zu supprimierende RacB-Zielprotein. Dadurch wird die Transkription und/oder Translation des Zielproteins unterdrückt. Die Hybridisierung kann auf konventionelle Art über die Bildung einer stabilen Duplex oder - im Fall von genomischer DNA - durch Bindung des anti- sense Nukleinsäuremoleküls mit der Duplex der genomischen DNA durch spezifische Wechselwirkung in der großen Furche der DNA-Helix entstehen.

Eine antisense Nukleinsäuresequenz geeignet zur Verminderung eines RacB-Proteins kann unter Verwendung der für dieses Protein kodierenden Nukleinsäuresequenz, beispielsweise der Nukleinsäuresequenz gemäß SEQ ID NO: 1, 3 oder 5, oder der Nukleinsäuresequenz kodierend für ein funktionelles Äquivalent derselben, beispielsweise einer Sequenz gemäß SEQ ID NO: 34, 36, 38, 40, 42, 44, 46, 48, 49, 51, 53, 55, 57, 61, 63, 65, 67 oder 69, nach den Basenpaarregeln von Watson und Crick abgeleitet werden. Die antisense Nukleinsäuresequenz kann zu der gesamten transkribierten mRNA des besagten Proteins komplementär sein, sich auf die kodierende Region beschränken oder nur aus einem Oligonukleotid bestehen, das zu einem Teil der kodierenden oder nicht-kodierenden Sequenz der mRNA komplementär ist. So kann das Oligonukleotid beispielsweise komplementär zu der Region sein, die den Translationsstart für das besagte Protein umfasst. Antisense-Nukleinsäuresequenzen können eine Länge von zum Beispiel 5, 10, 15, 20, 25, 30, 35, 40, 45 oder 50 Nukleotide haben, können aber auch länger sein und mindestens 100, 200, 500, 1000, 2000 oder 5000 Nukleotide umfassen. Antisense-Nukleinsäuresequenzen können rekombinant exprimiert oder chemisch bzw. enzymatisch unter Verwendung von dem Fachmann bekannten Verfahren synthetisiert werden. Bei der chemischen Synthese können natürlich oder modifizierte Nukleotide verwendet werden. Modifizierte Nukleotide können der antisense Nukleinsäuresequenz eine erhöhte biochemische Stabilität verleihen und zu einer erhöhten physikalischen Stabilität der Duplex gebildet aus antisense-Nukleinsäuresequenz und sense-Zielsequenz führen. Verwendet werden können beispielsweise Phosphorothioatderivative und Acridin-substitiierte Nukleotide wie 5-Fluorouracil, 5-Bromouracil, 5-Chlorouracil, 5-Iodouracil, Hypoxanthin, Xanthin, 4-Acetylcytosin, 5-(Carboxyhydroxylmethyl)uracil, 5-Carboxy-methylaminomethyl-2-thiouridin, 5-Carboxymethylaminomethyluracil, Dihydrouracil, β-D-Galactosylqueosin, Inosine, N6-Isopentenyladenin, 1-Methylguanin, 1-Methylinosin, 2,2-Dimethylguanin, 2-Methyladenin, 2-Methylguanin, 3-Methylcytosin, 5-Methylcytosin, N6-Adenin, 7-Methylguanin, 5-Methylaminomethyluracil, 5-Methoxyaminomethyl-2-thiouracil, β-D-mannosylqueosin, 5'-Methoxycarboxymethyluracil, 5-Methoxyuracil, 2-Methylthio-N6-isopentenyladenin, Uracil-5-oxyessigsäure, Pseudouracil, Queosine, 2-Thiocytosin, 5-Methyl-2-thiouracil, 2-Thiouracil, 4-Thiouracil, 5-Methyluracil, Uracil-5-oxyessigsäuremethylester, Uracil-5-oxyessigsäure, 5-Methyl- 2-thiouracil, 3-(3-Amino-3-N-2-carboxypropyl)uracil und 2,6-Diaminopurin.

In einer weiteren bevorzugten Ausführungsform kann die Expression eines RacB-Proteins durch Nukleotidsequenzen inhibiert werden, die komplementär zu der regulatorischen Region eines RacB-Gens (z.B. einem RacB Promoter und/oder Enhancer) sind und triple-helikale Strukturen mit der dortigen DNA-Doppelhelix ausbilden, so dass die Transkription des RacB-Gens vermindert wird. Entsprechende Verfahren sind beschrieben (Helene C (1991) Anticancer Drug Res 6(6):569-84; Helene C et al. (1992) Ann NY Acad Sci 660:27-36; Maher LJ (1992) Bioassays 14 (12):807-815).

In einer weiteren Ausführungsform kann das antisense Nukleinsäuremolekül eine α-anomere Nukleinsäure sein. Derartige α-anomere Nukleinsäuremoleküle bilden spezifische doppelsträngige Hybride mit komplementärer RNA in denen - im Unterschied zu den konventionellen β-Nukleinsäuren - die beiden Stränge parallel zueinander verlaufen (Gautier C et al. (1987) Nucleic Acids Res 15:6625-6641). Das antisense Nukleinsäuremolekül kann ferner auch 2'-O-Methylribonukleotide (Inoue et al. (1987) Nucleic Acids Res 15:6131-6148) oder chimäre RNA-DNA Analoge beinhalten (Inoue et al. (1987) FEBS Lett 215:327-330).

c) Einbringung einer RacB antisense-Nukleinsäuresequenz kombiniert mit einem Ribozym.

Vorteilhaft kann die oben beschriebene antisense-Strategie mit einem Ribozym-Verfahren gekoppelt werden. Katalytische RNA-Moleküle oder Ribozyme können an jede beliebige Ziel-RNA angepasst werden und spalten das Phosphodiester-Gerüst an spezifischen Positionen, wodurch die Ziel-RNA funktionell deaktiviert wird (Tanner NK (1999) FEMS Microbiol Rev 23(3):257-275). Das Ribozym wird dadurch nicht selber modifiziert, sondern ist in der Lage, weitere Ziel-RNA-Moleküle analog zu spalten, wodurch es die Eigenschaften eines Enzyms erhält. Der Einbau von Ribozymsequenzen in "antisense"-RNAs verleiht eben diesen "antisense"-RNAs diese enzymähnliche, RNA-spaltende Eigenschaft und steigert so deren Effizienz bei der Inaktivierung der Ziel-RNA. Die Herstellung und Verwendung entsprechender Ribozym-"antisense"-RNA-Moleküle ist beispielsweise beschrieben bei Haseloff et al. (1988) Nature 334: 585-591.

Auf diese Art können Ribozyme (z.B. "Hammerhead"-Ribozyme; Haselhoff und Gerlach (1988) Nature 334:585-591) verwendet werden, um die mRNA eines zu supprimierenden Enzyms - z.B. RacB - katalytisch zu spalten und die Translation zu verhindern. Die Ribozym-Technologie kann die Effizienz einer antisense-Strategie erhöhen. Verfahren zur Expression von Ribozymen zur Verminderung bestimmter Proteine sind beschrieben in (EP 0 291 533, EP 0 321 201, EP 0 360 257). In pflanzlichen Zellen ist eine Ribozym-Expression ebenfalls beschrieben (Steinecke P et al. (1992) EMBO J 11(4):1525-1530; de Feyter R et al. (1996) Mol Gen Genet. 250(3):329-338). Geeignete Zielsequenzen und Ribozyme können zum Beispiel wie bei "Steinecke P, Ribozymes, Methods in Cell Biology 50, Galbraith et al. eds, Academic Press, Inc. (1995), S. 449-460" beschrieben, durch Sekundärstrukturberechnungen von Ribozym- und Ziel-RNA sowie durch deren Interaktion bestimmt werden (Bayley CC et al. (1992) Plant Mol Biol. 18(2):353-361; Lloyd AM and Davis RW et al. (1994) Mol Gen Genet. 242(6):653-657). Beispielsweise können Derivate der Tetrahymena L-19 IVS RNA konstruiert werden, die komplementäre Bereiche zu der mRNA des zu supprimierenden RacB Proteins aufweisen (siehe auch US 4,987,071 und US 5,116,742). Alternativ können solche Ribozyme auch über einen Selektionsprozess aus einer Bibliothek diverser Ribozyme identifiziert werden (Bartel D und Szostak JW (1993) Science 261:1411-1418).

d) Einbringung einer RacB sense-Nukleinsäuresequenz zur Induktion eines Kosuppression

Die Expression einer RacB Nukleinsäuresequenz in sense-Orientierung kann zu einer Kosuppression des entsprechenden homologen, endogenen Gens führen. Die Expression von sense-RNA mit Homologie zu einem endogenen Gen kann die Expression desselben vermindern oder ausschalten , ähnlich wie es für antisense Ansätze beschrieben wurde (Jorgensen et al. (1996) Plant Mol Biol 31(5):957-973; Goring et al. (1991) Proc Natl Acad Sci USA 88:1770-1774; Smith et al. (1990) Mol Gen Genet 224:447-481; Napoli et al. (1990) Plant Cell 2:279-289; Van der Krol et al. (1990) Plant Cell 2:291-99). Dabei kann das eingeführte Konstrukt das zu vermindernde, homologe Gen ganz oder nur teilweise representieren. Die Möglichkeit zur Translation ist nicht erforderlich. Die Anwendung dieser Technologie auf Pflanzen ist beispielsweise beschrieben bei Napoli et al. (1990) The Plant Cell 2: 279-289 und in US 5,034,323.

Bevorzugt wird die Kosuppression unter Verwendung einer Sequenz realisert, die im wesentlichen identisch ist zu zumindest einem Teil der Nukleinsäuresequenz kodierend für ein RacB-Protein oder ein funktionelles Äquivalent desselben, beispielsweise der Nukleinsäuresequenz gemäß SEQ ID NO: 1, 3 oder 5, oder der Nukleinsäuresequenz kodierend für ein funktionelles Äquivalent derselben, beispielsweise einer Sequenz gemäß SEQ ID NO: 34, 36, 38, 40, 42, 44, 46, 48, 49, 51, 53, 55, 57, 61, 63, 65, 67 oder 69.

e) Einbringung von Nukleinsäuresequenzen kodierend für ein dominant-negatives RacB Protein

Die Funktion oder Aktivität eines RacB Protein kann effektiv auch durch Expression einer dominant-negativen Variante dieses RacB-Proteins realisiert werden. Verfahren zur Verminderung der Funktion bzw. Aktivität eines Protein mittels Koexpression seiner dominant-negativen Form sind dem Fachmann bekannt (Lagna G und Hemmati-Brivanlou A (1998) Current Topics in Developmental Biology 36:75-98; Perlmutter RM und Alberola-Ila J (1996) Current Opinion in Immunology 8(2):285-90; Sheppard D (1994) American Journal of Respiratory Cell & Molecular Biology. 11(1):1-6; Herskowitz I (1987) Nature 329(6136):219-22).

Eine dominant-negative RacB Variante kann beispielsweise durch Veränderung der Aminosäure Threonin an Position 20 bei den RacB Proteinen aus Mais, Reis oder Gerste in bevorzugt Asparagin-Säure realisiert werden. Das bevorzugt zu mutierende Threonin oder eventuell auch Serin (analog zu dem Threonin an Position 20 in RacB aus Mais, Reis oder Gerste) in RacB Homologen aus anderen Arten kann beispielsweise mittels Computer-unterstütztem Vergleich ("Alignment") ermittelt werden. Diese Mutationen zum Erreichen einer dominant-negativen RacB Variante werden bevorzugt auf der Ebene der Nukleinsäuresequenz kodierend für RacB Proteine durchgeführt. Eine entsprechende Mutation kann beispielsweise durch PCR vermittelte in vitro Mutagenese unter Verwendung entsprechender Oligonukleotidprimer, durch welche die gewünschte Mutation eingeführt wird, realisiert werden. Dazu werden dem Fachmann geläufige Verfahren verwendet. Beispielsweise kann zu diesem Zweck der "LA PCR in vitro Mutagenesis Kit" (Takara Shuzo, Kyoto) verwendet werden. Ein Verfahren zur Herstellung einer dominant-negativen Variante des RacB-Proteins aus Mais ist auch in WO 00/15815 (Beispiel 4, S. 69) beschrieben.

Besonders bevorzugt sind die unter den SEQ ID NO: 7, 8 und 9 beschriebenen dominant-negativen Varianten der RacB-Proteine aus Gerste, Reis oder Mais.

f) Einbringung DNA-oder Protein-bindende Faktoren gegen RacB Gene, -RNAs oder Proteine

Eine Verminderung einer RacB Genexpression ist auch mit spezifischen DNA-bindenden Faktoren z.B. mit Faktoren vom Typus der Zinkfingertranskriptionsfaktoren möglich. Diese Faktoren lagern sich an die genomische Sequenz des endogenen Zielgens, bevorzugt in den regulatorischen Bereichen, an und bewirken eine Repression des endogenen Gens. Die Verwendung eines solchen Verfahrens ermöglicht die Verminderung der Expression eines endogenen RacB Gens, ohne dass dessen Sequenz gentechnisch manipuliert werden muss. Entsprechende Verfahren zur Herstellung entsprechender Faktoren sind beschrieben (Dreier B et al. (2001) J Biol Chem 276(31): 29466-78; Dreier B et al. (2000) J Mol Biol 303(4):489-502; Beerli RR et al. (2000) Proc Natl Acad Sci USA 97 (4): 1495-1500; Beerli RR et al. (2000) J Biol Chem 275(42):32617-32627; Segal DJ and Barbas CF 3rd. (2000) Curr Opin Chem Biol 4(1):34-39; Kang JS and Kim JS (2000) J Biol Chem 275(12): 8742-8748; Beerli RR et al. (1998) Proc Natl Acad Sci USA 95(25) :14628- 14633; Kim JS et al. (1997) Proc Natl Acad Sci USA 94(8) :3616 -3620; Klug A (1999) J Mol Biol 293(2):215-218; Tsai SY et al. (1998) Adv Drug Deliv Rev 30(1-3):23-31; Mapp AK et al. (2000) Proc Natl Acad Sci USA 97(8):3930-3935; Sharrocks AD et al. (1997) Int J Biochem Cell Biol 29(12): 1371-1387; Zhang L et al. (2000) J Biol Chem 275(43):33850-33860).

Die Selektion dieser Faktoren kann unter Verwendung eines beliebigen Stückes eines RacB-Gens erfolgen. Bevorzugt liegt dieser Abschnitt im Bereich der Promotorregion. Für eine Genunterdrückung kann er aber auch im Bereich der kodierenden Exons oder Introns liegen. Die entsprechenden Abschnitte sind für den Fachmann mittels Datenbankabfrage aus der Genbank oder - ausgehend von einer RacB cDNA, deren Gen nicht in der Genbank vorhanden ist, durch Durchmusterung einer genomischen Bibliothek nach korrespondierenden genomischen Klonen erhältlich. Die dazu erforderlichen Verfahren sind dem Fachmann geläufig.

Ferner können Faktoren in eine Zelle eingebracht werden, die das RacB Zielprotein selber inhibieren. Die proteinbindenden Faktoren können z.B. Aptamere (Famulok M und Mayer G (1999) Curr Top Microbiol Immunol 243: 123-36) oder Antikörper bzw. Antikörperfragmente oder einzelkettige Antikörper sein. Die Gewinnung dieser Faktoren ist beschrieben und dem Fachmann bekannt. Beispielsweise wurde ein cytoplasmatischer scFv Antikörper eingesetzt, um die Aktivität des Phytochrom A Proteins in gentechnisch veränderten Tabakpflanzen zu modulieren (Owen M et al. (1992) Biotechnology (N Y) 10(7):790-794; Franken E et al. (1997) Curr Opin Biotechnol 8(4):411-416; Whitelam (1996) Trend Plant Sci 1:286-272).

Die Genexpression kann auch durch maßgeschneiderte, niedermolekulare synthetische Verbindungen unterdrückt werden, beispielsweise vom Polyamid-Typ (Dervan PB und Bürli RW (1999) Current Opinion in Chemical Biology 3:688-693; Gottesfeld JM et al. (2000) Gene Expr 9(1-2):77-91). Diese Oligomere bestehen aus den Bausteinen 3-(Dimethylamino) propylamin, N-Methyl-3-hydroxypyrrol, N-Methylimidazol und N-Methylpyrrole und können an jedes Stück doppelsträngiger DNA so angepasst werden, dass sie sequenzspezifisch in die große Furche binden und die Expression der dortigen Genesequenzen blockieren. Entsprechende Verfahren sind beschrieben (siehe unter anderem Bremer RE et al. (2001) Bioorg Med Chem. 9(8):2093-103; Ansari AZ et al. (2001) Chem Biol. 8(6): 583-92; Gottesfeld JM et al. (2001) J Mol Biol. 309(3):615-29; Wurtz NR et al. (2001) Org Lett 3(8):1201-3; Wang CC et al. (2001) Bioorg Med Chem 9(3):653-7; Urbach AR und Dervan PB (2001) Proc Natl Acad Sci USA 98(8): 4343-8; Chiang SY et al. (2000) J Biol Chem. 275(32):24246-54).

g) Einbringung von den RacB RNA-Abbau bewirkenden viralen Nukleinsäuresequenzen und Expressionskonstrukten

Die RacB Expression kann effektiv auch durch Induktion des spezifischen RacB RNA-Abbaus durch die Pflanze mit Hilfe eines viralen Expressionssystems (Amplikon) (Angell, SM et al. (1999) Plant J. 20(3):357-362) realisiert werden. Diese Systeme - auch als "VIGS" (viral induced gene silencing) bezeichnet - bringen Nukleinsäureseuqnzen mit Homologie zu den zu supprimierenden Transkripten mittels viraler Vektoren in die Pflanze ein. Die Transkription wird sodann - vermutlich mediiert durch pflanzliche Abwehrmechanismen gegen Viren - abgeschaltet. Entsprechende Techniken und Verfahren sind beschrieben (Ratcliff F et al. (2001) Plant J 25(2):237-45; Fagard M und Vaucheret H (2000) Plant Mol Biol 43(2-3):285-93; Anandalakshmi R et al. (1998) Proc Natl Acad Sci USA 95 (22):13079-84; Ruiz MT (1998) Plant Cell 10(6): 937-46).

h) Einbringung von Konstrukten zur Induktion einer homologen Rekombination an endogenen RacB-Genen beispielsweise zur Erzeugung von Knockout-Mutanten.

Zur Herstellung eines homolog rekombinanten Organismus mit verminderter RacB-Aktivität verwendet man beispielsweise ein Nukleinsäurekonstrukt, das zumindest einen Teil eines endogenen RacB Gens enthält, das durch eine Deletion, Addition oder Substitution mindestens eines Nukleotids so verändert wird, so dass die Funktionalität vermindert oder gänzlich aufgehoben wird. Die Veränderung kann auch die regulativen Elemente (z.B. den Promotor) des Gens betreffen, so dass die kodierende Sequenz unverändert bleibt, eine Expression (Transkription und/oder Translation) jedoch unterbleibt und vermindert wird.

Bei der konventionellen homologen Rekombination ist die veränderte Region an ihrem 5'- und 3'-Ende von weiteren Nukleinsäuresequenzen flankiert, die eine ausreichende Länge für die Ermöglichung der Rekombination aufweisen müssen. Die Länge liegt in der Regel in einem Bereich von mehreren einhundert Basen bis zu mehreren Kilobasen (Thomas KR und Capecchi MR (1987) Cell 51:503; Strepp et al. (1998) Proc Natl Acad Sci USA 95(8):4368-4373). Für die homologe Rekombination wird der Wirtsorganismus - zum Beispiel eine Pflanze - mit dem Rekombinationskonstrukt unter Verwendung der unten beschriebenen Verfahren transformiert und erfolgreich rekombinierte Klone unter Verwendung zum Beispiel einer Antibiotika- oder Herbizidresistenz selektioniert.

Homologe Rekombination ist ein relativ seltenes Ereignis in höheren Eukaryoten, vor allem in Pflanzen. Zufällige Integrationen in das Wirtsgenom überwiegen. Eine Möglichkeit die zufällig integrierten Sequenzen zu entfernen und so Zellklone mit einer korrekten homologen Rekombination anzureichern, besteht in der Verwendung eines sequenzspezifischen Rekombinationssystems wie in US 6,110,736 beschrieben, durch welche unspezifisch integrierte Sequenzen wieder deletiert werden können, was die Selektion erfolgreich über homologe Rekombination integrierter Ereignisse erleichtert. Eine Vielzahl von sequenzspezifischen Rekombinationssystemen kann verwendet werden, beispielhaft sind das Cre/lox-System des Bacteriophagen P1, das FLP/FRT System der Hefe, die Gin Rekombinase des Mu Phagen, die Pin Rekombinase aus E. coli und das R/RS System des pSR1 Plasmids genannt. Bevorzugt sind das Bacteriophagen P1 Cre/lox und das Hefe FLP/FRT System. Das FLP/FRT und cre/lox Rekombinasesystem wurde bereits in pflanzlichen Systemen angewendet (Odell et al. (1990) Mol Gen Genet 223: 369-378)

i) Einführung von Mutationen in endogene RacB Gene zur Erzeugung eines Funktionsverlustes (z.B. Generierung von Stopp-Kodons, Verschiebungen im Leseraster etc.)

Weitere geeignete Methoden zur Verminderung der RacB-Aktivität sind die Einführung von Nonsense-Mutationen in endogene RacB Gene zum Beispiel mittels Einführung von RNA/DNA-Oligonukleotiden in die Pflanze (Zhu et al. (2000) Nat Biotechnol 18(5):555-558) sowie die Generierung von Knockout-Mutanten mit Hilfe von z.B. T-DNA-Mutagenese (Koncz et al. (1992) Plant Mol Biol 20(5):963-976), ENU-(N-Ethyl-N-nitrosoharnstoff) - Mutagenese oder homolger Rekombination (Hohn B und Puchta (1999) H Proc Natl Acad Sci USA 96:8321-8323.). Punktmutationen können auch mittels DNA-RNA Hybriden erzeugt werden, die auch als "chimeraplasty" bekannt sind (Cole-Strauss et al. (1999) Nucl Acids Res 27(5):1323-1330; Kmiec (1999) Gene therapy American Scientist 87(3): 240-247).

Die Methoden der dsRNAi, der Kosuppression mittels sense-RNA und der "VIGS" ("virus induced gene silencing") werden auch als "post-transcriptional gene silencing" (PTGS) bezeichnet. PTGS-Verfahren wie auch die Verminderung der RacB-Funktion oder Aktivität mit dominant-negativen RacB-Varianten sind besonders vorteilhaft, weil die Anforderungen an die Homologie zwischen dem zu supprimierenden endogenem Gen und der transgen exprimierten sense- oder dsRNA-Nukleinsäuresequenz (bzw. zwischen dem endogenen Gen und seiner dominant-negativen Variante) geringer sind als beispielsweise bei einem klassischen antisense-Ansatz. Entsprechende Homologie-Kriterien sind bei der Beschreibung des dsRNAI-Verfahrens genannt und allgemein für PTGS-Verfahren oder dominant-negative Ansätze übertragbar. Aufgrund der hohen Homologie zwischen den RacB-Proteinen aus Mais, Reis und Gerste kann auf einen hohen Konservierungsgrad dieses Protein bei Pflanzen geschlossen werden. So kann man voraussichtlich unter Verwendung der RacB-Nukleinsäuresequenzen aus Gerste, Mais oder Reis auch die Expression von homologen RacB-Proteinen in anderen Arten effektiv supprimieren, ohne dass die Isolierung und Strukturaufklärung der dort vorkommenden RacB-Homologen zwingend erforderlich wäre. Dies erleichtert erheblich den Arbeitsaufwand. Analog kann man voraussichtlich auch unter Verwendung von dominant-negativen Varianten eines RacB-Proteins aus Reis, Mais oder Gerste die Funktion/Aktivität seines Homologs in anderen Pflanzenarten effektiv vermindern oder unterdrücken.

Alle Substanzen und Verbindungen die direkt oder indirekt eine Verminderung der Proteinmenge, RNA-Menge, Genaktivität oder Proteinaktivität eines RacB-Proteins bewirken, seien infolge unter der Bezeichnung "anti-RacB"-Verbindungen zusammengefasst. Der Begriff "anti-RacB"-Verbindung schließt explizit die in den oben beschriebenen Verfahren zum Einsatz kommenden Nukleinsäuresequenzen, Peptide, Proteine oder andere Faktoren ein.

"Einbringung" umfasst im Rahmen der Erfindung alle Verfahren, die dazu geeignet eine "anti-RacB"-Verbindung, direkt oder indirekt, in eine Pflanze oder eine Zelle, Kompartiment, Gewebe, Organ oder Samen derselben einzuführen oder dort zu generieren. Direkte und indirekte Verfahren sind umfasst. Die Einbringung kann zu einer vorübergehenden (transienten) Präsenz einer "anti-RacB"-Verbindung (beispielsweise einer dsRNA) führen oder aber auch zu einer dauerhaften (stabilen).

Gemäß der unterschiedlichen Natur der oben beschriebenen Ansätze kann die "anti-RacB"-Verbindung ihre Funktion direkt ausüben (zum Beispiel durch Insertion in ein endogenes RacB Gen). Die Funktion kann aber auch indirekt nach Transkription in eine RNA (zum Beispiel bei antisense Ansätzen) oder nach Transkription und Translation in ein Protein (zum Beispiel bei Bindungsfaktoren) ausgeübt werden. Sowohl direkte als auch indirekt wirkende "anti-RacB"-Verbindungen sind erfindungsgemäß umfasst.

Einführen umfasst beispielsweise Verfahren wie Transfektion, Transduktion oder Transformation. "Anti-RacB" Verbindungen umfasst somit beispielsweise auch rekombinante Expressionskonstrukte, die eine Expression (d.h. Transkription und ggf. Translation) beispielsweise einer RacB-dsRNA oder einer RacB "antisense"-RNA - bevorzugt in einer Pflanze oder einem Teil, Gewebe, Organ oder Samen derselben - bedingen.

In besagten Expressionskonstrukten steht ein Nukleinsäuremolekül, dessen Expression (Transkription und ggf. Translation) eine "anti-RacB"-Verbindung generiert, bevorzugt in funktioneller Verknüpfung mit mindestens einem genetischen Kontrollelement (beispielsweise einem Promotor), das eine Expression in einem Organismus, bevorzugt in Pflanzen, gewährleistet. Soll das Expressionskonstrukt direkt in die Pflanze eingeführt und die "anti-RacB"-Verbindung (beispielsweise die RacB dsRNA) dort in plantae erzeugt werden, so sind pflanzenspezifische genetische Kontrollelemente (beispielsweise Promotoren) bevorzugt. Die "anti-RacB"-Verbindung kann jedoch auch in anderen Organismen oder in vitro erzeugt und dann in die Pflanze eingebracht werden (wie in Beispiel 6 und 7 beschrieben). In diesem sind all prokaryotischen oder eukaryotischen genetischen Kontrollelemente (beispielsweise Promotoren) bevorzugt, die die Expression in den jeweils für die Herstellung gewählten Organismus erlauben.

Unter einer funktionellen Verknüpfung versteht man zum Beispiel die sequentielle Anordnung eines Promotors mit der zu exprimierenden Nukleinsäuresequenz (zum Beispiel einer "anti-RacB"-Verbindung) und ggf. weiterer regulativer Elemente wie zum Beispiel einem Terminator derart, dass jedes der regulativen Elemente seine Funktion bei der transgenen Expression der Nukleinsäuresequenz, je nach Anordnung der Nukleinsäuresequenzen zu sense oder antisense RNA, erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die transgen zu exprimierende Nukleinsäuresequenz hinter der als Promoter fungierenden Sequenz positioniert wird, so dass beide Sequenzen kovalent miteinander verbunden sind. Bevorzugt ist dabei der Abstand zwischen der Promotorsequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Die Herstellung einer funktionellen Verknüpfung als auch die Herstellung einer Expressionskassette kann mittels gängiger Rekombinations- und Klonierungstechniken realisiert werden, wie sie beispielsweise in Maniatis T, Fritsch EF und Sambrook J (1989) Molecular Cloning: A Laboratory Manual, Cold Spring Harbor Laboratory, Cold Spring Harbor (NY), in Silhavy TJ, Berman ML und Enquist LW (1984) Experiments with Gene Fusions, Cold Spring Harbor Laboratory, Cold Spring Harbor (NY), in Ausubel FM et al. (1987) Current Protocols in Molecular Biology, Greene Publishing Assoc. and Wiley Interscience und bei Gelvin et al. (1990) In: Plant Molecular Biology Manual beschrieben sind. Zwischen beide Sequenzen können aber auch weitere Sequenzen positioniert werden, die zum Beispiel die Funktion eines Linkers mit bestimmten Restriktionsenzymschnittstellen oder eines Signalpeptides haben. Auch kann die Insertion von Sequenzen zur Expression von Fusionsproteinen führen. Bevorzugt kann die Expressionskassette, bestehend aus einer Verknüpfung von Promoter und zu exprimierender Nukleinsäuresequenz, integriert in einem Vektor vorliegen und durch zum Beispiel Transformation in ein pflanzliches Genom insertiert werden.

Unter einer Expressionskassette sind aber auch solche Konstruktionen zu verstehen, bei denen ein Promoter - zum Beispiel durch eine homologe Rekombination - hinter ein endogenes RacB-Gen platziert wird, und durch Expression einer antisense RacB-RNA die erfindungsgemäße Verminderung eines RacB-Proteins bewirkt wird. Analog kann auch eine "anti-RacB" Verbindung (zum Beispiel eine Nukleinsäuresequenz kodierend für eine RacB dsRNA oder eine RacB antisense RNA) derart hinter einen endogenen Promotor platziert werden, dass der gleiche Effekt auftritt. Beide Ansätze führen zu Expressionskassetten im Sinne der Erfindung.

Pflanzenspezifische Promotoren meint grundsätzlich jeden Promotor, der die Expression von Genen, insbesondere Fremdgenen, in Pflanzen oder Pflanzenteilen, -zellen, -geweben, -kulturen steuern kann. Dabei kann die Expression beispielsweise konstitutiv, induzierbar oder entwicklungsabhängig sein.

Bevorzugt sind:

a) Konstitutive Promotoren

Bevorzugt sind Vektoren, die eine konstitutive Expression in Pflanzen ermöglichen (Benfey et al. (1989) EMBO J 8:2195-2202). "Konstitutiver" Promotor meint solche Promotoren, die eine Expression in zahlreichen, bevorzugt allen, Geweben über einen größeren Zeitraum der Pflanzenentwicklung, bevorzugt zu allen Zeitpunkten der Pflanzenentwicklung, gewährleisten. Vorzugsweise verwendet man insbesondere einen pflanzlichen Promotor oder einen Promotor, der einem Pflanzenvirus entstammt. Insbesondere bevorzugt ist der Promotor des 35S-Transkriptes des CaMV Blumenkohlmosaikvirus (Franck et al. (1980) Cell 21:285-294; Odell et al. (1985) Nature 313:810-812; Shewmaker et al. (1985) Virology 140:281-288; Gardner et al. (1986) Plant Mol Biol 6:221- 228) oder der 19S CaMV Promotor (US 5,352,605; WO 84/02913; Benfey et al. (1989) EMBO J 8:2195-2202). Ein weiterer geeigneter konstitutiver Promotor ist der "Rubisco small subunit (SSU)"-Promotor (US 4,962,028), der LeguminB-Promotor (GenBank Acc.-Nr. X03677), der Promotor der Nopalinsynthase aus Agrobacterium, der TR-Doppelpromotor, der OCS (Octopin Synthase) Promotor aus Agrobacterium, der Ubiquitin Promotor (Holtorf S et al. (1995) Plant Mol Biol 29: 637-649), den Ubiquitin 1 Promotor (Christensen et al. (1992) Plant Mol Biol 18:675-689; Bruce et al. (1989) Proc Natl Acad Sci USA 86:9692-9696), den Smas Promotor, den Cinnamylalkoholdehydrogenase-Promotor (US 5,683,439), die Promotoren der vakuolärer ATPase Untereinheiten oder der Promotor eines prolinreichen Proteins aus Weizen (WO 91/13991), sowie weitere Promotoren von Genen, deren konstitutive Expression in Pflanzen dem Fachmann bekannt ist. Als konstitutiver Promotor insbesondere bevorzugt ist der Promotor des Nitrilase-1 (nit1) Gens aus A. thaliana (GenBank Acc.-No.: Y07648.2, Nukleotide 2456-4340, Hillebrand et al. (1996) Gene 170: 197-200).

b) Gewebespezifische Promotoren

Bevorzugt sind ferner Promotoren mit Spezifitäten für die Antheren, Ovarien, Blüten, Blätter, Stengel, Wurzeln und Samen.

Samenspezifische Promotoren

wie zum Beispiel der Promotor des Phaseolins (US 5,504,200; Bustos MM et al. (1989) Plant Cell 1(9):839-53), des 2S Albumingens (Joseffson LG et al. (1987) J Biol Chem 262:12196-12201), des Legumins (Shirsat A et al. (1989) Mol Gen Genet 215(2): 326-331), des USP (unknown seed protein; Bäumlein H et al. (1991) Mol Gen Genet 225 (3):459-67), des Napin Gens (US 5,608,152; Stalberg K et al. (1996) L Planta 199:515-519), des Saccharosebindeproteins (WO 00/26388) oder der Legumin B4-Promotor (LeB4; Bäumlein H et al. (1991) Mol Gen Genet 225: 121-128; Baeumlein et al. (1992) Plant Journal 2(2):233-9; Fiedler U et al. (1995) Biotechnology (NY) 13(10):1090f), der Oleosin-Promoter aus Arabidopsis (WO 98/45461), der Bce4-Promoter aus Brassica (WO 91/13980). Weitere geeignete samenspezifische Promotoren sind die der Gene kodierend für das "High Molecular Weight Glutenin" (HMWG), Gliadin, Verzweigungsenzym, ADP Glucose Pyrophosphatase (AGPase) oder die Stärkesynthase. Bevorzugt sind ferner Promotoren, die eine samenspezifische Expression in Monokotyledonen wie Mais, Gerste, Weizen, Roggen, Reis etc. erlauben. Vorteilhaft eingesetzt werden können der Promoter des lpt2 oder lpt1-Gen (WO 95/15389, WO 95/23230) oder die Promotoren beschrieben in WO 99/16890 (Promotoren des Hordein-Gens, des Glutelin-Gens, des Oryzin-Gens, des Prolamin-Gens, des Gliadin-Gens, des Glutelin-Gens, des Zein-Gens, des Kasirin-Gens oder des Secalin-Gens).

Knollen-, Speicherwurzel- oder Wurzel-spezifische Promotoren wie beispielsweise der Patatin Promotor Klasse I (B33), der Promotor des Cathepsin D Inhibitors aus Kartoffel.

Blattspezifische Promotoren

wie Promotor der cytosolischen FBPase aus Kartoffel (WO 97/05900), der SSU Promotor (small subunit) der Rubisco (Ribulose-1,5-bisphosphatcarboxylase) oder der ST-LSI Promotor aus Kartoffel (Stockhaus et al. (1989) EMBO J 8:2445-2451). Ganz besonders bevorzugt sind Epidermis-spezifische Promotoren, wie beispielsweise der Promotor des OXLP-Gens ("Oxalat-Oxidase like protein"; Wei et al. (1998) Plant Mol. Biol. 36:101-112).

Blütenspezifische Promotoren

wie beispielsweise der Phytoen Synthase Promotor (WO 92/16635) oder der Promotor des P-rr Gens (WO 98/22593).

Antheren-spezifische Promotoren

wie den 5126-Promotor (US 5,689,049, US 5,689,051), den glob-1 Promotor und den γ-Zein Promotor.

c) Chemisch induzierbare Promotoren

Die Expressionskassetten können auch einen chemisch induzierbaren Promotor enthalten (Übersichtsartikel: Gatz et al. (1997) Annu Rev Plant Physiol Plant Mol Biol 48:89-108), durch den die Expression des exogenen Gens in der Pflanze zu einem bestimmten Zeitpunkt gesteuert werden kann. Derartige Promotoren, wie z.B. der PRP1 Promotor (Ward et al. (1993) Plant Mol Biol 22:361-366), durch Salicylsäure induzierbarer Promotor (WO 95/19443), ein durch Benzolsulfonamid-induzierbarer Promotor (EP 0 388 186), ein durch Tetrazyklininduzierbarer Promotor (Gatz et al. (1992) Plant J 2:397-404), ein durch Abscisinsäure induzierbarer Promotor (EP 0 335 528) bzw. ein durch Ethanol- oder Cyclohexanoninduzierbarer Promotor (WO 93/21334) können ebenfalls verwendet werden.

d) Stress- oder Pathogen-induzierbare Promotoren

Ferner sind Promotoren bevorzugt, die durch biotischen oder abiotischen Stress induziert werden wie beispielsweise der pathogen-induzierbare Promotor des PRP1-Gens (bzw. *gst1* Promotor) z.B. aus Kartoffel (WO 96/28561; Ward et al. (1993) Plant Mol Biol 22:361-366), der hitzeinduzierbare hsp70- oder hsp80-Promoter aus Tomate (US 5,187,267), der kälteinduzierare alpha-Amylase Promoter aus der Kartoffel (WO 96/12814), der licht-induzierbare PPDK Promotor oder der verwundungsinduzierte pinII-Promoter (EP-A 0 375 091). Weitere pathogen-induzierbare Promoren umfassen den Flachs *Fis1*-Promotor (WO 96/34949), den Vst1-Promotor (Schubert et al. (1997) Plant Mol Biol 34:417-426) sowie den EAS4 Sesquiterpene-Cyclase-Promotor aus Tabak (US 6,100,451).

Pathogen-induzierbare Promotoren umfassen die von Genen, die infolge eines Pathogenbefalls induziert werden wie beispielsweise Gene von PR-Proteinen, SAR-Proteinen, β-1,3-Glucanase, Chitinase usw. (beispielsweise Redolfi et al. (1983) Neth J Plant Pathol 89:245-254; Uknes, et al. (1992) Plant Cell 4:645-656; Van Loon (1985) Plant Mol Viral 4:111-116; Marineau et al. (1987) Plant Mol Biol 9:335-342; Matton et al. (1987) Molecular Plant-Microbe Interactions 2:325-342; Somssich et al. (1986) Proc Natl Acad Sci USA 83:2427-2430; Somssich et al. (1988) Mol Gen Genetics 2:93-98; Chen et al. (1996) Plant J 10:955-966; Zhang and Sing (1994) Proc Natl Acad Sci USA 91: 2507-2511; Warner, et al. (1993) Plant J 3:191-201; Siebertz et al. (1989) Plant Cell 1:961-968(1989).

Umfasst sind auch verwundungs-induzierbare Promotoren wie der des pinII Gens (Ryan (1990) Ann Rev Phytopath 28:425-449; Duan et al. (1996) Nat Biotech 14:494-498), des wun1 und wun2-Gens (US 5,428,148), des win1- und win2-Gens (Stanford et al. (1989) Mol Gen Genet 215:200-208), des Systemin (McGurl et al. (1992) Science 225: 1570-1573), des WIP1-Gens (Rohmeier et al. (1993) Plant Mol Biol 22:783-792; Eckelkamp et al. (1993) FEBS Letters 323:73-76), des MPI-Gens (Corderok et al. (1994) Plant J 6(2):141-150) und dergleichen.

Eine Quelle für weitere pathogen-induzierbare Promotoren stellt die PR-Genfamilie dar. Eine Reihe von Elementen in diesen Promotoren haben sich als vorteilhaft erwiesen. So vermittelt die Region -364 bis -288 im Promotor von PR-2d Salicylat-Spezifität (Buchel et al. (1996) Plant Mol Biol 30, 493-504). Die Sequenz 5'-TCATCTTCTT-3' taucht im Promotor der Gersten β-1,3-Glucanase und in mehr als 30 weiteren stressinduzierten Genen wiederholt auf. Diese Region bindet in Tabak ein nukleäres Protein, dessen Abundanz durch Salicylat erhöht wird. Die PR-1-Promotoren aus Tabak und Arabidopsis (EP-A 0 332 104, WO 98/03536) eignen sich ebenfalls als pathogen-induzierbare Promotoren. Bevorzugt, da besonders spezifisch durch Pathogen-induziert, sind die "acidic *PR-5*"-(aPR5)-Promotoren aus Gerste (Schweizer et al. (1997) Plant Physiol 114:79-88) und Weizen (Rebmann et al. (1991) Plant Mol Biol 16:329-331). aPR5-Proteine akkumulieren in ca. 4 bis 6 Stunden nach Pathogenbefall und zeigen nur eine sehr geringe Hintergrundsexpression (WO 99/66057). Ein Ansatz, um eine erhöhte pathogen-induzierte Spezifität zu erreichen, bildet die Herstellung synthetischer Promotoren aus Kombinationen von bekannten pathogen-responsiven Elementen (Rushton et al. (2002) Plant Cell 14, 749-762; WO 00/01830; WO 99/66057). Weitere pathogen-induzierbare Promotoren aus verschiedenen Arten sind dem Fachmann bekannt (EP-A 1 165 794; EP-A 1 062 356; EP-A 1 041 148; EP-A 1 032 684;

e) Entwicklungsabhängige Promotoren

Weitere geeignete Promotoren sind beispielsweise fruchtreifungs-spezifische Promotoren, wie beispielsweise der fruchtreifungs-spezifische Promotor aus Tomate (WO 94/21794, EP 409 625). Entwicklungsabhängige Promotoren schließt zum Teil die Gewebespezifischen Promotoren ein, da die Ausbildung einzelner Gewebe naturgemäß entwicklungsabhängig erfolgt.

Besonders bevorzugt sind konstitutive, sowie Blatt und/oder Stengel-spezifische, pathogen-induzierbare und epidermis-spezifische Promotoren, wobei pathogen-induzierbar und epidermis-spezifische Promotoren am meisten bevorzugt sind.

Es können ferner weitere Promotoren funktionell mit der zu exprimierenden Nukleinsäuresequenz verknüpft sein, die eine Expression in weiteren Pflanzengeweben oder in anderen Organismen, wie zum Beispiel *E. coli* Bakterien ermöglichen. Als Pflanzen Promotoren kommen im Prinzip alle oben beschriebenen Promotoren in Frage.

Die in den Expressionskassetten oder Vektoren enthaltenen Nukleinsäuresequenzen können mit weiteren genetischen Kontrollsequenzen neben einem Promoter funktionell verknüpft sein. Der Begriff der genetischen Kontrollsequenzen ist breit zu verstehen und meint all solche Sequenzen, die einen Einfluss auf das Zustandekommen oder die Funktion der erfindungsgemäßen Expressionskassette haben. Genetische Kontrollsequenzen modifizieren zum Beispiel die Transkription und Translation in prokaryotischen oder eukaryotischen Organismen. Vorzugsweise umfassen die Expressionskassetten 5'-stromaufwärts von der jeweiligen transgen zu exprimierenden Nukleinsäuresequenz den Promoter mit Spezifität für die embryonale Epidermis und/oder die Blüte und 3' -stromabwärts eine Terminatorsequenz als zusätzliche genetische Kontrollsequenz, sowie gegebenenfalls weitere übliche regulative Elemente, und zwar jeweils funktionell verknüpft mit der transgen zu exprimierenden Nukleinsäuresequenz. Genetische Kontrollsequenzen umfassen auch weitere Promotoren, Promotorelemente oder Minimalpromotoren, die die expressionssteuernden Eigenschaften modifizieren können. So kann durch genetische Kontrollsequenzen zum Beispiel die gewebespezifische Expression zusätzlich abhängig von bestimmten Stressfaktoren erfolgen. Entsprechende Elemente sind zum Beispiel für Wasserstress, Abscisinsäure (Lam E und Chua NH, J Biol Chem 1991; 266(26): 17131 -17135) und Hitzestress (Schoffl F et al., Molecular & General Genetics 217(2-3):246-53, 1989) beschrieben.

Weitere vorteilhafte Kontrollsequenzen sind beispielsweise in den gram-positiven Promotoren amy und SPO2, in den Hefe- oder Pilzpromotoren ADC1, MFa , AC, P-60, CYC1, GAPDH, TEF, rp28, ADH.

Prinzipiell können alle natürlichen Promotoren mit ihren Regulationssequenzen wie die oben genannten für das erfindungsgemäße Verfahren verwendet werden. Darüberhinaus können auch synthetische Promotoren vorteilhaft verwendet werden.

Genetische Kontrollsequenzen umfassen ferner auch die 5'-untranslatierte Regionen, Introns oder nichtkodierende 3'-Region von Genen wie beipielsweise das Actin-1 Intron, oder die Adh1-S Introns 1, 2 und 6 (allgemein: The Maize Handbook, Chapter 116, Freeling and Walbot, Eds., Springer, New York (1994)). Es ist gezeigt worden, dass diese eine signifikante Funktionen bei der Regulation der Genexpression spielen können. So wurde gezeigt, dass 5'-untranslatierte Sequenzen die transiente Expression heterologer Gene verstärken können. Beispielhaft für Translationsverstärker sei die 5'-Leadersequenz aus dem Tabak-Mosaik-Virus zu nennen (Gallie et al. (1987) Nucl Acids Res 15: 8693-8711) und dergleichen. Sie können ferner die Gewebsspezifität fördern (Rouster J et al. (1998) Plant J 15:435-440).

Die Expressionskassette kann vorteilhafterweise eine oder mehrere sogenannte "enhancer Sequenzen" funktionell verknüpft mit dem Promoter enthalten, die eine erhöhte transgene Expression der Nukleinsäuresequenz ermöglichen. Auch am 3'-Ende der transgen zu exprimierenden Nukleinsäuresequenzen können zusätzliche vorteilhafte Sequenzen inseriert werden, wie weitere regulatorische Elemente oder Terminatoren. Die transgen zu exprimierenden Nukleinsäuresequenzen können in einer oder mehreren Kopien im Genkonstrukt enthalten sein.

Als Kontrollsequenzen geeignete Polyadenylierungssignale sind pflanzliche Polyadenylierungssignale, vorzugsweise solche, die im wesentlichen T-DNA Polyadenylierungssignale aus *Agrobacterium tumefaciens*, insbesondere des Gens 3 der T-DNA (Octopin Synthase) des Ti-Plasmids pTiACHS entsprechen (Gielen et al. (1984) EMBO J 3:835 ff) oder funktionelle Äquivalente davon. Beispiele für besonders geeignete Terminatorsequenzen sind der OCS (Octopin-Synthase)-Terminator und der NOS (Nopalin-Synthase)-Terminator.

Als Kontrollsequenzen sind weiterhin solche zu verstehen, die eine homologe Rekombination bzw. Insertion in das Genom eines Wirtsorganismus ermöglichen oder die Entfernung aus dem Genom erlauben. Bei der homologen Rekombination kann zum Beispiel der natürliche Promoter eines bestimmten Gens gegen einen Promoter mit Spezifität für die embryonale Epidermis und/oder die Blüte ausgetauscht werden. Methoden wie die cre/lox-Technologie erlauben eine gewebespezifische, unter Umständen induzierbare Entfernung der Expressionskassette aus dem Genom des Wirtsorganismus (Sauer B (1998) Methods. 14(4):381-92). Hier werden bestimmte flankierende Sequenzen dem Zielgen angefügt (lox-Sequenzen), die später eine Entfernung mittels der cre-Rekombinase ermöglichen.

Eine Expressionskassetten und die von ihr abgeleiteten Vektoren können weitere Funktionselemente enthalten. Der Begriff Funktionselement ist breit zu verstehen und meint all solche Elemente, die einen Einfluss auf Herstellung, Vermehrung oder Funktion der erfindungsgemäßen Expressionskassetten, Vektoren oder transgenen Organismen haben. Beispielhaft aber nicht einschränkend seien zu nennen:

a) Selektionsmarker, die eine Resistenz gegen einen Metabolismusinhibitor wie 2-Desoxyglucose-6-phosphat (WO 98/45456), Antibiotika oder Biozide, bevorzugt Herbizide, wie zum Beispiel Kanamycin, G 418, Bleomycin, Hygromycin, oder Phosphinotricin etc. verleihen. Besonders bevorzugte Selektionsmarker sind solche die eine Resistenz gegen Herbizide verleihen. Beispielhaft seien genannt: DNA Sequenzen, die für Phosphinothricinacetyltransferasen (PAT) kodieren und Glutaminsynthaseinhibitoren inaktivieren (bar und pat Gen), 5-Enolpyruvylshikimat-3-phosphatsynthasegene (EPSP Synthasegene), die eine Resistenz gegen Glyphosat® (N-(phosphonomethyl)glycin) verleihen, das für das Glyphosat® degradierende Enzyme kodierende gox Gen (Glyphosatoxidoreduktase), das deh Gen (kodierend für eine Dehalogenase, die Dalapon inaktiviert), Sulfonylurea- und Imidazolinon inaktivierende Acetolactatsynthasen sowie bxn Gene, die für Bromoxynil degradierende Nitrilaseenzyme kodieren, das aasa-Gen, das eine Resistenz gegen das Antibiotikum Apectinomycin verleih, das Streptomycinphosphotransferase (SPT) Gen, das eine Resistenz gegen Streptomycin gewährt, das Neomycinphosphotransferas (NPTII) Gen, das eine Resistenz gegen Kanamycin oder Geneticidin verleiht, das Hygromycinphosphotransferase (HPT) Gen, das eine Resistenz gegen Hygromycin vermittelt, das Acetolactatsynthas Gen (ALS), das eine Resistenz gegen Sulfonylharnstoff-Herbizide verleiht (z.B. mutierte ALS-Varianten mit z.B. der S4 und/oder Hra Mutation).

b) Reportergene, die für leicht quantifizierbare Proteine kodieren und über Eigenfarbe oder Enzymaktivität eine Bewertung der Transformationseffizienz oder des Expressionsortes oder -zeitpunktes gewährleisten. Ganz besonders bevorzugt sind dabei Reporter-Proteine (Schenborn E, Groskreutz D. Mol Biotechnol. 1999; 13(1):29-44) wie das "green fluorescence protein" (GFP) (Sheen et al. (1995) Plant Journal 8(5):777-784; Haseloff et al. (1997) Proc Natl Acad Sci USA 94(6):2122-2127; Reichel et al.(1996) Proc Natl Acad Sci USA 93(12):5888-5893; Tian et al. (1997) Plant Cell Rep 16:267-271; WO 97/41228; Chui WL et al. (1996) Curr Biol 6:325-330; Leffel SM et al. (1997) Biotechniques. 23(5):912-8), die Chloramphenicoltransferase, eine Luziferase (Ow et al. (1986) Science 234:856-859; Millar et al. (1992) Plant Mol Biol Rep 10:324-414), das Aequoringen (Prasher et al. (1985) Biochem Biophys Res Commun 126(3):1259-1268), die β-Galactosidase, R-Locus Gen (kodieren ein Protein, das die Produktion von Anthocyaninpigmenten (rote Färbung) in pflanzlichen Gewebe reguliert und so eine direkte Analyse der Promotoraktivität ohne Zugabe zusätzlicher Hilfstoffe oder chromogener Substrate ermöglicht; Dellaporta et al., In: Chromosome Structure and Function: Impact of New Concepts, 18th Stadler Genetics Symposium, 11:263-282, 1988), ganz besonders bevorzugt ist die β-Glucuronidase (Jefferson et al., EMBO J. 1987, 6, 3901-3907).

c) Replikationsursprünge, die eine Vermehrung der erfindungsgemäßen Expressionskassetten oder Vektoren in zum Beispiel E.coli gewährleisten. Beispielhaft seien genannt ORI (origin of DNA replication), der pBR322 ori oder der P15A ori (Sambrook et al.: Molecular Cloning. A Laboratory Manual, 2nd ed. Cold Spring Harbor Laboratory Press, Cold Spring Harbor, NY, 1989).

d) Elemente, die für eine Agrobakterium vermittelte Pflanzentransformation erforderlich sind, wie zum Beispiel die rechte oder linke Begrenzung der T-DNA oder die vir-Region.

Zur Selektion erfolgreich homolog rekombinierter oder auch transformierter Zellen ist es in der Regel erforderlich, einen selektionierbaren Marker zusätzlich einzuführen, der den erfolgreich rekombinierten Zellen eine Resistenz gegen ein Biozid (zum Beispiel ein Herbizid), einen Metabolismusinhibitor wie 2-Desoxyglucose-6-phosphat (WO 98/45456) oder ein Antibiotikum verleiht. Der Selektionsmarker erlaubt die Selektion der transformierten Zellen von untransformierten (McCormick et al. (1986) Plant Cell Reports 5:81-84).

Die Einführung einer erfindungsgemäßen Expressionskassette in einen Organismus oder Zellen, Geweben, Organe, Teile bzw. Samen desselben (bevorzugt in Pflanzen bzw. pflanzliche Zellen, Gewebe, Organe, Teile oder Samen), kann vorteilhaft unter Verwendung von Vektoren realisiert werden, in denen die Expressionskassetten enthalten sind. Die Expressionskassette kann in den Vektor (zum Beispiel ein Plasmid) über eine geeignete Restriktionsschnittstelle eingeführt werden. Das entstandene Plasmid wird zunächst in E.coli eingeführt. Korrekt transformierte E.coli werden selektioniert, gezüchtet und das rekombinante Plasmid mit dem Fachmann geläufigen Methoden gewonnen. Restriktionsanalyse und Sequenzierung können dazu dienen, den Klonierungsschritt zu überprüfen.

Vektoren können beispielhaft Plasmide, Cosmide, Phagen, Viren oder auch Agrobacterien sein. In einer vorteilhaften Ausführungsform wird die Einführung der Expressionskassette mittels Plasmidvektoren realisiert. Bevorzugt sind solche Vektoren, die eine stabile Integration der Expressionskassette in das Wirtsgenom ermöglichen.

Die Herstellung eines transformierten Organismus (bzw. einer transformierten Zelle oder Gewebes) erfordert, dass die entsprechende DNA, RNA oder Protein in die entsprechende Wirtszelle eingebracht wird.

Für diesen Vorgang, der als Transformation (oder Transduktion bzw. Transfektion) bezeichnet wird, steht eine Vielzahl von Methoden zur Verfügung (Keown et al. (1990) Methods in Enzymology 185:527-537). So kann die DNA oder RNA beispielhaft direkt durch Mikroinjektion oder durch Bombardierung mit DNA-beschichteten Mikropartikeln eingeführt werden. Auch kann die Zelle chemisch, zum Beispiel mit Polyethylenglycol, permeabilisiert werden, so dass die DNA durch Diffusion in die Zelle gelangen kann. Die DNA kann auch durch Protoplastenfusion mit anderen DNA-enthaltenden Einheiten wie Minicells, Zellen, Lysosomen oder Liposomen erfolgen. Elektroporation ist eine weitere geeignete Methode zur Einführung von DNA, bei der die Zellen reversibel durch einen elektrischen Impuls permeabilisert werden. Entsprechende Verfahren sind beschrieben (beispielsweise bei Bilang et al. (1991) Gene 100:247-250; Scheid et al. (1991) Mol Gen Genet 228:104-112; Guerche et al. (1987) Plant Science 52:111-116; Neuhause et al. (1987) Theor Appl Genet 75:30-36; Klein et al. (1987) Nature 327:70-73 ; Howell et al. (1980) Science 208:1265; Horsch et al. (1985) Science 227:1229-1231 ; DeBlock et al. (1989) Plant Physiology 91:694-701; Methods for Plant Molecular Biology (Weissbach and Weissbach, eds.) Academic Press Inc. (1988); and Methods in Plant Molecular Biology (Schuler and Zielinski, eds.) Academic Press Inc. (1989)).

Bei Pflanzen werden dabei die beschriebenen Methoden zur Transformation und Regeneration von Pflanzen aus Pflanzengeweben oder Pflanzenzellen zur transienten oder stabilen Transformation genutzt. Geeignete Methoden sind vor allem die Protoplastentransformation durch Polyethylenglykol-induzierte DNA-Aufnahme, das biolistische Verfahren mit der Genkanone, die sogenannte "particle bombardment" Methode, die Elektroporation, die Inkubation trokkener Embryonen in DNA-haltiger Lösung und die Mikroinjektion.

Neben diesen "direkten" Transformationstechniken kann eine Transformation auch durch bakterielle Infektion mittels Agrobacterium tumefaciens oder Agrobacterium rhizogenes durchgeführt werden. Die Agrobacterium-vermittelte Transformation ist am besten für dicotyledone Pflanzenzellen geeignet. Die Verfahren sind beispielsweise beschrieben bei Horsch RB et al. (1985) Science 225: 1229f).

Werden Agrobacterien verwendet, so ist die Expressionskassette in spezielle Plasmide zu integrieren, entweder in einen Zwischenvektor (englisch: shuttle or intermediate vector) oder einen binären Vektor. Wird ein Ti oder Ri Plasmid zur Transformation verwendet werden soll, ist zumindest die rechte Begrenzung, meistens jedoch die rechte und die linke Begrenzung der Ti oder Ri Plasmid T-DNA als flankierende Region mit der einzuführenden Expressionskassette verbunden.

Bevorzugt werden binäre Vektoren verwendet. Binäre Vektoren können sowohl in E.coli als auch in Agrobacterium replizieren. Sie enthalten in der Regel ein Selektionsmarkergen und einen Linker oder Polylinker flankiert von der rechten und linken T-DNA Begrenzungssequenz. Sie können direkt in Agrobacterium transformiert werden (Holsters et al. (1978) Mol Gen Genet 163:181-187). Das Selektionsmarkergen erlaubt eine Selektion transformierter Agrobakteria und ist zum Beispiel das nptII Gen, das eine Resistenz gegen Kanamycin verleiht. Das in diesem Fall als Wirtsorganismus fungierende Agrobacterium sollte bereits ein Plasmid mit der vir-Region enthalten. Diese ist für die Übertragung der T-DNA auf die pflanzliche Zelle erforderlich. Ein so transformiertes Agrobacterium kann zur Transformation pflanzlicher Zellen verwendet werden. Die Verwendung von T-DNA zur Transformation pflanzlicher Zellen ist intensiv untersucht und beschrieben (EP 120 516; Hoekema, In: The Binary Plant Vector System, Offsetdrukkerij Kanters B.V., Alblasserdam, Chapter V; An et al. (1985) EMBO J 4:277-287). Verschiedene binäre Vektoren sind bekannt und teilweise kommerziell erhältlich wie zum Beispiel pBI101.2 oder pBIN19 (Clontech Laboratories, Inc. USA).

Weitere zur Expression in Pflanzen geeignet Promotoren sind beschrieben (Rogers et al. (1987) Meth in Enzymol 153:253-277; Schardl et al. (1987) Gene 61:1-11; Berger et al. (1989) Proc Natl Acad Sci USA 86:8402-8406).

Direkte Transformationstechniken eignen sich für jeden Organismus und Zelltyp eignen.

Im Falle von Injektion oder Elektroporation von DNA bzw. RNA in pflanzliche Zellen sind keine besonderen Anforderungen an das verwendete Plasmid gestellt. Einfache Plasmide wie die der pUC-Reihe können verwendet werden. Sollen vollständige Pflanzen aus den transformierten Zellen regeneriert werden, so ist es erforderlich, dass sich auf dem Plasmid ein zusätzliches selektionierbares Markergen befindet.

Stabil transformierte Zellen d.h. solche, die die eingeführte DNA integriert in die DNA der Wirtszelle enthalten, können von untransformierten selektioniert werden, wenn ein selektionierbarer Marker Bestandteil der eingeführten DNA ist. Als Marker kann beispielhaft jedes Gen fungieren, dass eine Resistenz gegen Antibiotika oder Herbizide (wie Kanamycin, G 418, Bleomycin, Hygromycin oder Phosphinotricin etc.) zu verleihen vermag (s.o.). Transformierte Zellen, die ein solches Markergen exprimieren, sind in der Lage, in der Gegenwart von Konzentrationen eines entsprechenden Antibiotikums oder Herbizides zu überleben, die einen untransformierten Wildtyp abtöten. Beispiel sind oben genannt und umfassen bevorzugt das bar Gen, dass Resistenz gegen das Herbizid Phosphinotricin verleiht (Rathore KS et al. (1993) Plant Mol Biol 21(5):871-884), das nptII Gen, dass Resistenz gegen Kanamycin verleiht, das hpt Gen, das Resistenz gegen Hygromycin verleiht, oder das EPSP-Gen, das Resistenz gegen das Herbizid Glyphosat verleiht. Der Selektionsmarker erlaubt die Selektion von transformierten Zellen von untransformierten (McCormick et al. (1986) Plant Cell Reports 5:81-84). Die erhaltenen Pflanzen können in üblicher Weise gezüchtet und gekreuzt werden. Zwei oder mehr Generationen sollten kultiviert werden, um sicherzustellen, dass die genomische Integration stabil und vererblich ist.

Die oben genannten Verfahren sind beispielsweise beschrieben in Jenes B et al.(1993) Techniques for Gene Transfer, in: Transgenic Plants, Vol. 1, Engineering and Utilization, herausgegeben von SD Kung und R Wu, AcademicPress, S. 128-143 sowie in Potrykus (1991) AnnuRevPlantPhysiol Plant Molec Biol 42:205-225). Vorzugsweise wird das zu exprimierende Konstrukt in einen Vektor kloniert, der geeignet ist, Agrobacterium tumefaciens zu transformieren, beispielsweise pBin19 (Bevan et al. (1984) Nucl Acids Res 12:8711f).

Sobald eine transformierte Pflanzenzelle hergestellt wurde, kann eine vollständige Pflanze unter Verwendung von dem Fachmann bekannten Verfahren erhalten werden. Hierbei geht man beispielhaft von Kalluskulturen aus. Aus diesen noch undifferenzierten Zellmassen kann die Bildung von Spross und Wurzel in bekannter Weise induziert werden. Die erhaltenen Sprösslinge können ausgepflanzt und gezüchtet werden.

Dem Fachmann sind solche Verfahren bekannt, um aus Pflanzenzellen, Pflanzenteile und ganze Pflanzen zu regenerieren. Beispielsweise werden hierzu Verfahren beschrieben von Fennell et al. (1992) Plant Cell Rep. 11: 567-570; Stoeger et al (1995) Plant Cell Rep. 14:273-278; Jahne et al. (1994) Theor Appl Genet 89:525-533 verwendet.

Das erfindungsgemäße Verfahren kann vorteilhaft mit weiteren Verfahren die eine Pathogenresistenz (beispielsweise gegen Insekten, Pilze, Bakterien, Nematoden etc.), Stressresistenz oder eine andere Verbesserung der pflanzlichen Eigenschaften bewirken kombiniert werden. Beispiele sind u.a. genannt bei Dunwell JM, Transgenic approaches to crop improvement, J Exp Bot. 2000;51 Spec No; Seite 487-96.

Ein weiterer Gegenstand der Erfindung betrifft ein Verfahren unter Verwendung von Nukleinsäuresequenzen kodierend für funktionelle Äquivalente des RacB Protein aus Gerste, bevorzugt die Nukleinsäuresequenz gemäß SEQ ID NO: 34, 36 oder 38, die dazu komplementäre Nukleinsäuresequenz und die durch Entartung (Degeneration) des genetischen Codes abgeleitete Sequenzen.

Ein weiterer Gegenstand der Erfindung betrifft ein Verfahren unter Verwendung von transgenen Expressionskassetten, die eine der obigen Nukleinsäuresequenzen umfassen. In den transgenen Expressionskassetten ist die Nukleinsäuresequenz kodierend für das RacB-Protein aus Gerste mit mindestens einem genetischen Kontrollelement nach obiger Definition derart verknüpft, das die Expression (Transkription und ggf. Translation) in einem beliebigen Organismus - bevorzugt in Pflanzen - realisiert werden kann. Dazu geeignete genetische Kontrollelemente sind oben beschrieben. Die transgenen Expressionskassetten können auch weitere Funktionselementen gemäß obiger Definition enthalten. Die insertierte Nukleinsäuresequenz kodierend für ein RacB-Protein aus Gerste kann in sense- oder antisense-Orientierung in die Expressionskassette insertiert sein, und damit zu Expression von sense- oder antisense-RNA führen. In dem erfindungsgemäßen Verfahren können transgene Vektoren, die die transgenen Expressionskassetten beinhalten, eingesetzt werden.

"Transgen" meint bezüglich zum Beispiel einer Nukleinsäuresequenz, einer Expressionskassette oder einem Vektor enthaltend besagte Nukleinsäuresequenz oder einem Organismus transformiert mit besagter Nukleinsäuresequenz, Expressionskassette oder Vektor alle solche durch gentechnische Methoden zustandegekommene Konstruktionen, in denen sich entweder

a) die RacB Nukleinsäuresequenz, oder

b) eine mit der RacB Nukleinsäuresequenz funktionell verknüpfte genetische Kontrollsequenz, zum Beispiel ein Promotor, oder

c) (a) und (b)

sich nicht in ihrer natürlichen, genetischen Umgebung befinden oder durch gentechnische Methoden modifiziert wurden, wobei die Modifikation beispielhaft eine Substitutionen, Additionen, Deletionen, Inversion oder Insertionen eines oder mehrerer Nukleotidreste sein kann. Natürliche genetische Umgebung meint den natürlichen chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommende Expressionskassette - beispielsweise die natürlich vorkommende Kombination des RacB-Promotors mit dem entsprechenden RacB-Gen - wird zu einer transgenen Expressionskassette, wenn diese durch nicht-natürliche, synthetische ("künstliche") Verfahren wie beispielsweise einer Mutagenisierung geändert wird. Entsprechende Verfahren sind beschrieben (US 5, 565, 350; WO 00/15815; siehe auch oben).

Ein anderer Gegenstand der Erfindung betrifft transgene Organismen, transformiert mit wenigstens einer der oben definierten Nukleinsäuresequenzen, Expressionskassetten oder Vektoren, sowie Zellen, Zellkulturen, Gewebe, Teile - wie zum Beispiel bei pflanzlichen Organismen Blätter, Wurzeln usw.- oder Vermehrungsgut abgeleitet von solchen Organismen. Organismus ist breit zu verstehen und meint prokaryotische und eukaryotische Organismen, bevorzugt pflanzliche Organismen.

Als transgene Organismen bevorzugte Wirts- oder Ausgangsorganismen sind vor allem Pflanzen gemäß der oben genannten Definition. Eingeschlossen sind im Rahmen der Erfindung alle Gattungen und Arten höherer und niedrigerer Pflanzen des Pflanzenreiches. Eingeschlossen sind ferner die reifen Pflanzen, Saatgut, Sprossen und Keimlinge, sowie davon abgeleitete Teile, Vermehrungsgut und Kulturen, zum Beispiel Zellkulturen. Reife Pflanzen meint Pflanzen zu jedem beliebigen Entwicklungsstadium jenseits des Keimlings. Keimling meint eine junge, unreife Pflanze in einem frühen Entwicklungsstadium. Insbesondere als Wirtsorganismen bevorzugte Pflanzen sind Pflanzen, auf die das erfindungsgemäße Verfahren zum Erzielen einer Pathogenresistenz gemäß oben genannten Kriterien angewendet werden kann. Ganz besonders bevorzugt sind monokotyle Pflanzen wie Weizen, Hafer, Hirse, Gerste, Roggen, Mais, Reis, Buchweizen, Sorghum, Triticale, Dinkel, Leinsamen, Zuckerrohr, als diktoyledone Kulturpflanzen wie Raps, Canola, Kresse, Arabidopsis, Kohlarten, Soja, Alfalfa, Erbse, Bohnengewächsen, Erdnuss, Kartoffel, Tabak, Tomate, Aubergine, Paprika, Sonnenblume, Tagetes, Salat, Calendula, Melone, Kürbis oder Zucchini.

Die Herstellung der transgenen Organismen kann mit den oben beschriebenen Verfahren zur Transformation oder Transfektion von Organismen realisiert werden.

Ein weiterer Gegenstand der Erfindung betrifft die Verwendung der erfindungsgemäßen, transgenen Organismen und der von ihnen abgeleiteten Zellen, Zellkulturen, Teile - wie zum Beispiel bei transgenen pflanzlichen Organismen Wurzeln, Blätter etc.-, und transgenes Vermehrungsgut wie Saaten oder Früchte, zur Herstellung von Nahrungs- oder Futtermitteln, Pharmazeutika oder Feinchemikalien.

Bevorzugt ist ferner ein Verfahren zur rekombinanten Herstellung von Pharmazeutika oder Feinchemikalien in Wirtsorganismen wobei ein Wirtsorganismus mit einer der oben beschriebenen Expressionskassetten transformiert wird und diese Expressionskassette ein oder mehrere Strukturgene enthält, die für die gewünschte Feinchemikalie kodieren oder die Biosynthese der gewünschten Feinchemikalie katalysieren, der transformierte Wirtsorganismus gezüchtet wird und die gewünschte Feinchemikalie aus dem Züchtungsmedium isoliert wird. Dieses Verfahren ist für Feinchemikalien wie Enzyme, Vitamine, Aminosäuren, Zucker, Fettsäuren, natürliche und synthetische Geschmacks-, Aroma- und Farbstoffe breit anwendbar. Besonders bevorzugt ist die Produktion von Tocopherolen und Tocotrienolen sowie Carotinoiden. Die Züchtung der transformierten Wirtsorganismen sowie die Isolierung aus den Wirtsorganismen bzw. aus dem Züchtungsmedium erfolgt mit dem Fachmann bekannten Verfahren. Die Produktion von Pharmazeutika, wie zum Beispiel Antikörpern oder Vakkzinen ist beschrieben bei Hood EE, Jilka JM (1999) Curr Opin Biotechnol 10(4): 382-6; Ma JK, Vine ND (1999) Curr Top Microbiol Immunol 236:275-92.

Sequenzen

1. SEQ ID NO: 1 : Nukleinsäuresequenz kodierend für das RacB Protein aus Gerste (Hordeum vulgare).

2. SEQ ID NO: 2 : Aminosäuresequenz kodierend für das RacB Protein aus Gerste (Hordeum vulgare).

3. SEQ ID NO: 3 : Nukleinsäuresequenz kodierend für das RacB Protein aus Reis (Oryza sativa).

4. SEQ ID NO: 4 : Aminosäuresequenz kodierend für das RacB Protein aus Reis (Oryza sativa).

5. SEQ ID-NO: 5 : Nukleinsäuresequenz kodierend für das RacB Protein aus Mais (Zea mays).

6. SEQ ID NO: 6 : Aminosäuresequenz kodierend für das RacB Protein aus Mais (Zea mays).

7. SEQ ID NO: 7 : Aminosäuresequenz kodierend für eine dominant-negative Variante des RacB Proteins aus (Hordeum vulgare).

8. SEQ ID NO: 8 : Aminosäuresequenz kodierend für eine dominant-negative Variante des RacB Proteins aus Reis (Oryza sativa).

9. SEQ ID NO: 9 : Aminosäuresequenz kodierend für eine dominant-negative Variante des RacB Proteins aus Mais (Zea mays).

10. SEQ ID NO: 10 Oligonukleotidprimer ONP-1
    5'-GGATCCGATGAGCGCGTCCAGGTT-3'

11. SEQ ID NO: 11 Oligonukleotidprimer ONP-2
    5'-GTCGACCTTCGCCCTTGTTCTTTGTC-3'

12. SEQ ID NO: 12 RACE-RacB Primer
    5'-gtgggcacatagtcggtggggaaggt-3'

13. SEQ ID NO: 13 GeneRacer™ 5'-Primer:
    5'-CGACTGGAGCACGAGGACACTGA-3

14. SEQ ID NO: 14 GeneRacer™ 5'-Nested Primer:
    5'-GGACACTGACATGGACTGAAGGAGTA-3

15. SEQ ID NO: 15 RacB-sense Primer
    5'-gttcatcaagtgcgtcaccgtg-3'

16. SEQ ID NO: 16 RacB-antisense Primer
    5'-ttagcttcctcagttcttccctg-3'

17. SEQ ID NO: 17 BAS-sense Primer
5'-cgcgccgcagccgagtacgac-3'

18. SEQ ID NO: 18 BAS-antisense Primer
5'-gtcacaaaaacacatgtaacc-3'

19. SEQ ID NO: 19 OXLP-sense Primer
5'-ggccgacatgcattcaccag-3'

20. SEQ ID NO: 20 OXLP-antisense Primer
5'-catctgatattgctgggtctg-3'

21. SEQ ID NO: 21 UBI-sense Primer
5'-ccaagatgcagatcttcgtga-3'

22. SEQ ID NO: 22 UBI-antisense Primer
5'-ttcgcgataggtaaaagagca-3'

23. SEQ ID NO: 23 M13-fwd Primer
5'-GTAAAACGACGGCCAGTG-3'

24. SEQ ID NO: 24 M13-Rev Primer
5'-GGAAACAGCTATGACCATG-3'

25. SEQ ID NO: 25 HvRop6 LEFT PRIMER
5'-GTGGAGGCGCGGCGAGA-3'

26. SEQ ID NO: 26 HvRop6 RIGHT PRIMER
5'-CCATGCTTCATCTCCATAGTCA-3'

27. SEQ ID NO: 27 HvRacD LEFT PRIMER
5'-ggatccCGATTCCATCAGGAAAGCAT-3'

28. SEQ ID NO: 28 HvRacD RIGHT PRIMER
5'-gtcgacGCGAGACACTGCAAAACAAA-3'

29. SEQ ID NO: 29 HvRop4 LEFT PRIMER
5'-GGATCCttctcgtccatttagccggc-3'

30. SEQ ID NO: 30 HvRop4 RIGHT PRIMER
5'-GTCGACTgatcacttgaagcatgccag-3'

31. SEQ ID NO: 31 RacB5'BamHI Primer
5'-GGATCCGATGAGCGCGTCCAGGTT-3'

32. SEQ ID NO: 32 RacB3'SalI Primer
5'-GTCGACCTTCGCCCTTGTTCTTTGTC-3'

33. SEQ ID NO: 33 V15 Mutagenese Primer
5'-ACCGTGGGGGACGTCGCCGTCGGCAAGAC-3'

34. SEQ ID NO: 34 : Nukleinsäuresequenz kodierend für das RacB-Homolog HvRop6 aus Gerste (Hordeum vulgare).

35. SEQ ID NO: 35 : Aminosäuresequenz kodierend für das RacB-Homolog HvRop6 aus Gerste (Hordeum vulgare).

36. SEQ ID NO: 36 : Nukleinsäuresequenz kodierend für das RacB-Homolog HvRacD aus Gerste (Hordeum vulgare).

37. SEQ ID NO: 37 : Aminosäuresequenz kodierend für das RacB-Homolog HvRacD aus Gerste (Hordeum vulgare).

38. SEQ ID NO: 38 : Nukleinsäuresequenz kodierend für das RacB-Homolog HvRop4 aus Gerste (Hordeum vulgare).

39. SEQ ID NO: 39 : Aminosäuresequenz kodierend für das RacB-Homolog HvRop4 aus Gerste (Hordeum vulgare).

40. SEQ ID NO: 40 : Nukleinsäureseguenz kodierend für das RacB-Homolog Zea mays ROP6 (GenBank Acc.-No.: AJ278665)

41. SEQ ID NO: 41 Aminosäuresequenz kodierend für das RacB-Homolog Zea mays ROP6

42. SEQ ID NO: 42 Nukleinsäuresequenz kodierend für das RacB-Homolog Oryza sativa subsp. japonica RACDP (RACD) (GenBank Acc.-No.: AF218381)

43. SEQ ID NO: 43 Aminosequenz kodierend für das RacB-Homolog Oryza sativa subsp. japonica RACDP

44. SEQ ID NO: 44 Nukleinsäuresequenz kodierend für das RacB-Homolog Oryza sativa ROP4 (GenBank Acc.-No.: AF380335)

45. SEQ ID NO: 45 Aminosäuresequenz kodierend für das RacB-Homolog Oryza sativa ROP4

46. SEQ ID NO: 46 Nukleinsäuresequenz kodierend für das RacB-Homolog Zea mays RACA (GenBank Acc.-No.: AF126052)

47. SEQ ID NO: 47 Aminosäuresequenz kodierend für das RacB-Homolog Zea mays RACA

48. SEQ ID NO: 48 Nukleinsäuresequenz kodierend für ein RacB-Homolog aus Hordeum vulgare (GenBank Acc.-No.: BM816965)

49. SEQ ID NO: 49 Nukleinsäuresequenz kodierend für ein RacB-Homolog aus Arabidopsis thaliana (At3g51300)

50. SEQ ID NO: 50 Aminosäuresequenz kodierend für ein RacB-Homolog aus Arabidopsis thaliana (At3g51300)

51. SEQ ID NO: 51 Nukleinsäuresequenz kodierend für ein RacB-Homolog aus Arabidopsis thaliana (At2g17800)

52. SEQ ID NO: 52 Aminosäuresequenz kodierend für ein RacB-Homolog aus Arabidopsis thaliana (At2g17800)

53. SEQ ID NO: 53 Nukleinsäuresequenz kodierend für ein RacB-Homolog aus Arabidopsis thaliana (At4g35950)

54. SEQ ID NO: 54 Aminosäuresequenz kodierend für ein RacB-Homolog aus Arabidopsis thaliana (At4g35950)

55. SEQ ID NO: 55 Nukleinsäuresequenz kodierend für ein RacB-Homolog aus Arabidopsis thaliana (At1g75840)

56. SEQ ID NO: 56 Aminosäuresequenz kodierend für ein RacB-Homolog aus Arabidopsis thaliana (At1g75840)

57. SEQ ID NO: 57 Nukleinsäuresequenz kodierend für ein RacB-Homolog aus Arabidopsis thaliana (At4g35020)

58. SEQ ID NO: 58 Aminosäuresequenz kodierend für ein RacB-Homolog aus Arabidopsis thaliana (At4g35020)

59. SEQ ID NO: 59 Nukleinsäuresequenz kodierend für ein RacB-Homolog aus Arabidopsis thaliana (At1g20090)

60. SEQ ID NO: 60 Aminosäuresequenz kodierend für ein RacB-Homolog aus Arabidopsis thaliana (At1g20090)

61. SEQ ID NO: 61 Nukleinsäuresequenz kodierend für ein RacB-Homolog aus Arabidopsis thaliana (At5g45970)

62. SEQ ID NO: 62 Aminosäuresequenz kodierend für ein RacB-Homolog aus Arabidopsis thaliana (At5g45970)

63. SEQ ID NO: 63 Nukleinsäuresequenz kodierend für ein RacB-Homolog aus Arabidopsis thaliana (At3g48040)

64. SEQ ID NO: 64 Aminosäuresequenz kodierend für ein RacB-Homolog aus Arabidopsis thaliana (At3g48040)

65. SEQ ID NO: 65 Nukleinsäuresequenz kodierend für ein RacB-Homolog aus Arabidopsis thaliana (At5g62880)

66. SEQ ID NO: 66 Aminosäuresequenz kodierend für ein RacB-Homolog aus Arabidopsis thaliana (At5g62880)

67. SEQ ID NO: 67 Nukleinsäuresequenz kodierend für ein RacB-Homolog aus Arabidopsis thaliana (At4g28950)

68. SEQ ID NO: 68 Aminosäuresequenz kodierend für ein RacB-Homolog aus Arabidopsis thaliana (At4g28950)

69. SEQ ID NO: 79 Nukleinsäuresequenz kodierend für ein RacB-Homolog aus Arabidopsis thaliana (At2g44690)

70. SEQ ID NO: 70 Aminosäuresequenz kodierend für ein RacB-Homolog aus Arabidopsis thaliana (At2g44690)

71. SEQ ID NO: 71 Oligonukleotidprimer Fra 186
5'-ATGAGCGCGTCCAGGTTCATA-3'

72. SEQ ID NO: 72 Oligonukleotidprimer Fra 187
5'-ATCAAACACGCCCTTCACGTT-3'

73. SEQ ID NO: 73 Transgener Expressionsvektor pSUN3NIT_AtRacB_s für Expression von Arbidopsis thaliana RacB in sense Orientierung

74. SEQ ID NO: 74 Transgener Expressionsvektor pSUN3NIT_AtRacB_as für Expression von Arbidopsis thaliana RacB in antisense Orientierung

75. SEQ ID NO: 75 Transgener Expressionsvektor pSUN3NIT_HvRacB_s für Expression eines Gerste RacB Fragmentes in sense Orientierung

76. SEQ ID NO: 76 Transgener Expressionsvektor pSUN3NIT_HvRacB_as für Expression eines Gerste RacB Fragmentes in antisense Orientierung

Abbildungen

1. Fig. 1: Vergleich der Aminosäuresequenzen von Gerste RacB, Reis RacB, Mais RacB, sowie humanen Rac1 und Rac2 Proteinen.
Grauhinterlegte Bereiche zeigen die Position des G1-Elementes (GXXXXGKS/T; Aminosäure 13 bis 20), der G2-Effektorregion (Aminosäure 29 bis 45), des G3-Elementes (LWDTAGQ; Aminosäure 58 bis 64), des G4-Elementes (TKXD; Aminosäure 118 bis 121), des G5 Elementes (EXS) und des C-terminalen Isoprenylierungsmotives (CXXX, HassanainHHetal. (2000) BiochemBiophys Res Commun. 272(3):783-8.) an. Bindestriche zeigen Sequenzlücken an. Sterne stellen identische Aminosäuren in allen Homologen dar. Aminosäuren die zwischen Gerste einerseits und Mais und Reis andererseits differieren sind weiß auf schwarzem Grund dargestellt. Die Position die zum Erhalt einer dominant-negativen RacB-Variante vorteilhaft verändert wird, ist oberhalb der Sequenz mit einem schwarzen Dreieck markiert.
2. Fig. 2: Expression von RacB in epidermalem Gewebe
RT-PCR von RNA aus den Gerste-Linien Pallas und BCPM1a12 (P10) 24 h nach Inokulation ("hai" hours after inoculation") mit BghA6. Zur Extraktion der RNA wurden abaxial epidermale Streifen (E, aus inokulierten Stellen der Blätter) vom Mesophyll und der adaxialen Epidermis (M) abgetrennt. Ubiquitin 1 (Ubi) fungierte als Marker für eine gewebsunspezifische Expression, OXLP als Positivkontrolle für die Genexpression in der Epidermis, Bas als Positivkontrolle für die Genexpression in Mesophyllzellen. RT-PCR wurde mit 25 Amplifikationszyklen wie unten beschrieben ausgeführt. RT-PCR-Produkte wurden im Gel denaturiert, geblotted und mittels antisense RNA-Sonden unter stringenten Bedingungen detektiert.
3. Fig. 3: *RacB* wird konstitutiv in verschiedenen resistenten Gerste-Linien exprimiert.
RNA wurde aus der Sorte Ingrid (*Mlo*, *Ror1,* Bgh suszeptibel), BCIngrid-*mlo5* (*mlo5, Ror1,* Bgh resistent) und A89 (*mlo5*, *ror1,* BghA6 moderat suszeptibel) unmittelbar vor Inokulation (0 Ø) bzw. 8, 15, 24 h nach Inokulation mit *Bgh*, sowie 24 h danach aus nicht-inokulierten Kontrollpflanzen (24 Ø) isoliert. Ubiquitin 1 (*Ubi*) wurde als Marker für eine konstitutive Expression verwendet, *OXLP* als Positivkontrolle für eine *Bgh*-induzierte Genexpression in der epidermalen Schicht Die OXLP-Expression wurde per Northern-Blot nachgewiesen. Die RT-PCR für *RacB* und *Ubi* wurde wie beschrieben mit 25 Amplifikationszyklen durchgeführt. Die PCR-Produkte wurden im Gel denaturiert, geblotted und mittels antisense RNA-Proben unter stringenten Bedingungen detektiert.

4. Fig. 4: "RNA Interference" mit *RacB*-dsRNA vermindert die Penetrationseffizienz des Echten Gerstenmehltau BghA6 in Gerste.

Die relative Penetrationseffizienz (RPE) wurde in sechs individuellen Experimenten bei Inokulation mit *Bgh* aus Gerste cv Pallas bestimmt. Die RPE errechnet sich als Differenz aus der Penetrationseffizienz bei RacB-dsRNA transformierten Zellen und der Penetrationseffizienz bei Kontroll-dsRNA transformierten Zellen (hier: durchschnittliche Penetrationseffizienz 57 %). Die prozentuale RPE (%-RPE) errechnet sich aus der RPE minus 1 und multipliziert mit 100.

$$\text{RPE} = \frac{[\text{PE bei RacB-dsRNA transformierten Zellen}]}{[\text{PE bei Kontroll-dsRNA transformierten Zellen}]}$$

$$\%\text{-RPE} = 100 * (\text{RPE}-1)$$

Die schwarzen Säulen stellen die %-RPE bei Evaluierung von mindesten 100 Interaktionsstellen für jeweils ein unabhängiges Experiment dar. Die weiße Säule stellt die durchschnittliche %-RPE der Experimente mit der RacB-dsRNA dar ("RACB-dsRNA"). Der Fehlerbalken gibt den Standardfehler an.

"Control" stellt die parallelen Experimente mit einer Kontroll-dsRNA.

Die %-RPE war in Zellen, die mit RacB-dsRNA beschossen wurden, deutlich vermindert im Vergleich zu Zellen, die mit einer Kontroll-dsRNA (TR: humaner Thyroidrezeptor-dsRNA) bombardiert wurden.

5. Fig 5: Einfluss des genetischen Hintergrundes auf die Funktion von RacB

Die %-RPE wurde in 5 unabhängigen Experimenten durch Inokulation von Gerste cv Pallas, Ingrid oder A89 mit BghA6 untersucht, die zuvor mit RacB-dsRNA transformiert wurden.

Die %-RPE ist in Pallas (*Mlo Ror1,* Schwarze Balken, Experiment 1 und 2) oder Ingrid (*Mlo Ror1,* Schwarze Balken, Experiment 3, 4 und 5) deutlich reduziert. %-RPE der suszeptiblen Mutante A89 (mlo5 ror1, schwarze Balken, Experimente 1 bis 5) war jedoch nicht vermindert. Weiße Balken geben den Mittelwert an, Fehlerbalken den Standardfehler.

6. Fig.6: Überexpression einer konstitutiv aktiven RacB-Mutante in Gerste der Sorte Pallas

Eine konstitutiv aktive Mutante von Gerste RACB (Austausch G->V an Position 15; RacB-V15) wurde unter Verwendung des Expressionskonstruktes pGY-RacBV15 in 5 unabhängigen Experimenten transient in Gerste der Sorte Pallas überexprimiert. Im Vergleich wurden entsprechende Versuche mit dem Vektor alleine ohne RacB-Insert (pGY) durchgeführt.

Die Expression einer konstituiven RacB-Mutante bewirkt eine signifikant erhöhte Anfälligkeit gegen Pathogenbefall mit Gerstenmehltau im Vergleich zu den Kontrollen (Fig. 6-A). Die relative Suszeptibilität gegenüber dem Pilzpathogen ist in allen Fällen erhöht (Fig. 6-B). Auch diese Ergebisse belegen die Schhlüsselfunktion von racB bei der Pathogenabwehr.

7. Fig. 7: Plasmidkarte zu Expressionsvektor pGY-1 (Schweizer P et al. (1999) Mol Plant Microbe Interact 12: 647-54; Shinshi H et al. (1990) Plant Mol Biol 14:357-368).

Beispiele

Allgemeine Methoden:

Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die im Rahmen der vorliegenden Erfindung durchgeführten Klonierungsschritte wie z.B. Restriktionsspaltungen, Agarosegelelektrophorese, Reinigung von DNA-Fragmenten, Transfer von Nukleinsäuren auf Nitrozellulose und Nylonmembranen, Verknüpfen von DNA-Fragmenten, Transformation von E. coli Zellen, Anzucht von Bakterien, Vermehrung von Phagen und Sequenzanalyse rekombinanter DNA werden wie bei Sambrook et al. (1989) Cold Spring Harbor Laboratory Press; ISBN 0-87969-309-6 beschrieben durchgeführt. Die Sequenzierung rekombinanter DNA-Moleküle erfolgt mit einem Laserfluoreszenz-DNA-Sequenzierer der Firma MWG-Licor nach der Methode von Sanger (Sanger et al. (1977) Proc Natl Acad Sci USA 74: 5463-5467).

Beispiel 1: Pflanzen, Pathogene und Inokulation

Die Gerstensorte Ingrid stammt von James McKey, University of Uppsala, Schweden. Die Sorte Pallas und die rückgekreuzte Linie BCIngrid-*mlo5* wurde von Lisa Munk, Department of Plant Pathology, Royal Veterinary and Agriculturai University, Kopenhagen, Dänemark zur Verfügung gestellt. Ihre Herstellung ist beschrieben (Kølster P et al. (1986)Crop Sci 26: 903-907). Die Linie A89 wurde durch Paul Schulze-Lefert (Max-Plank-Institut für Züchtungsforschung, Köln, Deutschland) bereitgestellt.

Das 12 bis 36 h im Dunkeln auf feuchtem Filterpapier vorgekeimte Saatgut wurde, wenn nicht anders beschrieben, zu je 5 Körnern an den Rand eines Vierkanttopfes (8x8cm) in Fruhstorfer Erde vom Typ P ausgelegt, mit Erde bedeckt und regelmäßig mit Leitungswasser gegossen. Alle Pflanzen wurden in Klimaschränken oder -kammern bei 16 bis 18°C, 50 bis 60 % relativer Luftfeuchte und einem 16-stündigen Licht / 8-stündigen Dunkelheitszyklus mit 3000 bzw. 5000 lux (50 bzw. 60 $\mu mols^{-1}m^{-2}$ Photonenflussdichte) 5 bis 8 Tage lang kultiviert und im Keimlingsstadium in den Versuchen verwendet. Bei Experimenten, in denen Applikationen an Primärblättern durchgeführt wurden, waren diese vollständig entwickelt.

Vor Durchführung der transienten Transfektionsexperimente wurden die Pflanzen in Klimaschränken oder -kammern bei tagsüber 24°C, nachts 20°C, 50 bis 60 % relativer Luftfeuchte und einem 16stündigen Licht / 8stündigen Dunkelheitszyklus mit 30000 lux kultiviert.

Für die Inokulation von Gerstenpflanzen wurde Echte Gerstenmehltau *Blumeria graminis* (DC) Speer f.sp. *hordei* Em. Marchal der Rasse A6 (Wiberg A (1974) Hereditas 77: 89-148) (BghA6) verwendet. Dieser wurde vom Institut für Biometrie, JLU Gießen bereitgestellt. Die Nachzucht des Inokulums erfolgte in Klimakammern zu den gleichen Bedingungen, wie sie oben für die Pflanzenbeschrieben sind, durch Übertragung der Konidien von befallenem Pflanzenmaterial auf regelmäßig angezogene, 7 Tage alte Gerstenpflanzen cv. Golden Promise bei einer Dichte von 100 Konidia/$mm^2$.

Die Inokulation mit BghA6 erfolgte unter Verwendung von 7 Tagen alten Keimlingen durch Abschütteln der Konidien bereits befallener Pflanzen in einem Inokulationsturm mit ca. 100 Konidien/$mm^2$ (soweit nicht anders angegeben).

Beispiel 2: RNA-Extraktion

Gesamt RNA wurde aus 8 bis 10 primären Blattsegmenten (Länge 5 cm) mittels "RNA Extraction Buffer" (AGS, Heidelberg, Germany) extrahiert.

Dazu wurden das zentrale Primärblattsegment von 5 cm Länge geerntet und in flüssigem Stickstoff in Mörsern homogenisiert. Das Homogenisat wurde bis zur RNA-Extraktion bei -70°C gelagert.

Aus dem tiefgefrorenen Blattmaterial wurde mit Hilfe eines RNA-Extraktions-Kits (AGS, Heidelberg) Gesamt-RNA extrahiert. Dazu wurden 200 mg des tiefgefrorenen Blattmaterials in einem Mikrozentrifugenröhrchen (2 mL) mit 1,7 mL RNA-Extraktionspuffer(AGS) überschichtet und sofort gut durchmischt. Nach Zugabe von 200 µL Chloroform wurde erneut gut gemischt und bei Raumtemperatur 45 min auf einem Horizontalschüttler bei 200 U/min geschüttelt. Anschließend wurde zur Phasentrennung 15 min bei 20000 g und 4°C zentrifugiert, die obere wässrige Phase in ein neues Mikrozentrifugenröhrchen überführt und die untere verworfen. Die wässrige Phase wurde erneut mit 900 µL Chloroform gereinigt, indem 3mal für 10 sec homogenisiert und erneut zentrifugiert (s.o.) und abgehoben wurde. Zur Fällung der RNA wurde dann 850 µL 2-Propanol hinzugegeben, homogenisiert und für 30 bis 60 min auf Eis gestellt. Im Anschluss daran wurde für 20 min zentrifugiert (s.o), vorsichtig der Überstand dekantiert, 2 mL 70 %iges Ethanol (-20°C) hinzu pipettiert, durchmischt und erneut 10 min zentrifugiert. Der Überstand wurde dann wiederum dekantiert und das Pelet vorsichtig mit einer Pipette von Flüssigkeitsresten befreit, bevor es an einem Reinluftarbeitsplatz im Reinluftstrom getrocknet wurde. Danach wurde die RNA in 50 µL DEPC-Wasser auf Eis gelöst, durchmischt und 5 min zentrifugiert (s.o.). 40 µl des Überstandes wurden als RNA-Lösung in ein neues Mikrozentrifugenröhrchen überführt und bei -70°C gelagert.

Die Konzentration der RNA wurde photometrisch bestimmt. Dazu wurde die RNA-Lösung 1:99 (v/v) mit destilliertem Wasser verdünnt und die Extinktion (Photometer DU 7400, Beckman) bei 260 nm gemessen ($E_{260\ nm}$ = 1 bei 40 µg RNA/mL). Gemäß der errechneten RNA-Gehalte wurden die Konzentrationen der RNA-Lösungen anschließend mit DEPC-Wasser auf 1 µg/µL angeglichen und im Agarosegel überprüft.

Zur Überprüfung der RNA-Konzentrationen im horizontalen Agarosegel (1 % Agarose in 1 x MOPS-Puffer mit 0,2 µg/mL Ethidiumbromid) wurde 1 µL RNA-Lösung mit 1 µL 10 x MOPS, 1 µL Farbmarker und 7 µL DEPC-Wasser versetzt, nach Ihrer Größe bei 120 V Spannung im Gel in 1 x MOPS-Laufpuffer über 1,5 h aufgetrennt und unter Um-Licht fotographiert. Eventuelle Konzentrationsunterschiede der RNA-Extrakte wurden mit DEPC-Wasser ausgeglichen und die Anpassung erneut im Gel überprüft.

Beipiel 3: Klonierung der RacB cDNA Sequenz aus Gerste

Die zur Isolation der HvRacB cDNA, ihrer Klonierung, Sequenzierung und Herstellung von Sonden benötigten cDNA Fragmente wurden mittel RT-PCR unter Verwendung des "One Step RT-PCR Kit" (Life Technologies, Karlsruhe, Deutschland oder Qiagen, Hilden, Deutschland) erhalten. Dazu wurde Gesamt-RNA aus Gerste-Sämlingen als Matrize verwendet. Die RNA wurde aus Pallas 3, 5 und 7 Tage nach Keimung isoliert. Darüberhinaus wurde RNA aus Pallas und den rückgekreuzten Linien mit *mlo5, Mlg* oder *Mla12* 1, 2 und 5 Tage nach Inokulation mit *Bgh*A6 am 7 Tag nach Keimung isoliert. Für die RT-PCR werden die folgenden Primer verwendet:

ONP-1 5'-GGATCCGATGAGCGCGTCCAGGTT-3' (SEQ ID NO: 10) und

ONP-2 5'-GTCGACCTTCGCCCTTGTTCTTTGTC-3' (SEQ ID NO: 11)

Für die Reaktion (25 μL-Ansatz) wurden je 1000 ng Gesamt-RNA, 0,4 mM dNTPs, je 0,6 mM OPN-1 und OPN-2 Primer, 10 μL RNase-Inhibitor und 1 μL Enzymmix in 1x RT-Puffer (*one step RT-PCR Kit,* Qiagen, Hilden) eingesetzt.
Folgendes Temperaturprogramm wird verwendet (PTC-100TM Modell 96V; MJ Research, Inc., Watertown, Massachussetts):

| | |
|---|---|
| 1 | Zyklus mit 30 min bei 50°C |
| 1 | Zyklus mit 150 sec bei 94°C |
| 30 | Zyklen mit 94°C für 45 sec, 55°C für 1 min und 72°C für 2 min |
| 1 | Zyklus mit 72°C für 7 min |

Die PCR Produkt wurde mittels 2% w/v Agarosegelelektrophorese aufgetrennt. Es wurde ein RT-PCR Produkt von insgesamt 642 bp erhalten, dass sich aus der RacB-Sequenz (SEQ ID NO: 1) und terminalen Sequenzen kodierend für Restriktionsendonukleasaeschnittstellen zusammensetzt. Das Fragment kodiert für ein offnes Leseraster von 591 bp kodierend für ein Polypeptid aus 197 Aminosäuren. Die entsprechende cDNA wurde aus einem Agarosegel isoliert und in den pGEM-T-Vektor (Promega, Mannheim, Deutschland) mittels T-Überhang-Ligation kloniert. Die cDNAs wurden ausgehend von der Plasmid-DNA unter Verwendung des "Thermo Sequenase Fluorescent Labeled Primer Cycle Sequencing Kit" (Amersham, Freiburg, Deutschland) sequenziert.
Da als Ausgangsprimer OPN-1 ein Primer von der RacB-Sequenz aus Reis abgeleitet worden ist (GenBank Acc.-No.: AF250327) wurde dieser Bereich (d.h. das 5'-Ende) der RacB-cDNA aus Gerste nochmals mittel der RACE-Technologie unter Verwendung des "GeneRacer Kit" (INVITROGENE Life Technologies) verifiziert. Dazu wurden 100 ng poly-A mRNA, 1 μL 10xCIP-Puffer, 10 Units RNAse-Inhibitor, 10 Units CIP ("calf intestinal phosphatase") und DEPC-behandeltes Wasser bis zu einem Gesamtvolumen von 10 μL für 1 h bei 50°C behandelt. Zur Präzipitation der RNA wurden weitere 90 μL DEPC-Wasser und 100 μL Phenol:chloroform hinzugegeben und intensiv für ca. 30 sec durchmischt. Nach 5 min Zentrifugation bei 20.000 g wurde die obere Phase mit 2 μL 10 mg/ml Mussel Glycogen, 10 μL 3 M Natriumacetat (pH 5,2) in einem neuen Mikroreaktionsgefäß versetzt. 220 μL 95 % Ethanol wurden hinzugegeben und die Mischung auf Eis inkubiert. Anschließend wurde die RNA durch Zentrifugation für 20 min bei 20.000 g und 4°C präzipitiert. Der Überstand wurde verworfen, 500 μL 75 % Ethanol hinzugegeben, kurz gevortext und wieder für 2 min zentrifugiert (20000 g). Der Überstand wurde wiederum verworfen, das Präzipitat für 2 min bei Raumtemperatur an der Luft getrocknet und anschließend in 6 μL DEPC-Wasser suspendiert. mRNA CAP-Strukturen wurden durch Zugage von 1 μL 10xTAP Puffer, 10 Units RNAsin und 1 Unit TAP ("tobacco acid pyrophosphatase") entfernt. Die Mischung wurde für 1 h bei 37°C inkubiert und anschließend auf Eis gekühlt. Die RNA wurde wiederum- wie oben beschrieben - präzipitiert und in ein Reaktionsgefäß mit 0,25 μg GeneRacer Oligonukleotid-Primer überführt. Der Oligonukleotid-Primer wurde in der RNA-Lösung resuspendiert, die Mischung für 5 min bei 70°C inkubiert und dann auf Eis gekühlt. 1 μL 10xLigasepuffer, 10 mM ATP, 1 Unit RNAsin und 5 Units T4 RNA-Ligase wurden hinzugegeben und der Reaktionsansatz für 1 h bei 37°C inkubiert. Die RNA wurde wiederum - wie oben beschrieben - präzipitiert und in 13 μl DEPC-Wasser resuspendiert. 10 pMol oligo-dT Primer wurden zu der RNA gegeben, sofort auf 70°C erhitzt und wieder auf Eis gekühlt. 1 μL jeder dNTP-Lösung (25 mM), 2 μL 10xRT buffer, 5u (1 μl) AMV reverse Transkriptase und 20 Units RNAsin wurden hinzugegeben und die Reaktionslösung für 1 h bei 42°C und anschließend für 15 min bei 85°C inkubiert. Die so hergestellte Erststrang-cDNA wurde bei -20°C gelagert.
Zur Amplifikation der 5'-cDNA-Endes wurde nachfolgender Primer verwendet:

RACE-RacB Primer:
5'-gtgggcacatagtcggtggggaaggt-3' (SEQ ID NO: 12)

GeneRacer™ 5'-Primer:
5'-CGACTGGAGCACGAGGACACTGA-3 (SEQ ID NO: 13)

GeneRacer™ 5'-Nested Primer:
5'-GGACACTGACATGGACTGAAGGAGTA-3 (SEQ ID NO: 14)

Der Ansatz (Gesamtvolumen 25 µL) hatte nachfolgende Zusammensetzung:

| | |
|---|---|
| 1 µl | Primer RACE-RacB (5 pmol/µL) , |
| 0,5 µl | GeneRacer 5 '-Primer (10 pmol/µL) |
| 2,5 µl | 10xPuffer Qiagen, |
| 2,5 µl | dNTPs (2mM) |
| 0,5 µl | cDNA |
| 0,2 µl | QiagenTAG (5 u/microL) |
| 17,8 µl | H2O |

Die PCR-Bedingungen lauteten:

| | |
|---|---|
| 94°C | 5 min Denaturierung |
| 5 Zyklen mit | 70°C 30 sek (Annealing),<br>72°C 1 min (Extension),<br>94°C 30 sek (Denaturierung) |
| 5 Zyklen mit | 68°C 30 sek (Annealing),<br>72°C 1 min (Extension),<br>94°C 30 sek (Denaturierung) |
| 28 Zyklen mit | 66°C 30 sek (Annealing),<br>72°C 1 min (Extension),<br>94°C 30 sek (Denaturierung) |
| 72°C<br>4°C | 10 min abschließende Extension Kühlung bis zur Weiterverarbeitung |

Die PCR ergab ein Produkt von ca. 400 bp Produkt. Davon ausgehend wurde eine "nested"-PCR mit deem RacB-spezifischen Oligonukleotidprimer und dem "GeneRacer Nested 5'-Primer" durchgeführt:

| | |
|---|---|
| 94°C | 5 min Denaturierung |
| 30 Zyklen mit | 64°C 30 sek (Annealing),<br>72°C 1 min (Extension),<br>94°C 30 sek (Denaturierung) |
| 72°C<br>4°C | 10 min abschließende Extension Kühlung bis zur Weiterverarbei |

Das erhaltene PCR-Produkt wurde über ein Gel isoliert, aus dem Gel extrahiert und in pGEM-T mittels T-Überhang-Ligation kloniert und sequenziert. Die Sequenz im Bereich des Primers OPN-1 war absolut identisch zu der von racB aus Reis, so dass mittels-Primers keine Punktmutationen erzeugt wurden. Die unter SEQ ID NO: 1 wiedergegebene Sequenz ist also identisch mit der RacB-Sequenz aus Gerste.

Beispiel 4: Reverse Transkription - Polymerasekettenreaktion (RT-PCR)

Für die semi-quantitative RT-PCR wurde der "OneStep RT-PCR Kit" (Qiagen, Hilden, Germany) verwendet. Dabei wurde RNA (herstellung s.o.) zuerst in cDNA übersetzt (Reverse Transkription) und in einer anschließenden PCR-Reaktion mit spezifischen Primern die gesuchte cDNA amplifiziert. Um die Ausgangsmenge an matrizen RNA abzuschätzen, wurde die Amplifikation während der exponentiellen Phase unterbrochen um Unterschiede in der Ziel-RNA wiederzuspiegeln. Die PCR Produkte wurden über ein Agarosegel aufgetrennt, denaturiert, auf Nylonmembranen geblottet und mit spezifischen, nicht-radioaktiv-markierten Sonden unter stringenten Standardbedingungen detektiert. Hybridisierung, Waschschritte und Immunodetektion erfolgten wie unter "Northern Blot" beschrieben.

Für die einzelnen Reaktionen (25 µL-Ansatz) wurden unter Verwendung des "*One Step RT-PCR Kit*" (Qiagen, Hilden, Deutschland) zusammengegeben:

1000 ng Gesamt-RNA einer bestimmten Probe
0,4 mM dNTPs,
jeweils 0,6 µM *sense*- und *antisense*-Primer
0,1 µl RNase-Inhibitor
1 µL Enzymmix in 1x RT-Puffer

Die cDNA-Synthese (reverse Transkription) erfolgte für 30 min bei 50°C. Anschließend wurde die Reverse Transkriptase für 15 min bei 95°C inaktiviert, was zugleich Aktivierung der DNA-Polymerase und Denaturierung der cDNA bewirkt. Anschließend folgt eine PCR gemäß nachfolgendem Programm:

| | | |
|---|---|---|
| 1 min 94 °C Denaturierung | | |
| 25 Zyklen mit | 1 min 54°C | Primeranlagerung |
| | 1 min 72°C | Primerverlängerung |
| | 10 min 72°C | Komplettierung der DNA-Doppelstränge |
| abschließend: | 4°C Abbruch der Reaktion | |

Die PCR-Produkte wurden im 1xTBE-Agarosegel mit Ethidiumbromid aufgetrennt.

Für die einzelnen Ansätze wurden mit den nachfolgenden Parren von Oligonukleotid-Primern amplifiziert:

a) Amplifikation eines 387 bp Fragment der Gerste RacB cDNA

| | |
|---|---|
| RacB-sense | 5'-gttcatcaagtgcgtcaccgtg-3' (SEQ ID NO: 15) |
| RacB-antisense | 5'-ttagcttcctcagttcttccctg-3'(SEQ ID NO: 16) |

b) Amplifikation eines 674 bp Fragment der Gerste *BAS* cDNA (GenBank Acc.-No Z34917)

| | |
|---|---|
| BAS-sense | 5'-cgcgccgcagccgagtacgac-3' (SEQ ID NO: 17) |
| BAS-antisense | 5'-gtcacaaaaacacatgtaacc-3' (SEQ ID NO: 18) |

c) Amplifikation eines 506 bp *OXLP* cDNA Fragments (GenBank Acc.-No X93171)

| | |
|---|---|
| OXLP-sense | 5'-ggccgacatgcattcaccag-3' (SEQ ID NO: 19) |
| OXLP-antisense | 5'-catctgatattgctgggtctg-3' (SEQ ID NO: 20) |

d) Amplifikation eines 513 bp *Ubi* cDNA Fragment (GenBank accession M60175)

| | |
|---|---|
| UBI-sense | 5'-ccaagatgcagatcttcgtga-3' (SEQ ID NO: 21) |
| UBI-antisense | 5'-ttcgcgataggtaaaagagca-3' (SEQ ID NO: 22) |

Alle erhaltenen Fragmente wurden zudem in den Vektor pGEM-T mittels T-Überhang-Ligation ligiert und dienten als Ausgangsplasmide für die Herstellung von Sonden (z.B. für Northern-Blot) bzw. dsRNA. Die einzelnen Konstrukte trugen die Bezeichnung pGEMT-RAC1, pGEMT-BAS, pGEMT-OXLP, pGEMT-UBI.

Beispiel 5: Northern-Blot Analyse

Zur Vorbereitung des Northern-Blottings wurde die RNA im Agarosegel unter denaturierenden Bedingungen aufgetrennt. Ein Teil RNA-Lösung (entsprechend 5 μg RNA) wurde dazu mit gleichem Volumen Probenpuffer (mit Ethidiumbromid) gemischt, 5 min bei 94°C denaturiert, 5 min auf Eis gestellt, kurz zentrifugiert und aufs Gel aufgetragen. Das 1 x MOPS-Gel (1,5 % Agarose, *ultra pure*) enthielt 5 Volumenprozent konzentrierte Formaldehydlösung (36,5 % [v/v]). Die RNA wurde bei 100 V 2 h lang aufgetrennt und anschließend geblottet.

Das Northern-Blotting erfolgte als aufwärtsgerichteter RNA-Transfer im Kapillarstrom. Das Gel wurde dazu zunächst 30 min in 25 mM Natriumhydrogen/dihydrogenphosphat-Puffer (pH 6,5) geschwenkt und zurechtgeschnitten. Ein Whatmanpapier wurde so vorbereitet, dass es auf einer horizontalen Platte auflag und auf 2 Seiten in eine Wanne mit 25 mM Natriumhydrogen/dihydrogenphosphat-Puffer (pH 6,5) ragte. Auf dieses Papier wurde das Gel aufgelegt, wobei nicht bedeckte Teile des Whatmanpapiers mit einer Plastikfolie abgedeckt wurden. Das Gel wurde dann mit einer positiv geladenen Nylonmembran (Boehringer-Mannheim) luftblasenfrei abgedekt, wonach die Membran wiederum mit saugfähigem Papier in mehreren Lagen etwa 5 cm hoch bedeckt wurde. Das saugfähige Papier wurde noch mit einer Glasplatte und einem 100 g Gewicht beschwert. Das Blotting erfolgte über Nacht bei Raumtemperatur. Die Membran wurde kurz in A. bidest. geschwenkt und zur RNA-Fixierung mit einer Lichtenergie von 125 mJ im *Crosslinker* (Biorad) UV-Licht bestrahlt. Die Überprüfung des gleichmäßigen RNA-Transfers auf die Membran erfolgte auf der UV-Lichtbank.

Zur Detektion von Gersten mRNA wurden 10 μg Gesamt-RNA aus jeder Probe über ein Agarosegel aufgetrennt und mittels Kapillartransfer auf eine positiv-geladene Nylonmembran geblottet. Die Detektion erfolgte mit dem DIG-Systeme.

Herstellung der Sonden: Zur Hybridisierung mit den zu detektierenden mRNAs wurden mit Digogygenin oder Fluoreszein markierte RNA Sonden hergestellt. Diese wurden durch in *vitro* Transkription eines PCR-Produktes mittels einer T7 oder SP6 RNA Polymerase mit markierten UTPs erstellt. Als Vorlage für die PCR gestützte Amplifikation dienten die oben beschriebenen Plasmidvektoren pGEMT-RAC1, pGEMT-BAS, pGEMT-OXLP, pGEMT-UBI.

Je nach Orienttierung des Inserts wurden unterschiedliche RNA-Polymerasen zur Herstellung des antisense-Stranges herangezogen. Die T7-RNA-Polymerase wurde für pGEMT-BAS und pGEMT-OXLP verwendet, die SP6-RNA-Polymerase für pGEMT-RAC1 und pGEMT-UBI.

Das Insert der einzelnen Vektoren wurde über PCR mit flankierenden Standard-Primern (M13 fwd und rev) amplifiziert. Die Reaktion lief dabei mit folgenden Endkonzentrationen in einem Gesamtvolumen von 50 μL PCR-Puffer (Silverstar) ab:

| | |
|---|---|
| M13-fwd: | 5'-GTAAAACGACGGCCAGTG-3' (SEQ ID NO: 23) |
| M13-Rev: | 5'-GGAAACAGCTATGACCATG-3' (SEQ ID NO: 24) |

10 % Dimethylsulfoxid (v/v)
je 2 ng/μL Primer (M13 *forward* und *reversed*)
1,5 mM $MgCl_2$,
0,2 mM dNTPs,
4 Units Taq-Polymerase (Silverstar),
2 ng/gL Plasmid-DNA.

Die Amplifikation verlief in einem *Thermocycler* (Perkin-Elmar 2400) temperaturgesteuert:

| | |
|---|---|
| 94°C | 3 min Denaturierung |
| 30 Zyklen mit | 94°C 30 sek (Denaturierung)<br>58°C 30 sek (Annealing),<br>72°C 1,2 min (Extension), |
| 72°C<br>4°C | 5 min abschließende Extension Kühlung bis zur Weiterverarbeitung |

Der Erfolg der Reaktion wurde im 1 %igen Agarosegel überprüft. Die Produkte wurden anschließend mit einem "*High Pure PCR-Product Purification Kit*" (Boehringer-Mannheim) aufgereinigt. Man erhielt etwa 40 μL Säuleneluat, das erneut im Gel überprüft und bei -20°C gelagert wurde.

Die RNA Polymerisation, die Hybridisierung und die Immunodetektion wurden weitestgehend nach Angaben des Herstellers des *Kits* zur nicht-radioaktiven RNA-Detektion durchgeführt (DIG System User's Guide, DIG-Luminescence detection Kit, Boehringer-Mannheim, Kogel et al. (1994) Plant Physiol 106:1264-1277). 4 µl gereinigtes PCR-Produkt wurden mit 2 µL Transskriptionspuffer, 2 µl NTP-Markierungsmix, 2 µl-NTP-Mix und 10 µl DEPC-Wasser versetzt. Anschließend wurden 2 µL der T7-RNA-Polymeraselösung zu pipettiert. Die Reaktion-wurde dann 2 h bei 37°C durchgeführt und anschließend mit DEPC-Wasser auf 100 µL aufgefüllt. Die RNA-Sonde wurde im Ethidiumbromidgel detektiert und bei -20°C gelagert.

Zur Vorbereitung der Hybridisierung wurden die Membranen zunächst 1 h bei 68°C in 2 x SSC (*Salt, Sodiumcitrate),* 0,1 % SDS-Puffer (*Sodiumdodecylsulfate*) geschwenkt, wobei der Puffer 2 bis 3 mal erneuert wurde. Die Membranen wurden anschließend an die Innenwand auf 68°C vorgeheizter Hybridisierungsröhren angelegt und 30 min mit 10 mL *Dig-Easy*-Hybridisierungspuffer im vorgeheizten Hybridisierungsofen inkubiert. Währenddessen wurden 10 µL Sondenlösung in 80 µL Hybridisierungspuffer bei 94°C für 5 min denaturiert, anschließend auf Eis gestellt und kurz zentrifugiert. Zur Hybridisierung wurde die Sonde dann in 10 mL 68°C warmem Hybridisierungspuffer überführt, und der Puffer in der Hybridisierungsröhre durch diesen Sondenpuffer ersetzt. Die Hybridisierung erfolgte dann ebenfalls bei 68°C über Nacht.

Vor Immundetektion von RNA-RNA Hybriden wurden die Blots stringent zweimal für jeweils 20 min in 0.1 % (w/v) SDS, 0.1 x SSC bei 68°C gewaschen.

Zur Immunodetektion wurden die Blots zunächst zweimal für 5 min bei RT in 2 x SSC, 0,1 % SDS geschwenkt. Anschließend erfolgten 2 stringente Waschschritte bei 68°C in 0,1 x SSC, 0,1 % SDS für je 15 min. Die Lösung wurde anschließend durch Waschpuffer ohne Tween ersetzt. Es wurde 1 min geschüttelt und die Lösung durch Blockingreagenz ausgetauscht. Nach weiteren 30 min Schütteln wurden 10 µL Anti-Fluoreszein-Antikörperlösung hinzugefügt und weitere 60 min geschüttelt. Es folgten zwei 15 minütige Waschschritte in Waschpuffer mit Tween. Die Membran wurde anschließend 2 min in Substratpuffer äquilibriert und nach Abtropfen auf eine Kopierfolie überführt. Auf der "RNA-Seite" der Membran wurde dann ein Gemisch aus 20 µL CDP-Star™ und 2 mL Substratpuffer gleichmäßig verteilt. Im Anschluss wurde die Membran mit einer zweiten Kopierfolie abgedeckt und an den Rändern mit Hitze luftblasenfrei und wasserdicht verschweißt. Die Membran wurde dann in einer Dunkelkammer für 10 min mit einem Roentgenfilm bedeckt und dieser anschließend entwickelt. Je nach Stärke der Lumineszenzreaktion wurde die Belichtungszeit variiert.

Wenn nicht extra gekennzeichnet waren die Lösungen im Lieferumfang des *Kits* enthalten (*DIG-Luminescence detection Kit,* Boehringer-Mannheim). Alle anderen wurden aus folgenden Stammlösungen durch Verdünnung mit autoklaviertem, destilliertem Wasser hergestellt. Alle Stammlösungen wurden, wenn nicht anders spezifiziert, mit DEPC (wie DEPC-Wasser) angesetzt und anschließend autoklaviert.

- DEPC-Wasser: Destilliertes Wasser wird über Nacht bei 37°C mit Diethylpyrokarbonat (DEPC, 0,1 %, w/v) behandelt und anschließend autoklaviert
- 10 x MOPS-Puffer: 0,2 M MOPS (Morpholin-3-propansulfonsäure), 0,05 M Natriumacetat, 0,01 M EDTA, pH mit 10 M NaOH auf pH 7,0 eingestellt
- 20 x SSC (Natriumchlorid-Natriumzitrat, *Salt-Sodiumcitrate*): 3 M NaClo, 0.3 M triNatriumcitrat x 2 $H_2O$, pH mit 4 M HCl auf pH 7,0 eingestellt.
- 1 % SDS (Natriumdodecylsufat, *Sodiumdodecylsulfate*)Natrium-dodecylsulfat (w/v), ohne DEPC
- RNA-Probenpuffer: 760 µL Formamid, 260 µL Formaldehyd, 100 µL Ethidiumbromid (10 mg/mL), 80 µL Glycerol, 80 µL Bromphenolblau (gesättigt), 160 µL 10 x MOPS, 100 µL Wasser.
- 10 x Waschpuffer ohne Tween: 1,0 M Maleinsäure, 1,5 M NaCl; ohne DEPC, mit NaOH (fest, ca. 77 g) und 10 M NaOH auf pH 7,5 einstellen.
- Waschpuffer mit Tween: aus Waschpuffer ohne Tween mit Tween (0,3 %, v/v)
- 10 x Blockingreagenz: 50 g Blockingpulver (Boehringer-Mannheim) in 500 mL Waschpuffer ohne Tween suspendieren.
- Substratpuffer:100 mM Tris (Trishydroxymethylamino-methan), 150 mM NaCl mit 4 M HCl auf pH 9,5 einstellen.
- 10 x Farbmarker: 50 % Glycerol (v/v), 1,0 mM EDTA pH 8,0, 0,25 % Bromphenolblau (w/v), 0,25 % Xylencyanol (w/v).

Beispiel 6: In vitro Synthese der RacB dsRNA

Alle Plasmide (pGEMT-RAC1, pGEMT-BAS, pGEMT-OXLP, pGEMT-UBI) die für die in vitro Transkription eingesetzt wurden beinhalten den T7 und SP6 Promotor (pGEM-T, Promega) an den jeweiligen Enden der insertierten Nukleinsäuresequenz, was die Synthese von sense- bzw. antisense RNA ermöglicht. Die Plasmide können mit geeigneten Restriktionsenzymen linearisiert werden, um eine korrekte Transkription der insertierten Nukleinsäuresequenz zu gewährleisten und ein Durchlesen in vektorielle Sequenzen zu verhindern.

Dazu wurden 10 µg Plasmid-DNA jeweils mit an der distal vom Promoter gelegenen Seite des Inserts geschnitten. Die geschnittenen Plasmide werden in 200 µl Wasser mit gleichem Volumen Phenol/Chloroform/Isoamylalkohol extrahiert, in ein neues Eppendorfreaktionsgefäß (RNAse frei) transferiert und 5 min bei 20000 g zentrifugiert. 180 µl der Plasmid-Lösung wurden mit 420 µl Ethanol versetzt, auf Eis gestellt und anschließend durch Zentrifugation für 30 min bei 20000 g und - 4°C präzipitiert. Das Präzipitat wurde in 10 µl TE Puffer aufgenommen.

Zur Herstellung der RacB-dsRNA wurde das Plasmid pGEMT-Rac1 mit SpeI verdaut und sense-RNA mit der T7-RNA-Polymerase transkribiert. Ferner wurde pGEMT-Rac1 mit NcoI verdaut und antisense-RNA mit der SP6-RNA-Polymerase transkribiert. RNA Polymerasen wurden von Roche Molecular Biology, Mannheim, Deutschland bezogen.

Jeder Transkriptionsansatz beinhaltete in einem Volumen of 40 µl:

| | |
|---|---|
| 2 µl | linearisierte Plasmid DNA (1 µg) |
| 2 µl | NTP' s (25 mM) (1,25 mM von jedem NTP) |
| 4 µl | 10xReaktionspuffer (Roche Molecular Biology), |
| 1 µl | RNAsin RNAsin (27 Units; Roche Molecular Biology), |
| 2 µl | RNA Polymerase (40 Units) |
| 29 µl | DEPC-Wasser |

Nach einer Inkubation von 2 h bei 37°C wurde jeweils ein Teil der Reaktionsansätze aus der Transktiption des "sense"- bzw. "anti-sense"-Stranges gemischt, für 5 min bei 95°C denaturiert und anschließend durch Abkühlung über 30 min auf eine Endtemperatur von 37°C miteinander hybridisiert ("annealing"). Alternativ kann nach der Denaturierung das Gemisch aus sense- und antisense-STrang auch für 30 min bei -20°C gekühlt werden. Das Proteinpräzipitat, das sich während Denaturierung und Hybridisierung bildet wurde durch kurze Zentrifugation bei 20.800 g abgetrennt und der Überstand direkt zur Beschichtung von Wolframpartikeln verwendet (s. unten). Zur Analyse wurden jeweils 1 µl jeden RNA-Stranges und der dsRNA auf einem nicht-denaturierenden Agarosegel aufgetrennt. Eine erfolgreiche Hybridisierung zeigte sich, durch eine Bandenverschiebung zu höherem Molekulargewicht im Vergleich zu den Einzelsträngen.

4 µl der dsRNA wurden Ethanol-präzipitiert (durch Zugabe von 6 µl Wasser, 1 µl 3M Natriumacetat-Lösung und 25 µl Ethanol, sowie Zenrifugation für mindestens 5 min bei 20000 g und 4°C) und in 500 µl Wasser resuspendiert. Das Absorbtionsspektrum zwischen 230 und 300 nm wurde gemessen, bzw. die Absorption bei 280 und 260 nm bestimmt, um die Reinheit und die Konzentration der dsRNA zu bestimmen. In der Regel wurden 80 bis 100 µg dsRNA mit einem $OD_{260}/OD_{280}$-Verhältnis von 1,80 bis 1,95 erhalten. Ein Verdau mit DNase I kann optional durchgeführt werden, beeinflusst jedoch nachfolgende Ergebnisse nicht wesentlich.

Als Kontroll-dsRNA fungierte die dsRNA des humanen Thyroidrezeptors (Ausgangsvektor pT7betaSal (Norman C et al. (1988) Cell 55(6):989-1003) zur Verfügung gestellt von Dr. Baniahmad, Institut für Genetik, Gießen, Deutschland; die Sequenz des Insert ist beschrieben unter der GenBank Acc.-No.: NM_000461). Für die Herstellung der sense-RNA wurde das Plasmid mit PvuII, für die antsense-RNA mit HindIII verdaut und die RNA dann mit T7- bzw. SP6 RNA-Polymerase transkribiert. Die einzelnen Verfahrensschritte zur Herstellung der Kontroll-dsRNA werden analog den oben für die RacB-dsRNA beschriebenen durchgeführt.

Beispiel 7: Transiente Transformation, RNAi und Evaluation der Pilzpathogenentwicklung

Gerste cv Pallas Blattsegmente wurden mit einer *RacB*-dsRNA zusammen mit einem GFP-Expressionsvektor transformiert. Anschließend wurden die Blätter mit Bgh inokuliert und das Ergebnis nach 48 h mittels Licht- und Fluoreszenzmikroskopie analysiert. Die Penetration in GFP-exprimierenden Zellen wurde mittels Detektion von Haustorien in lebenden Zellen und durch Bewertung der Pilzentwicklung auf eben diesen Zellen beurteilt. In allen sechs Experimenten führte die Bombardierung von Gerste cv Pallas mit *RacB*-dsRNA zu einer verminderten Anzahl von erfolgreich durch Bgh penetrierten Zellen im Vergleich zu Zellen die mit einer fremden Kontroll-dsRNA (humaner Thyroidhormonrezeptor dsRNA, TR) bombardiert wurden. Der resistenzinduzierende Effekt der *RacB*-dsRNA bedingte eine durchschnittliche Verminderung der Penetrationseffizienz durch Bgh um 44 % (Fig. 4).

Es wurde ein Verfahren zur transienten Transformation eingesetzt das bereits für die biolistische Einführung von dsRNA in epidermale Zellen von Gerstenblättern beschrieben wurde (Schweizer P et al. (1999) Mol Plant Microbe Interact 12:647-54; Schweizer P et al. (2000) Plant J 2000 24: 895-903). Wolframpartikel mit einem Durchmesser von 1,1 μm (Partikeldichte 25 mg/ml) wurden mit dsRNA (Herstellung siehe oben) zusammen mit Plasmid-DNA des Vektors *pGFP* (GFP unter Kontrolle des CaMV 35S Promotors) als Transformationsmarker beschichtet. Dazu wurden pro Schuss die nachfolgender Mengen an dsRNA bzw. Reporterplasmid zur Beschichtung verwendet: 1 μg pGFP und 2 μg dsRNA. Dopplesträngige RNA wurde mittels Verschmelzens von "sense" und "antisense"-RNA *in vitro* synthetisiert (s.o.).

Für Microcarrier-Präparation wurden 55 mg Wolframpartikel (M 17, Durchmesser 1,1 μm; Bio-Rad, München) zweimal mit 1 ml autoklaviertem Destilliertem Wasser und einmal mit 1 mL absolutem Ethanol gewaschen, getrocknet und in 1 ml 50 %igem Glycerin aufgenommen (ca. 50 mg/ml Stammlösung). Die Lösung wurde mit 50 %igem Glycerin auf 25 mg/ml verdünnt, vor Gebrauch gut gemischt und im Ultraschallbad suspendiert. Zur Microcarrier-Beschichtung wurden pro Schuss 1 μg Plasmid, 2 μg dsRNA (1 μL), 12,5 μl Wolframpartikel-Suspension (25 mg/ml), 12,5 μl 1 M $Ca(NO_3)_2$-Lösung (pH 10) tropfenweise unter ständigem Mischen zusammengegeben, 10 min bei RT stehengelassen, kurz zentrifugiert und 20 μl vom Überstand abgenommen. Der Rest mit den Wolframpartikel wird resuspendiert (Ultraschallbad) und ins Experiment eingesetzt.

Es wurden ca. 4 cm lange Segmente von Gerstenprimärblättern verwendet. Die Gewebe wurden auf 0,5 % Phytagar (GibcoBRL™ Life Technologies™, Karlsruhe) mit 20 μg/ml Benzimidazol in Petrischalen (6,5 cm Durchmesser) gelegt und direkt vor dem Partikelbeschuss an den Rändern mit einer Schablone mit einer rechteckigen Aussparung von 2,2 cm x 2,3 cm abgedeckt. Die Schalen wurden nacheinander auf den Boden der Vakuumkammer (Schweizer P et al. (1999) Mol Plant Microbe Interact 12:647-54) gestellt, über dem ein Nylonnetz (Maschenweite 0,2 mm, Millipore, Eschborn) als Diffusor auf einer Lochplatte eingeschoben war (5 cm über dem Boden, 11 cm unterhalb des Macrocarriers, s.u.), um Partikelklumpen zu zerstreuen und den Partikelstrom abzubremsen. Der oben an der Kammer angebrachte Macrocarrier (Plastik-Sterilfilterhalter, 13 mm, Gelman Sciences, Swinney, UK) wurde je Schuss mit 5,8 μL DNA-beschichteten Wolframpartikeln (Microcarrier, s.u.) beladen. Mit einer Membranvakuumpumpe (Vacuubrand, Wertheim) wurde der Druck um 0,9 bar in der Kammer reduziert und die Wolframpartikel mit 9 bar Heliumgasdruck auf die Oberfläche des Pflanzengewebes geschossen. Sofort danach wurde die Kammer belüftet. Zur Markierung transformierter Zellen wurden die Blätter mit dem Plasmid (pGFP; Vektor auf pUC18-Basis, CaMV 35S-Promoter/Terminator-Kassette mit insertiertem GFP-Gen; Schweizer P et al. (1999) Mol Plant Microbe Interact 12:647-54; zur Verfügung gestellt von Dr. P. Schweizer Schweizer P, Institut für Pflanzengenetik IPK, Gatersleben, Deutschland) beschossen. Vor dem Schießen eines anderen Plasmids wurde der Macrocarrier jeweils gründlich mit Wasser gereinigt. Nach vierstündiger Inkubation nach dem Beschuss bei leicht geöffneten Petrischalen, RT und Tageslicht wurden die Blätter mit 100 Konidien/$mm^2$ des Echten Gerstenmehltaupilzes (Rasse A6) inokuliert und für weitere 36 bis 48 h unter gleichen Bedingungen inkubiert.

Blattsegmente wurden mit den beschichteten Partikeln unter Verwendung einer "particle inflow gun" bombardiert. Pro Schuss wurden 312 μg Wolframpartikel appliziert. 4 h nach der Bombardierung wurde Inokulation mit *Blumeria graminis f.sp. hordei* Mehltau (Rasse A6) inokuliert und nach weiteren 40 h bezüglich der Infektionsanzeichen ausgewertet. Das Ergebnis (z.B. die Penetrationseffizienz, definiert als prozentualer Anteil angegriffener Zellen mit reifem Haustorium und einer Sekundärhyphe ("secondary elongating hyphae"), wurde mittels Fluoreszens- und Lichtmikroskopie analysiert. Eine Inokulation mit 100 Conidia/$mm^2$ ergibt eine Angriffsfrequenz von ca. 50 % der transformierten Zellen. Für jedes einzelne Experiment wurde eine minimale Anzahl von 100 Interaktionsstellen ausgewertet. Transformierte (GFP exprimierende) Zellen wurden unter Anregung mit blauem Licht identifiziert. Drei verschiedene Katagorien von transformierten Zellen konnten unterschieden werden:

1 Penetrierte Zellen, die ein leicht erkennbares Haustorium beinhalten. Eine Zelle mit mehr als einem Haustorium wurde als eine Zelle gewertet.

2. Zellen, die durch ein Pilz-Appressorium zwar angegriffen wurden, aber kein Haustorium beinhalten. Eine Zelle die mehrfach von Bgh angegriffen wurden, aber kein Haustorium enthält, wurde als eine Zelle gewertet.

3. Zellen die nicht durch Bgh angegriffen sind.

Stomatazellen und Stomatanebenzellen wurden von der Bewertung ausgeschlossen. Oberflächenstrukturen von Bgh wurden mittels Lichtmikroskopie oder Fluoreszenzfärbung des Pilzes mit 0,1 % Calcofluor (w/v in Wasser) für 30 sec analysiert. Die Entwicklung des Pilzes kann leicht durch Fluoreszenzmikroskopie nach Anfärbung mit Calcofluor evaliert werden. In RacB-dsRNA transformierten Zellen entwickelt der Pilz zwar ein primäres und ein appressoriales Keimschlauch ("Germ-Tube") aber kein Haustorium. Haustoriumausbildung ist eine Vorbedingung für die Bildung einer Sekundärhyphae.

Die relative Penetrationseffizien (RPE) errechnet sich als Differens aus der Penetrationseffizien bei transformierten Zellen (Transformation mit RacB- oder Kontrol-dsRNA) und der Penetrationseffizienz bei untransformierten Zellen (hier: durchschnittliche Penetrationseffizienz 57 %). Die prozentuale RPE (%-RPE) errechnet sich aus der RPE minus 1 und multipliziert mit 100.

$$RPE = \frac{[\text{PE bei RacB-dsRNA transformierten Zellen}]}{[\text{PE bei Kontroll-dsRNA transformierten Zellen}]}$$

$$\%\text{-RPE} = 100 * (\text{RPE}-1)$$

Der %-RPE-Wert (Abweichung von der durchschnittlichen Penetrationseffizienz der Kontrolle) dient der Bestimmung des Suszeptibilität von Zellen, die mit RacB-dsRNA transfiziert sind (Fig. 4).

Bei der Kontrol-dsRNA wurde bei fünf unabhängigen Versuchen kein Unterschied zwischen der Transfektion mit der Kontroll dsRNA und Wasser bezüglich der Penetrationseffizienz von *Bgh* beobachtet.

Ferner wurde die Abweichung der PE in verschiedenen Genotypen untersucht. Um die funktionelle Verbindung zum Mlo-Gen zu demonstrieren wurde ein *mlo5*-Genotyp (A89, *mlo5 ror1, Hintergrund:* Ingrid) eingesetzt, der nur moderat anfällig gegen einen *Bgh* Befall aufgrund einer Mutation des *Ror1*-Gen ist ( Freialdenhoven A et al. (1996) Plant Cell 8:5-14). In diesem doppelt-mutantem Genotyp wurde die Effizienz von *RacB*-dsRNA im Vergleich zu einem Wildtyp *Mlo*-Genotyp untersucht. In fünf unabhängigen Experimenten konnte jedoch keine Verhinderung der Haustorium-Ausbildung in A89 beobachtet werden. Wohingegend die PE bei parallelen Experimenten mit Pallas und Ingrid deutlich reduziert war (Fig. 5). Interessanterweise war der Effekt der *RacB*-dsRNA in Pallas ausgeprägter als in Ingrid (Fig. 5, Experiments 1 und 2 im Vergleich zu 3, 4 und 5).

Um einen Einfluss auf der dsRNA auf die Transformationsrate oder Überlebensrate der angegriffenen Zellen auszuschließen, wurde die Anzahl der GFP-exprimierenden Zellen zwischen Kontroll- und *RacB*-dsRNA Experimenten verglichen (Tabelle 7). Die *RacB*-dsRNA hatten keinen Einfluss auf die Gesamtanzahl- oder die Anzahl der angegriffenen GFP-exprimierenden Zellen.

Tabelle 7: Transformationsraten an Gerstenblätterm nach Bombardierung mit dsRNA

| Linie | Anzahl der GFP exprimierenden Zellen pro Schuss[a] | | | | $n^c$ |
|---|---|---|---|---|---|
| | Gesamt (Kontroll-dsRNA) | Angegriffen (Kontroll-dsRNA) | Gesamt (*RacB*-dsRNA) | Angegriffen (*RacB*-dsRNA) | |
| Pallas) (*Mlo Ror1*) | 34.3±4.6 | 16.0+.2.2 | 33.9±4.8 | 15.5±1.4 | 6 (21) |
| Ingrid (*Mlo Ror1*) | 51.0±8.9 | 27.6±8.7 | 49.915.6 | 31.5±7.8 | 3(11) |
| A89 (*mlo5 ror1*) | 34.4±5.4 | 18.1±4.0 | 34.1±5.5 | 16.7±3.8 | 5(22) |

a: 4 Blätter wurden pro Schuss bombardier. Angegeben sind Mittelwerte und Standardfehler
c: Anzahl der unabhängigen Versuche (Schüsse n jeweils für Kontroll- und RacB-dsRNA).

Beispiel 8: Konstitutiv aktive Mutante von RACB

Zur Positiv-Identifizierung von RACB als Suszeptibilitätsfaktor wurde eine putativ konstitutiv aktive Mutante von Gerste RACB erstellt (Austausch G->V an Position 15; RacB-V15) und in Gerste der Sorte Pallas überexprimiert. Zunächst wurde RACB full-lenght über RT-PCR dargestellt. Dazu wurden nachfolgende Oligonukleotid-primer eingesetzt:

RacB5'BamHI : 5'-GGATCCGATGAGCGCGTCCAGGTT-3' (SEQ ID NO: 31)

RacB3'SalI : 5'-GTCGACCTTCGCCCTTGTTCTTTGTC-3' (SEQ ID NO: 32)

Die cDNA wurde in pGEM-T kloniert und anschließend über die Primerschnittstellen ausgeschnitten und in pGY-1 (Schweizer P et al. (1999) Mol Plant Microbe Interact 12: 647-54; Fig. 7) über BamHI / SalI Schnittstellen kloniert. Das Konstrukt trägt die Bezeichnung pGY1-RacB.

Die Nukleinsäuresequenz kodierend für die konstitutiv aktive RacB-Mutante RACB-V15 wurde hergestellt mit dem "Transformer®Site-Directed Mutagenesis Kit" (Clonetech, Heidelberg) nach Anleitung des Herstellers. Als Ausgangsvektor wurde pGY1-RacB eingesetzt. Als Mutagenese-Primer wurde nachfolgendes Oligonukleotid verwendet:

V15 : 5'-ACCGTGGGGGACGTCGCCGTCGGCAAGAC-3' (SEQ ID NO: 33)

RACB-V15 wurde dann in 5 unabhängigen Experimenten transient in Gerste der Sorte Pallas unter Kontrolle des 35S CamV Promotors überexprimiert. Die Experimente wurden wie in Schultheiss et al. (Schultheiss H et al. (2002) Plant Physiol 128:1447-1454) beschrieben durchgeführt, nur wurde nach dem Partikelbeschuss 24 statt 4 h vor der Inokulation gewartet. Das coating der Partikel lief wie in Schweizer et al. (Schweizer P et al. (1999) Mol Plant Microbe Interact 12:647-54) beschrieben.

Die Expression einer konstituiven RacB-Mutante bewirkt eine signifikant erhöhte Anfälligkeit gegen Pathogenbefall mit Gerstenmehltau im Vergleich zu den Kontrollen. Auch diese Ergebisse belegen die Schlüsselfunktion von RacB bei der Pathogenabwehr. Die RACB-V15 Effekte (s.Fig 6-A/B) sind im t-test signifikant, wenn man einen zweiseitigen gepaarten Test macht. Die relative Suszeptibilität gegenüber dem Pilzpathogen ist in allen Fällen erhöht (Fig. 6-B).

Beispiel 9: Weitere HvRac-Homologe

Alle Vollänge-Sequenzen wurden mit spezifischen Primern aus RNA Isoliert und in pGEM-T kloniert und sequenzierT (Hückelhoven et al. (2001) Plant Mol Biol; Schultheiss et al. (2002) Plant Physiol 128:1447-1454). Die Sequenzen sind RacB z.T. sehr ähnlich.

a) HvRop6:

| | | |
|---|---|---|
| LEFT PRIMER | 5'-GTGGAGGCGCGGCGAGA-3' | (SEQ ID NO: 25) |
| RIGHT PRIMER | 5'-CCATGCTTCATCTCCATAGTCA-3' | (SEQ ID NO: 26) |

b) HvRacD:

| | | |
|---|---|---|
| LEFT PRIMER | 5'-ggatccCGATTCCATCAGGAAAGCAT-3' | (SEQ ID NO: 27) |
| RIGHT PRIMER | 5'-gtcgacGCGAGACACTGCAAAACAAA-3' | (SEQ ID NO: 28) |

c) HvRop4:

| | | |
|---|---|---|
| LEFT PRIMER | 5'-GGATCCttctcgtccatttagccggc-3' | (SEQ ID NO: 29) |
| RIGHT PRIMER | 5'-GTCGACtgatcacttgaagcatgccag-3' | (SEQ ID NO: 30) |

Beispiel 10: Herstellung von Sense- und Antisense-Konstrukten mit dem Gen A*tRacB zur* Expression in *Arabidopsis thaliana*

Ein Fragment eines *RacB*-Homologs aus *Arabidopsis* (MIPS-Code: AT4g35950; SEQ ID NO: 53; infolge AtRacB) wird via PCR aus einer Arabidopsis thaliana cDNA-Bibliothek isoliert. Die verwendeten Primersequenzen lauten:

Fra 186: 5'-ATGAGCGCGTCCAGGTTCATA-3' (SEQ ID NO: 71)

Fra 187: 5'-ATCAAACACGCCCTTCACGTT-3' (SEQ ID NO: 72)

Die Amplifikation verläuft in einem Thermocycler T3 der Firma Biometra temperaturgesteuert:

35 Zyklen mit 1 min 95°C, 0,5 min 59°C und 3 min 72°C. Abschließende Extension für 5 min bei 72°c.

Die PCR-Produkte wird in den Vektor pCR2.1 (gemäß *pCR Script Cloning Kit,* Firma Stratagene, Heidelberg) nach Herstellerangaben kloniert. Über das Restrikitionsenzym EcoRI (Firma Roche, Mannheim) wird ein Fragment aus dem Vektorkonstrukt geschnitten. Das Fragment läßt sich über eine Gelelektrophorese mit anschließender Aufreinigung über Anionenaustauschersäulen (QIAex Purification Kit, Fa. Qiagen, Hilden) isolieren. Entsprechend wird der binäre Vektor pSUN3-Nit über die Enzyme XmaI und EcoRI aufgeschnitten und einer Reinigung mittels Gelektrophorese mit anschließender Elution über Anionenaustauschersäulen (QIAex Purification Kit, Fa. Qiagen, Hilden) unterzogen.

Da für die Klonierung Insert und Vektor mit 5'-überhängenden Enden glatte Enden ("blunt ends") generiert werden müssen, wird sowohl das eluierte AtRacB-Fragment als auch das eluierte pSUN3-Nit-Fragment mit 2 µl dNTP-Mix I (je 10 mM dATP, dCTP, dGTP, dTTP; Firma Pharmacia, Freiburg) und 1,6 µl Klenow-Fragment (Fa. USB/ Amersham, Braunschweig, 2 U/µl) zum Auffüllen des Überhanges behandelt und 30 min bei 37°C inkubiert. Um eine Religation zu verhindern, werden die Vektoren zuerst über eine QIAquick Spin Column (Frima Qiagen,Hilden) gereinigt, mit CIAP (Calf Intestinal Alkaline Phosphatase, Fa. GibcoBRL, Eggenstein, 1 U/µl) behandelt und abschließend über ein 0,8%-iges Agarosegel aufgereinigt.

Für den folgenden Ligationsansatz in einem Gesamtvolumen von 50 µl wird zu den 10 µl geschnittener DNA noch 34 µl $H_20$, 5 µl Ligationspuffer und 1 µl T4 Ligase (Firma Roche, Mannheim) gegeben.

Dieser Ansatz wird über Nacht bei 16°C inkubiert. Anschließend wird die Ligase bei 65°C für 10 min inaktiviert. An die Ligation schließt sich wieder eine Fällung mit 0,1 Volumen Natriumacetat (pH5,2) und 2,5 Volumen Ethanol an. Nach Zentrifugation (30 min, 15000 g, 4°C) wird das Pellet in 70% Ethanol getrocknet und in 10 µl $H_20$ resuspendiert. 2 µl dieses Pellets werden durch Elektroporation (*E. coli*-Pulser, Firma Bio-Rad) in *Escherichia coli*-Bakterien Stamm DH5α transformiert. Die mit der DNA behandelten Bakterien werden auf LB-Platten plattiert, die das Antibiotikum Ampicillin (50 mg/l) enthalten. Nach einer Inkubation über 16 h bei 37°C werden von den gewachsenen Kolonien Bakterien abgestrichen und in jeweils Reagenzgläser mit 3 ml LB-Amp-Flüssigmedium überführt. Nach einer Inkubation von 16 h bei 37°C werden die dicht gewachsenen Kulturen zentrifugiert. Aus den Bakterienpellets wird mittels QIAprep DNA-Minipräparationskit (Firma Qiagen, Hilden) nach Herstellerangaben Plasmid-DNA isoliert und analytischen Verdaus mit verschiedenen Enzymkombinationen unterzogen. Durch diese Kontrollverdaus lassen sich Kontrukte isolieren, bei denen das Gen AtRacB in sense-Orientierung bzw. antisense-Orientierung hinter den in Pflanzen konstitutiv aktiven des Nitrilase-1 (nit1) Gens aus A. thaliana (GenBank Acc.-No.: Y07648.2, Nukleotide 2456-4340, Hillebrand et al. (1996) Gene 170:197-200) kloniert vorliegt. Diese Konstrukte werden mit pSUN3NIT_AtRacB_s (SEQ ID NO: 73) und pSUN3NIT_ atRacB_as (SEQ ID NO: 74) bezeichnet und für die Transformation von *Arabidopsis*-Pflanzen verwendet. Die Konstrukte beinhalten die vollständige Sequenz von AtRacB, so dass der Expressionsvektor pSUN3NIT_HvRacB_s, der das Fragment in sense-Orientierung enthält, befähigt ist ein funktionelles AtRacB-Protein zu exprimieren. Der Vektor dient primär als Negativkontrolle und ergibt in den meisten Fällen eine verminderte Pathogenresistenz, in einigen Fällen (s.u.) jedoch auch eine Erhöhung der Pathogenresistenz vermutlich über einen Cosuppressionseffekt.

Beispiel 11: Herstellung von Sense- und Antisense-Konstrukten mit dem Gen *HvRacB* zur Expression in *Arabidopsis thaliana*

Von dem Gen *HvRacB* sollen verschiedene nicht-funktionelle Fragmente zur Expression in Arabidopsis-Pflanzen hergestellt werden. Dazu wird das Plasmid, welches das *HvRacB*-Gen subkloniert in den bakteriellen Vektor pGEM-T enthielt mit den Enzymkombinationen BamHI / HindIII (Firma Roche, Mannheim) verdaut. Durch eine Behandlung mit der Klenow-Polymerase in Anwesenheit eines Gemischs von Nukleotiden (s.o.) werden überhängende 5'-Einzelstränge aufgefüllt. Das entstehende *HvRacB*-Fragment mit den auf diese Weise geglätteten Enden wird direkt in einen pSUN3NIT-Vektor kloniert, der in seiner Multiple Cloning Site mit den Enzymen BglII und SpeI (Firma Roche, Mannheim) geöffnet wird und dessen 5'-überhängende Enden mittels Behandlung mit Klenow-Polymerase (wie oben beschrieben) aufgefüllt werden. Für die Ligationsansätze jeweils in einem Gesamtvolumen von 50 µl werden zu den 10 µl geschnittener DNA noch 34 µl $H_20$, 5 µl Ligationspuffer und 1 µl T4 Ligase (Firma Roche, Mannheim) gegeben. Dieser Ansatz wird über Nacht bei 16°C inkubiert. Anschließend wird die Ligase bei 65°C für 10 min inaktiviert. An die Ligation schließt sich wieder eine Fällung mit 0,1 Volumen Natriumacetat (pH5,2) und 2,5 Volumen Ethanol an. Nach Zentrifugation (30 min, 15000 g, 4°C) wird das Pellet in 70% Ethanol getrocknet und in 10 µl $H_20$ resuspendiert. 2 µl dieses Pellets werden durch Elektroporation (*E. coli*-Pulser, Firma Bio-Rad) in *Escherichia coli*-Bakterien Stamm DH5α transformiert. Die mit der DNA behandelten Bakterien werden auf LB-Platten plattiert, die das Antibiotikum Ampicillin (50 mg/l) enthalten. Nach einer Inkubation über 16 h bei 37°C werden von den gewachsenen Kolonien Bakterien abgestrichen und in jeweils Reagenzgläser mit 3 ml LB-Amp-Flüssigmedium überführt. Nach einer Inkubation von 16 h bei 37°C werden die dicht gewachsenen Kulturen zentrifugiert. Aus den Bakterienpellets wird mittels QIAprep DNA-Minipräparationskit (Firma Qiagen, Hilden) nach Herstellerangaben Plasmid-DNA isoliert und analytischen Verdaus mit verschiedenen Enzymkombinationen unterzogen. Durch diese Kontrollverdaus lassen sich Konstrukte identifizieren, bei denen das entsprechende Genkonstrukt in sense bzw. in antisense-Orientierung hinter den in Pflanzen konstitutiv aktiven Promotor des Nitrilase-1 Gens aus A.thalina (s.o.) kloniert vorliegt. Diese Konstrukte werden mit pSUN3NIT_HvRacB_s (SEQ ID NO: 75) und pSUN3NIT_HvRacB_as (SEQ ID NO: 76) bezeichnet und für die Transformation von *Arabidopsis*-Pflanzen verwendet. Die Konstrukte beinhalten ein verkürztes Fragment von hvRacB, so dass auch der Expressionsvektor pSUN3NIT_HvRacB_s, der das Fragment in sense-Orientierung enthält, nicht befähigt ist ein funktionelles HvRacB-Protein zuexprimieren. Der Vektor dient primär als NegativKontrolle, ergibt aber auch in einigen Fällen (s.u.) eine Erhöhung der Pathogenresistenz vermutlich über einen Cosuppressionseffekt.

Beispiel 12: Transformation von Arabidopsis thaliana und Analyse der Pilzresistenz

Wildtyp *A.thaliana* Pflanzen (Columbia) werden mit dem *Agrabacterium tumefaciens* Stamm (EHA105) auf Grundlage einer modifizierten Methode (Steve Clough und Andrew Bent (1998) Plant J 16(6):735-743) der Vacuum Infiltrationsmethode nach Bechtold et al.(Bechtold N et al. (1993) CR Acad Sci Paris, Life Sciences 316:1194-1199).

Die verwendeten *A.tumefaciens* Zellen werden im Vorfeld mit den Plasmiden pSUN3NIT_AtRacB_s (SEQ ID NO: 73), pSUN3NIT_atRacB as (SEQ ID NO: 74), pSUN3NIT_HvRacB_s (SEQ ID NO: 75) und pSUN3NIT_HvRacB_ as (SEQ ID NO: 76) transformiert.

Samen der Agrobacterium-transformierten Primärtransformanden werden auf Grundlage der Kanamycin-Resistenz selektioniert. Antibiotika resistente Keimlinge werden in Erde gepflanzt und als vollentwickelte Pflanzen zur biochemischen Analyse verwendet.

Zur Analyse der Resistenz der transgene *Arabidopsis*-Pflanzen gegenüber pathogene Pilzen werden Inokulationen mit den biotrophen Pilzen *Peronospora parasitica* und *Erysiphe cichoracearum* vorgenommen.

a) Peronospora parasitica

5 bis 8 Wochen alte Pflanzen werden mit einer Konidiensporensuspension (ca. $10^6$ Sporen / ml) besprüht. Die inokulierten Pflanzen werden über Nacht in einem Kühlschrank bei ca. 16°C mit einer Plastiktüte überdeckt dunkel und feucht gehalten. Nach einem Tag wird die Plastiktüte etwas geöffnet und später vollständig entfernt. Sechs Tage nach Inokulation werden die Pflanzen wieder über Nacht mit der Plastiktüte zugedeckt, wodurch die Sporulation induziert wird. Am folgenden Tag werden die Blätter auf das Auftreten von Konidiophoren untersucht. Das interzelluläre Wachstum des Pilzes führt in den nächsten Tagen zur Induktion von schwachen Chlorosen bis hin zu starken Nekrosen in den Blättern. Diese Symptome werden quantifiziert und auf Signifikanz getestet.

b) Erysiphe cichoracearum

Der biotrophe Mehltau-Pilz wird auf *Arabidopsis*-Pflanzen kultiviert. Zur Infektion der 4 Wochen alten transgenen RacB-exprimierenden *Arabidopsis*-Pflanzen werden mit einem feinen Pinsel Konidienträger auf der Oberfläche der Blätter abgenommen und auf die Blätter der transgene Pflanzen gestrichen. Die Pflanzen werden für 7 Tage bei 20°C inkubiert. 7 Tage nach Inokulation werden die Konidienträger auf den Blättern sichtbar, und es treten in den folgenden Tagen Chlorosen und Nekrosen zutage. Diese Symptome werden quantifiziert und auf Signifikanz getestet.

c) Ergebnisse

Die transgenen Arabidopsis Pflanzen die antisense-Sequenzen für AtRacB oder HvRacB expimieren, zeigen in zeigen sowohl gegen Peronospora parasitica als auch gegen Erysiphe cichoracearum eine signifikant erhöhte Resistenz im Vergleich zu nicht-transgenen Wildtyp-Pflanzen.

Die transgenen Arabidopsis Pflanzen die sense-Sequenzen für das vollständige AtRacB expimieren, zeigen in zeigen in den meisten Fällen sowohl gegen Peronospora parasitica als auch gegen Erysiphe cichoracearum eine signifikant erhöhte Anfälligkeit im Vergleich zu nicht-transgenen Wildtyp-Pflanzen. In einigen Fällen kann jedoch auch eine erhöhte Resistenz (vermutlich über einen Cosuppressionseffekt) beobachtet werden.

Die transgenen Arabidopsis Pflanzen die sense-Sequenzen für das ein Fragment von HvRacB expimieren, zeigen in einigen Fällen sowohl gegen Peronospora parasitica als auch gegen Erysiphe cichoracearum eine signifikant erhöhte Resistenz im Vergleich zu nicht-transgenen Wildtyp-Pflanzen.

## Patentansprüche

1. Verfahren zum Erzielen oder Erhöhen der Resistenz gegen mindestens ein Pathogen in Pflanzen, **dadurch gekennzeichnet, dass** nachfolgende Arbeitsschritte umfasst sind

   a) Verminderung der Proteinmenge, Aktivität oder Funktion eines RacB-Proteins in einer Pflanze oder einem Gewebe, Organ, Teil oder Zelle derselben und
   b) Auswahl der Pflanzen, bei denen - im Unterschied oder Vergleich zur Ausgangspflanze - die Resistenz gegen mindestens ein Pathogen besteht oder erhöht ist.

2. Verfahren nach Anspruch 1, **dadurch gekennzeichnet, dass** das RacB Protein ausgewählt ist aus der Gruppe der Proteine bestehend aus

   a) einem Polypeptid gemäß SEQ ID NO: 2, 4 oder 6, und
   b) einem funktionellen Äquivalent eines Polypeptides gemäß SEQ ID NO: 2, 4 oder 6.

3. Verfahren nach Anspruch 2, **dadurch gekennzeichnet, dass** das funktionelle Äquivalent eine Homologie von mindestens 64 % zu einem der Polypeptide gemäss SEQ ID NO: 2, 4 oder 6 hat.

4. Verfahren nach Anspruch 2oder 3, wobei das funktionelle Äquivalent eine Homologie von mindestens 90 % zu einem der Polypeptide gemäss SEQ ID NO: 2, 4 oder 6 hat.

5. Verfahren nach einem der Ansprüche 1 bis 4, **dadurch gekennzeichnet, dass** die Verminderung des RacB-Proteins gewährleistet wird durch Anwendung eines Verfahrens ausgewählt aus der Gruppe bestehend aus

   a) Einbringen einer doppelsträngigen RacB RNA-Nukleinsäuresequenz (RacB-dsRNA) oder einer deren Expression gewährleistenden Expressionskassette oder Expressionskassetten,
   b) Einbringen einer RacB antisense-Nukleinsäuresequenz oder einer deren Expression gewährleistenden Expressionskassette,
   c) Einbringen einer RacB antisense-Nukleinsäuresequenz kombiniert mit einem Ribozym oder einer deren Expression gewährleistenden Expressionskassette,
   d) Einbringen von RacB sense-Nukleinsäuresequenzen zur Induktion einer Kosuppression oder einer deren Expression gewährleistenden Expressionskassette,
   e) Einbringen einer Nukleinsäuresequenz kodierend für dominant-negatives RacB Protein oder einer deren Expression gewährleistenden Expressionskassette,
   f) Einbringen DNA-oder Protein-bindende Faktoren gegen RacB -Gene, -RNAs oder -Proteine oder einer deren Expression gewährleistenden Expressionskassette,
   g) Einbringen von den RacB RNA-Abbau bewirkende virale Nukleinsäuresequenzen und Expressionskonstrukten oder einer deren Expression gewährleistenden Expressionskassette,
   h) Einbringen von Konstrukten zur Induktion einer homologen Rekombination an endogenen RacB-Genen und
   i) Einführung von Mutationen in ein endogenes RacB Gen.

6. Verfahren nach einem der Ansprüche 1 bis 5, umfassend

(a) die stabile Transformation einer pflanzlichen Zelle mit einer rekombinanten Expressionskassette enthaltend in funktioneller Verknüpfung mit einem in Pflanzen aktiven Promotor eine Nukleinsäuresequenz kodierend für

a) eine doppelsträngigen RacB RNA-Nukleinsäuresequenz oder
b) eine RacB antisense-Nukleinsäuresequenz oder
c) eine RacB antisense-Nukleinsäuresequenz kombiniert mit einem Ribozym oder
d) eine RacB sense-Nukleinsäuresequenzen zur Induktion einer Kosuppression oder
e) ein dominant-negatives RacB Protein
f) DNA-oder Protein-bindende Faktoren gegen RacB-Gene, -RNAs oder -Proteine
g) den RacB RNA-Abbau bewirkende virale Nukleinsäuresequenzen

(b) Regeneration der Pflanze aus der pflanzlichen Zelle, und
(c) Expression besagter Nukleinsäuresequenz in einer Menge und für eine Zeit hinreichend um eine Pathogenresistenz in besagter Pflanze zu erzeugen oder zu erhöhen.

**7.** Verfahren nach einem der Ansprüche 1 bis 6, **dadurch gekennzeichnet, dass** das Pathogen ausgewählt aus der Gruppe bestehend aus Bakterien, Pilzen, Insekten, Viren und Nematoden.

**8.** Verfahren nach einem der Ansprüche 1 bis 7, **dadurch gekennzeichnet, dass** das Pathogen ausgewählt ist aus der Gruppe der Pilze bestehend aus Plasmodiophoramycota, Oomycota, Ascomycota, Chytridiomyceten, Zygomyceten, Basidiomycota und Deuteromyceten.

**9.** Verfahren nach einem der Ansprüche 1 bis 8, **dadurch gekennzeichnet, dass** die Pflanze aus den monokotyledonen und dikotyledonen Pflanzen ausgewählt ist.

**10.** Verfahren nach einem der Ansprüche 1 bis 9, **dadurch gekennzeichnet, dass** die Pflanze ausgewählt ist aus der Gruppe bestehend aus Weizen, Hafer, Hirse, Gerste, Roggen, Mais, Reis, Buchweizen, Sorghum, Triticale, Dinkel, Leinsamen oder Zuckerrohr.

**11.** Verfahren nach einem der Ansprüche 1 bis 10, **dadurch gekennzeichnet, dass** dasRacB-Protein aus Gerste eines gemäß SEQ ID NO: 2 ist.

**12.** Verfahren nach einem der Ansprüche 4 bis 10, **dadurch gekennzeichnet, dass** die dominant negative Variante nach Anspruch 6 beschrieben ist durch SEQ ID NO: 7.

**13.** Transgener pflanzlicher Organismus oder eine Zelle, Zellkultur, ein Gewebe, ein Teil oder Vermehrungsgut von dieser, erhalten gemäß eines Verfahrens nach den Ansprüchen 1 bis 12.

**14.** Transgener pflanzlicher Organismus nach Anspruch 13, wobei die Pflanze ausgewählt ist aus der Gruppe der Pflanzen bestehend aus Weizen, Hafer, Hirse, Gerste, Roggen, Mais, Reis, Buchweizen, Sorghum, Triticale, Dinkel, Leinsamen, Zuckerrohr, Raps, Canola, Kresse, Arabidopsis, Kohlarten, Soja, Alfalfa, Erbse, Bohnengewächsen, Erdnuss, Kartoffel, Tabak, Tomate, Aubergine, Paprika, Sonnenblume, Tagetes, Salat, Calendula, Melone, Kürbis und Zucchini.

## Claims

**1.** A method of generating or increasing the resistance to at least one pathogen in plants, which comprises the following steps

a) reducing the amount, activity or function of an RacB protein in a plant or a tissue, organ, part or cell thereof, and
b) selecting those plants in which, as opposed or as compared to the original plant, the resistance to at least one pathogen exists or is increased.

**2.** The method according to claim 1, wherein the RacB protein is selected from the group of the proteins consisting of

a) a polypeptide as shown in SEQ ID NO: 2, 4 or 6, and
b) a functional equivalent of a polypeptide as shown in SEQ ID NO: 2, 4 or 6.

**3.** The method according to claim 2, wherein the functional equivalent has at least 64% homology with one of the polypeptides as shown in SEQ ID NO: 2, 4 or 6.

**4.** The method according to claim 2 or 3, wherein the functional equivalent has at least 90% homology with one of the polypeptides as shown in SEQ ID NO: 2, 4 or 6.

**5.** The method according to any of claims 1 to 4, wherein the reduction of the RacB protein is ensured by using a method selected from the group consisting of

a) introduction of a double-stranded RacB RNA nucleic acid sequence (RacB dsRNA) or an expression cassette (s) ensuring the expression thereof,
b) introduction of an RacB antisense nucleic acid sequence or an expression cassette ensuring expression thereof,
c) introduction of an RacB antisense nucleic acid sequence in combination with a ribozyme or an expression cassette ensuring expression thereof,
d) introduction of RacB sense nucleic acid sequences for inducing cosuppression or an expression cassette ensuring expression thereof,
e) introduction of a nucleic acid sequence encoding dominant-negative RacB protein or an expression cassette ensuring expression thereof,
f) introduction of DNA- or protein-binding factors against RacB genes, RacB RNAs or RacB proteins or an expression cassette ensuring expression thereof,
g) introduction of viral nucleic acid sequences and expression constructs causing RacB RNA degradation or an expression cassette ensuring expression thereof,
h) introduction of constructs for inducing a homologous recombination on endogenous RacB genes, and
i) introduction of mutations into an endogenous RacB gene.

**6.** The method according to any of claims 1 to 5, comprising

(a) the stable transformation of a plant cell with a recombinant expression cassette comprising a nucleic acid sequence encoding

a) a double-stranded RacB RNA nucleic acid sequence or
b) an RacB antisense nucleic acid sequence or
c) an RacB antisense nucleic acid sequence combined with a ribozyme or
d) an RacB sense nucleic acid sequence for inducing cosuppression or
e) a dominant-negative RacB protein or
f) DNA- or protein-binding factors against RacB genes, RacB RNAs or RacB proteins
g) viral nucleic acid sequences causing RacB RNA degradation

in operable linkage with a promoter which is active in plants,
(b) regeneration of the plant from the plant cell, and
(c) expression of said nucleic acid sequence in an amount and over a period of time sufficient to generate or increase a pathogen resistance in said plant.

**7.** The method according to any of claims 1 to 6, wherein the pathogen is selected from the group consisting of bacteria, fungi, insects, viruses and nematodes.

**8.** The method according to any of claims 1 to 7, wherein the pathogen is selected from the group of fungi consisting of Plasmodiophoramycota, Oomycota, Ascomycota, Chytridiomycetes, Zygomycetes, Basidiomycota and Deuteromycetes.

**9.** The method according to any of claims 1 to 8, wherein the plant is selected from the monocots and dicots.

**10.** The method according to any of claims 1 to 9, wherein the plant is selected from the group consisting of wheat, oats, millet, barley, rye, maize, rice, buckwheat, sorghum, triticale, spelt, linseed or sugar cane.

**11.** The method according to any of claims 1 to 10, wherein the barley RacB protein is one as shown in SEQ ID NO: 2.

**12.** The method according to any of claims 4 to 10, wherein the dominant-negative variant according to claim 6 is described by SEQ ID NO: 7.

**13.** A transgenic plant organism or a cell, cell culture, a tissue, a part of propagation material thereof, obtained by a method according to claims 1 to 12.

**14.** The transgenic plant organism according to claim 13, the plant being selected from the group of plants consisting of wheat, oats, millet, barley, rye, maize, rice, buckwheat, sorghum, triticale, spelt, linseed, sugar cane, oil seed rape, canola, cress, Arabidopsis, cabbages, soya, alfalfa, pea, beans, peanut, potato, tobacco, tomato, eggplant, bell pepper, sunflower, Tagetes, lettuce, Calendula, melon, pumpkin/squash and zucchini.

**Revendications**

**1.** Procédé pour l'obtention ou l'augmentation de la résistance à au moins un agent pathogène chez des plantes, **caractérisé en ce qu'**il comprend les étapes d'opérations suivantes

a) diminution de la quantité de protéine, de l'activité ou de la fonction d'une protéine RacB dans une plante ou un tissu, un organe, une partie ou une cellule de celle-ci et
b) choix des plantes dans lesquelles - à la différence ou en comparaison de la plante de départ - la résistance à au moins un agent pathogène existe ou est augmentée.

**2.** Procédé selon la revendication 1, **caractérisé en ce que** la protéine RacB est choisie dans le groupe des protéines constitué par

a) un polypeptide selon SEQ ID n° 2, 4 ou 6, et
b) un équivalent fonctionnel d'un polypeptide selon SEQ ID n° 2, 4 ou 6.

**3.** Procédé selon la revendication 2, **caractérisé en ce que** l'équivalent fonctionnel a une homologie d'au moins 64 % avec un polypeptide selon SEQ ID n° 2, 4 ou 6.

**4.** Procédé selon la revendication 2 ou 3, **caractérisé en ce que** l'équivalent fonctionnel a une homologie d'au moins 90 % avec un polypeptide selon SEQ ID n° 2, 4 ou 6.

**5.** Procédé selon l'une quelconque des revendications 1 à 4, **caractérisé en ce que** la diminution de la protéine RacB est assurée par application d'un procédé choisi dans l'ensemble consistant en

a) l'introduction d'une séquence d'acide nucléique ARN double brin de RacB (ARNdb-RacB) ou d'une cassette d'expression ou de cassettes d'expression assurant son expression,
b) l'introduction d'une séquence d'acide nucléique antisens de RacB ou d'une cassette d'expression assurant son expression,
c) l'introduction d'une séquence d'acide nucléique antisens de RacB associée à un ribozyme ou à une cassette d'expression assurant son expression,
d) l'introduction d'une séquence d'acide nucléique sens de RacB pour l'induction d'une expression, ou d'une cassette d'expression assurant son expression,
e) l'introduction d'une séquence d'acide nucléique codant pour une protéine RacB négative-dominante ou d'une cassette d'expression assurant son expression,
f) l'introduction de facteurs se liant à l'ADN ou à une protéine, contre des gènes, des ARN ou des protéines RacB, ou d'une cassette d'expression assurant leur expression,
g) l'introduction de séquences d'acide nucléique viral et de produits de construction d'expression, provoquant la dégradation d'ARN de RacB, ou d'une cassette d'expression assurant leur expression,
h) l'introduction de produits de construction pour l'induction d'une recombinaison homologue avec des gènes de RacB endogènes et
i) l'introduction de mutations dans un gène de RacB endogène.

**6.** Procédé selon l'une quelconque des revendications 1 à 5, comprenant

(a) la transformation stable d'une cellule végétale par une cassette d'expression recombinante contenant, en liaison fonctionnelle avec un promoteur actif chez des plantes, une séquence d'acide nucléique codant pour

a) une séquence d'acide nucléique ARN double brin de RacB ou
b) une séquence d'acide nucléique antisens de RacB ou
c) une séquence d'acide nucléique antisens de RacB associée à un ribozyme ou
d) une séquence d'acide nucléique sens de RacB pour l'induction d'une cosuppression ou
e) une protéine RacB négative-dominante
f) des facteurs se liant à l'ADN ou à une protéine, contre des gènes, des ARN ou des protéines RacB
g) des séquences d'acide nucléique viral provoquant la dégradation d'ARN de RacB

(b) la régénération de la plante à partir de la cellule végétale, et
(c) l'expression de ladite séquence d'acide nucléique en une quantité suffisante et pendant un temps suffisant pour engendrer ou pour augmenter une résistance à un agent pathogène chez ladite plante.

**7.** Procédé selon l'une quelconque des revendications 1 à 6, **caractérisé en ce que** l'agent pathogène est choisi dans le groupe constitué par les bactéries, les champignons, les insectes, les virus et les nématodes.

**8.** Procédé selon l'une quelconque des revendications 1 à 7, **caractérisé en ce que** l'agent pathogène est choisi dans le groupe des champignons constitué par les *Plasmodiophoramycota,* les *Oomycota,* les *Ascomycota,* les chytridiomycètes, les zygomycètes, les basidiomycètes et les deutéromycètes.

**9.** Procédé selon l'une quelconque des revendications 1 à 8, **caractérisé en ce que** la plante est choisie parmi les plantes monocotylédones et les plantes dicotylédones.

**10.** Procédé selon l'une quelconque des revendications 1 à 9, **caractérisé en ce que** la plante est choisie dans le groupe constitué par le blé, l'avoine, le millet, l'orge, le seigle, le maïs, le riz, le sarrasin, le sorgho, le triticale, l'épeautre, le lin et la canne à sucre.

**11.** Procédé selon l'une quelconque des revendications 1 à 10, **caractérisé en ce que** la protéine RacB provenant de l'orge est une protéine selon SEQ ID n° 2.

**12.** Procédé selon l'une quelconque des revendications 4 à 10, **caractérisé en ce que** la variante négative dominante selon la revendication 6 est décrite par SEQ ID n° 7.

**13.** Organisme végétal transgénique ou une cellule, une culture de cellules, un tissu, une partie ou un matériel de multiplication de celui-ci, obtenu conformément à un procédé selon les revendications 1 à 12.

**14.** Organisme végétal transgénique selon la revendication 13, dans lequel la plante est choisie dans le groupe des plantes constitué par le blé, l'avoine, le millet, l'orge, le seigle, le maïs, le riz, le sarrasin, le sorgho, le triticale, l'épeautre, le lin, la canne à sucre, le colza, le canola (colza à faible teneur en acide érucique), le cresson, l'arabidopsis, les variétés de chou, le soja, la luzerne, le pois, les légumineuses, l'arachide, la pomme de terre, le tabac, la tomate, l'aubergine, le poivron, le tournesol, tagetes, la salade, calendula, le melon, la courge et la courgette.

# FIG.1

```
RACB H.vul._     MSASRFIKCVTVGDGAVGKTCMLISYTSNTFPTDYVPTVFDNFSANVVVDGNTVNLGLWD 60
RACB_Z.mays_     MSASRFIKCVTVGDGAVGKTCMLISYTSNTFPTDYVPTVFDNFSANVVVDGNTVNLGLWD 60
RACB_O.sat.      MSASRFIKCVTVGDGAVGKTCMLISYTSNTFPTDYVPTVFDNFSANVVVDGNTVNLGLWD 60
RAC2_H.sap.      MQA---IKCVVVGDGAVGKTC LLISYTTNAFPGEYI PTVFDNYSANVMVDSKPVNLGLWD 57
RAC1_H.sap.      MQA---IKCVVVGDGAVGKTC LLISYTTNAFPGEYI PTVFDNYSANVMVDGKPVNLGLWD 57
                 *  *    **** ** ****** ***  ***** *  **   * ****** *** * **    ****** *

RACB_H.vul._     TAGQEDYNRLRPLSYRGADVFLLAFSL ISKASYENVSKKW IPELKHYAPGVP I I LVGTKL 120
RACB_Z.mays_     TAGQEDYNRLRPLSYRGADVFLLAFSL ISKASYENVSKKW IPELKHYAPGVP I I LVGTKL 120
RACB_O.sat.      TAGQEDYNRLRPLSYRGADVFLLAFSL ISKASYENVSKKW IPELKHYAPGVP I I LVGTKL 120
RAC2_H.sap.      TAGQEDYDRLRPLSYPQTDVFL ICFSLVSPASYENVRAKWFPEVRHHCPSTPI I LVGTKL 117
RAC1_H.sap.      TAGQEDYDRLRPLSYPQTDVFL ICFSLVSPASFENVRAKWYPEVRHHCPNTPI I LVGTKL 117
                 **** *** *******    ****   *** * ** ***   * * **   *   *  *********

RACB H.vul._     DLRDDKQFFVDHPG--AVP ITTAQGEELKKL IGAPYY IECSSKTQLNVKGVFDAA IKVVL 178
RACB_Z.mays_     DLRDDKQFFVDHPG--AVP ITTAQGEELRKQ IGAPYYIECSSKTQLNVKGVFDAA IKVVL 178
RACB_O.sat.      DLRDDKQFFVDHPG--AVP ITTAQGEELRKQ IGAPYYIECSSKTQLNVKGVFDAA IKVVL 178
RAC2_H.sap.      DLRDDKDT IEKLKEKKLAP ITYPQGLALAKE IDSVKYLECSALTQRGLKTVFDEA IRAVL  177
RAC1_H.saP.      DLRDDKDT IEKLKEKKLTP ITYPQGLAMAKE IGAVKYLECSALTQRGLKTVFDEA IRAVL  177
                 *****                ***  **    *  *    * ***  **   * *** **  **

RACB_H.vul.      QPPKAKKKKKAQRGACS IL 197
RACB_Z.mays_     QPPKAKKKKKVQRGACS IL 197
RACB_O.sat.      QPPKAKKKKKAQRGACS IL 197
RAC2_H.sap.      CPQPTRQQKR----ACSLL 192
RAC1_H.sap.      CPPPVKKRKR----KCLLL 192
                  *      *      *  *
```

FIG.2

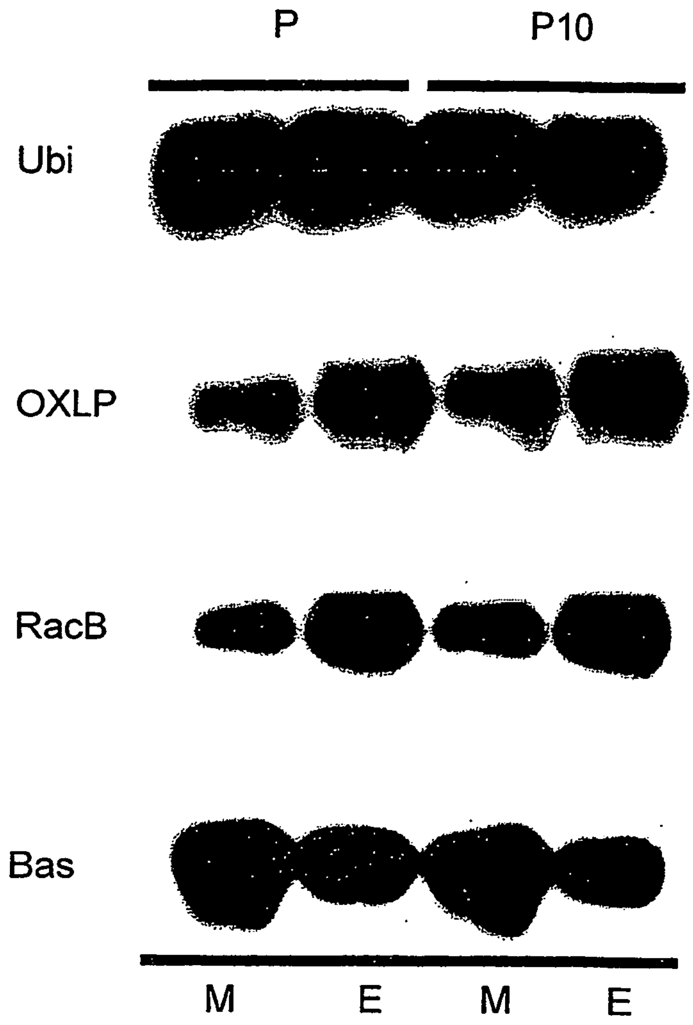

# FIG.3

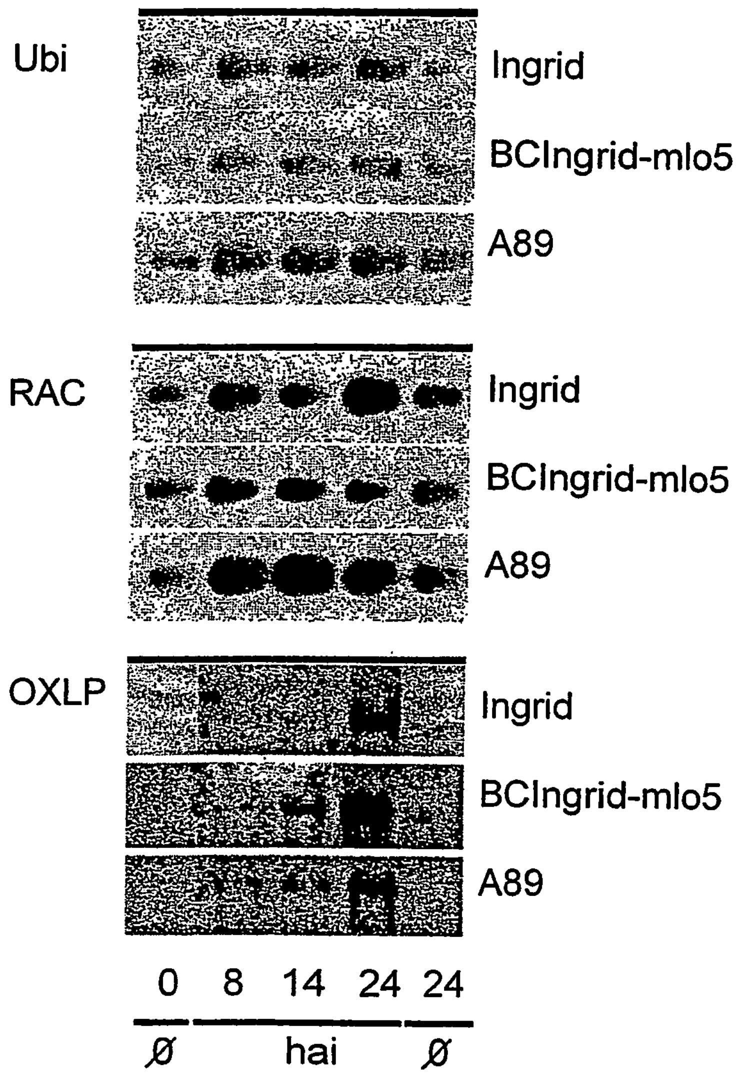
Ubi
Ingrid
BCIngrid-mlo5
A89
RAC
Ingrid
BCIngrid-mlo5
A89
OXLP
Ingrid
BCIngrid-mlo5
A89
0 8 14 24 24
Ø hai Ø

# FIG.4

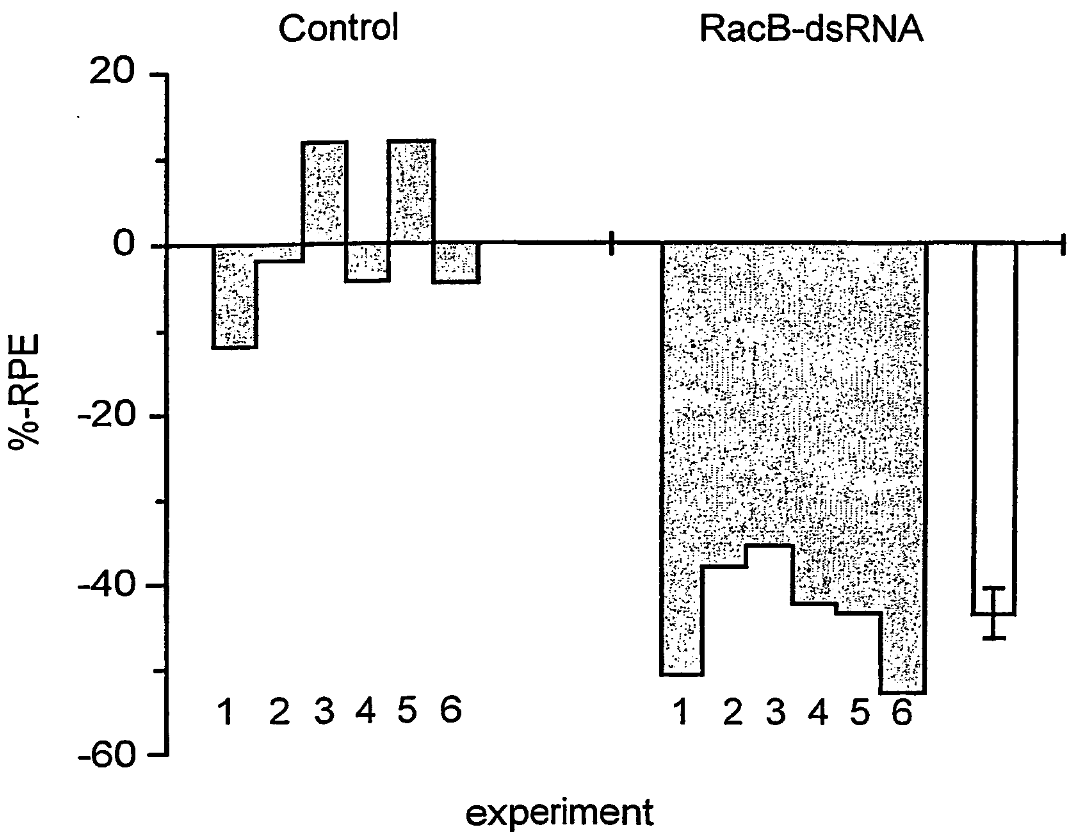
Control
RacB-dsRNA
20
0
-20
-40
-60
%-RPE
1 2 3 4 5 6
1 2 3 4 5 6
experiment

FIG.5

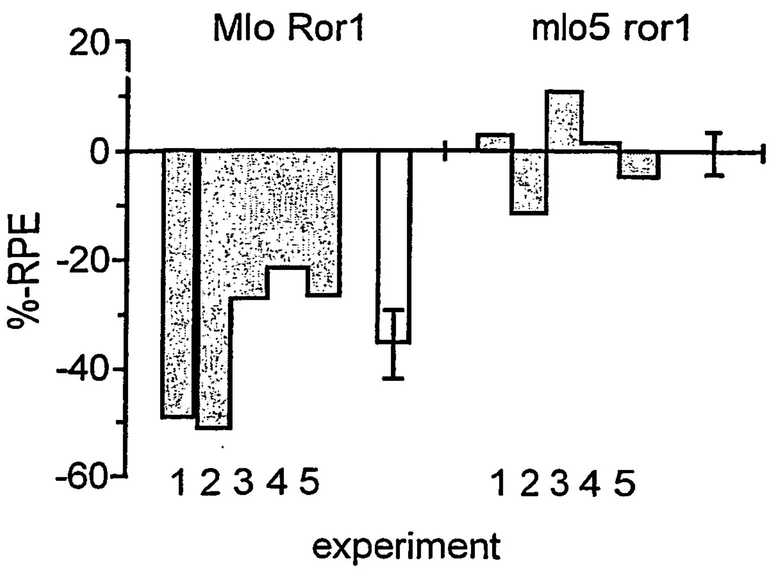

# FIG.6

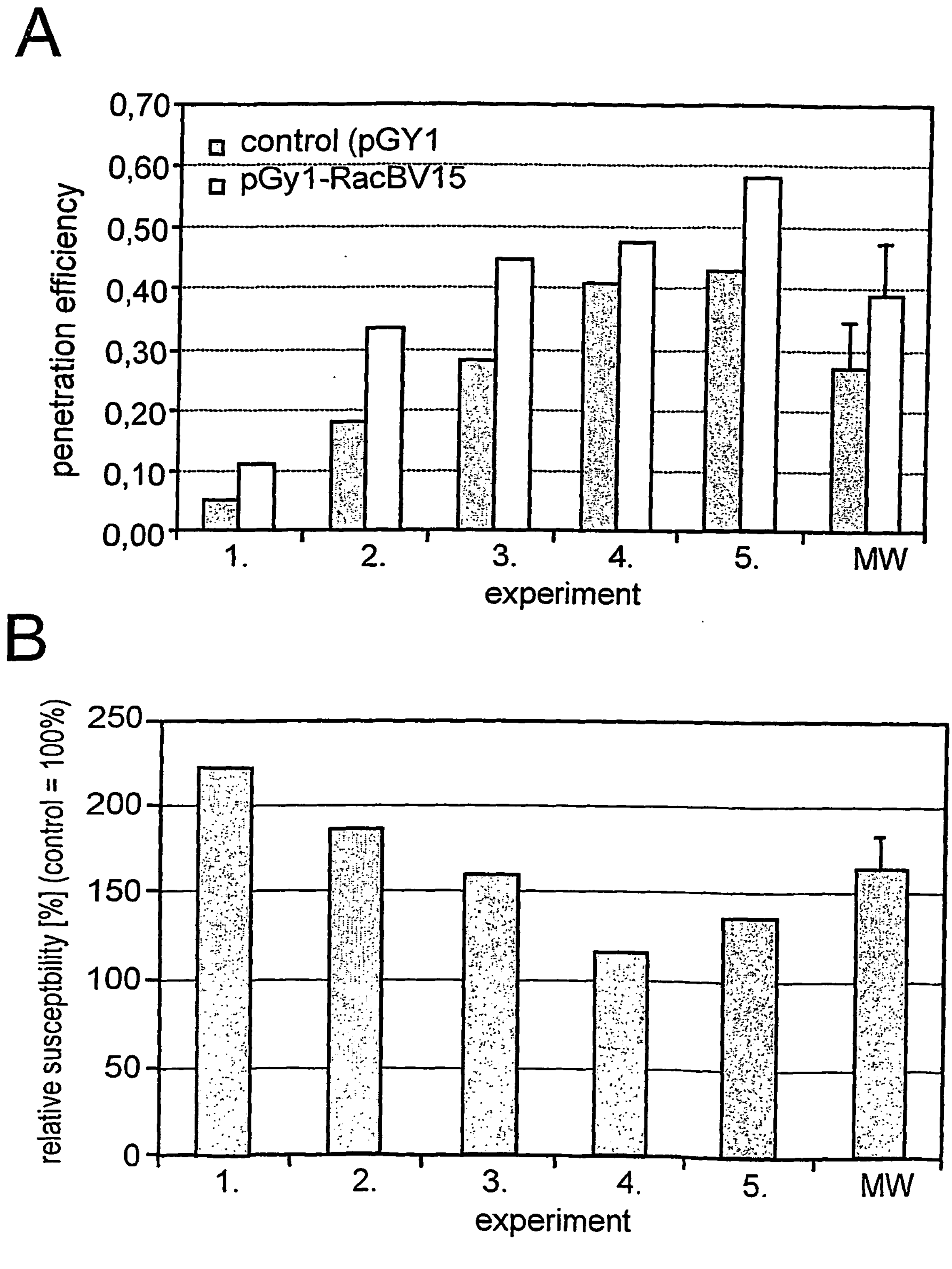
A
0,70
0,60
0,50
0,40
0,30
0,20
0,10
0,00
control (pGY1
pGy1-RacBV15
penetration efficiency
1.
2.
3.
4.
5.
MW
experiment
B
250
200
150
100
50
0
relative susceptbility [%] (control = 100%)
1.
2.
3.
4.
5.
MW
experiment

FIG.7

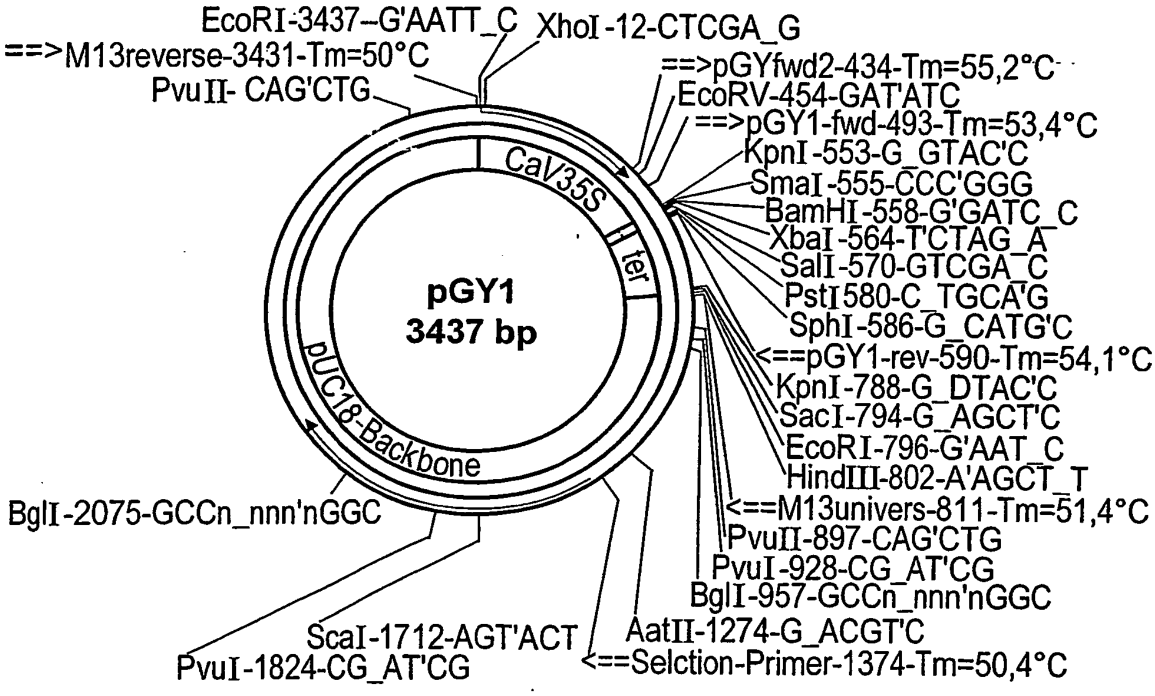
EcoRI-3437--G'AATT_C
XhoI-12-CTCGA_G
==>M13reverse-3431-Tm=50°C
PvuII- CAG'CTG
==>pGYfwd2-434-Tm=55,2°C
EcoRV-454-GAT'ATC
==>pGY1-fwd-493-Tm=53,4°C
KpnI-553-G_GTAC'C
SmaI-555-CCC'GGG
BamHI-558-G'GATC_C
XbaI-564-T'CTAG_A
SalI-570-GTCGA_C
PstI580-C_TGCA'G
SphI-586-G_CATG'C
<==pGY1-rev-590-Tm=54,1°C
KpnI-788-G_DTAC'C
SacI-794-G_AGCT'C
EcoRI-796-G'AAT_C
HindIII-802-A'AGCT_T
<==M13univers-811-Tm=51,4°C
PvuII-897-CAG'CTG
PvuI-928-CG_AT'CG
BglI-957-GCCn_nnn'nGGC
AatII-1274-G_ACGT'C
<==Selction-Primer-1374-Tm=50,4°C
ScaI-1712-AGT'ACT
PvuI-1824-CG_AT'CG
BglI-2075-GCCn_nnn'nGGC
CaV35S
ter
pUC18-Backbone
pGY1
3437 bp

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9804586 A
- WO 0001722 A
- WO 9947552 A
- WO 0015815 A
- WO 9932619 A
- WO 9953050 A
- WO 0068374 A
- WO 0044914 A
- WO 0044895 A
- WO 0049035 A
- WO 0063364 A
- WO 9732016 A
- US 5593874 A
- US 5698425 A
- US 5712135 A
- US 5789214 A
- US 5804693 A
- US 4801340 A
- EP 0291533 A
- EP 0321201 A
- EP 0360257 A
- US 4987071 A
- US 5116742 A
- US 5034323 A
- US 6110736 A
- US 5352605 A
- WO 8402913 A
- US 4962028 A
- US 5683439 A
- WO 9113991 A
- US 5504200 A
- US 5608152 A
- WO 0026388 A
- WO 9845461 A
- WO 9113980 A
- WO 9515389 A
- WO 9523230 A
- WO 9916890 A
- WO 9705900 A
- WO 9216635 A
- WO 9822593 A
- US 5689049 A
- US 5689051 A
- WO 9519443 A
- EP 0388186 A
- EP 0335528 A
- WO 9321334 A
- WO 9628561 A
- US 5187267 A
- WO 9612814 A
- EP 0375091 A
- WO 9634949 A
- US 6100451 A
- US 5428148 A
- EP 0332104 A
- WO 9803536 A
- WO 9966057 A
- WO 0001830 A
- EP 1165794 A
- EP 1062356 A
- EP 1041148 A
- EP 1032684 A
- WO 9421794 A
- EP 409625 A
- WO 9845456 A
- WO 9741228 A
- EP 120516 A
- US 5565350 A


### In der Beschreibung aufgeführte Nicht-Patentliteratur


- **Dunwell JM.** *J Exp Bot,* 2000, vol. 51 (487-96
- **Vaeck et al.** *Nature,* 1987, vol. 328, 33-37
- **Broglie et al.** *Science,* 1991, vol. 254, 1194-1197
- **Ward et al.** *Plant Cell,* 1991, vol. 3, 1085-1094
- **Uknes et al.** *Plant Cell,* 1992, vol. 4 (6), 645-656
- **Friedrich et al.** *Plant J,* 1996, vol. 10 (1), 61-70
- **Lawton et al.** *Plant J.,* 1996, vol. 10, 71-82
- **Büschges R et al.** *Cell,* 1997, vol. 88, 695-705
- **Jorgensen JH.** *Euphytica,* 1977, vol. 26, 55-62
- **Lyngkjaer MF et al.** *Plant Pathol,* 1995, vol. 44, 786-790
- **Schulze-Lefert P ; Vogel J.** *Trends Plant Sci.,* 2000, vol. 5, 343-348
- **Jorgensen JH ; Mortensen K.** *Phytopathology,* 1977, vol. 67, 678-685

- **Freialdenhoven A et al.** *Plant Cell,* 1996, vol. 8, 5-14
- **Peterhänsel C et al.** *PLANT CELL,* 1997, vol. 9, 1397-1409
- **Wolter M et al.** *Mol Gen Genet,* 1993, vol. 239, 122-128
- **Jarosch B et al.** *Mol Plant Microbe Interact,* 1999, vol. 12, 508-514
- **Kumar J et al.** *Phytopathology,* 2001, vol. 91, 127-133
- **Irani K ; Goldschmidt-Clermont PJ.** *Biochem Pharmacol,* 1998, vol. 55, 1339-1346
- **Kwong et al.** *J Biol Chem,* 1995, vol. 270 (34), 19868-19872
- **Dusi et al.** *Biochem J,* 1996, vol. 314, 409-412
- **Diekmann et al.** *Science,* 1994, vol. 265, 531-533
- **Purgin et al.** *The Plant Cell,* 1997, vol. 9, 2077-2091
- **Kleinberg et al.** *Biochemistry,* 1994, vol. 33, 2490-2495
- **Prigmore et al.** *Journal of Biol Chem,* 1995, vol. 27 (18), 10717-10722
- **Irani et al.** *Science,* 1997, vol. 275, 1649-1652
- **Low et al.** *Advances in Molecular Genetics of Plant-Microbe Interactions,* 1994, vol. 3, 361-369
- **Mehdy et al.** *Plant Physiol,* 1994, vol. 105, 467-472
- **Sundaresan et al.** *Biochem J,* 1996, vol. 318, 379-382
- **Symon M.** *TIBS,* 1996, vol. 21, 178-181
- **Hill et al.** *Cell,* 1995, vol. 81, 1159-1170
- **Chandra et al.** *Proc Natl Acad Sci USA,* 1996, vol. 93, 13393-13397
- **Winge et al.** *Plant Mol Biol,* 1997, vol. 35, 483-495
- **Lin et al.** *The Plant Cell,* 1997, vol. 9, 1647-1659
- **Groom QJ et al.** *Plant J,* 1996, vol. 10, 515-522
- **Hassanain HH et al.** *Biochem Biophys Res Commun,* 2000, vol. 272 (3), 783-788
- *Proc Natl Acad Sci USA,* 2001, vol. 98, 759-764
- **Valster AH et al.** *Trends Cell Biol,* 2000, vol. 10 (4), 141-146
- **Kawasaki T et al.** *Proc Natl Acad Sci USA,* 1999, vol. 96, 10922-10926
- **Ono E et al.** *Proc Natl Acad Sci USA,* 2001, vol. 98, 759-764
- **Schweizer et al.** *Plant J,* 2000, vol. 24, 895-903
- **Devoto A et al.** *J Biol Chem,* 1999, vol. 274 (49), 34993-5004
- **Benard V et al.** *J Biol Chem,* 1999, vol. 274 (19), 13198-204
- **Burstein ES.** *Oncogene,* 1998, vol. 17 (12), 1617-23
- **Altschul et al.** *Nucleic Acids Res.,* 1997, vol. 25, 3389ff
- **Hassanain HH et al.** *Biochem Biophys Res Commun.,* 2000, vol. 272 (3), 783-8
- **Sambrook J ; Fritsch EF ; Maniatis T et al.** Molecular Cloning (A Laboratory Manual). Cold Spring Harbor Laboratory Press, 1989, 9.31-9.57
- Current Protocols in Molecular Biology. John Wiley &Sons, 1989, 6.3.1-6.3.6
- **Matzke MA et al.** *Plant Mol Biol,* 2000, vol. 43, 401-415
- **Fire A. et al.** *Nature,* 1998, vol. 391, 806-811
- **Schweizer P et al.** *Plant J,* 2000, vol. 24, 895-903
- **Waterhouse PM et al.** *Proc Natl Acad Sci USA,* 1998, vol. 95, 13959-64
- **Sheehy et al.** *Proc Natl Acad Sci USA,* 1988, vol. 85, 8805-8809
- **Mol JN et al.** *FEBS Lett,* 1990, vol. 268 (2), 427-430
- **Helene C.** *Anticancer Drug Res,* 1991, vol. 6 (6), 569-84
- **Helene C et al.** *Ann NY Acad Sci,* 1992, vol. 660, 27-36
- **Maher LJ.** *Bioassays,* 1992, vol. 14 (12), 807-815
- **Gautier C et al.** *Nucleic Acids Res,* 1987, vol. 15, 6625-6641
- **Inoue et al.** *Nucleic Acids Res,* 1987, vol. 15, 6131-6148
- **Inoue et al.** *FEBS Lett,* 1987, vol. 215, 327-330
- **Tanner NK.** *FEMS Microbiol Rev,* 1999, vol. 23 (3), 257-275
- **Haseloff et al.** *Nature,* 1988, vol. 334, 585-591
- **Haselhoff ; Gerlach.** *Nature,* 1988, vol. 334, 585-591
- **Steinecke P et al.** *EMBO J,* 1992, vol. 11 (4), 1525-1530
- **de Feyter R et al.** *Mol Gen Genet.,* 1996, vol. 250 (3), 329-338
- **Steinecke P et al.** Ribozymes, Methods in Cell Biology. Academic Press, Inc, 1995, vol. 50, 449-460
- **Bayley CC et al.** *Plant Mol Biol.,* 1992, vol. 18 (2), 353-361
- **Lloyd AM ; Davis RW et al.** *Mol Gen Genet.,* 1994, vol. 242 (6), 653-657
- **Bartel D ; Szostak JW.** *Science,* 1993, vol. 261, 1411-1418
- **Jorgensen et al.** *Plant Mol Biol,* 1996, vol. 31 (5), 957-973
- **Goring et al.** *Proc Natl Acad Sci USA,* 1991, vol. 88, 1770-1774

- **Smith et al.** *Mol Gen Genet,* 1990, vol. 224, 447-481
- **Napoli et al.** *Plant Cell,* 1990, vol. 2, 279-289
- **Van der Krol et al.** *Plant Cell,* 1990, vol. 2, 291-99
- **Napoli et al.** *The Plant Cell,* 1990, vol. 2, 279-289
- **Lagna G ; Hemmati-Brivanlou A.** *Current Topics in Developmental Biology,* 1998, vol. 36, 75-98
- **Perlmutter RM ; Alberola-Ila J.** *Current Opinion in Immunology,* 1996, vol. 8 (2), 285-90
- **Sheppard D.** *American Journal of Respiratory Cell & Molecular Biology,* 1994, vol. 11 (1), 1-6
- **Herskowitz I.** *Nature,* 1987, vol. 329 (6136), 219-22
- **Dreier B et al.** *J Biol Chem,* 2001, vol. 276 (31), 29466-78
- **Dreier B et al.** *J Mol Biol,* 2000, vol. 303 (4), 489-502
- **Beerli RR et al.** *Proc Natl Acad Sci USA,* 2000, vol. 97 (4), 1495-1500
- **Beerli RR et al.** *J Biol Chem,* 2000, vol. 275 (42), 32617-32627
- **Segal DJ ; Barbas CF.** *Curr Opin Chem Biol,* 2000, vol. 4 (1), 34-39
- **Kang JS ; Kim JS.** *J Biol Chem,* 2000, vol. 275 (12), 8742-8748
- **Beerli RR et al.** *Proc Natl Acad Sci USA,* 1998, vol. 95 (25), 14628-14633
- **Kim JS et al.** *Proc Natl Acad Sci USA,* 1997, vol. 94 (8), 3616-3620
- **Klug A.** *J Mol Biol,* 1999, vol. 293 (2), 215-218
- **Tsai SY et al.** *Adv Drug Deliv Rev,* 1998, vol. 30 (1-3), 23-31
- **Mapp AK et al.** *Proc Natl Acad Sci USA,* 2000, vol. 97 (8), 3930-3935
- **Sharrocks AD et al.** *Int J Biochem Cell Biol,* 1997, vol. 29 (12), 1371-1387
- **Zhang L et al.** *J Biol Chem,* 2000, vol. 275 (43), 33850-33860
- **Famulok M ; Mayer G.** *Curr Top Microbiol Immunol,* 1999, vol. 243, 123-36
- **Owen M et al.** *Biotechnology (N Y),* 1992, vol. 10 (7), 790-794
- **Franken E et al.** *Curr Opin Biotechnol,* 1997, vol. 8 (4), 411-416
- **Whitelam.** *Trend Plant Sci,* 1996, vol. 1, 286-272
- **Dervan PB ; Bürli RW.** *Current Opinion in Chemical Biology,* 1999, vol. 3, 688-693
- **Gottesfeld JM et al.** *Gene Expr,* 2000, vol. 9 (1-2), 77-91
- **Bremer RE et al.** *Bioorg Med Chem.,* 2001, vol. 9 (8), 2093-103
- **Ansari AZ et al.** *Chem Biol.,* 2001, vol. 8 (6), 583-92
- **Gottesfeld JM et al.** *J Mol Biol.,* 2001, vol. 309 (3), 615-29
- **Wurtz NR et al.** *Org Lett,* 2001, vol. 3 (8), 1201-3
- **Wang CC et al.** *Bioorg Med Chem,* 2001, vol. 9 (3), 653-7
- **Dervan PB.** *Proc Natl Acad Sci USA,* 2001, vol. 98 (8), 4343-8
- **Chiang SY et al.** *J Biol Chem.,* 2000, vol. 275 (32), 24246-54
- **Angell, SM et al.** *Plant J.,* 1999, vol. 20 (3), 357-362
- **Ratcliff F et al.** *Plant J,* 2001, vol. 25 (2), 237-45
- **Fagard M ; Vaucheret H.** *Plant Mol Biol,* 2000, vol. 43 (2-3), 285-93
- **Anandalakshmi R et al.** *Proc Natl Acad Sci USA,* 1998, vol. 95 (22), 13079-84
- **Ruiz MT.** *Plant Cell,* 1998, vol. 10 (6), 937-46
- **Thomas KR ; Capecchi MR.** *Cell,* 1987, vol. 51, 503
- **Strepp et al.** *Proc Natl Acad Sci USA,* 1998, vol. 95 (8), 4368-4373
- **Odell et al.** *Mol Gen Genet,* 1990, vol. 223, 369-378
- **Zhu et al.** *Nat Biotechnol,* 2000, vol. 18 (5), 555-558
- **Koncz et al.** *Plant Mol Biol,* 1992, vol. 20 (5), 963-976
- **Hohn B ; Puchta.** *Proc Natl Acad Sci USA,* 1999, vol. 96, 8321-8323
- **Cole-Strauss et al.** *Nucl Acids Res,* 1999, vol. 27 (5), 1323-1330
- **Kmiec.** *Gene therapy American Scientist,* 1999, vol. 87 (3), 240-247
- **Maniatis T ; Fritsch EF ; Sambrook J.** Molecular Cloning: A Laboratory Manual. Cold Spring Harbor Laboratory, 1989
- **Silhavy TJ ; Berman ML ; Enquist LW.** Experiments with Gene Fusions. Cold Spring Harbor Laboratory, 1984
- **Ausubel FM et al.** Current Protocols in Molecular Biology. Greene Publishing Assoc. and Wiley Interscience, 1987
- **Gelvin et al.** *Plant Molecular Biology Manual,* 1990
- **Benfey et al.** *EMBO J,* 1989, vol. 8, 2195-2202
- **Franck et al.** *Cell,* 1980, vol. 21, 285-294
- **Odell et al.** *Nature,* 1985, vol. 313, 810-812
- **Shewmaker et al.** *Virology,* 1985, vol. 140, 281-288
- **Gardner et al.** *Plant Mol Biol,* 1986, vol. 6, 221-228
- **Holtorf S et al.** *Plant Mol Biol,* 1995, vol. 29, 637-649
- **Christensen et al.** *Plant Mol Biol,* 1992, vol. 18, 675-689

- **Bruce et al.** *Proc Natl Acad Sci USA,* 1989, vol. 86, 9692-9696
- **Hillebrand et al.** *Gene,* 1996, vol. 170, 197-200
- **Bustos MM et al.** *Plant Cell,* 1989, vol. 1 (9), 839-53
- **Josefsson LG et al.** *J Biol Chem,* 1987, vol. 262, 12196-12201
- **Shirsat A et al.** *Mol Gen Genet,* 1989, vol. 215 (2), 326-331
- **Bäumlein H et al.** *Mol Gen Genet,* 1991, vol. 225 (3), 459-67
- **Stalberg K et al.** *L Planta,* 1996, vol. 199, 515-519
- **Bäumlein H et al.** *Mol Gen Genet,* 1991, vol. 225, 121-128
- **Baeumlein et al.** *Plant Journal,* 1992, vol. 2 (2), 233-9
- **Fiedler U et al.** *Biotechnology (NY),* 1995, vol. 13 (10), 1090f
- **Stockhaus et al.** *EMBO J,* 1989, vol. 8, 2445-2451
- **Wei et al.** *Plant Mol. Biol.,* 1998, vol. 36, 101-112
- **Gatz et al.** *Annu Rev Plant Physiol Plant Mol Biol,* 1997, vol. 48, 89-108
- **Ward et al.** *Plant Mol Biol,* 1993, vol. 22, 361-366
- **Gatz et al.** *Plant J,* 1992, vol. 2, 397-404
- **Schubert et al.** *Plant Mol Biol,* 1997, vol. 34, 417-426
- **Redolfi et al.** *Neth J Plant Pathol,* 1983, vol. 89, 245-254
- **Uknes et al.** *Plant Cell,* 1992, vol. 4, 645-656
- **Van Loon.** *Plant Mol Viral,* 1985, vol. 4, 111-116
- **Marineau et al.** *Plant Mol Biol,* 1987, vol. 9, 335-342
- **Matton et al.** *Molecular Plant-Microbe Interactions,* 1987, vol. 2, 325-342
- **Somssich et al.** *Proc Natl Acad Sci USA,* 1986, vol. 83, 2427-2430
- **Somssich et al.** *Mol Gen Genetics,* 1988, vol. 2, 93-98
- **Chen et al.** *Plant J,* 1996, vol. 10, 955-966
- **Zhang ; Sing.** *Proc Natl Acad Sci USA,* 1994, vol. 91, 2507-2511
- **Warner et al.** *Plant J,* 1993, vol. 3, 191-201
- **Siebertz et al.** *Plant Cell,* 1989, vol. 1, 961-968
- **Ryan.** *Ann Rev Phytopath,* 1990, vol. 28, 425-449
- **Duan et al.** *Nat Biotech,* 1996, vol. 14, 494-498
- **Stanford et al.** *Mol Gen Genet,* 1989, vol. 215, 200-208
- **McGurl et al.** *Science,* 1992, vol. 225, 1570-1573
- **Rohmeier et al.** *Plant Mol Biol,* 1993, vol. 22, 783-792
- **Eckelkamp et al.** *FEBS Letters,* 1993, vol. 323, 73-76
- **Corderok et al.** *Plant J,* 1994, vol. 6 (2), 141-150
- **Buchel et al.** *Plant Mol Biol,* 1996, vol. 30, 493-504
- **Schweizer et al.** *Plant Physiol,* 1997, vol. 114, 79-88
- **Rebmann et al.** *Plant Mol Biol,* 1991, vol. 16, 329-331
- **Rushton et al.** *Plant Cell,* 2002, vol. 14, 749-762
- **Lam E ; Chua NH.** *J Biol Chem,* 1991, vol. 266 (26), 17131-17135
- **Schoffl F et al.** *Molecular & General Genetics,* 1989, vol. 217 (2-3), 246-53
- The Maize Handbook. Springer, 1994
- **Gallie et al.** *Nucl Acids Res,* 1987, vol. 15, 8693-8711
- **Rouster J et al.** *Plant J,* 1998, vol. 15, 435-440
- **Gielen et al.** *EMBO J,* 1984, vol. 3, 835 ff
- **Sauer B.** *Methods,* 1998, vol. 14 (4), 381-92
- **Schenborn E ; Groskreutz D.** *Mol Biotechnol.,* 1999, vol. 13 (1), 29-44
- **Sheen et al.** *Plant Journal,* 1995, vol. 8 (5), 777-784
- **Haseloff et al.** *Proc Natl Acad Sci USA,* 1997, vol. 94 (6), 2122-2127
- **Reichel et al.** *Proc Natl Acad Sci USA,* 1996, vol. 93 (12), 5888-5893
- **Tian et al.** *Plant Cell Rep,* 1997, vol. 16, 267-271
- **Chui WL et al.** *Curr Biol,* 1996, vol. 6, 325-330
- **Leffel SM et al.** *Biotechniques,* 1997, vol. 23 (5), 912-8
- **Ow et al.** *Science,* 1986, vol. 234, 856-859
- **Millar et al.** *Plant Mol Biol Rep,* 1992, vol. 10, 324-414
- **Prasher et al.** *Biochem Biophys Res Commun,* 1985, vol. 126 (3), 1259-1268
- **Dellaporta et al.** Chromosome Structure and Function: Impact of New Concepts. *18th Stadler Genetics Symposium,* 1988, vol. 11, 263-282
- **Jefferson et al.** *EMBO J.,* 1987, vol. 6, 3901-3907
- **Sambrook et al.** Molecular Cloning. A Laboratory Manual. Cold Spring Harbor Laboratory Press, 1989
- **McCormick et al.** *Plant Cell Reports,* 1986, vol. 5, 81-84
- **Keown et al.** *Methods in Enzymology,* 1990, vol. 185, 527-537
- **Bilang et al.** *Gene,* vol. 100, 247-250
- **Scheid et al.** *Mol Gen Genet,* 1991, vol. 228, 104-112

- **Guerche et al.** *Plant Science,* 1987, vol. 52, 111-116
- **Neuhause et al.** *Theor Appl Genet,* 1987, vol. 75, 30-36
- **Klein et al.** *Nature,* 1987, vol. 327, 70-73
- **Howell et al.** *Science,* 1980, vol. 208, 1265
- **Horsch et al.** *Science,* 1985, vol. 227, 1229-1231
- **DeBlock et al.** *Plant Physiology,* 1989, vol. 91, 694-701
- Methods for Plant Molecular Biology. Academic Press Inc, 1988
- Methods in Plant Molecular Biology. Academic Press Inc, 1989
- **Horsch RB et al.** *Science,* 1985, vol. 225, 1229f
- **Holsters et al.** *Mol Gen Genet,* 1978, vol. 163, 181-187
- **Hoekema.** The Binary Plant Vector System. Offset-drukkerij Kanters B.V,
- **An et al.** *EMBO J,* 1984, vol. 4, 277-287
- **Rogers et al.** *Meth in Enzymol,* 1987, vol. 153, 253-277
- **Schardl et al.** *Gene,* 1987, vol. 61, 1-11
- **Berger et al.** *Proc Natl Acad Sci USA,* 1989, vol. 86, 8402-8406
- **Rathore KS et al.** *Plant Mol Biol,* 1993, vol. 21 (5), 871-884
- **McCormick et al.** *Cell Reports,* 1986, vol. 5, 81-84
- Techniques for Gene Transfer, in: Transgenic Plants. **Jenes B et al.** Engineering and Utilization. AcademicPress, 1993, vol. 1, 128-143
- **Potrykus.** *AnnuRevPlantPhysiol Plant Molec Biol,* 1991, vol. 42, 205-225
- **Bevan et al.** *Nucl Acids Res,* 1984, vol. 12, 8711f
- **Fennell et al.** *Plant Cell Rep.,* 1992, vol. 11, 567-570
- **Stoeger et al.** *Plant Cell Rep.,* 1995, vol. 14, 273-278
- **Jahne et al.** *Theor Appl Genet,* 1994, vol. 89, 525-533
- **Dunwell JM.** Transgenic approaches to crop improvement. *J Exp Bot.,* 2000, vol. 51, 487-96
- **Hood EE ; Jilka JM.** *Curr Opin Biotechnol,* 1999, vol. 10 (4), 382-6
- **Ma JK ; Vine ND.** *Curr Top Microbiol Immunol,* 1999, vol. 236, 275-92
- **HassanainHH et al.** *BiochemBiophys Res Commun.,* 2000, vol. 272 (3), 783-8
- **Schweizer P et al.** *Mol Plant Microbe Interact,* 1999, vol. 12, 647-54
- **Shinshi H et al.** *Plant Mol Biol,* 1990, vol. 14, 357-368
- Voet, Voet. Wiley Press, 896-897
- **Sambrook et al.** VOET, VOET. Cold Spring Harbor Laboratory Press, 1989
- **Sanger et al.** *Proc Natl Acad Sci USA,* 1977, vol. 74, 5463-5467
- **Kølster P et al.** *Crop Sci,* 1986, vol. 26, 903-907
- **Wiberg A.** *Hereditas,* 1974, vol. 77, 89-148
- DIG System User's Guide, DIG-Luminescence detection Kit, Boehringer-Mannheim. *Plant Physiol,* 1994, vol. 106, 1264-1277
- **Norman C et al.** *Cell,* 1988, vol. 55 (6), 989-1003
- **Schweizer P et al.** *Mol Plant Microbe Interact,* 1999, vol. 2, 647-54
- **Schultheiss H et al.** *Plant Physiol,* 2002, vol. 128, 1447-1454
- **Hückelhoven et al.** *Plant Mol Biol,* 2001
- **Schultheiss et al.** *Plant Physiol,* 2002, vol. 128, 1447-1454
- **Steve Clough ; Andrew Bent.** *Plant J,* 1998, vol. 16 (6), 735-743
- **Bechtold N et al.** *CR Acad Sci Paris, Life Sciences,* 1993, vol. 316, 1194-1199